# EUROPEAN PATENT APPLICATION

(11) **EP 4 421 070 A1**
(43) Date of publication of application: **28.08.2024**
(21) Application number: 22883978.3
(22) Date of filing: 18.10.2022
(51) Int. Cl.: C07D 403/06, A61K 31/498, A61K 31/5377, A61K 31/4985, A61P 11/00, A61P 43/00, C07D 401/06, C07D 241/40, C07D 401/12, C07D 487/04

(54) **NOVEL COMPOUND AND PHARMACEUTICAL COMPOSITION COMPRISING SAME**

(30) Priority: 18.10.2021 KR 20210138794; 17.10.2022 KR 20220133578
(71) Applicant: Sapiensbio Inc., Seongnam-si, Gyeonggi-do 13207 (KR)
(72) Inventor: YOON, Dong-Oh, Seongnam-si, Gyeonggi-do 13207 (KR); RYOO, Kanghyun, Seongnam-si, Gyeonggi-do 13207 (KR); LEE, Hyunseung, Seongnam-si, Gyeonggi-do 13207 (KR); RYU, Incheol, Seongnam-si, Gyeonggi-do 13207 (KR); JEONG, Eun Jeong, Seongnam-si, Gyeonggi-do 13207 (KR); LEE, Myoungwoo, Seongnam-si, Gyeonggi-do 13207 (KR); LEE, Jin Woo, Seongnam-si, Gyeonggi-do 13207 (KR); KIM, Je Hak, Seongnam-si, Gyeonggi-do 13207 (KR)
(74) Representative: Dehns
(86) International application number: PCT/KR2022/015857
(87) International publication number: WO 2023/068755

(57) **Abstract**

The present invention relates to a novel compound that is effective in inhibiting fibrosis. The inventors confirmed that the compound has an effect of inhibiting actin polymerization and not only reduces the levels of all types of fibrosis biomarkers but also effectively inhibits the expression of α-SMA, which is a major causative factor of fibrosis. Therefore, the novel compound according to the present invention is expected to be effectively used as a preparation having an excellent effect of alleviating and inhibiting fibrosis to prevent and treat a fibrosis disease.

## Description

### [Technical Field]

The present invention relates to a novel compound and a pharmaceutical composition including the same.

This application claims priority to and the benefit of Korean Patent Application No. 10-2021-0138794, filed on October 18, 2021, and Korean Patent Application No. 10-2022-0133578, filed on October 17, 2022, the disclosure of which is incorporated herein by reference in their entirety.

### [Background Art]

Idiopathic pulmonary fibrosis (IPF) is a disease in which the fibrosis of lung parenchyma chronically progresses and is characterized by the infiltration of immune cells into the alveolar wall, inducing fibrosis and causing serious structural changes in lung tissue, resulting in a gradual decline in lung function. If the fibrosis of the lung parenchyma continues to progress due to IPF, respiratory failure may occur and even lead to death. However, since there is no effective treatment, IPF is a highly fatal disease that has a 5-year survival rate of less than 40%, and a 10-year survival rate of less than 15%, after diagnosis. Pirfenidone and nintedanib are currently used clinically as IPF therapeutic drugs were approved by the U.S. FDA, but the improvement in lung function exhibits a low treatment effect of only about 10%. In addition, both treatments show no significant difference in extending the patient's life expectancy after diagnosis. Accordingly, there is a need for the development of a treatment for pulmonary fibrosis through a new mechanism.

In damaged tissue, inflammatory proteins such as TGF-β and TNF-α are produced and secreted out of cells, causing functional and structural changes in the cytoskeleton and a healing process. In normal conditions, after the healing process, inflammatory proteins and the accumulated extracellular matrix (ECM) are reduced and a recovery process occurs, but in pathological conditions, the accumulation of the secreted ECM continues without normal recovery proceeding. This is one of the main mechanisms causing fibrosis. Cells respond to external physical stimuli by causing changes in gene expression, cell morphology, and the cytoskeleton through changes in intracellular signal transmission (mechanotransduction) and cell-cell interactions. These cellular changes serve as an important driving force for cell movement and cell surface remodeling. When damaged tissue cannot be restored to its original state through repeated abnormal healing processes due to the continuous influx of various external factors, the damaged tissue is converted into fibrotic tissue.

Epithelial-mesenchymal transition (EMT) and fibroblast-myofibroblast transition (FMT) are the key mechanisms causing pulmonary fibrosis. In the damaged tissue of the alveoli, the inflammatory cytokines such as TGF-β are secreted, and in the activated epithelial layer, EMT results from the breakdown of cell connective tissue caused by an inflammatory signal, and the epithelial cells are converted into fibroblasts. In addition, when fibroblasts present in the subepithelial layer receive mechanical and physical damage from external stimuli, stress fibers are expressed via the mechanotransduction pathway. In addition, it is effectively activated by cytokines causing fibrosis, and fibroblasts are converted into myofibroblasts by FMT. Changes of the cytoskeleton occur from the above process. The cytoskeleton consists of microtubules, actin microfilaments, and intermediate filaments and not only maintains the skeletal structure of cells but also relates to various roles such as cell movement and interaction with an external environment. Among them, the actin microfilament is a microfilament consisting of globular actin (G-actin) as a monomer, and a filamentous actin (F-actin) as a polymeric filament, and affects the mobility of cells such as lamellipodia or filopodia, and is also very important in cell transformation (EMT and FMT). Cell transformation is achieved by changes in the cytoskeleton, and one of the factors that cause changes in the cytoskeleton is actin polymerization.

Particularly, the control of cytoskeletal changes is important in fibrosis, and an abnormal fibrosis mechanism may be improved by the control of actin polymerization. Cells control cell mobility through actin polymerization and form stress fibers to cause cytoskeletal remodeling, and target proteins involved therein are actin-binding proteins. Arp2/3 complex, which is one of the target proteins, acts on nucleation among various steps that control the mobility of the cytoskeleton and serves to form filament branches, and consists of 7 subunits (Arp2, Arp3, ARPC1, ARPC2, ARPC3, ARPC4, and ARPC5). Here, the Arp2/3 complex affects the amplification of actin microfilaments through actin branching and is associated with the speed of actin network formation in fibroblasts. Accordingly, by controlling the function of the Arp2/3 complex, cell transformation may be controlled by suppressing changes in the actin network and cytoskeleton due to actin polymerization.

Pulmonary fibrosis occurs between tissue and blood vessels surrounding the alveoli, and particularly, IPF is a disease in which tissue is gradually hardened by the accumulation of ECM due to myofibroblasts. This disease causes the contraction of myofibroblasts induced by changes in the cytoskeleton of fibroblasts, resulting in the induction of tissue hardening and reduced respiratory function of the lungs. Changes of the cytoskeleton are associated with the activation of myofibroblasts, and the key biomarker for the activation of myofibroblasts is known to be α-smooth muscle actin (α-SMA). Recent study results reveal that, when IPF-derived fibroblasts were treated with the cytokine TGF-β, α-SMA expression was increased. Accordingly, a hallmark of the activation of myofibroblasts involved in fibrosis is α-SMA expression. From previous study results, the improvement in cell transformation by the control of the cytoskeleton is believed to be a mechanism for treating IPF. Therefore, it is intended to develop a drug for treating various fibrosis-related diseases including IPF by developing a material for controlling the function of the Arp2/3 complex involved in actin polymerization that controls cytoskeletal changes.

### [Disclosure]

### [Technical Problem]

The present invention was devised to solve the above problems, the inventors prepared a novel compound, and confirmed that the compound can control actin polymerization to treat cytoskeleton-related diseases such as pulmonary fibrosis, and thus completed the present invention based on this.

The present invention is directed to providing a compound represented by Chemical Formula 1 or 2 below, an enantiomer thereof, a diastereomer thereof, or a pharmaceutically acceptable salt thereof:

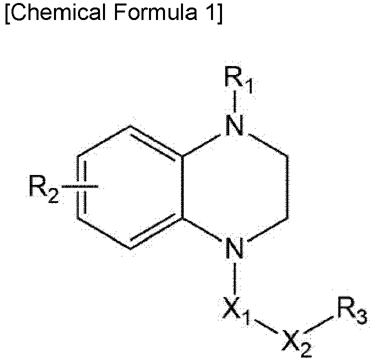

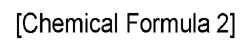

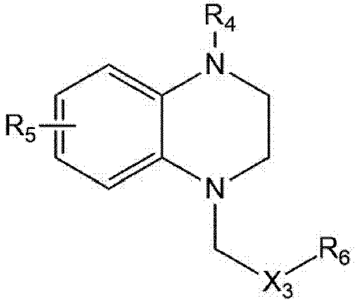

The present invention is also directed to providing a pharmaceutical composition for preventing or treating a pulmonary fibrosis-related disease, which includes the compound represented by Chemical Formula 1 or 2, an enantiomer thereof, a diastereomer thereof, or a pharmaceutically acceptable salt thereof as an active ingredient.

The present invention is also directed to providing a kit for preventing or treating a pulmonary fibrosis-related disease, which includes the composition.

However, technical problems to be solved in the present invention are not limited to the above-described problems, and other problems which are not described herein will be fully understood by those of ordinary skill in the art from the following descriptions.

### [Technical Solution]

The present invention provides a compound represented by Chemical Formula 1 or 2 below, an enantiomer thereof, a diastereomer thereof, or a pharmaceutically acceptable salt thereof: 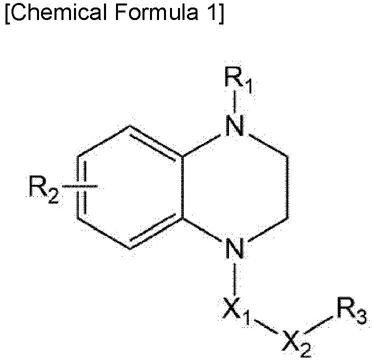 (In Chemical Formula 1,
X₁ is CO or CH₂;
X₂ is (CH₂)ₙ, n is an integer of 1 or 2, and X₂ is unsubstituted or has one H substituted with a methyl group or an amino group;
R₁ is a halogen-substituted or unsubstituted C₃-C₁₀ aryl group, a halogen-substituted or unsubstituted benzyl group, a 6-membered heteroaryl-substituted or unsubstituted C₁-C₁₀ alkyl group, a C₂-C₁₀ heteroaryl group, a C₃-C₁₀ cycloalkyl group, a C₂-C₁₀ heterocycloalkyl group, a C₁-C₅ alkyl-substituted or unsubstituted benzoyl group, or a C₁-C₅ alkyl-substituted or unsubstituted acyl group;
R₂ is H or a halogen;
R₃ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a methyl-substituted or unsubstituted C₂-C₁₀ heterocycloalkylamine group, a 6-membered heteroaryl-substituted or unsubstituted C₁-C₃ alkylamine group, or 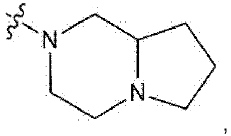 wherein the heterocycloalkyl group is substituted with one or more selected from the group consisting of O, OH, and a C₁-C₅ alkyl group or unsubstituted; and
the heterocycloalkyl group, the heteroaryl group, or the heterocycloalkylamine group each independently includes one or more selected from the group consisting of N, O, and SO₂.) 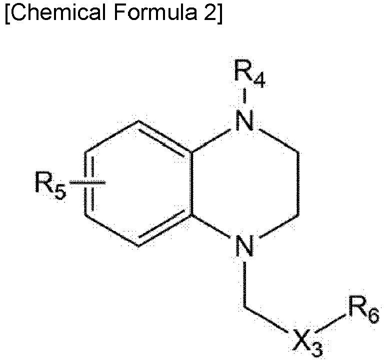 (In Chemical Formula 2,
   X₃ is CO or CH₂;
   R₄ is a halogen-substituted or unsubstituted C₃-C₁₀ aryl group, a halogen-substituted or unsubstituted benzyl group, a 6-membered heteroaryl-substituted or unsubstituted C₁-C₁₀ alkyl group, a C₂-C₁₀ heteroaryl group, a C₃-C₁₀ cycloalkyl group, a C₂-C₁₀ heterocycloalkyl group, a C₁-C₅ alkyl-substituted or unsubstituted benzoyl group, or a C₁-C₅ alkyl-substituted or unsubstituted acyl group;
   R₅ is H or a halogen;
   R₆ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a methyl-substituted or unsubstituted C₂-C₁₀ heterocycloalkylamine group, a 6-membered heteroaryl-substituted or unsubstituted C₁-C₃ alkylamine group, or 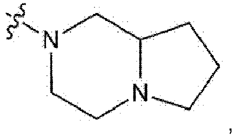 wherein the heterocycloalkyl group is substituted with one or more selected from the group consisting of O, OH, and a C₁-C₅ alkyl group or unsubstituted; and
   the heterocycloalkyl group, the heteroaryl group, or the heterocycloalkylamine group each independently includes one or more selected from the group consisting of N, O, and SO₂.)

In one embodiment of the present invention, in Chemical Formula 1, R₁ is a halogen-substituted or unsubstituted phenyl group, a halogen-substituted or unsubstituted benzyl group, a pyridyl group, a C₃-C₆ cycloalkyl group, a benzoyl group, a C₄-C₅ alkyl-substituted or unsubstituted acyl group, or a pyridyl-substituted or unsubstituted C₁-C₄ alkyl group;
R₃ is an N-containing C₂-C₆ heterocycloalkyl group, an N-containing C₂-C₆ heteroaryl group, a morphonyl group, 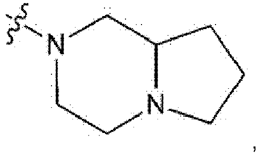 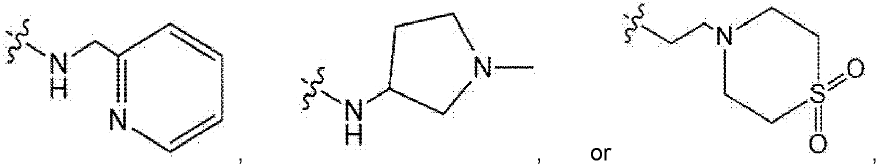 wherein the heterocycloalkyl group may be substituted with one or more selected from the group consisting of O, OH, and a C₁-C₃ alkyl group or unsubstituted, but the present invention is not limited thereto. More preferably, the heterocycloalkyl group is a 5-membered or 6-membered heterocycloalkyl group. In addition, the heteroaryl group may be a 5-membered or 6-membered heteroaryl group.

In another embodiment of the present invention, in Chemical Formula 2, R₄ is a halogen-substituted or unsubstituted phenyl group, and R₆ is an N-containing C₂-C₆ heterocycloalkyl group, a morphonyl group, or 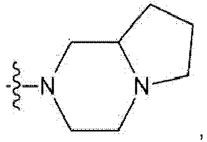 wherein the heterocycloalkyl group may be substituted with OH or a C₁-C₃ alkyl group or unsubstituted, but the present invention is not limited thereto. More preferably, the heterocycloalkyl group is a 5-membered or 6-membered heterocycloalkyl group.

In still another embodiment of the present invention, the compound represented by Chemical Formula 1 or 2 below may be anyone selected from the group consisting of Compounds 1 to 208 in Table 1 below, but the present invention is not limited thereto.

The present invention also provides a pharmaceutical composition for preventing or treating a pulmonary fibrosis-related disease, which includes the compound represented by Chemical Formula 1 or 2, an enantiomer thereof, a diastereomer thereof, or a pharmaceutically acceptable salt thereof as an active ingredient.

The present invention also provides a method of preventing or treating a pulmonary fibrosis-related disease, which includes administering the compound represented by Chemical Formula 1 or 2, an enantiomer thereof, a diastereomer thereof, or a pharmaceutically acceptable salt thereof to a subject in need thereof.

The present invention also provides a use of the compound represented by Chemical Formula 1 or 2, an enantiomer thereof, a diastereomer thereof, or a pharmaceutically acceptable salt thereof for preventing, alleviating, or treating a pulmonary fibrosis-related disease.

The present invention also provides a use of the compound represented by Chemical Formula 1 or 2, an enantiomer thereof, a diastereomer thereof, or a pharmaceutically acceptable salt thereof for preparing a drug for treating a pulmonary fibrosis-related disease.

The present invention also provides a kit for preventing or treating a pulmonary fibrosis-related disease, which includes the composition according to the present invention.

In one embodiment of the present invention, the composition may inhibit actin polymerization, but the present invention is not limited thereto.

In another embodiment of the present invention, the composition may reduce the level or activity of one or more selected from the group consisting of α-SMA, COL1A1, COL4A1, fibronectin, and phosphorylated SMAD2 (p-SMAD2), but the present invention is not limited thereto.

In still another embodiment of the present invention, the pulmonary fibrosis-related disease may be one or more selected from the group consisting of pulmonary fibrosis, idiopathic pulmonary fibrosis (IPF), desquamative interstitial pneumonia, non-specific interstitial pneumonia, cryptogenic organizing pneumonia, respiratory bronchiolitis-associated interstitial lung disease, acute interstitial pneumonia, lymphatic interstitial pneumonia, idiopathic pleuroparenchymal fibroelastosis, and chronic obstructive pulmonary disease (COPD), but the present invention is not limited thereto.

In yet another embodiment of the present invention, the composition may be used to treat a pulmonary fibrosis-related disease accompanied by hyperactivation of actin polymerization, but the present invention is not limited thereto. In other words, the composition may show an excellent therapeutic effect on a pulmonary fibrosis-related disease accompanied by hyperactivation of actin polymerization.

### [Advantageous Effects]

The present invention relates to a novel compound having a fibrosis inhibition effect. The present inventors confirmed that the compound has an effect of inhibiting actin polymerization and not only reduces the levels of various types of fibrosis biomarkers, but also effectively inhibits the expression of a major causative factor of fibrosis, α-SMA. Therefore, the novel compound according to the present invention, as a preparation with an excellent effect of alleviating and inhibiting fibrosis, is expected to be effectively used in the prevention and treatment of a fibrosis disease.

### [Description of Drawings]

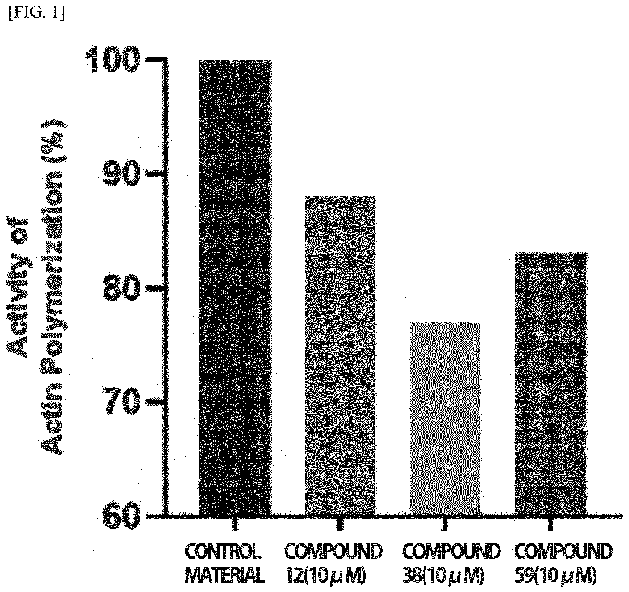

FIG. 1 shows the results of comparing maximum polymerization rates by treating an actin monomer with a compound according to the present invention and then inducing actin polymerization to measure the actin polymerization-inhibiting activity of the compound (control material: compound-untreated control).

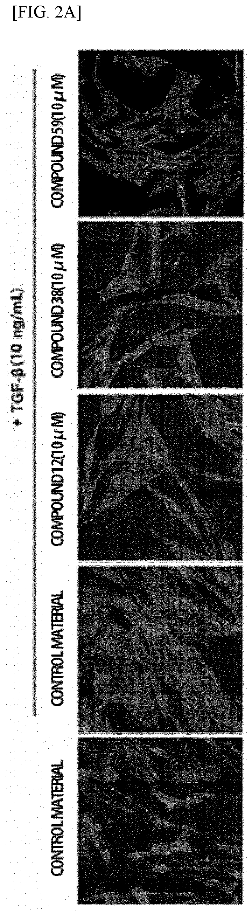

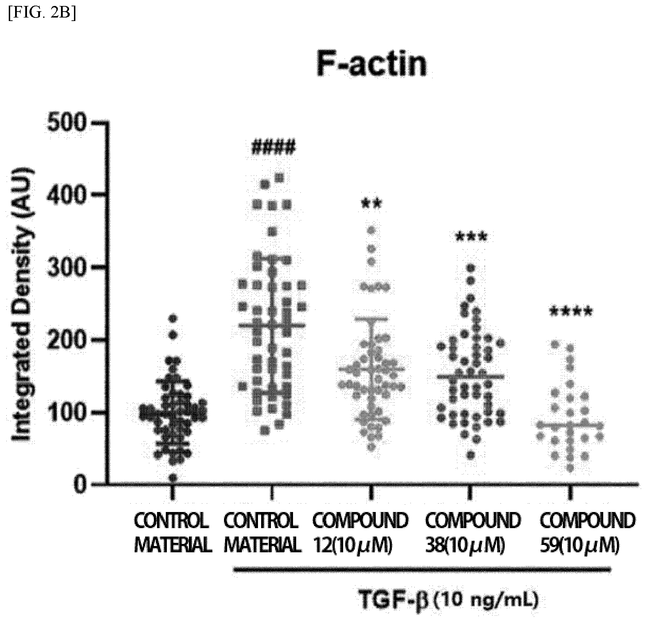

FIGS. 2A and 2B show the images of F-actin and a cell nucleus photographed using a confocal microscope (FIG. 2A) and the degree of F-actin polymerization (FIG. 2B) after treatment of the compound along with a fibrosis-causing cytokine to measure the F-actin polymerization-inhibiting activity of the compound according to the present invention.

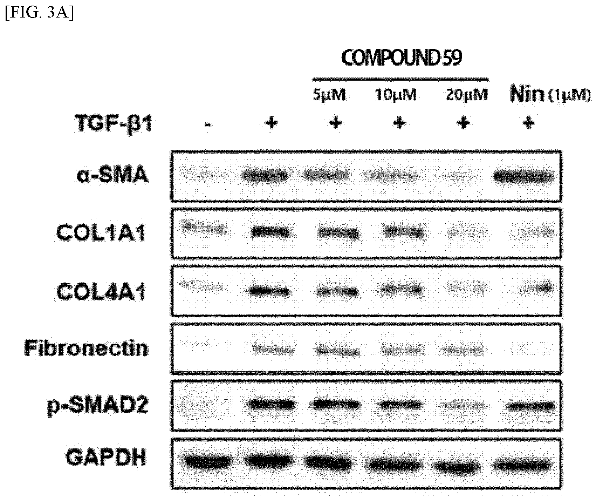

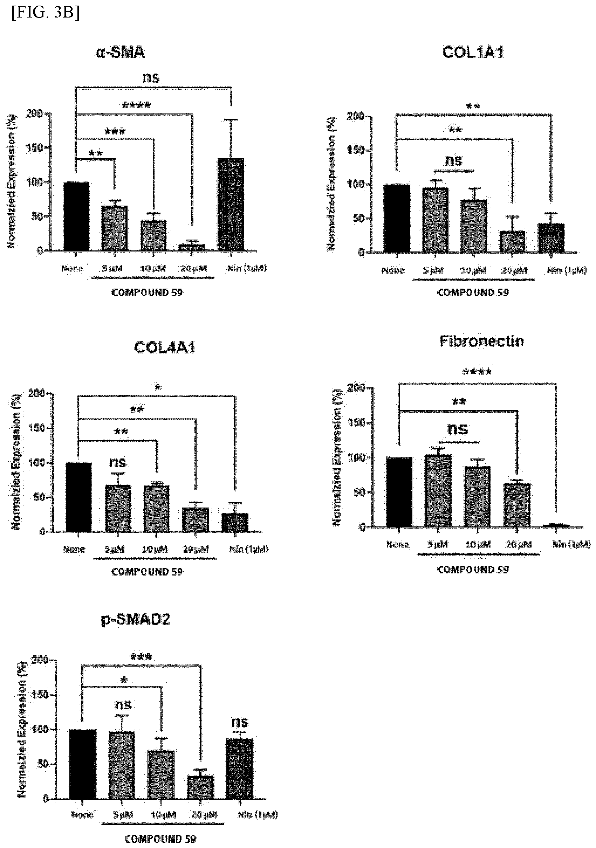

FIGS. 3A and 3B show the results of confirming α-SMA, COL1A1, COL4A1, fibronectin, and p-SMAD2 protein levels through Western blotting (FIG. 3A) and quantifying the protein levels (FIG. 3B) after treating lung tissue-derived fibroblasts with the compound according to the present invention or a pulmonary fibrosis treatment (nintedanib, "Nin") to confirm the fibrosis factor control effect of the compound.

### [Modes of the Invention]

The present invention relates to a novel compound, which was completed by confirming that the compound is effective in not only inhibiting actin polymerization but also inhibiting the production of various types of fibrosis-related factors, including α-SMA.

Specifically, in one embodiment of the present invention, 208 types of novel compounds according to the present invention were prepared (Example 1).

In another embodiment of the present invention, as a result of inducing actin polymerization after treating an actin monomer with the compound according to the present invention, the degree of activation of actin polymerization was significantly reduced by the compound, confirming that the compound according to the present invention can effectively inhibit actin polymerization (Example 2).

In still another embodiment of the present invention, as a result of treating cells treated with a fibrosis promoting factor with the compound according to the present invention, F-actin polymerization was effectively inhibited, confirming that the compound of the present invention can inhibit the activation of myofibroblasts and the progression of fibrosis through inhibition of F-actin polymerization (Example 3).

In yet another embodiment of the present invention, as a result of observing changes in the expression of fibrosis-related factors by treating lung tissue-derived fibroblasts with the compound according to the present invention, it was confirmed that the compound according to the present invention significantly reduces the levels of fibrosis biomarkers (COL1A1, COL4A1, fibronectin, and p-SMAD2), and particularly, more effectively inhibits α-SMA, compared to a pulmonary fibrosis treatment, nintedanib (Example 4).

The above results proved that the novel compound according to the present invention effectively inhibits various types of fibrosis-inducing factors, and particularly, significantly suppresses actin polymerization, which is a major cause of fibrosis, and thus the compound according to the present invention is expected to be used widely in the pulmonary fibrosis treatment field.

Hereinafter, the present invention will be described in detail.

The present invention provides a compound represented by Chemical Formula 1 or 2 below, an enantiomer thereof, a diastereomer thereof, or a pharmaceutically acceptable salt thereof: 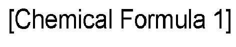 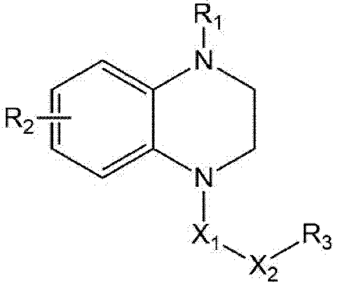 (In Chemical Formula 1,
X₁ is CO or CH₂;
X₂ is (CH₂)ₙ, n is an integer of 1 or 2, and X₂ is unsubstituted or has one H substituted with a methyl group or an amino group;
R₁ is a halogen- or C₁-C₁₀ alkyl-substituted or unsubstituted C₃-C₁₀ aryl group, a halogen-substituted or unsubstituted benzyl group, a C₂-C₁₀ heteroaryl-substituted or unsubstituted C₁-C₁₀ alkyl group, a C₃-C₁₀ alkyl-substituted or unsubstituted C₂-C₁₀ heteroaryl group, a C₃-C₁₀ cycloalkyl group, a C₂-C₁₀ heterocycloalkyl group, a halogen- or C₁-C₅ alkyl-substituted or unsubstituted benzoyl group, or a C₁-C₅ alkyl- or C₁-C₅ haloalkyl-substituted or unsubstituted acyl group;
R₂ is H or a halogen;
R₃ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a C₁-C₁₀ alkyl-substituted or unsubstituted C₂-C₁₀ heterocycloalkylamine group, a C₂-C₁₀ heteroaryl-substituted or unsubstituted C₁-C₃ alkylamine group, or 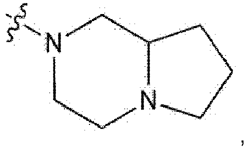 wherein the heterocycloalkyl group is substituted with one or more selected from the group consisting of O, OH, and a C₁-C₅ alkyl group, or unsubstituted, and the cycloalkyl group is substituted with one or more selected from the group consisting of a C₁-C₁₀ alkyl group, a C₁-C₁₀ alkoxy group, and a C₁-C₁₀ alkylether group or unsubstituted; and
the heterocycloalkyl group, the heteroaryl group, or the heterocycloalkylamine group each independently includes one or more selected from the group consisting of N, O, and SO₂.) 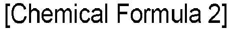 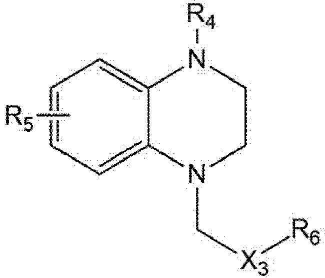 (In Chemical Formula 2,
   X₃ is CO or CH₂;
   R₄ is a halogen- or C₁-C₁₀ alkyl-substituted or unsubstituted C₃-C₁₀ aryl group, a halogen-substituted or unsubstituted benzyl group, a C₂-C₁₀ heteroaryl-substituted or unsubstituted C₁-C₁₀ alkyl group, a C₃-C₁₀ alkyl-substituted or unsubstituted C₂-C₁₀ heteroaryl group, a C₃-C₁₀ cycloalkyl group, a C₂-C₁₀ heterocycloalkyl group, a halogen- or C₁-C₅ alkyl-substituted or unsubstituted benzoyl group, or a C₁-C₅ alkyl- or C₁-C₅ haloalkyl-substituted or unsubstituted acyl group;
   Rs is H or a halogen;
   R₆ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a C₁-C₁₀ alkyl-substituted or unsubstituted C₂-C₁₀ heterocycloalkylamine group, a C₂-C₁₀ heteroaryl-substituted or unsubstituted C₁-C₃ alkylamine group, or 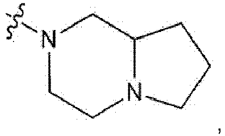 wherein the heterocycloalkyl group is substituted or unsubstituted with one or more selected from the group consisting of O, OH, and a C₁-C₅ alkyl group, and the cycloalkyl group is substituted or unsubstituted with one or more selected from the group consisting of a C₁-C₁₀ alkyl group, a C₁-C₁₀ alkoxy group, and a C₁-C₁₀ alkylether group; and
   the heterocycloalkyl group, the heteroaryl group, or the heterocycloalkylamine group each independently includes one or more selected from the group consisting of N, O, and SO₂.)

The present invention also provides a pharmaceutical composition for preventing or treating a pulmonary fibrosis-related disease, which includes the compound represented by Chemical Formula 1 or 2, an enantiomer thereof, a diastereomer thereof, or a pharmaceutically acceptable salt thereof as an active ingredient.

In the present invention, X₂ is (CH₂)ₙ, n is an integer of 1 or 2, wherein X₂ may be unsubstituted or have one H that is substituted with a methyl or amino group. Preferably, when n is 1, X₂ may be unsubstituted or have one H that is substituted with a methyl group (CH₃). In addition, when n is 2, X₂ may be unsubstituted or have one H that is substituted with an amino group (NH₂). In one embodiment of the present invention, X₂ may be selected from the group consisting of 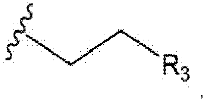 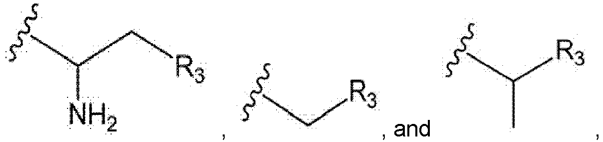 but the present invention is not limited thereto.

In one embodiment of the present invention, X₂ and R₃ may be linked via C or N, but the present invention is not limited thereto. For example, X₂ may be linked to C or N of R₃, but the present invention is not limited thereto.

In the present invention, "CO" refers to a carbonyl group. That is, in the present invention, X₁ and X₃ may each be independently a carbonyl group or hydrogen-binding C (e.g., CH₂).

In the present invention, "halogen" includes F, Cl, Br, or I.

In the present invention, "alkyl" refers to a fully saturated branched or unbranched (or straight-chain or linear) hydrocarbon. In the present invention, the alkyl may be a C₁-C₂₀, C₁-C₁₅, C₁-C₁₂, C₁-C₁₀, C₁-C₈, C₁-C₆, C₁-C₅, or C₁-C₃ alkyl, but the present invention is not limited thereto. The alkyl may include, for example, methyl, ethyl, and isomers such as n-propyl, isopropyl, cyclopropyl, cyclopropylmethyl, n-butyl, isobutyl, sec-butyl, n-pentyl, isopentyl, neopentyl, iso-amyl, n-hexyl, 3-methylhexyl, 2,2-dimethylpentyl, 2,3-dimethylpentyl, and n-heptyl without limitation. Preferably, in the present invention, the alkyl may be an aminoalkyl or haloalkyl. The "aminoalkyl" may refer to an alkyl group in which at least one H is substituted with an N atom, and the "haloalkyl" may refer to an alkyl group in which at least one H is substituted with a halogen atom.

In the present invention, "alkoxy" refers to alkyl bonding to a single oxygen atom (-O-R). In the present invention, the alkoxy may be C₁-C₁₀, C₁-C₈, C₁-C₆, C₁-C₅, C₁-C₃, or C₁-C₂ alkoxy, but the present invention is not limited thereto. The alkoxy may refer to, for example, methoxy, ethoxy, phenoxy, or butoxy.

In the present invention, "cycloalkyl" refers to a saturated or partially unsaturated non-aromatic cyclic hydrocarbon group. In the present invention, the cycloalkyl includes both monocyclic and polycyclic hydrocarbon rings. In the present invention, the cycloalkyl may be C₃-C₂₀, C₃-C₁₅, C₃-C₁₂, C₃-C₁₀, C₃-C₈, C₃-C₆, or C₃-C₅ cycloalkyl, but the present invention is not limited thereto. In other words, the cycloalkyl group may be, for example, cyclopropyl, cyclopropylmethyl, cyclobutyl, cyclopentyl, cyclohexyl, or cycloheptyl. In addition, the cycloalkyl group may be a polycyclic ring in which a five-membered ring and a six-membered ring are bonded. In addition, the cycloalkyl according to the present invention may be a cycloalkylamine group that bonds to an amine group.

In the present invention, "heterocycloalkyl" refers to a cyclic hydrocarbon group that includes one or more hetero atoms in the ring in addition to carbon atoms. The hetero atom may be one or more selected from the group consisting of B, N, O, P, S, and SO₂. Preferably, the heterocycloalkyl according to the present invention may include N and further include O or SO₂. Here, the SO₂-containing heterocycloalkyl group means including -SO₂- in the ring skeleton. Specifically, the heterocycloalkyl may be, for example, a heterocyclic amine bonded to an amine group. Alternatively, the heterocycloalkyl group may include piperidinyl, pyrrolidinyl, morphonyl, piperazinyl, aziridinyl, pyrrolopyrazinyl, azetidinyl, 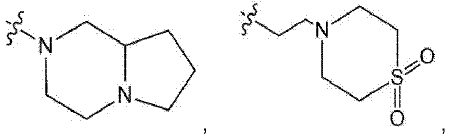 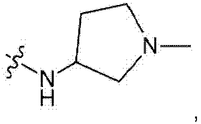 and all possible isomers thereof without limitation.

In the present invention, "aryl" refers to an aromatic system (aromatic ring) including one or more rings, which is used alone or in combination, and also includes a group in which an aromatic ring is fused to one or more carbon rings. In the present invention, the aryl may be a C₃-C₂₀, C₃-C₁₅, C₃-C₁₂, C₃-C₁₀, C₃-C₈, C₃-C₆, or C₅-C₆ aryl, but the present invention is not limited thereto.

In the present invention, "heteroaryl" refers to a monocyclic or bicyclic organic compound, which includes one or more hetero atoms in the ring in addition to carbon atoms. The hetero atom may be one or more selected from the group consisting of B, N, O, P, S, and SO₂. Preferably, the heteroaryl may include at least one N (i.e., at least one C constituting the ring is substituted with N). Alternatively, in the present invention, the heteroaryl may be an aromatic amine.

The aryl and heteroaryl (aromatic ring) according to the present invention may include, for example, phenyl, biphenyl, benzyl, benzoyl, benzidine, toluyl, thienyl, furyl, naphthyl, imidazolyl, pyrrolyl, thiazolyl, oxazolyl, isoxazolyl, pyranyl, pyrazinyl, pyrolinyl, pyridinyl (pyridyl), piperazinyl, pyrimidinyl, pyrazinyl, pyridazinyl, pyrazolyl, thiadiazolyl, triazolyl, indolyl, azaindolyl, indazolyl, azaindazolyl, benzimidazolyl, benzofuranyl, benzothienyl, benzoisoxazolyl, benzoxazolyl, benzopyrazolyl, benzothiazolyl, benzothiadiazolyl, benzotriazolyl, adeninyl, quinolinyl, isoquinolinyl, naphthalenyl, tetrahydronaphthyl, 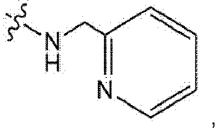 and all possible isomers thereof without limitation.

In the present invention, "alkylamine" refers to an amine group bonded to an alkyl group. The alkylamine may be an amine group which binds to a C₁-C₁₀, C₁-C₈, C₁-C₆, C₁-C₅, or C₁-C₃ alkyl group, but the present invention is not limited thereto.

In the present invention, hetero atom-containing substituents (a heterocycloalkyl group, a heteroaryl group, and a heterocycloalkylamine group) may include 1 to 5, 1 to 4, 1 to 3, 1 or 2, or 1 hetero atom in its skeletal structure, but the present invention is not limited thereto.

In the present invention, the term "substituted" in "substituted or unsubstituted" means that one or more hydrogen atoms in an organic compound are substituted by introducing another atomic group when forming a derivative, and a substituent refers to the introduced atomic group. In a special case, an object to be substituted may be an atom other than a hydrogen atom (e.g., C, forming the skeletal structure, is substituted with a different atom). The functional groups mentioned in the present invention may each independently have one or more hydrogen atoms substituted with other atomic groups. In the present invention, "substitution" includes single substitution, double substitution, triple substitution, or quadruple substitution. The atomic group is preferably selected from OH, halogen, C₁-C₁₀ alkyl, C₁-C₁₀ haloalkyl, C₁-C₁₀ aminoalkyl, C₁-C₁₀ alkoxy, C₃-C₂₀ cycloalkyl, C₂-C₂₀ heterocycloalkyl, C₃-C₂₀ aryl, or C₂-C₂₀ heteroaryl, but the present invention is not limited thereto. The atomic group may be substituted with any one of the functional groups known in the art, including the functional groups described herein.

In the present invention, unless specially stated otherwise, "being substituted or unsubstituted" may mean that at least one H (hydrogen atom) is substituted with a different functional group or unsubstituted. For example, "halogen-substituted or unsubstituted C₃-C₁₀ aryl group" may mean that a C₃-C₁₀ aryl group is unsubstituted or at least one H is substituted with a halogen.

In the present invention, the term "isomer" refers to a compound which has the same molecular formula but differs in the linkage scheme or spatial arrangement of constituent atoms in the molecule. Isomers include, for example, structural isomers and stereoisomers. The stereoisomer may be a diastereomer or an enantiomer. The enantiomer refers to an isomer which does not overlap its mirror image like the relationship between the left hand and right hand and is also called an optical isomer. The enantiomers are classified into R (rectus: clockwise) and S (sinister: counterclockwise) when there are four or more different substituents at a chiral center carbon. The diastereomers refer to stereoisomers that are not mirror images and may be classified into cis-trans isomers due to different spatial arrangements of atoms.

In the present invention, the term "pharmaceutically acceptable salt" includes all salts that are derived from pharmaceutically acceptable inorganic acids, organic acids, or bases.

The term "pharmaceutically acceptable" used herein refers to a compound or composition that is suitable for use in contact with tissue of a subject (e.g., a human) due to a reasonable benefit/risk ratio without excessive toxicity, irritation, allergic reaction or other problems or complications and is included in the scope of sound medical judgment.

Examples of suitable acids may include hydrochloric acid, bromic acid, sulfuric acid, nitric acid, perchloric acid, hydroiodic acid, fumaric acid, maleic acid, phosphoric acid, glycolic acid, lactic acid, salicylic acid, succinic acid, toluene-p-sulfonic acid, tartaric acid, (+)-L-tartaric acid, di-L-tartaric acid, acetic acid, trichloroacetic acid or trifluoroacetic acid, 2,2-dichloroacetic acid, acylated amino acids, adipic acid, alginic acid, ascorbic acid, L-aspartic acid, 4-acetaminobenzoic acid, (+)-camphoric acid, camphorsulfonic acid, (+)-(1S)-camphorsulfonic acid, capric acid, caproic acid, caprylic acid, cinnamic acid, cyclomic acid, dodecylsulfonic acid, ethane-1,2-disulfonic acid, ethanesulfonic acid, 2-hydroxyethanesulfonic acid, galactaric acid, gentisic acid, glucoheptanoic acid, D-gluconic acid, D-glucuronic acid, L-glutamic acid, a-oxo-glutaric acid, hippuric acid, (+)-L-lactic acid, (+-)-DL-lactic acid, lactobionic acid, (-)-L-malic acid, (+-)-DL-mandelic acid, citric acid, methanesulfonic acid, formic acid, benzoic acid, malonic acid, gluconic acid, naphthalene-2-sulfonic acid, naphthalene-1,5-disulfonic acid, benzenesulfonic acid, 1-hydroxy-2-naphthoic acid, nicotinic acid, oleic acid, orotic acid, oxalic acid, di-oxalic acid, palmitic acid, palmic acid, L-pyroglutamic acid, salicylic acid, 4-amino-salicylic acid, sebacic acid, stearic acid, tannic acid, thiocyanic acid, camsylic acid, and undecylic acid. An acid addition salt may be prepared by a conventional method, for example, by dissolving a compound in an excessive amount of acid aqueous solution and precipitating the salt using a water-miscible organic solvent such as methanol, ethanol, acetone or acetonitrile. Alternatively, the acid addition salt may be prepared by heating equimolar amounts of compound and an acid or alcohol in water, and evaporating or drying the mixture, or suction filtering the precipitated salt.

The range of the compound of the present invention may include not only pharmaceutically acceptable salts, but also all isomers, hydrates, and solvates, which can be prepared by conventional methods.

In the present invention, "fibrosis-related disease" may include all diseases directly or indirectly caused by fibrosis and also include diseases accompanied by fibrosis. Fibrosis refers to a phenomenon in which connective tissue is formed within tissue as the extracellular matrix including collagen is secreted by activated fibroblasts. Fibrosis contributes to wound recovery by forming scar tissue, but if connective tissue accumulates excessively, it can paralyze the normal structure and function of a corresponding organ. In the present invention, fibrosis-related diseases include pulmonary fibrosis, skin fibrosis, pancreatic fibrosis, systemic sclerosis, cardiac fibrosis, and macular degeneration. Preferably, in the present invention, the fibrosis-related disease is a pulmonary fibrosis-related disease.

Specifically, the pulmonary fibrosis-related disease may be, but is not limited to, one or more selected from the group consisting of pulmonary fibrosis, idiopathic pulmonary fibrosis (IPF), desquamative interstitial pneumonia, non-specific interstitial pneumonia, cryptogenic organizing pneumonia, respiratory bronchiolitis-associated interstitial lung disease, acute interstitial pneumonia, lymphatic interstitial pneumonia, idiopathic pleuroparenchymal fibroelastosis, and chronic obstructive pulmonary disease (COPD). However, any disease that has pulmonary fibrosis or can be directly or indirectly caused by pulmonary fibrosis is included without limitation. Preferably, in the present invention, the pulmonary fibrosis-related disease is accompanied by hyperactivation of actin polymerization.

In the present invention, "pulmonary fibrosis" is a respiratory disease that causes breathing difficulties due to hardening of lung tissue and specifically refers to a condition in which a normal lung structure is destroyed and hardened due to excessive accumulation of fibrous connective tissue in the lungs. As fibrosis progresses in the lungs, the lung wall thickens and the amount of oxygen supplied to the blood decreases, resulting in shortness of breath. The most common type of pulmonary fibrosis is idiopathic pulmonary fibrosis (IPF), and the main symptom is shortness of breath during exercise. For IPF treatment, nintedanib, which is an anti-fibrotic drug, is used, but the therapeutic effect is known to be only 10%.

In the present invention, the composition may inhibit actin polymerization. The actin includes F-actin. "Actin filaments" are the main component constituting the cytoskeleton of eukaryotes. Actin filaments are assembled from actin monomers through actin polymerization, which hydrolyzes ATP to ADP. While actin polymerization is necessary for controlling cell movement, excessive actin polymerization promotes the expression of alpha-smooth muscle actin (α-SMA) protein and induces the activation of myofibroblasts, a heterogeneous cell population consisting of fibrosis-promoting cells, resulting in tissue fibrosis. The inventors confirmed through specific experiments that the compound according to the present invention can effectively inhibit actin polymerization, and thus fibrosis can be fundamentally treated and alleviated.

In addition, in the present invention, the composition is characterized by reducing the levels or activity of one or more proteins selected from the group consisting of α-SMA, COL1A1, COL4A1, fibronectin, and phosphorylated-SMAD2 (p-SMAD2). The proteins are biomarkers for fibrosis, and particularly, the "α-SMA" protein is a marker for activated myofibroblasts, which are the main effector cells of fibrosis. Myofibroblasts with increased α-SMA expression are known to induce fibrosis by producing fibrotic proteins such as collagen. The inventors confirmed through specific examples that the compounds according to the present invention exhibit an excellent effect of inhibiting α-SMA expression in fibroblasts compared to a conventional pulmonary fibrosis treatment, nintedanib, showing that the compounds according to the present invention have an excellent effect of inhibiting pulmonary fibrosis compared to nintedanib.

The content of the compound in the composition of the present invention can be appropriately adjusted according to the symptoms of a disease, the degree of the progression of symptoms, and a patient's condition, and may be, for example, 0.0001 to 99.9 wt%, or 0.001 to 50 wt% based on the total weight of the composition, but the present invention is not limited thereto. The content ratio is a value based on a dry content from which a solvent is removed.

The pharmaceutical composition according to the present invention may further include appropriate carriers, excipients, and diluents, which are generally used in the preparation of pharmaceutical compositions. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated and used in the form of powders, granules, tablets, sustained-release tablets, enteric tablets, sublingual tablets, troches, pills, capsules, hard capsules, soft capsules, sustained-release capsules, enteric capsules, granules, sustained-release granules, enteric granules, powders, dry extracts, liquids, suspensions, soft extracts, fluid extracts, lemonades, or external preparations such as aromatic water, oils, spirits, tinctures, inhalants, elixirs, injections, perfusates, sterile injectable solutions, eye drops, plasters, lotions, pastes, sprays, patches, or aerosols according to conventional methods, and the external preparations may be formulated as a cream, a gel, a patch, a spray, an ointment, a plaster, a lotion, a liniment, a paste, or a cataplasma.

Carriers, excipients, and diluents, which can be included in the pharmaceutical composition according to the present invention, may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

In the case of formulation, the composition according to the present invention may be prepared using a diluent or an excipient such as a filler, a thickening agent, a binder, a wetting agent, a disintegrant, and a surfactant, which are commonly used.

Additives for a tablet, a powder, a granule, a capsule, a pill, or a troche according to the present invention may include excipients such as corn starch, potato starch, wheat starch, lactose, sucrose, glucose, fructose, di-mannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose (HPMC) 1928, HPMC 2208, HPMC 2906, HPMC 2910, propylene glycol, casein, calcium lactate, and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol, and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, petrolatum, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine, and light anhydrous silicic acid.

Additives for liquid formulations according to the present invention may include water, diluted hydrochloric acid, diluted sulfuric acid, sodium citrate, sucrose monostearate, polyoxyethylene sorbitol fatty acid ester (Tween ester), polyoxyethylene monoalkyl ether, lanolin ether, lanolin ester, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose.

The syrup according to the present invention may include a solution of white sugar, a different type of sugar, or a sweetener, and if necessary, a fragrance, a colorant, a preservative, a stabilizer, a suspending agent, an emulsifier, or a thickener.

The emulsion according to the present invention may include distilled water, and if necessary, an emulsifier, a preservative, a stabilizer, or a fragrance.

The suspension according to the present invention may include a suspending agent such as acacia, tragacanth, methylcellulose, carboxymethylcellulose, sodium carboxymethylcellulose, microcrystalline cellulose, sodium alginate, hydroxypropylmethyl cellulose (HPMC), HPMC 1828, HPMC 2906, or HPMC 2910, and if necessary, a surfactant, a preservative, a stabilizer, a colorant, or a fragrance.

Injections according to the present invention may include a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetraacetic acid; an antioxidant such as sodium bisulfide 0.1%, sodium formaldehyde sulfoxylate, thiourea, disodium ethylenediaminetetraacetate or acetone sodium bisulfite; an analgesic such as benzyl alcohol, chlorobutanol, procaine hydrochloride, glucose or calcium gluconate; or a suspending agent such as sodium CMC, sodium alginate, Tween 80 or aluminum monostearate.

Suppositories according to the present invention may include a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium (A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57).

Solid preparations for oral administration include tablets, pills, powder, granules, and capsules, and such solid preparations are formulated by mixing the extract with at least one excipient, for example, starch, calcium carbonate, sucrose, lactose, or gelatin. Additionally, in addition to simple excipients, lubricants such as magnesium stearate, and talc are also used.

Liquid preparations for oral administration include a suspension, a liquid for internal use, an emulsion, or a syrup may be used, and a generally-used simple diluent such as water or liquid paraffin, as well as various types of excipients, for example, a wetting agent, a sweetener, a fragrance and a preservative may be included. Preparations for parenteral administration include sterilized aqueous solutions, non-aqueous solvents, suspensions, emulsions, lyophilized formulations, and suppositories. As the non-aqueous solvent or suspension, propylene glycol, polyethylene glycol, a vegetable oil such as olive oil, or an injectable ester such as ethyl oleate may be used.

The pharmaceutical composition according to the present invention is administered at a pharmaceutically effective amount. The "pharmaceutically effective amount" used herein refers to an amount sufficient for treating a disease at a reasonable benefit/risk ratio applicable to medical treatment, and an effective dosage may be determined by parameters including the type and severity of a patient's disease, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art.

The pharmaceutical composition of the present invention may be administered into a subject via various routes. All administration routes may be considered, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention is determined according to the type of drug as an active ingredient as well as various related parameters such as a disease to be treated, an administration route, a patient's age, sex, body weight, and the severity of a disease. Specifically, the effective amount of the composition according to the present invention may be changed according to a patient's age, sex or body weight, and generally, 0.001 to 500 mg, and preferably 0.01 to 100 mg per kg of body weight may be administered daily or every other day, or one to three times a day. However, the effective amount may be increased or decreased depending on the route of administration, the severity of obesity, sex, a body weight or age, and thus the above dosage does not limit the scope of the present invention in any way.

In the present invention, "individual" refers to a subject in need of treatment for a disease, and more specifically, a mammal such as a human or a non-human primate, a mouse, a rat, a dog, a cat, a horse, or a cow.

In the present invention, "administration" refers to providing a given composition of the present invention to a subject by any appropriate method.

In the present invention, "prevention" refers to all actions involved in inhibiting or delaying the onset of a desired disease, "treatment" refers to all actions involved in improving or beneficially changing a disease and its metabolic abnormalities by administering the pharmaceutical composition according to the present invention, and "alleviation" refers to all actions involved in reducing parameters related to a desired disease, e.g., the severity of symptoms, by administering the composition according to the present invention.

In addition, the present invention provides a kit for preventing or treating a pulmonary fibrosis-related disease, which includes the composition according to the present invention. In the present invention, "kit" refers to a combination of materials and devices for preventing or treating pulmonary fibrosis using the compound according to the present invention, and there is no limitation on the specific form. The kit according to the present invention may not only include the compound according to the present invention for preventing and/or treating a pulmonary fibrosis-related disease, but also one or more types of component compositions, solutions, or devices, which are suitable for preventing, alleviating, or treating a disease.

In addition, the present invention provides a method of preparing the compound according to the present invention, which includes the following steps:
(M1) reacting fluorinated nitrobenzene with any one or more selected from the group consisting of aniline, fluoroaniline, and cyclohexylamine; or
(M2) reacting tetrahydroquinoxaline with any one or more selected from the group consisting of di-tert-butyl-dicarbonate, acetone, and triacetoxyborohydride in an organic solvent.

In (M1), the reaction may be performed at 50 to 200 °C, 50 to 150 °C, or 100 to 200 °C, and then cooled to room temperature, but the present invention is not limited thereto.

In (M2), the reaction may be performed at 10 to 50 °C, 10 to 40 °C, 10 to 30 °C, 10 to 20 °C, or room temperature, but the present invention is not limited thereto.

The preparation method may further include extracting a reaction product with an organic solvent after (M1) or (M2), and then concentrating the extracted reaction product under reduced pressure. The organic solvent may be a halogenated alkyl solution, and preferably, dichloromethane, but the present invention is not limited thereto.

Throughout the specification, when one part "includes" a component, it means that it may also include other components rather than excluding components unless specifically stated otherwise. The term "approximately" or "substantially" used herein are used at, or in proximity to, numerical values when allowable manufacturing and material tolerances, which are inherent in the meanings, are presented. These terms are used to prevent the unfair use of the disclosures in which correct or absolute values are cited to help in understanding the present invention by unscrupulous infringers.

Throughout the specification, the term "combination thereof" included in the Markush-type expression refers to a mixture or combination of one or more selected from the group consisting of constituents described in the Markush-type expression, that is, one or more selected from the group consisting of the components.

Hereinafter, preferred examples are presented to allow the present invention to be better understood. However, the following examples are merely provided to understand the present invention more easily, and the content of the present invention is not limited by the following examples.

### [Examples]

### Example 1. Preparation of compounds

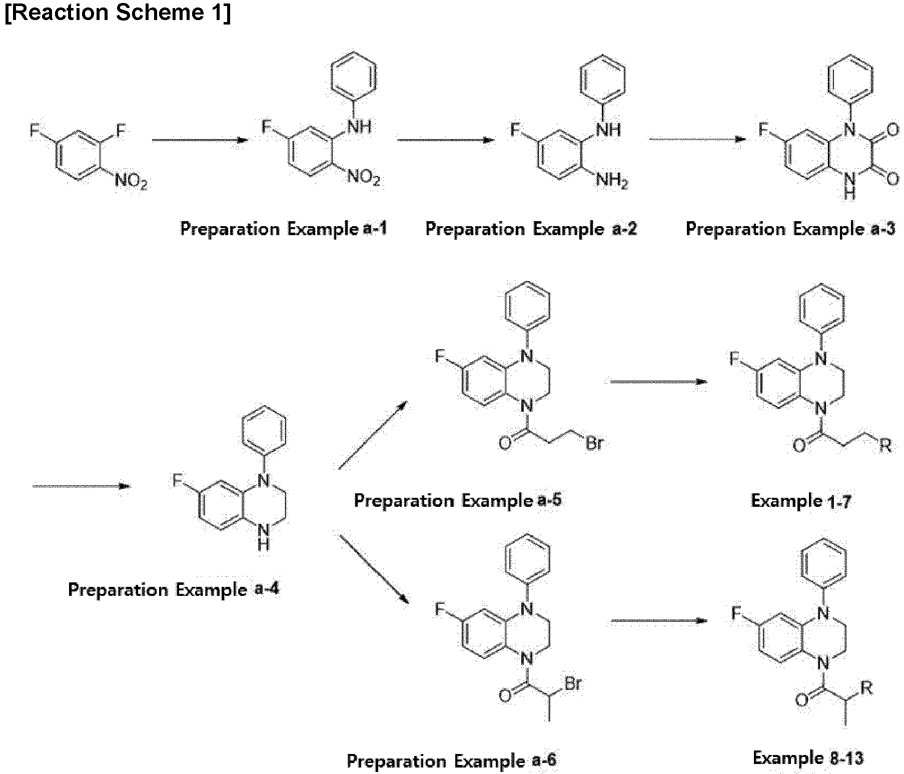

In Reaction Scheme 1,
R is the same as defined in Chemical Formula 1 described above.

### <Preparation Example a-1> Preparation of 5-fluoro-2-nitro-N-phenylaniline

A solution in which 2,4-difluoro-1-nitrobenzene (1.0 equiv) and aniline (1.0 equiv) were mixed was stirred at 130 °C for 24 hours and cooled to room temperature. After the reaction was terminated by adding water, the reaction solution was extracted with dichloromethane. An organic layer was washed with brine, dried with MgSO₄, filtered, and then concentrated under reduced pressure. The obtained residue was purified by silica column chromatography using an ethyl acetate/hexane mixture, thereby preparing 5-fluoro-2-nitro-*N*-phenylaniline.

Yellow solid (yield 63%); ¹H NMR (400 MHz, CDCl₃) δ 9.65 (s, 1H), 8.26 (dd, 1H, *J* = 9.4, 5.9 Hz), 7.45 (m, 2H), 7.31-7.26 (m, 3H), 6.80 (dd, 1H, *J* = 11.3, 2.3 Hz), 6.48 (ddd, 1H, *J* = 9.5, 6.9, 2.7 Hz).

### <Preparation Example a-2> Preparation of 5-fluoro-N¹-phenylbenzene -1,2-diamine

A mixture in which 5-fluoro-2-nitro-*N*-phenylaniline (1.0 equiv) obtained in Preparation Example a-1 and tin(II) chloride dehydrate (3.0 equiv) were dissolved in ethyl acetate (0.5 M) was stirred at 90 °C for 5 hours. The reaction solution was cooled to room temperature, poured into water, neutralized with a 10M NaOH aqueous solution, and then extracted with ethyl acetate. An organic layer was washed with brine, dried with MgSO_{4,} filtered, and then concentrated under reduced pressure. The obtained residue was purified by silica column chromatography using an ethyl acetate/hexane mixture, thereby preparing 5-fluoro-*N*¹-phenylbenzene-1,2-diamine.
Brown oil (yield 86%); ¹H NMR (400 MHz, CDCl₃) δ 7.28-7.24 (m, 2H), 6.93-6.86 (m, 3H), 6.75-6.71 (m, 1H), 6.66 (td, 1H, *J* = 8.2, 2.8 Hz), 5.34 (s, 1H), 3.89 (s, 2H); LC/MS ESI (+): 202.7 (M+1)

### <Preparation Example a-3> Preparation of 7-fluoro-1-phenylquinoxaline-2,3(1H,4H)-dione

A reaction solution in which 5-fluoro-*N*¹-phenylbenzene-1,2-diamine (1.0 equiv) obtained in Preparation Example a-2 was dissolved in diethyl oxalate (6.0 equiv) was stirred at 160 °C for 24 hours. The reaction solution was cooled to room temperature, filtered and dried, thereby preparing 7-fluoro-1-phenylquinoxaline-2,3(1H,4H)-dione.
White solid (yield 86%); ¹H NMR (400 MHz, DMSO-*d*₆) δ 12.11 (s, 1H), 7.64-7.54 (m, 3H), 7.40-7.38 (m, 2H), 7.22 (dd, 1H, *J* = 8.8, 5.3 Hz), 7.0 (td, 1H, *J* = 8.6, 2.7 Hz), 5.99 (dd, 1H, *J* = 10.2, 2.7 Hz); LC/MS ESI (+): 256.6 (M+1)

### <Preparation Example a-4> Preparation of 7-fluoro-1-phenyl-1,2,3,4-tetrahydroquinoxaline

A borane-THF complex (1 M) (3.0 equiv) was slowly added to a solution in which 7-fluoro-1-phenylquinoxaline-2,3(1H,4H)-dione (1.0 equiv) obtained in Preparation Example a-3 was dissolved in THF (0.5 M) at room temperature and then stirred at 65 °C for 16 hours. After cooling the reaction solution to room temperature, water was added to terminate the reaction, the reaction product was neutralized with a saturated NaHCO₃ aqueous solution and extracted with ethyl acetate. An organic layer was washed with brine, dried with MgSO₄, filtered, and then concentrated under reduced pressure. The obtained residue was purified by silica column chromatography using an ethyl acetate/hexane mixture, thereby preparing 7-fluoro-1-phenyl-1,2,3,4-tetrahydroquinoxaline.
Brown solid (yield 38%); ¹H NMR (400 MHz, CDCl₃) δ 7.38-7.34 (m, 2H), 7.24-7.20 (m, 2H), 7.13-7.09 (m, 1H), 6.52-6.46 (m, 2H), 6.36 (td, 1H, *J* = 8.4, 2.7 Hz), 3.71-3.68 (m, 2H), 3.46-3.44 (m, 2H); LC/MS ESI (+): 228.9 (M+1)

### <Preparation Example a-5> Preparation of 3-bromo-1-(6-fluoro-4-phenyl-3,4-dihydroquinoxalin-1(2H)-yl)propan-1-one

7-Fluoro-1-phenyl-1,2,3,4-tetrahydroquinoxaline (1.0 equiv) obtained in Preparation Example a-4 was dissolved in dichloromethane (0.2 M), and then 3-bromopropanoyl chloride (1.1 equiv) was slowly added at -78 °C. The reaction solution was stirred at room temperature for 1.5 hours, water was added to terminate the reaction, and extraction was performed with dichloromethane. An organic layer was washed with brine, dried with MgSO₄, filtered, and distilled under reduced pressure. The obtained residue was purified by silica column chromatography using an ethyl acetate/hexane mixture, thereby preparing 3-bromo-1-(6-fluoro-4-phenyl-3,4-dihydroquinoxalin-1(2H)-yl)propan-1-one.

White solid (yield 94%); ¹H NMR (400 MHz, CDCl₃) δ 7.42 (m, 2H), 7.24 (m, 3H), 6.97 (brs, 1H), 6.45-6.37 (m, 2H), 4.06 (brs, 2H), 3.75-3.68 (m, 4H), 3.17 (t, 2H, *J* = 6.3 Hz).

### <Preparation Example a-6> Preparation of 2-bromo-1-(6-fluoro-4-phenyl-3,4-dihydroquinoxalin-1(2H)-yl)propan-1-one

7-Fluoro-1-phenyl-1,2,3,4-tetrahydroquinoxaline (1.0 equiv) obtained in Preparation Example a-4 was dissolved in dichloromethane (0.2 M), and then 2-bromopropionyl chloride (1.1 equiv) was slowly added at -78 °C. The reaction solution was stirred at room temperature for 1.5 hours, water was added to terminate the reaction, and then extraction was performed with dichloromethane. An organic layer was washed with brine, dried with MgSO₄, filtered, and then distilled under reduced pressure. The obtained residue was purified by silica column chromatography using an ethyl acetate/hexane mixture, thereby preparing 2-bromo-1-(6-fluoro-4-phenyl-3,4-dihydroquinoxalin-1(2H)-yl)propan-1-one.

Light purple solid (yield 83%); ¹H NMR (400 MHz, CDCl₃) δ 7.44-7.40 (m, 2H), 7.27-7.25 (m, 3H), 7.14 (m, 1H), 6.47-6.38 (m, 2H), 5.04 (m, 1H), 4.18 (brs, 1H), 3.90 (brs, 1H), 3.78-3.69 (m, 2H), 1.86 (d, 3H, *J* = 6.7 Hz).

### <Example 1-1> Synthesis of 1-(6-fluoro-4-phenyl-3,4-dihydroquinoxalin-1 (2H)-yl)-3-(pyrrolidin-1-yl)propan-1-one

Pyrrolidine (3.0 equiv) was added to a solution in which 3-bromo-1-(6-fluoro-4-phenyl-3,4-dihydroquinoxalin-1(2H)-yl)propan-1-one (1.0 equiv) obtained in Preparation Example a-5 was dissolved in THF (0.2 M) and then stirred at 90 °C for 3 hours. After cooling the resulting solution to room temperature, water was added to terminate the reaction, and extraction was performed with ethyl acetate. An organic layer was washed with brine, dried with MgSO₄, filtered, and then distilled under reduced pressure. The obtained residue was purified by silica column chromatography using a methanol/dichloromethane mixture, thereby synthesizing 1-(6-fluoro-4-phenyl-3,4-dihydroquinoxalin-1(2H)-yl)-3-(pyrrolidin-1-yl)propan-1-one.
White solid (yield 58%); ¹H NMR (400 MHz, CDCl₃) δ 7.45-7.35 (m, 2H), 7.25-7.21 (m, 3H), 7.09-6.09 (m, 1H), 6.45-6.35 (m, 2H), 4.06-3.96 (m, 2H), 3.71 (t, 2H, *J* = 5.3 Hz), 2.90-2.77 (m, 4H), 2.55-2.40 (m, 4H), 1.80-1.69 (m, 4H); LC/MS ESI (+): 354.1 (M+1)

### <Example 1-2> Synthesis of 1-(6-fluoro-4-phenyl-3,4-dihydroquinoxalin-1(2H)-yl)-3-morpholinopropan-1-one

1-(6-Fluoro-4-phenyl-3,4-dihydroquinoxalin-1(2H)-yl)-3-morpholinopropan-1-one was synthesized in the same manner as in Example 1-1, except that morpholine was used instead of pyrrolidine.
White solid (yield 44%); ¹H NMR (400 MHz, CDCl₃) δ 7.45-7.38 (m, 2H), 7.25-7.22 (m, 3H), 7.09-6.97 (m, 1H), 6.45-6.36 (m, 2H), 4.07-3.97 (m, 2H), 3.72 (t, 2H, *J* = 5.5 Hz), 3.69-3.61 (m, 4H), 2.80-2.70 (m, 4H), 2.46-2.34 (m, 4H); LC/MS ESI (+): 369.9 (M+1)

### <Example 1-3> Synthesis of 1-(6-fluoro-4-phenyl-3,4-dihydroquinoxalin-1(2H)-yl)-3-(piperidin-1-yl)propan-1-one

1-(6-Fluoro-4-phenyl-3,4-dihydroquinoxaline-1(2H)-yl)-3-(piperidin-1-yl)propan-1-one was synthesized in the same manner as in Example 1-1, except piperidine was used instead of pyrrolidine.
White solid (yield 53%); ¹H NMR (400 MHz, CDCl₃) δ 7.45-7.38 (m, 2H), 7.25-7.21 (m, 3H), 7.11-6.99 (m, 1H), 6.45-6.35 (m, 2H), 4.05-3.97 (m, 2H), 3.71 (t, 2H, *J* = 5.3 Hz), 2.80-2.67 (m, 4H), 2.43-2.27 (m, 4H), 1.58-1.49 (m, 4H), 1.45-1.36 (m, 2H); LC/MS ESI (+): 367.9 (M+1)

### <Example 1-4> Synthesis of 1-(6-fluoro-4-phenyl-3,4-dihydroquinoxalin-1(2H)-yl)-3-(4-methylpirperazin-1-yl)propan-1-one

1-(6-Fluoro-4-phenyl-3,4-dihydroquinoxaline-1 (2H)-yl)-3-(4-methylpiperazin-1-yl)propan-1-one was synthesized in the same manner as in Example 1-1, except 1-methylpiperazine was used instead of pyrrolidine.
White solid (yield 62%); ¹H NMR (400 MHz, CDCl₃) δ 7.45-7.38 (m, 2H), 7.25-7.21 (m, 3H), 7.11-6.98 (m, 1H), 6.45-6.36 (m, 2H), 4.05-3.97 (m, 2H), 3.72 (t, 2H, *J* = 5.5 Hz), 2.80-2.71 (m, 4H), 2.60-2.29 (m, 8H), 2.26 (s, 3H); LC/MS ESI (+): 383.1 (M+1)

### <Example 1-5> Synthesis of (R)-1-(6-fluoro-4-phenyl-3,4-dihydroquinoxalin-1(2H)-yl)-3-(2-methylpyrrolidin-1-yl)porpan-1-one

(R)-1-(6-fluoro-4-phenyl-3,4-dihydroquinoxaline-1(2H)-yl)-3-(2-methylpyrrolidin-1-yl)propan-1-one was synthesized in the same manner as in Example 1-1, except that (R)-2-methylpyrrolidine was used instead of pyrrolidine.
Yellow solid (yield 65%); ¹H NMR (400 MHz, CD₃OD) δ 7.41-7.50 (m, 2H), 7.35-7.20 (m, 4H), 6.51-6.36 (m, 1H), 6.34-6.21 (m, 1H), 4.14-3.95 (m, 2H) , 3.87-3.67 (m, 2H), 3.56-3.34 (m, 2H), 3.13-2.92 (m, 4H), 2.87-2.72 (m, 1H), 2.30-2.10 (m, 1H), 2.08-1.85 (m, 2H), 1.71-1.64 (m, 1H), 1.32 (s, 3H); LC/MS ESI (+): 368.1 (M+1)

### <Example 1-6> Synthesis of (R)-1-(6-fluoro-4-phenyl-3,4-dihydroquinoxalin-1(2H)-yl)-3-(2-methylpiperidin-1-yl)propan-1-one

(R)-1-(6-fluoro-4-phenyl-3,4-dihydroquinoxaline-1(2H)-yl)-3-(2-methylpiperidin-1-yl)propan-1-one was synthesized in the same manner as in Example 1-1, except that (R)-2-methylpiperidine was used instead of pyrrolidine.
Brown oil (yield 65%); ¹H NMR (400 MHz, CDCl₃) δ 7.45-7.37 (m, 2H), 7.26-7.21 (m, 3H), 7.16-7.05 (m, 1H), 6.50-6.35 (m, 2H), 4.12-3.93 (m, 2H), 3.83-3.65 (m, 2H), 3.47-3.35 (m, 1H), 3.25-2.96 (m, 5H), 2.72-2.59 (m, 1H), 2.01 (s, 2H), 1.85-1.71 (m, 4H), 1.52-1.26 (m, 4H); LC/MS ESI (+): 382.1 (M+1)

### <Example 1-7> Synthesis of 1-(6-fluoro-4-phenyl-3,4-dihydroquinoxalin-1(2H)-yl)-3-(1H-imidazol-1-yl)propan-1-one

1-(6-Fluoro-4-phenyl-3,4-dihydroquinoxaline-1(2H)-yl)-3-(1H-imidazol-1-yl)propan-1-one was synthesized in the same manner as in Example 1-1, except that imidazole was used instead of pyrrolidine.
Yellow oil (yield 59%); ¹H NMR (400 MHz, CD₃OD) δ 7.60-7.54 (m, 1H),7.49- 7.41 (m, 2H), 7.31-7.17 (m, 3H), 7.06-6.94 (m, 2H), 6.91-6.85 (m, 1H), 6.43-6.38 (m, 1H), 6.22-6.18 (m, 1H), 4.42-4.34 (m, 2H), 3.95 (d, 2H, *J* = 5.1 Hz), 3.57-3.47(m, 2H), 3.10 (t, 2H, *J* = 6.3 Hz); LC/MS ESI(+): 351.2 (M+1)

### <Example 1-8> Synthesis of 1-(6-fluoro-4-phenyl-3,4-dihydroquinoxalin-1(2H)-yl)-2-((R)-2-methylpiperidin-1-yl)propan-1-one

(R)-2-methylpiperidine (3.0 equiv) was added to a solution in which 2-bromo-1-(6-fluoro-4-phenyl-3,4-dihydroquinoxalin-1(2H)-yl)propan-1-one (1.0 equiv) obtained in Preparation Example a-6 was dissolved in THF (0.2 M) and then stirred at 120 °C for 3 hours. The resulting solution was cooled to room temperature, water was added to terminate the reaction, and then extraction was performed with ethyl acetate. An organic layer was washed with brine, dried with MgSO₄, filtered, and distilled under reduced pressure. The obtained residue was purified by silica column chromatography using a methanol/dichloromethane mixture, thereby synthesizing 1-(6-fluoro-4-phenyl-3,4-dihydroquinoxaline-1(2H)-yl)-2-((R)-2-methylpiperidin-1-yl)propan-1-one.
White solid (yield 32%); ¹H NMR (400 MHz, CDCl₃) δ 7.47-7.39 (m, 2H), 7.25-7.21 (m, 1H), 7.04-6.95 (m, 1H), 6.50-6.41 (m, 2H), 4.75-4.64 (m, 1H), 4.52-4.41 (m, 1H), 4.16-3.96 (m, 1H), 3.92-3.82 (m, 1H), 3.70-3.58 (m, 1H), 3.44-3.32 (m, 1H), 3.20-3.09 (m, 1H), 2.99-2.79 (m, 2H), 2.63-2.48 (m, 1H), 1.69-1.59 (m, 3H), 1.50-1.39 (m, 4H), 1.35-1.27 (m, 2H), 1.88 (s, 3H); LC/MS ESI (+): 381.9 (M+1)

### <Example 1-9> Synthesis of 1-(6-fluoro-4-phenyl-3,4-dihydroquinoxalin-1(2H)-yl)-2-morpholinopropan-1-one

1-(6-fluoro-4-phenyl-3,4-dihydroquinoxaline-1(2H)-yl)-2-morpholinopropan-1-one was synthesized in the same manner as in Example 1-8, except that morpholine was used instead of (R)-2-methylpiperidine.
Yellow solid (yield 18%); ¹H NMR (400 MHz, CDCl₃) δ 7.46-7.38 (m, 2H), 7.25-7.17 (m, 4H), 6.48-6.35 (m, 2H), 4.27-4.16 (m, 1H), 4.02-3.94 (m, 1H), 3.92-3.83 (m, 1H), 3.81-3.61 (m, 6H), 2.65-2.45 (m, 4H), 1.26 (d, 3H, *J* = 6.3 Hz); LC/MS ESI (+): 370.0 (M+1)

### <Example 1-10> Synthesis of 1-(6-fluoro-4-phenyl-3,4-dihydroquinoxalin-1(2H)-yl)-2-(pyrrolidin-1-yl)propan-1-one

1-(6-Fluoro-4-phenyl-3,4-dihydroquinoxalin-1(2H)-yl)-2-(pyrrolidin-1-yl)propan-1-one was synthesized in the same manner as in Example 1-8, except that pyrrolidine was used instead of (R)-2-methylpiperidine.
Yellow oil (yield 43%); ¹H NMR (400 MHz, CDCl₃) δ 7.45-7.37 (m, 2H), 7.26-7.18 (m, 3H), 7.18-7.04 (m, 1H), 6.49-6.38 (m, 2H), 4.33-4.21 (m, 1H), 4.01-3.89 (m, 1H), 3.82-3.75 (m, 2H), 3.73-3.67 (m, 1H), 2.71-2.51 (m, 4H), 1.79-1.69 (m, 4H), 1.34 (d, 3H, *J* = 6.7 Hz); LC/MS ESI (+): 354.1 (M+1)

### <Example 1-11> Synthesis of 1-(6-fluoro-4-phenyl-3,4-dihydroquinoxaline-1(2H)-yl)-2-(4-methylpiperazin-1-yl)propan-1-one

1-(6-Fluoro-4-phenyl-3,4-dihydroquinoxaline-1(2H)-yl)-2-(4-methylpiperazin-1-yl)propan-1-one was synthesized in the same manner as in Example 1-8, except that 1-methylpiperazine was used instead of (R)-2-methylpiperidine.
Yellow solid (yield 14%); ¹H NMR (400 MHz, CDCl₃) δ 7.45-7.37 (m, 2H), 7.25-7.18 (m, 4H), 6.45-6.39 (m, 2H), 4.20-4.11 (m, 1H), 3.99-3.91 (m, 2H), 3.79-3.68 (m, 2H), 2.71-2.30 (m, 8H), 2.25 (s, 3H), 1.26 (s, 3H); LC/MS ESI (+): 383.0 (M+1)

### <Example 1-12> Synthesis of 1-(6-fluoro-4-phenyl-3,4-dihydroquinoxaline-1(2H)-yl)-2-(piperidin-1-yl)propan-1-one

1-(6-Fluoro-4-phenyl-3,4-dihydroquinoxaline-1(2H)-yl)-2-(piperidin-1-yl)propan-1-one was synthesized in the same manner as in Example 1-8, except that piperidine was used instead of (R)-2-methylpiperidine.
White solid (yield 42%); ¹H NMR (400 MHz, CDCl₃) δ 7.47-7.35 (m, 2H), 7.25-7.20 (m, 3H), 6.44-6.34 (m, 2H), 4.33-4.14 (m, 1H), 3.97-3.70 (m, 4H), 2.55-2.42 (m, 4H), 1.58-1.43 (m, 4H), 1.43-1.33 (m, 2H), 1.24 (d, 3H, *J* = 7.0 Hz); LC/MS ESI (+): 368.1 (M+1)

### <Example 1-13> Synthesis of 1-(6-fluoro-4-phenyl)-3,4-dihydroquinoxaline-1(2H)-yl)-2-((R)-2-methylpyrrolidin-1-yl)propan-1-one

1-(6-Fluoro-4-phenyl)-3,4-dihydroquinoxaline-1(2H)-yl)-2-((R)-2-methylpyrrolidin-1-yl)propan-1-one was synthesized in the same manner as in Example 1-8, except that (R)-2-methylpyrrolidine was used instead of (R)-2-methylpiperidine.
Yellow oil (yield 16.4%); ¹H NMR (400 MHz, CDCl₃) δ 7.45-7.38 (m, 2H), 7.25-7.19 (m, 3H), 7.00-6.93 (m, 1H), 6.48-6.38 (m, 2H), 4.84-4.69 (m, 1H), 4.42-4.29 (m, 1H), 3.86 (dt, 1H, *J* = 11.3, 3.9 Hz), 3.67-3.55 (m, 1H), 3.41-3.27 (m, 1H), 3.16-2.97 (m, 2H), 2.66-2.54 (m, 1H), 1.90-1.76 (m, 1H), 1.73-1.57 (m, 3H), 1.48-1.38 (m, 3H), 1.36-1.28 (m, 1H), 0.84-0.72 (m, 2H); LC/MS ESI (+): 368.2 (M+1)

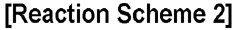

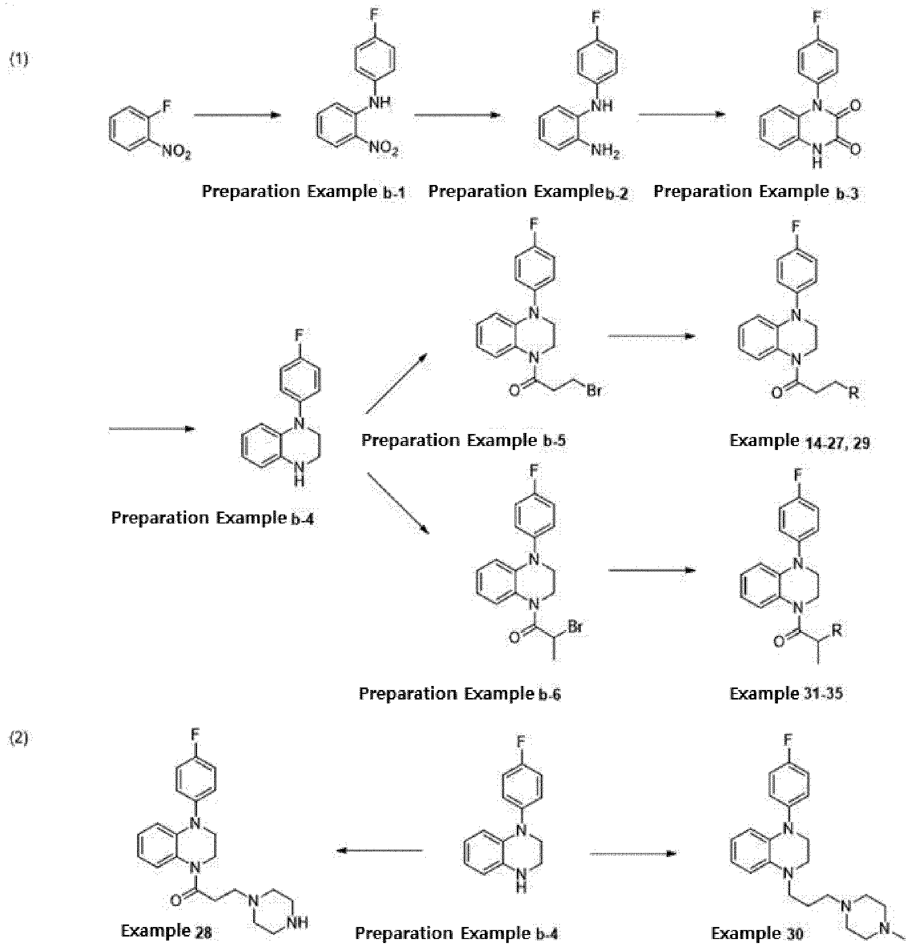

### <Preparation method b> Preparation of 1-(4-fluorophenyl)-1,2,3,4-tetrahydroquinoxaline

1-(4-Fluorophenyl)-1,2,3,4-tetrahydroquinoxaline according to the present invention was prepared through the processes of Preparation Examples b-1 to b-6 below.

### <Preparation Example b-1> Preparation of N-(4-fluorophenyl)-2-nitroaniline

*N*-(4-Fluorophenyl)-2-nitroanile was prepared in the same manner as in Preparation Example a-1, except that 1-fluoro-2-nitrobenzene was used instead of 2,4-difluoro-1-nitrobenzene.

Yellow solid (yield 30%); ¹H NMR (400 MHz, CDCl₃) δ 9.40 (s, 1H), 8.21 (dd, 1H, *J* = 8.6, 1.6 Hz), 7.38-7.34 (m, 1H), 7.27-7.22 (m, 2H), 7.15-7.09 (m, 2H), 7.05 (dd, 1.H, *J* = 8.6, 1.2 Hz), 6.77 (ddd, 1H, *J* = 8.4, 6.8, 1.2 Hz).

### <Preparation Example b-2> Preparation of N¹-(4-fluorophenyl)benzen-1,2-diamine

*N*¹-(4-Fluorophenyl)benzen-1,2-diamine was prepared in the same manner as in Preparation Example a-2, except that *N*-(4-fluorophenyl)-2-nitroaniline obtained in Preparation Example b-1 was used instead of 5-fluoro-*N*-(4-fluorophenyl)-2-nitroaniline.
Orange solid (yield 61%); ¹H NMR (400 MHz, CDCl₃) δ 7.06 (dd, 1H, *J* = 7.8, 1.2 Hz), 7.01 (td, 1H, *J* = 7.6, 1.2 Hz), 6,95-6.89 (m, 2H), 6.81 (dd, 1H, *J* = 7.8, 1.2 Hz), 6.76 (td, 1H, *J* = 7.6, 1.2 Hz), 6.73-6.67 (m, 2H), 5.09 (s, 1H), 3.74 (s, 2H); LC/MS ESI (+): 202.6 (M+1)

### <Preparation Example b-3> Preparation of 1-(4-fluorophenyl)quinoxalin-2,3(1H,4H)-dione

1-(4-Fluorophenyl)quinoxalin-2,3(1H,4H)-dione was prepared in the same manner as in Preparation Example a-3, except that *N*¹-(4-fluorophenyl)benzen-1,2-diamine obtained in Preparation Example b-2 was used instead of 5-fluoro-*N*¹-phenylbenzene-1,2-diamine.
White solid (yield 81%); ¹H NMR (400 MHz, DMSO-*d*₆) δ 12.11 (s, 1H), 7.45 (d, 4H, *J* = 6.7 Hz), 7.21 (m, 1H), 7.13 (td, 1H, *J* = 7.6, 1.2 Hz), 6.98 (m, 1H), 6.32 (dd, 1H, *J* = 8.2, 0.8 Hz); LC/MS ESI (+): 256.6 (M+1)

### <Preparation Example b-4> Preparation of 1-(4-fluorophenyl)-1,2,3,4-tetrahydroquinoxaline

1-(4-Fluorophenyl)-1,2,3,4-tetrahydroquinoxaline was prepared in the same manner as in Preparation Example a-4, except that 1-(4-fluorophenyl)quinoxalin-2,3(1H,4H)-dione obtained in Preparation Example b-3 was used instead of 7-fluoro-1-phenylquinoxaline-2,3(1H,4H)-dione.
Brown solid (yield 38%); ¹H NMR (400 MHz, CDCl₃) δ 7.18-7.13 (m, 2H), 7.05-6.99 (m, 2H), 6.70-6.53 (m, 4H), 3.66-3.64 (m, 2H), 3.48-3.45 (m, 2H); LC/MS ESI (+): 228.8 (M+1)

### <Preparation Example b-5> Preparation of 3-bromo-1-(4-(4-fluorophenyl)-3,4-dihydroquinoxalin-1(2H)-yl)propan-1-one

### 3-Bromo-1-(4-(4-fluorophenyl)-3,4-dihydroquinoxalin-1(2H)-yl)propan-1-one was prepared in the same manner as in Preparation Example a-5, except that 1-(4-fluorophenyl)-1,2,3,4-tetrahydroquinoxaline obtained in Preparation Example b-4 was used instead of 7-fluoro-1-phenyl-1,2,3,4-tetrahydroquinoxaline.

Yellow solid (yield 88%); ¹H NMR (400 MHz, CDCl₃) δ 7.24-7.19 (m, 2H), 7.12-7.03 (m, 3H), 6.96 (t, 1H, *J* = 7.6 Hz), 6.77-6.71 (m, 1H), 6.65-6.60 (m, 1H), 4.07 (m, 2H), 3.71 (m, 4H), 3.21 (t, 2H, *J* = 6.5 Hz).

### <Preparation Example b-6> Preparation of 2-bromo-1-(4-(4-fluorophenyl)-3,4-dihydroquinoxalin-1(2H)-yl)propan-1-one

2-Bromo-1-(4-(4-fluorophenyl)-3,4-dihydroquinoxalin-1(2H)-yl)propan-1-one was prepared in the same manner as in Preparation Example a-6, except that 1-(4-fluorophenyl)-1,2,3,4-tetrahydroquinoxaline obtained in Preparation Example b-4 was used instead of 7-fluoro-1-phenyl-1,2,3,4-tetrahydroquinoxaline.

Ivory solid (yield 62%); ¹H NMR (400 MHz, CDCl₃) δ 7.25-7.21 (m, 2H), 7.18 (d, 1H, *J* = 7.4 Hz), 7.12-7.06 (m, 2H), 7.01 (t, 1H, *J* = 7.2 Hz), 6.76 (td, 1H, *J*= 7.6, 1.2 Hz), 6.65 (d, 1H, *J* = 8.2 Hz), 5.12 (m, 1H), 4.21 (brs, 1H), 3.91 (brs, 1H), 3.76-3.68 (m, 2H), 1.87 (d, 3H, *J* = 6.3 Hz).

### <Example 1-14> Synthesis of 1-(4-(4-fluorophenyl)-3,4-dihydroxyquinoxalin-1(2H)-yl)-3-(pyrrolidin-1-yl)propan-1-one

Pyrrolidine (3.0 equiv) was added to a solution in which 3-bromo-1-(6-fluoro-4-phenyl-3,4-dihydroquinoxalin-1(2H)-yl)propan-1-one (1.0 equiv) obtained in Preparation Example b-5 was dissolved in THF (0.2 M) and then stirred at 90 °C for 3 hours. The resulting solution was cooled to room temperature, water was added to terminate the reaction, and then extraction was performed with ethyl acetate. An organic layer was washed with brine, dried with MgSO₄, filtered, and distilled under reduced pressure. The obtained residue was purified by silica column chromatography using a methanol/dichloromethane mixture, thereby synthesizing 1-(4-(4-fluorophenyl)-3,4-dihydroquinoxaline-1(2H)-yl)-3-(pyrrolidin-1-yl)propan-1-one.
White solid (yield 44%); ¹H NMR (400 MHz, CDCl₃) δ 7.23-7.18 (m, 2H), 7.15-7.05 (m, 3H), 6.97-6.89 (m, 1H), 6.73 (dd, 1H, *J* = 7.8, 1.2 Hz), 6.64 (d, 1H, *J* = 8.2 Hz), 4.04 (t, 2H, *J* = 5.1 Hz), 3.68 (t, 2H, *J* = 5.5 Hz), 2.88-2.80 (m, 4H), 2.52-2.43 (m, 4H), 1.78-1.70 (m, 4H); LC/MS ESI (+): 354.0 (M+1)

### <Example 1-15> Synthesis of 1-(4-(4-fluorophenyl)-3,4-dihydroquinoxalin-1(2H)-yl)-3-morpholinopropan-1-one

1-(4-(4-Fluorophenyl)-3,4-dihydroquinoxaline-1(2H)-yl)-3-morpholinopropan-1-one was synthesized in the same manner as in Example 1-14, except that morpholine was used instead of pyrrolidine.
White solid (yield 44%); ¹H NMR (400 MHz, CDCl₃) δ 7.23-7.18 (m, 2H), 7.15-7.06 (m, 3H), 6.97-6.89 (m, 1H), 6.73 (m, 1H), 6.65 (d, 1H, *J* = 8.2 Hz), 4.04 (t, 2H, *J* = 5.3 Hz), 3.70 (t, 2H, *J* = 5.5 Hz), 3.67-3.62 (m, 4H), 2.84-2.72 (m, 4H), 2.44-2.35 (m, 4H); LC/MS ESI (+): 370.0 (M+1)

### <Example 1-16> Synthesis of 1-(4-(4-fluorophenyl)-3,4-dihydroxyquinoxalin-1(2H)-yl)-3-(piperidin-1-yl)propan-1-one

1-(4-(4-Fluorophenyl)-3,4-dihydroquinoxaline-1(2H)-yl)-3-(piperidin-1-yl)propan-1-one was synthesized in the same manner as in Example 1-14, except that piperidine was used instead of pyrrolidine.
White solid (yield 46%); ¹H NMR (400 MHz, CDCl₃) δ 7.23-7.18 (m, 2H), 7.15-7.05 (m, 3H), 6.96-6.89 (m, 1H), 6.76-6.69 (m, 1H), 6.64 (d, 1H, *J=* 8.2 Hz), 4.03 (t, 2H, *J* = 5.5 Hz), 3.69 (t, 2H, *J* = 5.5 Hz), 2.85-2.69 (m, 4H), 2.43-2.28 (m, 4H), 1.53-1.48 (m, 4H), 1.45-1.35 (m, 2H); LC/MS ESI (+): 368.1 (M+1)

### <Example 1-17> Synthesis of 1-(4-(4-fluorophenyl)-3,4-dihydroquinoxalin-1(2H)-yl)-3-(4-methylpiperazin-1-yl)propan-1-one di-p-toluene sulfonate

1-(4-(4-Fluorophenyl)-3,4-dihydroquinoxaline-1(2H)-yl)-3-(4-methylpiperazin-1-yl)propan-1-one was synthesized in the same manner as in Example 1-14, except that 1-methylpiperazine was used instead of pyrrolidine. The obtained 1-(4-(4-fluorophenyl)-3,4-dihydroquinoxaline-1(2H)-yl)-3-(4-methylpiperazin-1-yl)propan-1-one (3.3 g, 8.57 mmol) was dissolved in ethyl acetate (30 mL) and stirred at room temperature for 10 minutes. *p*-toluene sulfonic acid (3.34 g, 17.58 mmol) was dissolved in ethyl acetate (25 mL) and slowly added to the prepared reaction solution, followed by stirring at room temperature for 1 hour. 1-(4-(4-fluorophenyl)-3,4-dihydroquinoxaline-1(2H)-yl)-3-(4-methylpiperazin-1-yl)propan-1-one di-p-toluene sulfonate was synthesized by filtering, washing, and drying the resulting solid.
White solid (yield 54.3%); ¹H NMR (400 MHz, CD₃OD) δ 7.69 (d, 4H, *J* = 8.2 Hz), 7.30-7.24 (m, 2H), 7.23 (d, 4H, *J* = 7.8 Hz), 7.05-6.84 (m, 2H), 6.67-6.66 (m, 1H), 6.64-6.56 (m, 1H), 4.11-3.81 (m, 4H), 3.81-3.43 (m, 10H), 3.26-3.15 (m, 2H), 3.02 (s, 3H), 2.36 (s, 6H); LC/MS ESI (+): 383.0 (M+1)

### <Example 1-18> Synthesis of (R)-1-(4-(4-fluorophenyl)-3,4-dihydroqunoxalin-1(2H)-yl)-3-(2-methylpyrrolidin-1-yl)propan-1-one

(R)-1-(4-(4-fluorophenyl)-3,4-dihydroquinoxaline-1(2H)-yl)-3-(2-methylpyrrolidin-1-yl)propan-1-one was synthesized in the same manner as in Example 1-14, except that (R)-2-methylpyrrolidine was used instead of pyrrolidine.
Pale yellow oil (yield 52%); ¹H NMR (400 MHz, CD₃OD) δ 7.31-7.26 (m, 2H), 7.25-7.18 (m, 1H), 7.17-7.11 (m, 2H), 7.01-6.91 (m, 1H), 6.79-6.71 (m, 1H), 6.63 (d, 1H, *J* = 7.8 Hz), 4.05-3.98 (m, 2H), 3.76-3.67 (m, 2H), 3.24-3.11 (m, 1H), 2.94-2.84 (m, 3H), 2.51-2.41 (m, 1H), 2.41-2.30 (m, 1H), 2.17-2.05 (m, 1H), 2.00-1.88 (m, 1H), 1.76-1.61 (m, 2H), 1.44-1.31 (m, 1H), 1.08 (d, 3H, *J* = 4.7 Hz); LC/MS ESI (+): 368.1 (M+1)

### <Example 1-19> Synthesis of (R)-1-(4-(4-fluorophenyl)-3,4-dihydroquinoxalin-1(2H)-yl)-3-(2-methylpiperadin-1-yl)propan-1-one

(R)-1-(4-(4-fluorophenyl)-3,4-dihydroquinoxaline-1(2H)-yl)-3-(2-methylpiperidin-1-yl)propan-1-one was synthesized in the same manner as in Example 1-14, except that (R)-2-methylpiperidine was used instead of pyrrolidine.
Brown oil (yield 50%); ¹H NMR (400 MHz, CDCl₃) δ 7.25-7.19 (m, 2H), 7.18-7.04 (m, 3H), 6.99-6.90 (m, 1H), 6.80-6.73 (m, 1H), 6.64 (d, 1H, *J* = 7.4 Hz), 3.97-3.08 (m, 2H), 3.75-3.65 (m, 2H), 3.41-3.27 (m, 1H), 3.14-2.94 (m, 4H), 2.87-2.73 (m, 1H), 2.62-2.46 (m, 1H), 2.03-1.99 (m, 2H), 1.79-1.66 (m, 3H), 1.45-1.33 (m, 1H), 1.31-1.18 (m, 3H); LC/MS ESI (+): 382.0 (M+1)

### <Example 1-20> Synthesis of 1-(4-(4-fluorophenyl)-3,4-dihydroquinoxalin-1(2H)-yl)-3-(hexahydropyrrolo[1,2-a]pyrazin-2(1H)-yl)propan-1-one

1-(4-(4-Fluorophenyl)-3,4-dihydroquinoxaline-1(2H)-yl)-3-(hexahydropyrrolo[1,2-a]pyrazin-2(1H)-yl)propan-1-one was synthesized in the same manner as in Example 1-14, except that octahydropyrrolo[1,2-a]pyrazine was used instead of pyrrolidine.
Pale yellow oil (yield 96%); ¹H NMR (400 MHz, CD₃OD) δ 7.33-7.27 (m, 2H), 7.26-7.19 (m, 1H), 7.18-7.11 (m, 2H), 7.03-6.93 (m, 1H), 6.80-6.72 (m, 1H), 6.73-6.63 (m, 1H), 4.02 (t, 2H, *J* = 5.5 Hz), 3.73 (t, 2H, *J* = 5.3 Hz), 3.27-3.16 (m, 2H), 3.03-2.68 (m, 9H), 2.55-2.41 (m, 1H), 2.33-2.18 (m, 1H), 2.02-1.89 (m, 3H), 1.68-1.54 (m, 1H); LC/MS ESI (+): 409.1 (M+1)

### <Example 1-21> Synthesis of 1-(4-(4-fluorophenyl)-3,4-dihydroquinoxalin-1(2H)-yl)-3-((pyridin-2-ylmethyl)amino)propan-1-one

1-(4-(4-Fluorophenyl)-3,4-dihydroquinoxaline-1(2H)-yl)-3-((pyridin-2-ylmethyl)amino)propan-1-one was synthesized in the same manner as in Example 1-14, except that 2-(aminomethyl)pyridine was used instead of pyrrolidine.
Pale yellow oil (yield 96%); ¹H NMR (400 MHz, CD₃OD) δ 8.47 (d, 1H, *J* = 4.7 Hz), 7.82-7.72 (m, 1H), 7.42 (d, 1H, *J* = 6.7 Hz), 7.29-7.09 (m, 7H), 6.98-6.89 (m, 1H), 6.73 (t, 1H, *J* = 7.4 Hz), 6.60 (d, 1H, *J* = 8.6 Hz), 4.04-3.96 (m, 2H), 3.90-3.80 (m, 2H), 3.74-3.66 (m, 2H), 2.97-2.83 (m, 4H); LC/MS ESI (+): 391.0 (M+1)

### <Example 1-22> Synthesis of 3-(4-cyclopropylpiperazin-1-yl)-1-(4-(4-fluorophenyl)-3,4-dihydroquinoxalin-1(2H)-yl)propan-1-one

3-(4-Cyclopropylpiperazin-1-yl)-1-(4-(4-fluorophenyl)-3,4-dihydroquinoxalin-1(2H)-yl)propan-1-one was synthesized in the same manner as in Example 1-14, except that 1-cyclopropylpiperazine was used instead of pyrrolidine.
White solid (yield 66.7%); ¹H NMR (400 MHz, CD₃OD) δ 7.32-7.25 (m, 2H), 7.25-7.11 (m, 3H), 7.01-6.91 (m, 1H), 6.78-6.72 (m, 1H), 6.65 (d, 1H, *J* = 7.8 Hz), 4.01 (t, 2H, *J* = 5.5 Hz), 3.73 (t, 2H, *J* = 5.3 Hz), 2.93-2.85 (m, 2H), 2.85-2.77 (m, 2H), 2.75-2.43 (m, 8H), 1.69-1.60 (m, 1H), 0.51-0.44 (m, 2H), 0.42-0.35 (m, 2H); LC/MS ESI (+): 383.1 (M+1)

### <Example 1-23> Synthesis of (S)-1-(4-(4-fluorophenyl)-3,4-dihydroquinoxalin-1(2H)-yl)-3-(3-hydroxypyrrolidin-1-yl)propan-1-one

(S)-1-(4-(4-fluorophenyl)-3,4-dihydroquinoxaline-1(2H)-yl)-3-(3-hydroxypyrrolidin-1-yl)propan-1-one was synthesized in the same manner as in Example 1-14, except that (S)-3-hydroxypyrrolidine was used instead of pyrrolidine.
Yellow oil (yield 76%); ¹H NMR (400 MHz, CD₃OD) δ 7.31- 7.28 (m, 2H), 7.22-7.18 (m, 1H), 7.17-7.11 (m, 2H), 7.02-6.92 (m, 1H), 6.78-6.72 (m, 1H), 6.62 (d, 1H, *J* = 8.2 Hz), 4.37-4.31 (m, 2H), 4.08-3.96 (m, 2H), 3.79-3.66 (m, 2H), 3.07 (m, 7H), 2.75-2.63 (m, 2H), 2.14-2.06 (m, 1H), 1.80-1.70 (m, 1H); LC/MS ESI(+): 370.3 (M+1)

### <Example 1-24> Synthesis of 3-(1,1-dioxydothiomorpholino)-1-(4-(4-fluorophenyl)-3,4-dihydroquinoxalin-1(2H)-yl)propan-1-one

3-(1,1-Dioxydothiomorpholino)-1-(4-(4-fluorophenyl)-3,4-dihydroquinoxalin-1(2H)-yl)propan-1-one was synthesized in the same manner as in Example 1-14, except that thiomorpholine 1,1-dioxide was used instead of pyrrolidine.
White solid (yield 73%); ¹H NMR (400 MHz, CD₃OD) δ 7.35-7.27 (m, 2H), 7.24-7.19 (m, 1H), 7.19-7.10 (m, 2H), 7.01-6.93 (m, 1H), 6.77-6.73 (m, 1H), 6.63 (d, 1H, *J* = 8.2 Hz), 4.01 (t, 2H, *J* = 5.5 Hz), 3.73 (t, 2H, *J* = 5.5 Hz), 3.02-2.96 (m, 4H), 2.94-2.77 (m, 8H); LC/MS ESI(+): 418.3 (M+1)

### <Example 1-25> Synthesis of 1-(4-(4-fluorophenyl)-3,4-dihydroquinoxaline-1(2H)-yl)-3-(1H-imidazol-1-yl)propan-1-one

1-(4-(4-Fluorophenyl)-3,4-dihydroquinoxaline-1(2H)-yl)-3-(1H-imidazol-1-yl)propan-1-one was synthesized in the same manner as in Example 1-14, except that imidazole was used instead of pyrrolidine.
Red oil (yield 52%); ¹H NMR (400 MHz, CD₃OD) δ 7.61-7.54 (m, 1H), 7.27-7.19 (m, 2H), 7.18-7.10 (m, 2H), 7.03-6.98 (m, 2H), 6.95-6.91 (m, 1H), 6.99-6.85 (m, 1H), 6.74-6.66 (m, 1H), 6.54 (d, 1H, *J* = 8.2 Hz), 4.38 (t, 2H, *J* = 6.3 Hz), 3.96 (t, 2H, *J* = 5.3 Hz), 3.56-3.48 (m, 2H), 3.12 (t, 2H, *J* = 6.3 Hz); LC/MS ESI(+): 351.2 (M+1)

### <Example 1-26> Synthesis of (R)-1-(4-(4-fluorophenyl)-3,4-dihydroquinoxaline-1(2H)-yl)-3-((1-methylpyrrolidin-3-yl)amino)propan-1-one

(R)-1-(4-(4-fluorophenyl)-3,4-dihydroquinoxaline-1(2H)-yl)-3-((1-methylpyrrolidin-3-yl)amino)propan-1-one was synthesized in the same manner as in Example 1-14, except that (R)-1-methylpyrrolidin-3-amine was used instead of pyrrolidine.
Brown oil (yield 58%); ¹H NMR (400 MHz, CD₃OD) δ 7.32-7.26 (m, 2H), 7.26-7.08 (m, 3H), 7.01-6.89 (m, 1H), 6.79-6.69 (m, 1H), 6.62 (d, 1H, *J* = 8.1 Hz), 4.01 (t, 2H, *J* = 5.4 Hz), 3.72 (t, 2H, *J* = 5.3 Hz), 2.95-2.76 (m, 5H), 2.69-2.52 (m, 2H), 2.41-2.25 (m, 4H), 2.20-2.07 (m, 1H), 1.61 (d, 1H, *J* = 5.1 Hz), 1.29 (s, 1H); LC/MS ESI (+): 383.4 (M+1)

### <Example 1-27> Synthesis of (S)-1-(4-(4-fluorophenyl)-3,4-dihydroquinoxaline-1(2H)-yl)-3-((1-methylpyrrolidin-3-yl)amino)propan-1-one

(S)-1-(4-(4-fluorophenyl)-3,4-dihydroquinoxaline-1(2H)-yl)-3-((1-methylpyrrolidin-3-yl)amino)propan-1-one was synthesized in the same manner as in Example 1-14, except that (S)-1-methylpyrrolidin-3-amine was used instead of pyrrolidine.
Brown oil (yield 60%); ¹H NMR (400 MHz, CD₃OD) δ 7.33-7.25 (m, 2H), 7.25-7.09 (m, 3H), 7.01-6.89 (m, 1H), 6.79-6.69 (m, 1H), 6.62 (d, 1H, *J* = 7.9 Hz), 4.05-3.97 (t, 2H, *J* = 5.4 Hz), 3.72 (t, 2H, *J* = 5.2 Hz), 2.93-2.78 (m, 5H), 2.68-2.52 (m, 2H), 2.41-2.24 (m, 4H), 2.19-2.05 (m, 1H), 1.61 (d, 1H, *J* = 5.4 Hz), 1.29 (s, 1H); LC/MS ESI (+): 383.4 (M+1)

### <Example 1-28> Synthesis of 1-(4-(4-fluorophenyl)-3,4-dihydroquinoxalin-1 (2H)-yl)-3-(piperazin-1 -yl)propan-1 -one

TEA (92 µL, 0.662 mmol) and 1-boc-piperazine (187 mg, 0.994 mmol) were added to a solution in which 3-bromo-1-(6-fluoro-4-phenyl-3,4-dihydroquinoxalin-1(2H)-yl)propan-1-one (120 mg, 0.331 mmol) obtained in Preparation Example b-5 was dissolved in THF (1.5 mL) and stirred at 90 °C for 3 hours. The resulting solution was cooled to room temperature, concentrated under reduced pressure to obtain a concentrate. The concentrate was dissolved in dichloromethane (1 mL), TFA (265 µL, 3.313 mmol) was added thereto, and the resulting mixture was stirred at room temperature for 3 hours. The reaction was terminated by adding water, and extraction was performed with dichloromethane. An organic layer was washed with brine, dried with MgSO₄, filtered, and distilled under reduced pressure. The obtained residue was purified by silica column chromatography using a dichloromethane/methanol mixture, thereby synthesizing 1-(4-(4-fluorophenyl)-3,4-dihydroquinoxaline-1(2H)-yl)-3-(piperazin-1-yl)propan-1-one.
Yellow oil (yield 58%); ¹H NMR (400 MHz, CD₃OD) δ 7.34-7.26 (m, 2H), 7.25-7.19 (m, 1H), 7.19-7.11 (m, 2H), 7.01-6.93 (m, 1H), 6.79-6.72 (m, 1H), 6.65 (d, 1H, *J* = 8.2 Hz), 4.01 (t, 2H, *J* = 5.7 Hz), 3.78-3.70 (m, 2H), 2.85 (t, 2H, *J* = 6.9 Hz), 2.81-2.73 (m, 4H), 2.70 (d, 2H, *J* = 6.7 Hz), 2.45-2.31 (m, 4H); LC/MS ESI(+): 369.3 (M+1)

### <Example 1-29> Synthesis of 1-(4-(4-fluorophenyl)-3,4-dihydroquinoxaline-1(2H)-yl)-3-(1H-imidazol-5-yl)propan-1-one

HBTU (3.0 equiv), TEA (2.2 equiv), and 3-(imidazole-5-yl)propanic acid (1.2 equiv) were added to a solution in which 1-(4-fluorophenyl)-1,2,3,4-tetrahydroquinoxaline (1.0 equiv) obtained in Preparation Example b-4 was dissolved in DMF (0.2 M) and stirred at 100 °C for 4 hours. The resulting solution was cooled to room temperature, water was added to terminate the reaction, and extraction was performed with dichloromethane. An organic layer was washed with brine, dried with MgSO₄, filtered, and distilled under reduced pressure. The obtained residue was purified by silica column chromatography using a dichloromethane/methanol mixture, thereby synthesizing 1-(4-(4-fluorophenyl)-3,4-dihydroquinoxaline-1(2H)-yl)-3-(1H-imidazol-5-yl)propan-1-one.
Brown solid (yield 9%); ¹H NMR (400 MHz, CD₃OD) δ 7.55-7.49 (m, 1H), 7.30-7.22 (m, 2H), 7.19-7.02 (m, 3H), 6.96-6.90 (m, 1H), 6.80-6.66 (m, 2H), 6.59 (d, 1H, *J* = 8.3 Hz), 3.99 (t, 2H, *J* = 5.5 Hz), 3.66-3.53 (m, 2H), 3.04-2.85 (m, 4H); LC/MS ESI (+): 351.3 (M+1)

### <Example 1-30> Synthesis of 1-(4-fluorophenyl)-4-(3-(4-methylpiperazin-1-yl)propyl)-1,2,3,4-tetrahydroquinoxaline

1-Bromo-3-chloropropane (130 µL, 1.315 mmol) and Cs₂CO₃ (214 mg, 0.657 mmol) were added to a solution in which 1-(4-fluorophenyl)-1,2,3,4-tetrahydroquinoxaline (100 mg, 0.438 mmol) obtained in Preparation Example b-4 was dissolved in DMF (2 mL) and then stirred at 80 °C for 6 hours. The resulting solution was cooled to room temperature, water was added to terminate the reaction, and extraction was performed with dichloromethane. An organic layer was washed with brine, dried with MgSO₄, filtered, and concentrated under reduced pressure. The concentrate obtained thereby was dissolved in THF (1.5 mL), and 1-methlypiperazine (82 µL, 1.315 mmol) and TEA (122 µL, 0.876 mmol) were added, followed by stirring at 80 °C for 3 hours. The resulting solution was cooled to room temperature, water was added to terminate the reaction, and extraction was performed with dichloromethane. An organic layer was washed with brine, dried with MgSO₄, filtered, and distilled under reduced pressure. The obtained residue was purified by silica column chromatography using a dichloromethane/methanol mixture, thereby obtaining 1-(4-fluorophenyl)-4-(3-(4-methylpiperazin-1-yl)propyl)-1,2,3,4-tetrahydroquinoxaline.
Yellow oil (yield 14%); ¹H-NMR (400 MHz, CDCl₃) δ 7.11 (dd, 1H, *J* = 8.6, 4.7 Hz), 7.03-6.99 (m, 2H), 6.78-6.69 (m, 2H), 6.63 (d, 1H, *J* = 7.8 Hz), 6.51-6.47 (m, 1H), 3.66-3.59 (m, 2H), 3.41-3.33 (m, 4H), 2.57-2.37 (m, 10H), 2.30 (s, 3H), 1.86-1.78 (m, 2H); LC/MS ESI (+): 369.4 (M+1)

### <Example 1-31> Synthesis of 1-(4-(4-fluorophenyl)-3,4-dihydroquinoxaline-1 (2H)-yl)-2-((R)-2-methylpi peridin-1-yl)propan-1-one

(R)-2-Methylpiperidine (3.0 equiv) was added to a solution in which 2-bromo-1-(4-(4-fluorophenyl)-3,4-dihydroquinoxalin-1(2H)-yl)propan-1-one (1.0 equiv) obtained in Preparation Example b-6 was dissolved in THF (0.2 M) and stirred at 90 °C for 3 hours. The resulting solution was cooled to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using an ethyl acetate/hexane mixture, thereby obtaining 1-(4-(4-fluorophenyl)-3,4-dihydroquinoxaline-1(2H)-yl)-2-((R)-2-methylpiperidin-1-yl)propan-1-one.
Yellow solid (yield 32%); ¹H-NMR (400 MHz, CDCl₃) δ 7.23-7.15 (m, 2H), 7.11-6.91 (m, 1H), 6.79-6.72 (m, 1H), 6.71-6.65 (m, 1H), 4.87-4.66 (m, 1H), 4.57-4.40 (m, 1H), 3.88-3.80 (m, 1H), 3.70-3.57 (m, 1H), 3.46-3.29 (m, 1H), 3.15-3.05 (m, 1H), 2.83-2.72 (m, 1H), 2.58-2.44 (m, 1H), 1.66-1.52 (m, 3H), 1.50-1.37 (m, 4H), 1.34-1.21 (m, 2H), 0.95-0.72 (m, 3H); LC/MS ESI (+): 381.7 (M+1)

### <Example 1-32> Synthesis of 1-(4-(4-fluorophenyl)-3,4-dihydroquinoxalin-1(2H)-yl)-2-morpholinopropan-1-one

1-(4-(4-Fluorophenyl)-3,4-dihydroquinoxaline-1(2H)-yl)-2-morpholinopropan-1-one was synthesized in the same manner as in Example 1-31, except that morpholine was used instead of (R)-2-methylpiperidine.
White solid (yield 11%); ¹H NMR (400 MHz, CDCl₃) δ 7.23-7.17 (m, 3H), 7.12-7.04 (m, 2H), 7.01-6.92 (m, 1H), 6.78-6.71 (m, 1H), 6.68 (d, 1H, *J* = 7.4 Hz), 4.31-4.20 (m, 1H), 4.10-3.99 (m, 1H), 3.93-3.81 (m, 1H), 3.80-3.59 (m, 6H), 2.57-2.49 (m ,4H), 1.27 (d, 3H, *J* = 7.0 Hz); LC/MS ESI (+): 369.7 (M+1)

### <Example 1-33> Synthesis of 1-(4-(4-fluorophenyl)-3,4-dihydroquinoxaline-1(2H)-yl)-2-(pyrrolidin-1-yl)propan-1-one

1-(4-(4-Fluorophenyl)-3,4-dihydroquinoxaline-1(2H)-yl)-2-(pyrrolidin-1-yl)propan-1-one was synthesized in the same manner as in Example 1-31, except that pyrrolidine was used instead of (R)-2-methylpiperidine.
Colorless oil (yield 61%); ¹H NMR (400 MHz, CDCl₃) δ 7.20-7.17 (m, 2H), 7.11-7.06 (m, 3H), 7.03-6.94 (m, 1H), 6.79-6.73 (m, 1H), 6.70 (d, 1H, *J* = 7.8 Hz), 4.35-4.23 (m, 1H), 4.15-4.00 (m, 1H), 3.91-3.79 (m, 1H), 3.78-3.68 (m, 2H), 2.79-2.56 (m, 4H), 1.82-1.72 (m, 4H), 1.38 (d, 3H, *J* = 6.7 Hz); LC/MS ESI (+): 354.1 (M+1)

### <Example 1-34> Synthesis of 1-(4-(4-fluorophenyl)-3,4-dihydroquinoxaline-1(2H)-yl)-2-(4-methylpiperazin-1-yl)propan-1-one

1-(4-(4-Fluorophenyl)-3,4-dihydroquinoxaline-1(2H)-yl)-2-(4-methylpiperazin-1-yl)propan-1-one was synthesized in the same manner as in Example 1-31, except that 1-methylpiperazine was used instead of (R)-2-methylpiperidine.
White solid (yield 28%); ¹H NMR (400 MHz, CDCl₃) δ 7.24-7.18 (m, 3H), 7.12-7.03 (m, 2H), 7.00-6.91 (m, 1H), 6.77-6.64 (m, 2H), 4.29-4.16 (m, 1H), 4.11-4.00 (m, 1H), 3.95-3.82 (m, 1H), 3.78-3.66 (m, 2H), 2.71-2.32 (m, 8H), 2.27 (s, 3H), 1.27 (d, 3H, *J* = 6.7 Hz); LC/MS ESI (+): 383.1 (M+1)

### <Example 1-35> Synthesis of 1-(4-(4-fluorophenyl)-3,4-dihydroquinoxaline-1(2H)-yl)-2-(piperidin-1-yl)propan-1-one

1-(4-(4-Fluorophenyl)-3,4-dihydroquinoxaline-1(2H)-yl)-2-(piperidin-1-yl)propan-1-one was synthesized in the same manner as in Example 1-31, except that piperidine was used instead of (R)-2-methylpiperidine.
White solid (yield 5%); ¹H NMR (400 MHz, CDCl₃) δ 7.24-7.14 (m, 3H), 7.11-7.04 (m, 2H), 6.99-6.89 (m, 1H), 6.76-6.70 (m, 1H), 6.70-6.62 (m, 1H), 4.37-4.23 (m, 1H), 4.11-3.96 (m, 1H), 3.89-3.58 (m, 4H), 2.53-2.42 (m, 3H), 1.55-1.43 (m, 4H), 1.42-1.34 (m, 2H), 1.26 (d, 3H, *J* = 6.7 Hz); LC/MS ESI (+): 367.7 (M+1)

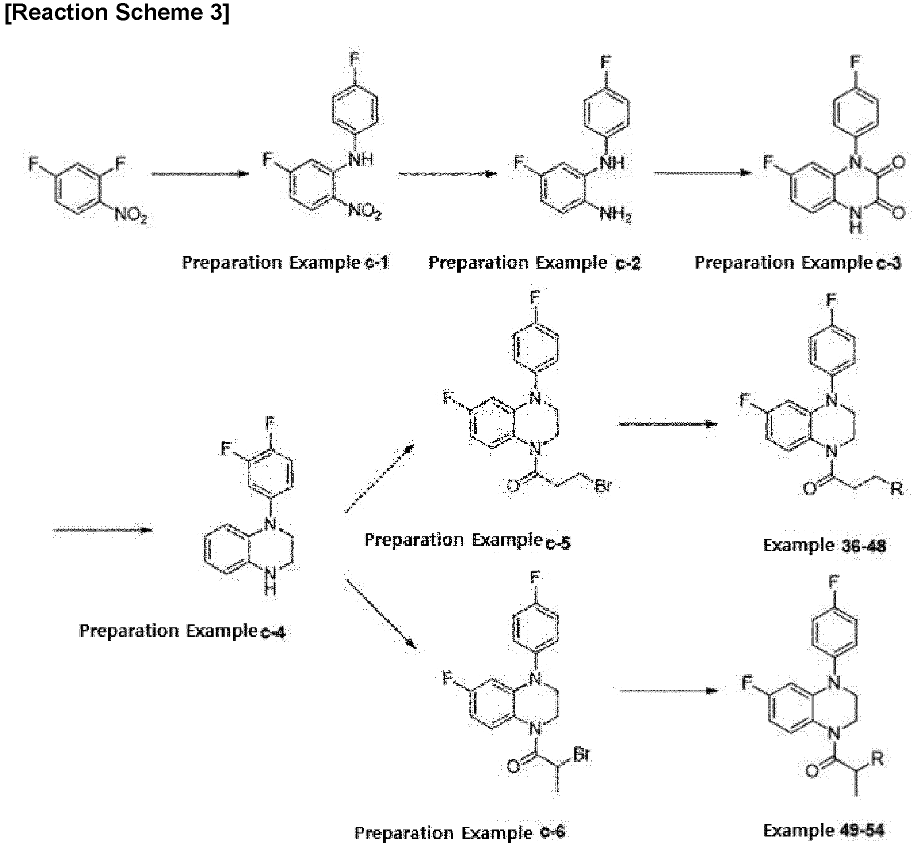

### <Preparation Method c> Preparation of 7-fluoro-1-(4-fluorophenyl)-1,2,3,4-tetrahydroquinoxaline

7-Fluoro-1-(4-fluorophenyl)-1,2,3,4-tetrahydroquinoxaline according to the present invention was prepared through the processes of Preparation Examples c-1 to c-6 below.

### <Preparation Example c-1> Preparation of 5-fluoro-N-(4-fluorophenyl)-2-nitroaniline

5-Fluoro-N-(4-fluorophenyl)-2-nitroaniline was prepared in the same manner as in Preparation Example a-1, except that 2,4-difluoro-1-nitrobenzene and 4-fluoroaniline were used instead of 2,4-difluoro-1-nitrobenzene and aniline.

Yellow solid (yield 63%); ¹H NMR (400 MHz, CDCl₃) δ 9.54 (s, 1H), 8.26 (dd, 1H, *J* = 9.4, 6.3 Hz), 7.27-7.23 (m, 2H), 7.18-7.13 (m, 2H), 6.63 (dd, 1H, *J* = 11.3, 2.7 Hz), 6.48 (ddd, 1H, *J* = 9.5, 6.9, 2.7 Hz).

### <Preparation Example c-2> Preparation of 5-fluoro-N¹-(4-fluorophenyl)benzene-1,2-diamine

5-Fluoro-*N*¹-(4-fluorophenyl)benzene-1,2-diamine was prepared in the same manner as in Preparation Example a-2, except that 5-fluoro-*N*-(4-fluorophenyl)-2-nitroaniline obtained in Preparation Example c-1 was used instead of 5-fluoro-2-nitro-*N-*phenylaniline.
Orange solid (yield 56%); ¹H NMR (400 MHz, CDCl₃) δ 7.00-6.94 (m, 2H), 6.86-6.82 (m, 2H), 6.79 (dd, 1H, *J* = 10.0, 2.9 Hz), 6.70 (dd, 1H, *J* = 8.6, 5.9 Hz), 6.62 (td, 1H, *J* = 8.3, 2.9 Hz), 5.27 (s, 1H), 3.49 (s, 2H); LC/MS ESI (+): 220.6 (M+1)

### <Preparation Example c-3> Preparation of 7-fluoro-1-(4-fluorophenyl)quinoxalin-2,3(1H,4H)-dione

7-Fluoro-1-(4-fluorophenyl)quinoxalin-2,3(1H,4H)-dione was prepared in the same manner as in Preparation Example a-3, except that 5-fluoro-*N*¹-(4-fluorophenyl)benzene-1,2-diamine obtained in Preparation Example c-2 was used instead of 5-fluoro-*N*¹-phenylbenzene-1,2-diamine.
Ivory solid (yield 68%); ¹H NMR (400 MHz, DMSO-*d*₆) δ 12.13 (s, 1H), 7.47 (d, 4H, *J* = 6.7 Hz), 7.23 (dd, 1H, *J* = 8.8, 5.3 Hz), 7.03 (td, 1H, *J* = 8.6, 2.7 Hz), 6.08 (dd, 1H, *J* = 10.4, 2.5 Hz); LC/MS ESI (+): 274.9 (M+1)

### <Preparation Example c-4> Preparation of 7-fluoro-1-(4-fluorophenyl)-1,2,3,4-tetrahydroquinoxaline

7-Fluoro-1-(4-fluorophenyl)-1,2,3,4-tetrahydroquinoxaline was prepared in the same manner as in Preparation Example a-4, except that 7-fluoro-1-(4-fluorophenyl)quinoxalin-2,3(1H,4H)-dione obtained in Preparation Example c-3 was used instead of 7-fluoro-1-phenylquinoxaline-2,3(1H,4H)-dione.
Light purple solid (yield 72%); ¹H NMR (400 MHz, CDCl₃) δ 7.21-7.16 (m, 2H), 7.09-7.03 (m, 2H), 6.49 (dd, 1H, *J* = 8.6, 5.5 Hz), 6.36-6.27 (m, 2H), 3.66-3.63 (m, 2H), 3.46-3.44 (m, 2H); LC/MS ESI (+): 246.8 (M+1)

### <Preparation Example c-5> Preparation of 3-bromo-1-(6-fluoro-4-(4-fluorophenyl)-3,4-dihydroquinoxalin-1(2H)-yl)propan-1-one

3-Bromo-1-(6-fluoro-4-(4-fluorophenyl)-3,4-dihydroquinoxalin-1(2H)-yl)propan-1-one was prepared in the same manner as in Preparation Example a-5, except that 7-fluoro-1-(4-fluorophenyl)-1,2,3,4-tetrahydroquinoxaline obtained in Preparation Example c-4 was used instead of 7-fluoro-1-phenyl-1,2,3,4-tetrahydroquinoxaline.

White solid (yield 96%); ¹H NMR (400 MHz, CDCl₃) δ 7.25-7.20 (m, 2H), 7.15-7.09 (m, 2H), 7.02-6.93 (m, 1H), 6.44-6.39 (m, 1H), 6.27-6.22 (m, 1H), 4.10-4.02 (m, 2H), 3.74-3.65 (m, 4H), 3.20-3.12 (m, 2H).

### <Preparation Example c-6> Preparation of 2-bromo-1-(6-fluoro-4-(4-fluorophenyl)-3,4-dihydroquinoxalin-1(2H)-yl)propan-1-one

2-Bromo-1-(6-fluoro-4-(4-fluorophenyl)-3,4-dihydroquinoxalin-1(2H)-yl)propan-1-one was synthesized in the same manner as in Preparation Example a-6, except that 7-fluoro-1-(4-fluorophenyl)-1,2,3,4-tetrahydroquinoxaline obtained in Preparation Example c-4 was used instead of 7-fluoro-1-phenyl-1,2,3,4-tetrahydroquinoxaline.

Purple solid (yield 63%); ¹H NMR (400 MHz, CDCl₃) δ 7.25-7.21 (m, 2H), 7.15-7.10 (m, 3H), 6.44 (td, 1H, *J* = 8.2, 2.7 Hz), 6.26 (d, 1H, *J* = 9.4 Hz), 5.03 (m, 1H), 4.22 (brs, 1H), 3.85 (brs, 1H), 3.74-3.62 (m, 2H), 1.87 (d, 3H, *J* = 6.7 Hz).

### <Example 1-36> Synthesis of 1-(6-fluoro-4-(4-fluorophenyl)-3,4-dihydroquinoxaline-1(2H)-yl)-3-(pyrrolidin-1-yl)propan-1-one

Pyrrolidine (3.0 equiv) was added to a solution in which 3-bromo-1-(6-fluoro-4-(4-fluorophenyl)-3,4-dihydroquinoxalin-1(2H)-yl)propan-1-one (1.0 equiv) obtained in Preparation Example c-5 was dissolved in THF (0.2 M) and stirred at 90 °C for 3 hours. The resulting solution was cooled to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using a methanol/dichloromethane mixture, thereby synthesizing 1-(6-fluoro-4-(4-fluorophenyl)-3,4-dihydroquinoxaline-1(2H)-yl)-3-(pyrrolidin-1-yl)propan-1-one.
White solid (yield 30%); ¹H NMR (400 MHz, CDCl₃) δ 7.24-7.19 (m, 2H), 7.16-7.08 (m, 2H), 7.08-6.97 (m, 1H), 6.44-6.36 (m, 1H), 6.29-6.21 (m, 1H), 4.06-3.97 (m, 2H), 3.66 (t, 2H, *J* = 5.1 Hz), 2.88-2.75 (m, 4H), 2.54-2.41 (m, 4H), 1.79-1.71 (m, 4H); LC/MS ESI (+): 372.1 (M+1)

### <Example 1-37> Synthesis of 1-(6-fluoro-4-(4-fluorophenyl)-3,4-dihydroquinoxaline-1(2H)-yl)-3-morpholinopropan-1-one

1-(6-Fluoro-4-(4-fluorophenyl)-3,4-dihydroquinoxaline-1(2H)-yl)-3-morpholinopropan-1-one was synthesized in the same manner as in Example 1-36, except that morpholine was used instead of pyrrolidone.
White solid (yield 61%); ¹H NMR (400 MHz, CDCl₃) δ 7.25-7.17 (m, 2H), 7.16-7.09 (m, 2H), 7.08-6.97 (m, 1H), 6.45-6.37 (m, 1H), 6.30-6.22 (m, 1H), 4.06-3.96 (m, 2H), 3.73-3.62 (m, 6H), 2.76 (s, 4H), 2.47-2.35 (m, 4H); LC/MS ESI (+): 388.1 (M+1)

### <Example 1-38> Synthesis of 1-(6-fluoro-4-(4-fluorophenyl)-3,4-dihydroquinoxaline-1(2H)-yl)-3-(piperidin-1-yl)propan-1-one

1-(6-Fluoro-4-(4-fluorophenyl)-3,4-dihydroquinoxaline-1(2H)-yl)-3-(piperidin-1-yl)propan-1-one was synthesized in the same manner as in Example 1-36, except that piperidine was used instead of pyrrolidine.
White solid (yield 54%); ¹H NMR (400 MHz, CDCl₃) δ 7.25-7.18 (m, 2H), 7.15-7.09 (m, 2H), 7.08-7.01 (m, 1H), 6.45-6.37 (m, 1H), 6.29-6.21 (m, 1H), 4.06-3.95 (m, 2H), 3.67 (t, 2H, *J* = 5.1 Hz), 2.81-2.68 (m, 4H), 2.45-2.28 (m, 4H), 1.62-1.49 (m, 4H), 1.47-1.36 (m, 2H); LC/MS ESI (+): 386.0 (M+1)

### <Example 1-39> Synthesis of 1-(6-fluoro-4-(4-fluorophenyl)-3,4-dihydroquinoxaline-1(2H)-yl)-3-(4-methylpiperazin-1-yl)propan-1-one

1-(6-Fluoro-4-(4-fluorophenyl)-3,4-dihydroquinoxaline-1 (2H)-yl)-3-(4-methylpiperazin-1-yl)propan-1-one was synthesized in the same manner as in Example 1-36, except that 1-methylpiperazine was used instead of pyrrolidine.
White solid (yield 67%); ¹H NMR (400 MHz, CDCl₃) δ 7.25-7.19 (m, 2H), 7.15-7.09 (m, 2H), 7.08-6.97 (m, 1H), 6.44-6.35 (m, 1H), 6.29-6.21 (m, 1H), 4.04-3.95 (m, 2H), 3.67 (t, 2H, *J* = 5.1 Hz), 2.76 (s, 4H), 2.62-2.29 (m, 8H), 2.27 (s, 3H); LC/MS ESI (+): 401.1 (M+1)

### <Example 1-40> Synthesis of (R)-1-(6-fluoro-4-(4-fluorophenyl)-3,4-dihydroquinoxaline-1(2H)-yl)-3-(2-methylpyrrolidin-1-yl)propan-1-one

(R)-1-(6-Fluoro-4-(4-fluorophenyl)-3,4-dihydroquinoxaline-1(2H)-yl)-3-(2-methylpyrrolidin-1-yl)propan-1-one was synthesized in the same manner as in Example 1-36, except that (R)-2-methylpyrrolidine was used instead of pyrrolidine.
Yellow solid (yield 66%); ¹H NMR (400 MHz, CD₃OD) δ 7.38-7.28 (m, 2H), 7.28-7.16 (m, 3H), 6.51-6.36 (m, 1H), 6.25-6.11 (m, 1H), 4.14-3.94 (m, 2H), 3.83-3.64 (m, 2H), 3.58-3.35 (m, 2H), 3.20-2.93 (m, 4H), 2.93-2.72 (m, 1H), 2.30-2.12 (m, 1H), 2.08-1.87 (m, 2H), 1.73-1.57 (m, 1H), 1.47-1.27 (m, 3H); LC/MS ESI (+): 386.1 (M+1)

### <Example 1-41> Synthesis of (R)-1-(6-fluoro-4-(4-fluorophenyl)-3,4-dihydroquinoxaline-1(2H)-yl)-3-(2-methylpiperidin-1-yl)propan-1-one

(R)-1-(6-Fluoro-4-(4-fluorophenyl)-3,4-dihydroquinoxaline-1(2H)-yl)-3-(2-methylpiperidin-1-yl)propan-1-one was synthesized in the same manner as in Example 1-36, except that (R)-2-methylpiperidine was used instead of pyrrolidine.
Brown oil (yield 76%); ¹H NMR (400 MHz, CDCl₃) δ 7.25-7.18 (m, 2H), 71.4-7.07 (m, 3H), 6.48-6.39 (m, 1H), 6.28-6.18 (m, 1H), 4.05-3.93 (m, 2H), 3.76-3.60 (m, 2H), 3.83-3.47 (m, 1H), 3.25-2.95 (m, 5H), 2.71-2.57 (m, 1H), 2.02-1.99 (m, 2H), 1.88-1.67 (m, 4H), 1.65-1.55 (m, 1H), 1.49-1.24 (m, 3H); LC/MS ESI (+): 400.0 (M+1)

### <Example 1-42> Synthesis of 1-(6-fluoro-4-(4-fluorophenyl)-3,4-dihydroquinoxaline-1(2H)-yl)-3-(hexahydropyrrolo[1,2-a]pyrazin-2(1H)-yl)propan-1-one

1-(6-Fluoro-4-(4-fluorophenyl)-3,4-dihydroquinoxaline-1(2H)-yl)-3-(hexahydropyrrolo[1,2-a]pyrazin-2(1H)-yl)propan-1-one was synthesized in the same manner as in Example 1-36, except that octahydropyrrolo[1,2-a]pyrazine was used instead of pyrrolidine.
Pale yellow solid (yield 55%); ¹H NMR (400 MHz, CDCl₃) δ 7.25-7.19 (m, 2H), 7.18-7.09 (m, 2H), 7.08-6.96 (m, 1H), 6.47-6.39 (m, 1H), 6.31-6.22 (m, 1H), 4.06-3.93 (m, 2H), 3.74-3.62 (m, 2H), 3.39-2.64 (m, 12H), 2.58-2.42 (m, 1H), 2.12-1.94 (m, 3H), 1.86-1.72 (m, 1H); LC/MS ESI (+): 427.0 (M+1)

### <Example 1-43> Synthesis of 3-(4-cyclopropylpiperazin-1-yl)-1-(6-fluoro-4-(4-fluorophenyl)-3,4-dihydroquinoxalin-1(2H)-yl)propan-1-one

3-(4-Cyclopropylpiperazin-1-yl)-1-(6-fluoro-4-(4-fluorophenyl)-3,4-dihydroquinoxalin-1(2H)-yl)propan-1-one was synthesized in the same manner as in Example 1-36, except that 1-cyclopropylpiperazine was used instead of pyrrolidine.
Pale yellow solid (yield 57%); ¹H NMR (400 MHz, CD₃OD) δ 7.37-7.29(m, 2H), 7.26-7.14 (m, 3H), 6.49-6.39 (m, 1H), 6.26-6.16 (m, 1H), 4.00 (t, 2H, *J* = 5.3 Hz), 3.75-3.67 (m, 2H), 2.84-2.78 (m, 2H), 2.73-2.58 (m, 6H), 2.53-2.33 (m, 4H), 1.68-1.59 (m, 1H), 0.51-0.44 (m, 2H), 0.41-0.35 (m, 2H); LC/MS ESI (+): 427.0 (M+1)

### <Example 1-44> Synthesis of (S)-1-(6-fluoro-4-(4-fluorophenyl)-3,4-dihydroquinoxaline-1(2H)-yl)-3-(3-hydroxypyrrolidin-1-yl)propan-1-one

(S) -1-(6-fluoro-4-(4-fluorophenyl)-3,4-dihydroquinoxaline-1(2H)-yl)-3-(3-hydroxypyrrolidin-1-yl)propan-1-one was synthesized in the same manner as in Example 1-36, except that (S)-3-hydroxypyrrolidine was used instead of pyrrolidine.
Yellow oil (yield 58%); ¹H NMR (400 MHz, CD₃OD) δ 7.33 (dd, 2H, *J* = 8.8, 4.9 Hz), 7.28-7.14 (m, 3H), 6.49-6.41 (m, 1H), 6.23-6.15 (m, 1H), 4.42-4.36 (m, 1H), 4.04-3.98 (m, 2H), 3.74-3.68 (m, 2H), 3.20-2.68 (m, 8H), 2.19-2.11 (m, 1H), 1.88-1.76 (m, 1H); LC/MS ESI (+): 388.3 (M+1)

### <Example 1-45> Synthesis of 1-(6-fluoro-4-(4-fluorophenyl)-3,4-dihydroquinoxaline-1(2H)-yl)-3-(1H-imidazol-1-yl)propan-1-one

1-(6-Fluoro-4-(4-fluorophenyl)-3,4-dihydroquinoxaline-1(2H)-yl)-3-(1H-imidazol-1-yl)propan-1-one was synthesized in the same manner as in Example 1-36, except that imidazole was used instead of pyrrolidine.
Colorless oil (yield 65%); ¹H NMR (400 MHz, CDCl₃) δ 7.49-7.41 (m, 1H), 7.22-7.06 (m, 4H), 7.03-6.97 (m, 1H), 6.89-6.81 (m, 1H), 6.79-6.62 (m, 1H), 6.38-6.34 (m, 1H), 6.20-6.16 (m, 1H), 4.36 (t, 2H, *J* = 6.1 Hz), 3.93-4.03 (m, 2H), 3.53-3.45 (m, 2H), 3.00 (t, 2H, *J* = 6.1 Hz); LC/MS ESI (+): 369.2 (M+1)

### <Example 1-46> Synthesis of 1-(6-fluoro-4-(4-fluorophenyl)-3,4-dihydroquinoxaline-1(2H)-yl)-3-(1H-imidazol-5-yl)propan-1-one

1-(6-Fluoro-4-(4-fluorophenyl)-3,4-dihydroquinoxaline-1(2H)-yl)-3-(1H-imidazol-5-yl)propan-1-one was synthesized in the same manner as in Example 1-29, except that 7-fluoro-1-(4-fluorophenyl)-1,2,3,4-tetrahydroquinoxaline obtained in Preparation Example c-4 was used instead of 1-(4-fluorophenyl)-1,2,3,4-tetrahydroquinoxaline.
Purple solid (yield 14%); ¹H NMR (400 MHz, CD₃OD) δ 7.56-7.50 (m, 1H), 7.35-7.25 (m, 2H), 7.24-7.13 (m, 2H), 7.10-7.04 (m, 1H), 6.75-6.67 (m, 1H), 6.42-6.38 (m, 1H), 6.23-6.04 (m, 1H), 3.97 (t, 2H, *J* = 5.3 Hz), 3.56 (m, 2H), 2.98-2.88 (m, 4H); LC/MS ESI (+): 369.3 (M+1)

### <Example 1-47> Synthesis of (S)-2-amino-1-(6-fluoro-4-(4-fluorophenyl)-3,4-dihydroquinoxaline-1(2H)-yl)-3-(1H-imidazol-4-yl)propan-1-one

T3P (50% in EA, 3.0 equiv) and DIPEA (3.0 equiv) were added to a solution in which *N*_{α}-(*tert*-butoxycarbonyl)-*L*-histidine (1.0 equiv) was dissolved in ethyl acetate (0.5 M) and stirred for 5 minutes, and then a solution in which 7-fluoro-1-(4-fluorophenyl)-1,2,3,4-tetrahydroquinoxaline (1.0 equiv) obtained in Preparation Example c-4 was dissolved in ethyl acetate (0.5 M) was added. The reaction mixture was stirred at 70 °C for 12 hours, water was added to terminate the reaction, followed by extraction with ethyl acetate. An organic layer was washed with brine, dried with MgSO₄, filtered, and concentrated under reduced pressure. TFA (10.0 equiv) was added to a solution in which the obtained concentrate was dissolved in dichloromethane (0.5 M) and stirred at room temperature for 24 hours. After terminating the reaction by adding water, extraction was performed with dichloromethane. An organic layer was washed with brine, dried with MgSO₄, filtered, and distilled under reduced pressure. The obtained residue was purified by MPLC reverse phase column chromatography, thereby synthesizing (S)-2-amino-1-(6-fluoro-4-(4-fluorophenyl)-3,4-dihydroquinoxaline-1(2H)-yl)-3-(1H-imidazol-4-yl)propan-1-one.
White solid (yield 21%); ¹H NMR (400 MHz, CD₃OD) δ 7.57-7.47 (m, 1H), 7.29-7.22 (m, 2H), 7.22-7.18 (m, 2H), 7.13-7.02 (m, 1H), 6.74-6.60 (m, 1H), 6.44-6.35 (m, 1H), 6.15-6.08 (m, 1H), 4.51-4.36 (m, 2H), 3.71-3.58 (m, 1H), 3.42-3.25 (m, 1H), 3.24-3.13 (m, 1H), 3.03-2.93 (m, 1H), 2.80-2.70 (m, 1H); LC/MS ESI (+): 384.1 (M+1)

### <Example 1-48> Synthesis of (R)-2-amino-1-(6-fluoro-4-(4-fluorophenyl)-3,4-dihydroquinoxaline-1(2H)-yl)-3-(1H-imidazol-4-yl)propan-1-one

(R)-2-amino-1-(6-fluoro-4-(4-fluorophenyl)-3,4-dihydroquinoxaline-1(2H)-yl)-3-(1H-imidazol-4-yl)propan-1-one was synthesized in the same manner as in Example 1-47, except that *N*_{α}-(*tert*-butoxycarbonyl)-*D*-histidine was used instead of *N*_{α}-(*tert-*butoxycarbonyl)-L-histidine.
White solid (yield 16%); ¹H NMR (400 MHz, CD₃OD) δ 7.57-7.46 (m, 1H), 7.31-7.21 (m, 2H), 7.24-7.16 (m, 2H), 7.15-7.05 (m, 1H), 6.75-6.65 (m, 1H), 6.47-6.36 (m, 1H), 6.18-6.08 (m, 1H), 4.53-4.48 (m, 2H), 3.73-3.60 (m, 1H), 3.43-3.32 (m, 1H), 3.25-3.14 (m, 1H), 3.05-2.95 (m, 1H), 2.84-2.72 (m, 1H); LC/MS ESI (+): 384.0 (M+1)

### <Example 1-49> Synthesis of 1-(6-fluoro-4-(4-fluorophenyl)-3,4-dihydroquinoxaline-1(2H)-yl)-2-((R)-2-methylpiperidin-1-yl)propan-1-one

(R)-2-Methylpiperidine (3.0 equiv) was added to a solution in which 2-bromo-1-(6-fluoro-4-(4-fluorophenyl)-3,4-dihydroquinoxalin-1(2H)-yl)propan-1-one (1.0 equiv) obtained in Preparation Example c-6 was dissolved in THF (0.2 M) and stirred at 90 °C for 3 hours. The resulting solution was cooled to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using a methanol/dichloromethane mixture, thereby synthesizing 1-(6-fluoro-4-(4-fluorophenyl)-3,4-dihydroquinoxaline-1(2H)-yl)-2-((R)-2-methylpiperidin-1-yl)propan-1-one.
Yellow solid (yield 32%); ¹H NMR (400 MHz, CDCl₃) δ 7.25-7.16 (m, 2H), 7.25-7.08 (m, 2H), 7.05-6.92 (m, 1H), 6.49-6.37 (m, 1H), 6.33-6.19 (m, 1H), 4.78-4.61 (m, 1H), 4.52-4.37 (m, 2H), 4.14-3.94 (m, 1H), 3.86-3.74 (m, 1H) 3.67-3.55 (m, 1H), 3.45-3.29 (m, 1H), 3.18-3.05 (m, 1H), 2.96-2.76 (m, 2H), 2.61-2.46 (m, 1H), 1.69-1.55 (m, 3H), 1.48-1.38 (m, 3H), 1.36-1.26 (m, 2H), 1.17-1.07 (m, 1H), 0.93-0.81 (m, 2H); LC/MS ESI (+): 400.1 (M+1)

### <Example 1-50> Synthesis of 1-(6-fluoro-4-(4-fluorophenyl)-3,4-dihydroquinoxaline-1(2H)-yl)-2-morpholinopropan-1-one

1-(6-Fluoro-4-(4-fluorophenyl)-3,4-dihydroquinoxaline-1(2H)-yl)-2-morpholinopropan-1-one was synthesized in the same manner as in Example 1-49, except that morpholine was used instead of (R)-2-methylpiperidine.
White solid (yield 43%); ¹H NMR (400 MHz, CDCl₃) δ 7.24-7.16 (m, 3H), 7.16-7.08 (m, 2H), 6.44-6.37 (m, 1H), 6.31-6.23 (m, 1H), 4.23-4.09 (m, 1H), 4.03-3.84 (m, 2H), 3.72-3.62 (m, 6H), 2.63-2.53 (m, 2H), 2.53-2.45 (m, 2H), 1.25 (d, 3H, *J* = 5.9 Hz); LC/MS ESI (+): 387.7 (M+1)

### <Example 1-51> Synthesis of 1-(6-fluoro-4-(4-fluorophenyl)-3,4-dihydroquinoxaline-1(2H)-yl)-2-(pyrrolidin-1-yl)propan-1-one

1-(6-Fluoro-4-(4-fluorophenyl)-3,4-dihydroquinoxaline-1(2H)-yl)-2-(pyrrolidin-1-yl)propan-1-one was synthesized in the same manner as in Example 1-49, except that pyrrolidine was used instead of (R)-2-methylpiperidine.
Ivory solid (yield 46%); ¹H NMR (400 MHz, CDCl₃) δ 7.22-7.19 (m, 2H), 7.17-7.10 (m, 2H), 7.07-6.99 (m, 1H), 6.49-6.41 (m, 1H), 6.34-6.26 (m, 1H), 4.44-4.26 (m, 1H), 4.14-3.93 (m, 2H), 3.79-3.66 (m, 2H), 3.26-3.06 (m, 2H), 3.04-2.83 (m, 2H), 2.02-1.87 (m, 4H), 1.52-1.42 (m, 3H); LC/MS ESI (+): 371.8 (M+1)

### <Example 1-52> Synthesis of 1-(6-fluoro-4-(4-fluorophenyl)-3,4-dihydroquinoxaline-1(2H)-yl)-2-(4-methylpiperazin-1-yl)propan-1-one

1-(6-Fluoro-4-(4-fluorophenyl)-3,4-dihydroquinoxaline-1(2H)-yl)-2-(4-methylpiperazin-1-yl)propan-1-one was synthesized in the same manner as in Example 1-49, except that 1-methylpiperazine was used instead of (R)-2-methylpiperidine.
White solid (yield 29%); ¹H NMR (400 MHz, CDCl₃) δ 7.25-7.16 (m, 3H), 7.15-7.07 (m, 2H), 6.44-6.36 (m, 1H), 6.31-6.20 (m, 1H), 4.27-4.06 (m, 1H), 4.04-3.84 (m, 2H), 3.76-3.61 (m, 2H), 2.82-2.32 (m, 8H), 2.27 (s, 3H), 1.29-1.21 (m, 3H); LC/MS ESI (+): 401.1 (M+1)

### <Example 1-53> Synthesis of 1-(6-fluoro-4-(4-fluorophenyl)-3,4-dihydroquinoxaline-1(2H)-yl)-2-(piperidin-1-yl)propan-1-one

1-(6-Fluoro-4-(4-fluorophenyl)-3,4-dihydroquinoxaline-1(2H)-yl)-2-(piperidin-1-yl)propan-1-one was synthesized in the same manner as in Example 1-49, except that piperidine was used instead of (R)-2-methylpiperidine.
White solid (yield 18%); ¹H NMR (400 MHz, CDCl₃) δ 7.28-7.26 (m, 1H), 7.24-7.17 (m, 2H), 7.16-7.08 (m, 2H), 6.44-6.37 (m, 1H), 6.30-6.21 (m, 1H), 4.29-4.13 (m, 1H), 3.99-3.81 (m, 2H), 3.78-3.61 (m, 2H), 2.55-2.42 (m, 4H), 1.55-1.44 (m, 4H), 1.44-1.36 (m, 2H), 1.23 (d, 3H, *J* = 6.7 Hz); LC/MS ESI (+): 386.0 (M+1)

### <Example 1-54> Synthesis of 1-(6-fluoro-4-(4-fluorophenyl)-3,4-dihydroquinoxaline-1(2H)-yl)-2-((R)-2-methylpyrrolidin-1-yl)propan-1-one

1-(6-Fluoro-4-(4-fluorophenyl)-3,4-dihydroquinoxaline-1(2H)-yl)-2-((R)-2-methylpyrrolidin-1-yl)propan-1-one was synthesized in the same manner as in Example 1-49, except that (R)-2-methylpyrrolidine was used instead of (R)-2-methylpiperidine.
Pale yellow oil (yield 17.9%); ¹H NMR (400 MHz, CDCl₃) δ 7.23-7.15 (m, 2H), 7.15-7.07 (m, 2H), 7.02-6.91 (m, 1H), 6.47-6.39 (m, 1H), 6.31-6.22 (m, 1H), 4.79-4.66 (m, 1H), 4.41-4.28 (m, 1H), 3.82-3.74 (m, 1H), 3.64-3.52 (m, 1H), 3.40-3.26 (m, 1H), 3.14-2.96 (m, 2H), 2.67-2.53 (m, 1H), 1.91-1.81 (m, 1H), 1.80-1.64 (m, 3H), 1.44 (d, 3H, *J* = 5.5 Hz), 1.36-1.29 (m, 1H), 0.81-0.72 (m, 2H); LC/MS ESI (+): 386.2 (M+1)

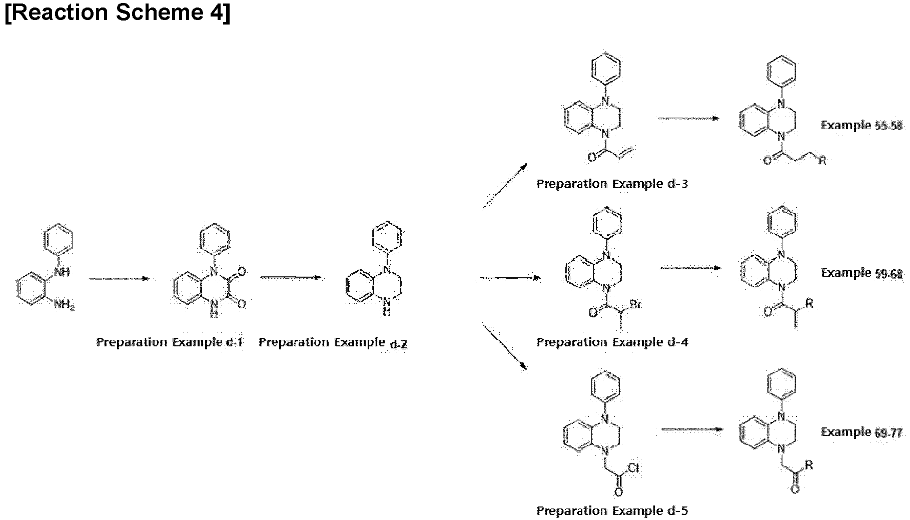

### <Preparation Method d> Preparation of 1-phenyl-1,2,3,4-tetrahydroquinoxaline

1-Phenyl-1,2,3,4-tetrahydroquinoxaline according to the present invention was synthesized through the processes of Preparation Examples d-1 to d-4 below.

### <Preparation Example d-1> Preparation of 1-phenyl-1,4-dihydroquinoxaline-2,3-dione

1-Phenyl-1,4-dihydroquinoxaline-2,3-dione was prepared in the same manner as in Preparation Example a-3, except that 2-aminodiphenylamine was used instead of 5-fluoro-*N*¹-phenylbenzene-1,2-diamine.
Ivory solid (yield 82%); ¹H NMR (400 MHz, DMSO-*d*₆) δ 12.14 (s, 1H), 7.65-7.62 (m, 2H), 7.56 (m, 1H), 7.41-7.39 (m, 2H), 7.24 (dd, 1H, *J* =7.8, 1.2 Hz), 7.14 (td, 1H, *J* =7.6, 1.2 Hz), 6.98 (m, 1H), 6.32 (dd, 1H, *J* =8.2, 0.8 Hz); LC/MS ESI (+): 239.0 (M+1)

### <Preparation Example d-2> Preparation of 1-phenyl-1,2,3,4-tetrahydroquinoxaline

1-Phenyl-1,2,3,4-tetrahydroquinoxaline was prepared in the same manner as in Preparation Example a-4, except that 1-phenyl-1,4-dihydroquinoxaline-2,3-dione obtained in Preparation Example d-1 was used instead of 7-fluoro-1-phenylquinoxaline-2,3(1H,4H)-dione.
Yellow solid (yield 89%); ¹H NMR (400 MHz, CDCl₃) δ 7.37-7.32 (m, 2H), 7.24-7.22 (m, 2H), 7.05 (m, 1H), 6.87 (dd, 1H, *J* =8.0, 1.4 Hz), 6.74 (m, 1H), 6.64-6.57 (m, 2H), 3.89 (brs, 1H), 3.73 (t, 2H, *J* =4.7 Hz), 3.47(t, 2H, *J* =4.7 Hz); LC/MS ESI (+): 210.9 (M+1)

### <Preparation Example d-3> Preparation of 1-(4-phenyl-3,4-dihydroquinoxalin-1(2H)-yl)prop-2-en-1-one

1-(4-Phenyl-3,4-dihydroquinoxalin-1(2H)-yl)prop-2-en-1-one was prepared in the same manner as in Preparation Example a-5, except that 1-phenyl-1,2,3,4-tetrahydroquinoxaline obtained in Preparation Example d-2 was used instead of 7-fluoro-1-phenyl-1,2,3,4-tetrahydroquinoxaline.

Pale yellow solid (yield 91%); ¹H NMR (400 MHz, CDCl₃) δ 7.42-7.36 (m, 2H), 7.29-7.24 (m, 2H), 7.22-7.14 (m, 1H), 7.03 (d, 1H, *J* = 7.4 Hz), 6.99-6.92 (m, 1H), 6.80-6.71 (m, 3H), 6.49 (dd, 1H, *J* = 16.8, 2.0 Hz), 5.74 (dd, 1H, *J* = 10.2, 2.0 Hz), 4.14 (t, 2H, *J* = 5.7 Hz), 3.78 (t, 2H, *J* = 5.5 Hz).

### <Preparation Example d-4> Preparation of 2-bromo-1-(4-phenyl-3,4-dihydroquinoxalin-1(2H)-yl)propan-1-one

2-Bromo-1-(4-phenyl-3,4-dihydroquinoxalin-1(2H)-yl)propan-1-one was prepared in the same manner as in Preparation Example a-6, except that 1-phenyl-1,2,3,4-tetrahydroquinoxaline obtained in Preparation Example d-2 was used instead of 7-fluoro-1-phenyl-1,2,3,4-tetrahydroquinoxaline.

Pale yellow solid (yield 91%); ¹H NMR (400 MHz, CDCl₃) δ 7.40-7.36 (m, 2H), 7.27-7.25 (m, 2H), 7.20-7.16 (m, 2H), 7.01 (m, 1H), 6.82-6.75 (m, 2H), 5.12 (d, 1H, *J* = 5.1 Hz), 4.17 (brs, 1H), 3.97 (brs, 1H), 3.81-3.76 (m, 2H), 1.87 (d, 3H, *J* = 6.3 Hz).

### <Preparation Example d-5> Preparation of 2-(4-phenyl-3,4-dihydroquinoxalin-1(2H)-yl)acetyl chloride

After dissolving 1-phenyl-1,2,3,4-tetrahydroquinoxaline (1.0 equiv) obtained in Preparation Example d-2 and triethylamine (3.0 equiv) in dichloromethane (0.5 M), 2-bromoacetyl chloride (1.5 equiv) was slowly added at -10 °C. The reaction solution was stirred at room temperature for 3 hours, and water was added to terminate the reaction, followed by extraction with dichloromethane. An organic layer was washed with brine, dried with MgSO₄, filtered, and distilled under reduced pressure. The obtained residue was purified by silica column chromatography using a hexane/ethyl acetate mixture, thereby synthesizing 2-(4-phenyl-3,4-dihydroquinoxalin-1(2H)-yl)acetyl chloride.

Ivory oil (yield 73%); ¹H NMR (400 MHz, CDCl₃) δ 7.42-7.37 (m, 2H), 7.25-7.24 (m, 2H), 7.22-7.11 (m, 2H), 7.10-6.97 (m, 1H), 6.79-6.75 (m, 2H), 4.35 (s, 2H), 4.08 (t, 2H, *J* = 5.3 Hz), 3.78 (t, 2H, *J* = 5.5 Hz).

### <Example 1-55> Synthesis of 3-morpholino-1-(4-phenyl-3,4-dihydroquinoxalin-1(2H)-yl)propan-1-one

Morpholine (3.0 equiv) and DIPEA (3.0 equiv) were mixed and stirred at room temperature for 5 minutes. A mixture in which 1-(4-phenyl-3,4-dihydroquinoxalin-1(2H)-yl)prop-2-en-1-one (1.0 equiv) obtained in Preparation Example d-3 was dissolved in DMF (0.1 M) was added to the reaction solution and stirred at 110 °C for 3 hours. After cooling to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using a hexane/ethyl acetate mixture, thereby synthesizing 3-morpholino-1-(4-phenyl-3,4-dihydroquinoxalin-1(2H)-yl)propan-1-one.
Yellow oil (yield 36%); ¹H NMR (400 MHz, CDCl₃) δ 7.41-7.20 (m, 5H), 7.12 (t, 1H, *J* = 7.4 Hz), 6.97-6.86 (m, 1H), 6.76-6.66 (m, 2H), 3.88 (t, 2H, *J* = 5.5 Hz), 3.66 (t, 2H, *J* = 5.3 Hz), 3.50-3.37 (m, 4H), 2.69 (t, 2H, *J* = 7.0 Hz), 2.53 (t, 2H, *J* = 6.7 Hz), 2.30-2.13 (m, 4H); LC/MS ESI (+): 352.1 (M+1)

### <Example 1-56> Synthesis of 3-(4-methylpiperazin-1-yl)-1-(4-phenyl-3,4-dihydroquinoxalin-1(2H)-yl)propan-1-one

3-(4-Methylpiperazin-1-yl)-1-(4-phenyl-3,4-dihydroquinoxalin-1(2H)-yl)propan-1-one was synthesized in the same manner as in Example 1-55, except that 1-methylpiperazine was used instead of morpholine.
Colorless oil (yield 39%); ¹H NMR (400 MHz, CD₃OD) δ 7.40-7.34 (m, 2H), 7.25-7.22 (m, 2H), 7.19-7.05 (m, 2H), 7.01-6.94 (m, 1H), 6.84 (d, 1H, *J* = 7.8 Hz), 6.80-6.72 (m, 1H), 4.04 (t, 2H, *J* = 5.3 Hz), 3.75 (t, 2H, *J* = 5.7 Hz), 2.84-2.75 (m, 4H), 2.63-2.39 (m, 12H), 2.33 (s, 3H); LC/MS ESI (+): 365.1 (M+1)

### <Example 1-57> Synthesis of (R)-3-(2-methylpyrrolidin-1-yl)-1-(4-phenyl-3,4-dihydroquinoxalin-1(2H)-yl)propan-1-one

(R)-3-(2-Methylpyrrolidin-1-yl)-1-(4-phenyl-3,4-dihydroquinoxalin-1(2H)-yl)propan-1-one was synthesized in the same manner as in Example 1-55, except that (R)-2-methylpyrrolidine was used instead of morpholine.
Yellow oil (yield 45%); ¹H NMR (400 MHz, CDCl₃) δ 7.41-7.33 (m, 2H), 7.25-7.22 (m, 2H), 7.17-7.10 (m, 2H), 6.99-6.91 (m, 1H), 6.85-6.75 (m, 2H), 4.09-4.01 (m, 2H), 3.79-3.70 (m, 2H), 3.42-3.27 (m, 2H), 3.10-2.92 (m, 3H), 2.89-2.65 (m, 2H), 2.37-2.36 (m, 1H), 2.04-1.96 (m, 1H), 1.94-1.83 (m, 1H), 1.83-1.70 (m, 1H), 1.65-1.52 (m, 1H), 1.25 (m, 4H); LC/MS ESI (+): 350.1 (M+1)

### <Example 1-58> Synthesis of 3-(3-hydroxypiperidin-1-yl)-1-(4-phenyl-3,4-dihydroquinoxalin-1(2H)-yl)propan-1-one

3-(3-Hydroxypiperidin-1-yl)-1-(4-phenyl-3,4-dihydroquinoxalin-1(2H)-yl)propan-1-one was synthesized in the same manner as in Example 1-55, except that 3-hydroxypiperidine was used instead of morpholine.
Light brown solid (yield 82%); ¹H NMR (400 MHz, CDCl₃) δ 7.45-6.70 (m, 9H), 4.20-3.90 (m, 2H), 3.75 (t, 3H, *J* = 5.6 Hz), 2.92-2.66 (m, 4H), 2.52-1.19 (m, 9H); LC/MS ESI (+): 366.0 (M+1)

### <Example 1-59> Synthesis of 2-((R)-2-methylpiperidin-1-yl)-1-(4-phenyl-3,4-dihydroquinoxalin-1(2H)-yl)propan-1-one phosphate

(R)-2-Methylpiperidine (3.0 equiv) was added to a solution in which 2-bromo-1-(4-phenyl-3,4-dihydroquinoxalin-1(2H)-yl)propan-1-one (1.0 equiv) obtained in Preparation Example d-4 was dissolved in THF (0.2 M) and stirred at 90 °C for 24 hours. After cooling to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using a hexane/ethyl acetate mixture, thereby synthesizing 2-((R)-2-methylpiperidin-1-yl)-1-(4-phenyl-3,4-dihydroquinoxalin-1(2H)-yl)propan-1-one. The obtained 2-((R)-2-methylpiperidin-1-yl)-1-(4-phenyl-3,4-dihydroquinoxalin-1(2H)-yl)propan-1-one (2 g, 5.502 mmol) was dissolved in ethyl acetate (75 mL) and stirred at room temperature for 10 minutes. 85% phosphoric acid (0.506 mL, 8.253 mmol) was dissolved in ethyl acetate (25 mL), slowly added to the prepared reaction solution, and then stirred at room temperature for 1 hour. The resulting solid was filtered, washed, and dried, thereby synthesizing 2-((R)-2-methylpiperidin-1-yl)-1-(4-phenyl-3,4-dihydroquinoxalin-1(2H)-yl)propan-1-one phosphate.
White solid (yield 11.4%); ¹H NMR (400 MHz, CD₃OD) δ 7.45-7.37 (m, 2H), 7.24-7.30 (m, 2H), 7.23-7.06 (m, 3H), 6.90-6.81 (m, 2H), 4.75-4.56 (m, 2H), 4.14-3.83 (m, 2H), 3.77-3.67 (m, 1H), 3.65-3.50 (m, 3H), 3.43-3.33 (m, 1H), 1.96-1.81 (m, 4H), 1.77-1.65 (m, 5H), 1.60-1.34 (m, 2H), 1.15 (d, 3H, *J* = 6.7 Hz); LC/MS ESI (+): 363.7 (M+1)

### <Example 1-60> Synthesis of 2-morpholino-1-(4-phenyl-3,4-dihydroquinoxalin-1(2H)-yl)propan-1-one

Morpholine (3.0 equiv) was added to a solution in which 2-bromo-1-(4-phenyl-3,4-dihydroquinoxalin-1(2H)-yl)propan-1-one (1.0 equiv) obtained in Preparation Example d-4 was dissolved in THF (0.2 M) and then stirred at 90 °C for 24 hours. After cooling to room temperature, water was added to terminate the reaction, and extraction was performed with ethyl acetate. An organic layer was washed with brine, dried with MgSO₄, filtered, and distilled under reduced pressure. The obtained residue was purified by silica column chromatography using a hexane/ethyl acetate mixture, thereby synthesizing 2-morpholino-1-(4-phenyl-3,4-dihydroquinoxalin-1(2H)-yl)propan-1-one.
Yellow oil (yield 32%); ¹H NMR (400 MHz, CDCl₃) δ 7.40-7.32 (m, 2H), 7.25-7.17 (m, 3H), 7.17-7.11 (m, 1H), 7.01-6.93 (m, 1H), 6.91-6.82 (m, 1H), 6.79-6.72 (m, 1H), 4.38-4.25 (m, 1H), 4.11-3.99 (m, 1H), 3.90-3.78 (m, 2H), 3.78-3.69 (m, 1H), 3.67-3.62 (m, 4H), 2.63-2.45 (m, 4H), 1.28 (d, 3H, *J*= 7.0 Hz); LC/MS ESI (+): 352.2 (M+1)

### <Example 1-61> Synthesis of 1-(4-phenyl-3,4-dihydroquinoxalin-1(2H)-yl)-2-(pyrrolidin-1-yl)propan-1-one

1-(4-Phenyl-3,4-dihydroquinoxalin-1(2H)-yl)-2-(pyrrolidin-1-yl)propan-1-one was synthesized in the same manner as in Example 1-60, except that pyrrolidine was used instead of (R)-2-methylpiperidine).
Yellow oil (yield 11%); ¹H NMR (400 MHz, CDCl₃) δ 7.40-7.32 (m, 2H), 7.22 (d, 2H, *J* = 7.8 Hz), 7.16-7.11 (m, 2H), 7.03-6.95 (m, 1H), 6.91-6.84 (m, 1H), 6.81-6.74 (m, 1H), 4.40-4.28 (m, 1H), 4.07-3.95 (m, 1H), 3.83-3.72 (m, 3H), 2.69-2.52 (m, 4H), 1.79-1.67 (m, 4H), 1.36 (d, 3H, *J* = 6.7 Hz); LC/MS ESI (+): 336.2 (M+1)

### <Example 1-62> Synthesis of 2-(4-methylpiperazin-1-yl)-1-(4-phenyl-3,4-dihydroquinoxalin-1(2H)-yl)propan-1-one

2-(4-Methylpiperazin-1-yl)-1-(4-phenyl-3,4-dihydroquinoxalin-1(2H)-yl)propan-1-one was synthesized in the same manner as in Example 1-60, except that 1-methylpiperazine was used instead of (R)-2-methylpiperidine.
Yellow oil (yield 38%); ¹H NMR (400 MHz, CDCl₃) δ 7.40-7.32 (m, 2H) 7.25-7.19 (m, 3H), 7.14-7.09 (m, 1H), 7.01-6.92 (m, 1H), 6.90-6.81 (m, 1H), 6.78-6.71 (m, 1H), 4.33-4.20 (m, 1H), 4.13-4.00 (m, 1H), 3.92-3.67 (m, 3H), 2.68-2.47 (m, 4H), 2.45-2.30 (m, 4H), 2.23 (s, 3H), 1.26 (d, 3H, *J* = 6.7 Hz); LC/MS ESI (+): 365.3 (M+1)

### <Example 1-63> Synthesis of 1-(4-phenyl-3,4-dihydroquinoxalin-1(2H)-yl)-2-(piperidin-1-yl)propan-1-one

1-(4-Phenyl-3,4-dihydroquinoxalin-1(2H)-yl)-2-(piperidin-1-yl)propan-1-one was synthesized in the same manner as in Example 1-60, except that piperidine was used instead of (R)-2-methylpiperidine.
Yellow oil (yield 49%); ¹H NMR (400 MHz, CDCl₃) δ 7.42-7.32 (m, 2H), 7.25-7.19(m, 3H), 7.17-7.11 (m, 1H), 7.01-6.91 (m, 1H), 6.89-6.80 (m, 1H), 6.79-6.70 (m, 1H), 4.41-4.27 (m, 1H), 4.14-3.99 (m, 1H), 3.88-3.67 (m, 3H), 2.54-2.41 (m, 4H), 1.52-1.46 (m, 4H), 1.40-1.33 (m, 2H), 1.26 (d, 3H, *J* = 6.7 Hz); LC/MS ESI (+): 350.3 (M+1)

### <Example 1-64> Synthesis of 2-(hexahydropyrrolo[1,2-a]pyrazin-2(1H)-yl)-1-(4-phenyl-3,4-dihydroquinoxalin-1(2H)-yl)propan-1-one

2- (Hexahydropyrrolo[1,2-a]pyrazin-2(1H)-yl)-1-(4-phenyl-3,4-dihydroquinoxalin-1(2H)-yl)propan-1-one was synthesized in the same manner as in Example 1-60, except that octahydropyrrolo[1,2-a]pyrazine was used instead of (R)-2-methylpiperidine.
Yellow solid (yield 31%); ¹H NMR (400 MHz, CD₃OD) δ 7.43-7.32 (m, 2H), 7.30-7.17 (m, 3H), 7.17-7.09 (m, 1H), 7.09-6.99 (m, 1H), 6.96-6.79 (m, 2H), 4.51-4.37 (m, 1H), 4.36-4.24 (m, 1H), 3.95-3.83 (m, 1H), 3.79-3.57 (m, 2H), 3.32-3.23 (m, 2H), 3.07-2.78 (m, 6H), 2.64-2.44 (m, 2H), 2.13-1.92 (m, 3H), 1.79-1.52 (m, 1H), 1.31 (d, 3H, *J* = 6.7 Hz); LC/MS ESI (+): 391.2 (M+1)

### <Example 1-65> Synthesis of 2-((1-methylpyrrolidin-3-yl)amino)-1-(4-phenyl-3,4-dihydroquinoxalin-1(2H)-yl)propan-1-one

2-((1-Methylpyrrolidin-3-yl)amino)-1-(4-phenyl-3,4-dihydroquinoxalin-1(2H)-yl)propan-1-one was synthesized in the same manner as in Example 1-60, except that 1-methylpyrrolidin-3-amine was used instead of (R)-2-methylpiperidine.
Yellow oil (yield 43%); ¹H NMR (400 MHz, CD₃OD) δ 7.43-7.33 (m, 2H), 7.32-7.23 (m, 2H), 7.19-7.08 (m, 2H), 7.08-6.97 (m, 1H), 6.89-6.74 (m, 2H), 4.47-4.33 (m, 1H), 4.14-3.96 (m, 1H), 3.91-3.81 (m, 1H), 3.79-3.64 (m, 2H), 3.24-3.11 (m, 1H), 2.87-2.75 (m, 1H), 2.67-2.53 (m, 2H), 2.51-2.37 (m, 1H), 2.30-2.19 (m, 3H), 2.09-2.16 (m, 1H), 1.96-2.16 (m, 2H), 1.35-1.59 (m, 1H), 1.27 (m, 3H); LC/MS ESI (+): 365.2 (M+1)

### <Example 1-66> Synthesis of 2-(4-cyclopropylpiperazin-1-yl)-1-(4-phenyl-3,4-dihydroquinoxalin-1(2H)-yl)propan-1-one

2-(4-Cyclopropylpiperazin-1-yl)-1-(4-phenyl-3,4-dihydroquinoxalin-1(2H)-yl)propan-1-one was synthesized in the same manner as in Example 1-60, except that 1-cyclopropylpiperazine was used instead of (R)-2-methylpiperidine.
Yellow oil (yield 17.7%); ¹H NMR (400 MHz, CD₃OD) δ 7.41-7.31 (m, 2H), 7.29-7.23 (m, 2H), 7.23-7.17 (m, 1H), 7.16-7.09 (m, 1H), 7.08-6.97 (m, 1H), 6.91-6.84 (m, 1H), 6.84-6.77 (m, 1H), 4.53-4.40 (m, 1H), 4.16-4.07 (m, 1H), 3.93-3.84 (m, 1H), 3.75-3.55 (m, 2H), 2.68-2.42 (m, 8H), 1.64-1.56 (m, 1H), 1.26 (d, 3H, *J*= 7.0 Hz), 0.48-0.38 (m, 2H), 0.38-0.25 (m, 2H); LC/MS ESI (+): 391.2 (M+1)

### <Example 1-67> Synthesis of 2-methyl-1-(4-phenyl-3,4-dihydroquinoxalin-1(2H)-yl)-3-((pyridin-2-ylmethyl)amino)propan-1-one

2-Methyl-1-(4-phenyl-3,4-dihydroquinoxalin-1(2H)-yl)-3-((pyridin-2-ylmethyl)amino)propan-1-one was synthesized in the same manner as in Example 1-60, except that 2-(aminomethyl)pyridine was used instead of (R)-2-methylpiperidine.
Yellow oil (yield 63%); ¹H NMR (400 MHz, CD₃OD) δ 8.48-8.40 (m, 1H), 7.77-7.69 (m, 1H), 7.43-7.33 (m, 3H), 7.30-7.21 (m, 3H), 7.18-7.11 (m, 1H), 7.07-6.91 (m, 2H), 6.81-6.73 (m, 1H), 6.72-6.66 (m, 1H), 4.21-4.12 (m, 1H), 4.12-3.94 (m, 2H), 3.90-3.71 (m, 5H), 1.32-1.18 (m, 3H); LC/MS ESI (+): 373.1 (M+1)

### <Example 1-68> Synthesis of 1-(2-((R)-2-methylpiperidin-1-yl)propyl)-4-phenyl-1,2,3,4-tetrahydroquinoxaline

A borane-THF complex (1 M) (3.0 equiv) was slowly added to a solution in which 2-((R)-2-methylpiperidin-1-yl)-1-(4-phenyl-3,4-dihydroquinoxalin-1 (2H)-yl)propan-1-one (1.0 equiv) obtained in Example 1-59 was dissolved in THF (0.5 M) at room temperature and then stirred at 70 °C for 24 hours. After cooling the reaction solution to room temperature, water was added to terminate the reaction, the reaction product was neutralized with a saturated NaHCO₃ aqueous solution and extracted with ethyl acetate. An organic layer was washed with brine, dried with MgSO₄, filtered, and concentrated under reduced pressure. The obtained residue was purified by silica column chromatography using a hexane/ethyl acetate mixture, thereby synthesizing 1-(2-((R)-2-methylpiperidin-1-yl)propyl)-4-phenyl-1,2,3,4-tetrahydroquinoxaline.
Yellow oil (yield 21%); ¹H NMR (400 MHz, CDCl₃) δ 7.33-7.27 (m, 2H), 7.14 (d, 2H, *J* = 7.4 Hz), 7.03-6.97 (m, 1H), 6.84-6.76 (m, 2H), 6.67 (d, 1H, *J =* 7.8 Hz), 6.55-6.48 (m, 1H), 3.70-3.63 (m, 2H), 3.58-3.42 (m, 3H), 3.40-3.32 (m, 1H), 3.27-3.17 (m, 1H), 3.04-2.95 (m, 1H), 2.92-2.83 (m, 1H), 2.49-2.39 (m, 1H), 1.72-1.57 (m, 4H), 1.47-1.30 (m, 2H), 1.24-1.13 (m, 6H); LC/MS ESI (+): 350.1 (M+1)

### <Example 1-69> Synthesis of 1-morpholino-2-(4-phenyl-3,4-dihydroquinoxalin-1(2H)-yl)ethan-1-one

Morpholine (3.0 equiv) and DIPEA (3.0 equiv) were mixed and stirred at room temperature for 5 minutes. A mixture in which 2-(4-phenyl-3,4-dihydroquinoxalin-1(2H)-yl)acetyl chloride (1.0 equiv) obtained in Preparation Example d-5 was dissolved in DMF (0.1 M) was added to the reaction solution and then stirred at 110 °C for 3 hours. After cooling to room temperature, water was added to terminate the reaction, and extraction was then performed with ethyl acetate. An organic layer was washed with brine, dried with MgSO₄, filtered, and distilled under reduced pressure. The obtained residue was purified by silica column chromatography using a hexane/ethyl acetate mixture, thereby synthesizing 1-morpholino-2-(4-phenyl-3,4-dihydroquinoxalin-1(2H)-yl)ethan-1-one.
Ivory solid (yield 32%); ¹H NMR (400 MHz, CDCl₃) δ 7.56-7.41 (m, 1H), 7.40-7.33 (m, 2H), 7.28-7.22 (m, 2H), 7.16-7.08 (m, 1H), 6.93-6.84 (m, 1H), 6.72-6.63 (m, 2H), 3.96-3.86 (m, 2H), 3.74-3.62 (m, 2H), 3.55-3.44 (m, 4H), 2.44-2.35 (m, 4H); LC/MS ESI (+): 338.1 (M+1)

### <Example 1-70> Synthesis of (R)-1-(2-methylpiperidin-1-yl)-2-(4-phenyl-3,4-dihydroquinoxalin-1(2H)-yl)ethan-1-one

(R)-1-(2-Methylpiperidin-1-yl)-2-(4-phenyl-3,4-dihydroquinoxalin-1(2H)-yl)ethan-1-one was synthesized in the same manner as in Example 1-69, except that (R)-2-methylpiperridine was used instead of morpholine.
Pale yellow oil (yield 36.8%); ¹H NMR (400 MHz, CD₃OD) δ 7.48-7.32 (m, 2H), 7.32-7.21 (m, 3H), 7.20-7.09 (m, 1H), 7.05-6.89 (m, 1H), 6.88-6.68 (m, 2H), 4.21-4.10 (m, 1H), 4.00-3.85 (m, 1H), 3.83-3.64 (m, 3H), 2.90-2.74 (m, 1H), 2.56-2.37 (m, 3H), 1.73-1.42 (m, 5H), 1.07-0.82 (m, 4H); LC/MS ESI (+): 350.1 (M+1)

### <Example 1-71> Synthesis of (S)-1-(2-methylpiperidin-1-yl)-2-(4-phenyl-3,4-dihydroquinoxalin-1(2H)-yl)ethan-1-one

(S)-1-(2-Methylpiperidin-1-yl)-2-(4-phenyl-3,4-dihydroquinoxalin-1(2H)-yl)ethan-1-one was synthesized in the same manner as in Example 1-69, except that (S)-2-methylpiperidine was used instead of morpholine.
Pale yellow oil (yield 61.1%); ¹H NMR (400 MHz, DMSO-*d*₆) δ 7.52-7.33 (m, 2H), 7.32-7.20 (m, 3H), 7.20-7.05 (m, 1H), 7.03-6.82 (m, 1H), 6.80-6.62 (m, 2H), 4.10-4.00 (m, 1H), 3.97-3.54 (m, 4H), 2.76-2.58 (m, 1H), 2.47-2.26 (m, 3H), 1.61-1.38 (m, 3H), 1.38-1.05 (m, 2H), 1.01-0.67 (m, 4H); LC/MS ESI (+): 350.1 (M+1)

### <Example 1-72> Synthesis of (R)-1-(2-methylpyrrolidin-1-yl)-2-(4-phenyl-3,4-dihydroquinoxalin-1(2H)-yl)ethan-1-one

(R)-1-(2-Methylpyrrolidin-1-yl)-2-(4-phenyl-3,4-dihydroquinoxalin-1(2H)-yl)ethan-1-one was synthesized in the same manner as in Example 1-69, except that (R)-2-methylpyrrolidine was used instead of morpholine.
Brown oil (yield 27%); ¹H NMR (400 MHz, CDCl₃) δ 7.41-7.33 (m, 2H), 7.25-7.21 (m, 3H), 7.19-7.12 (m, 1H), 6.98-6.91 (m, 1H), 6.80 (d, 1H, *J =* 8.2 Hz), 6.78-6.72 (m, 1H), 4.26-4.17 (m, 1H), 3.94-3.87 (m, 2H), 3.80-3.72 (m, 2H), 3.25-3.14 (m, 2H), 2.55-2.46 (m, 1H), 2.43-2.33 (m, 1H), 1.97-1.85 (m, 1H), 1.81-1.62 (m, 2H), 1.46-1.38 (m, 1H), 1.04 (d, 3H, *J* = 4.7 Hz); LC/MS ESI (+): 336.1 (M+1)

### <Example 1-73> Synthesis of 1-(4-methylpiperazin-1-yl)-2-(4-phenyl-3,4-dihydroquinoxalin-1(2H)-yl)ethan-1-one

1-(4-Methylpiperazin-1-yl)-2-(4-phenyl-3,4-dihydroquinoxalin-1(2H)-yl)ethan-1-one was synthesized in the same manner as in Example 1-69, except that 1-methylpiperazine was used instead of morpholine.
Ivory solid (yield 34%); ¹H NMR (400 MHz, CDCl₃) δ 7.40-7.34 (m, 2H), 7.34-7.28 (m, 1H), 7.25-7.21 (m, 2H), 7.19-7.13 (m, 1H), 6.99-6.91 (m, 1H), 6.80 (d, 1H, *J =* 8.2 Hz), 6.77-6.71 (m, 1H), 4.05 (t, 2H, *J* = 5.5 Hz), 3.75 (t, 2H, *J* = 5.3 Hz), 3.38 (s, 2H), 2.75-2.34 (m, 8H), 2.27 (s, 3H); LC/MS ESI (+): 351.2 (M+1)

### <Example 1-74> Synthesis of 1-(4-methylpiperidin-1-yl)-2-(4-phenyl-3,4-dihydroquinoxalin-1(2H)-yl)ethan-1-one

1-(4-Methylpiperidin-1-yl)-2-(4-phenyl-3,4-dihydroquinoxalin-1(2H)-yl)ethan-1-one was synthesized in the same manner as in Example 1-69, except that 4-methylpiperridine was used instead of morpholine.
Pale yellow oil (yield 39.4%); ¹H NMR (400 MHz, DMSO-*d*₆) δ 7.59-6.59 (m, 9H), 3.94 (d, 2H, *J* = 5.7 Hz), 3.71 (s, 2H), 3.29 (s, 2H), 2.75 (s, 2H), 2.07-1.92 (m, 2H), 1.62-0.93 (m, 6H), 0.85 (d, 3H, *J* = 6.4 Hz); LC/MS ESI (+): 350.2 (M+1)

### <Example 1-75> Synthesis of 1-(hexahydropyrrolo[1,2-a]pyrazin-2(1H)-yl)-2-(4-phenyl-3,4-dihydroquinoxalin-1(2H)-yl)ethan-1-one

1-(Hexahydropyrrolo[1,2-a]pyrazin-2(1H)-yl)-2-(4-phenyl-3,4-dihydroquinoxalin-1(2H)-yl)ethan-1-one was synthesized in the same manner as in Example 1-69, except that octahydropyrrolo[1,2-a]pyrazine was used instead of morpholine.
Pale yellow oil (yield 42%); ¹H NMR (400 MHz, CDCl₃) δ 7.37 (dd, 2H, *J* = 8.5, 7.3 Hz), 7.26-7.20 (m, 3H), 7.16 (dd, 1H, *J* = 8.1, 6.7 Hz), 6.94 (d, 1H, *J* = 8.1 Hz), 6.84-6.71 (m, 2H), 4.15-3.97 (m, 2H), 3.76 (t, 2H, *J* = 5.6 Hz), 3.50-3.36 (m, 2H), 3.11-2.87 (m, 3H), 2.40-1.55 (m, 10H); LC/MS ESI (+): 377.2 (M+1)

### <Example 1-76> Synthesis of 1-(4-cyclopropylpiperazin-1-yl)-2-(4-phenyl-3,4-dihydroquinoxalin-1(2H)-yl)ethan-1-one

1-(4-Cyclopropylpiperazin-1-yl)-2-(4-phenyl-3,4-dihydroquinoxalin-1(2H)-yl)ethan-1-one was synthesized in the same manner as in Example 1-69, except that 1-cyclopropylpiperazine was used instead of morpholine.
Pale yellow oil (yield 46.9%); ¹H NMR (400 MHz, DMSO-*d*₆) δ 7.62-6.50 (m, 9H), 4.04-3.65 (m, 3H), 3.33-3.17 (m, 6H), 2.39 (s, 5H), 1.60-1.50 (m, 1H), 0.45-0.18 (m, 4H); LC/MS ESI (+): 377.0 (M+1)

### <Example 1-77> Synthesis of 1-(3-hydroxypiperidin-1-yl)-2-(4-phenyl-3,4-dihydroquinoxalin-1(2H)-yl)ethan-1-one

1-(3-Hydroxypiperidin-1-yl)-2-(4-phenyl-3,4-dihydroquinoxalin-1(2H)-yl)ethan-1-one was synthesized in the same manner as in Example 1-69, except that 3-hydroxypiperidine was used instead of morpholine.
Pale yellow oil (yield 83.8%); ¹H NMR (400 MHz, CDCl₃) δ 7.48-6.58 (m, 9H), 4.19-4.07 (m, 1H), 3.98 (d, 1H, *J=* 9.5 Hz), 3.86-3.67 (m, 3H), 3.63-3.32 (m, 2H), 2.72-2.12 (m, 4H), 1.82-1.36 (m, 5H); LC/MS ESI (+): 352.1 (M+1)

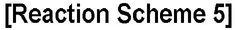

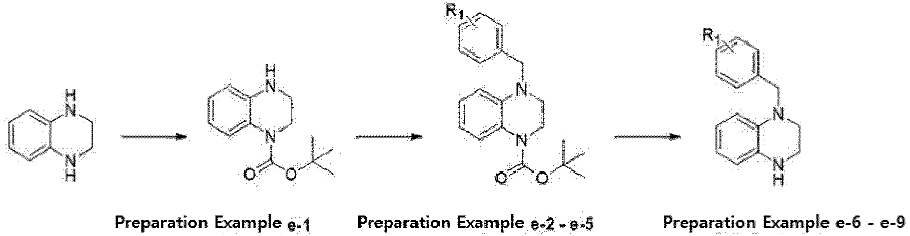

### <Preparation Method e> Preparation of benzyl- or chloro-substituted benzyl-1,2,3,4-tetrahydroquinoxaline

Benzyl- or chloro-substituted benzyl-1,2,3,4-tetrahydroquinoxaline according to the present invention was prepared through the processes of Preparation Examples e-1 to e-9 below.

### <Preparation Example e-1> Preparation of tert-butyl-3,4-dihydroquinoxalin-1(2H)-carboxylate

A 0.02M NaOH aqueous solution (2 equiv) was added to a mixture in which 1,2,3,4-tetrahydroquinoxaline (1.0 equiv) and di-tert-butyl-dicarbonate (1.2 equiv) were dissolved in THF (0.1 M) and stirred at room temperature for 24 hours. The reaction solution was concentrated under reduced pressure and then extracted with dichloromethane. An organic layer was dried with MgSO₄, filtered, and then concentrated under reduced pressure. The obtained residue was purified by silica column chromatography using a hexane/ethyl acetate mixture, thereby preparing tert-butyl-3,4-dihydroquinoxalin-1(2H)-carboxylate.
Yellow solid (yield 94%); ¹H NMR (400 MHz, DMSO-*d*₆) δ 7.29 (d, 1H, *J* = 8.1 Hz), 6.79 (ddd, 1H, *J* = 8.5, 7.2, 1.5 Hz), 6.54 (dd, 1H, *J* = 8.0, 1.5 Hz), 6.45 (ddd, 1H, *J* = 8.5, 7.2, 1.5 Hz), 6.01 (s, 1H), 3.60-3.53 (m, 2H), 3.27-3.20 (m, 2H), 1.44 (s, 9H); LC/MS ESI (+): 202.7 (M+1)

### <Preparation Example e-2> Preparation of tert-butyl-4-benzyl-3,4-dihydroquinoxalin-1(2H)-carboxylate

Benzyl bromide (1.2 equiv) was added to a mixture in which tert-butyl-3,4-dihydroquinoxalin-1(2H)-carboxylate (1.0 equiv) obtained in Preparation Example e-1 and DIPEA (4 equiv) were dissolved in DMF (1.8 M) and stirred at 130 °C for 2 hours. The reaction solution was cooled to room temperature and poured into water, and then extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing tert-butyl-4-benzyl-3,4-dihydroquinoxalin-1(2H)-carboxylate.
Brown oil (yield 100%); ¹H NMR (400 MHz, CDCl₃) δ 7.46 (d, 1H, *J* = 8.0 Hz), 7.37 - 7.22 (m, 5H), 6.91 (td, 1H, *J* = 7.8, 1.6 Hz), 6.64 (t, 2H, *J* = 8.6 Hz), 4.52 (s, 2H), 3.87 - 3.80 (m, 2H), 3.47 - 3.40 (m, 2H), 1.53 (s, 9H); LC/MS ESI (+): 325.2 (M+1)

### <Preparation Example e-3> Preparation of tert-butyl-4-(2-chlorobenzyl)-3,4-dihydroquinoxalin-1(2H)-carboxylate

Tert-butyl-4-(2-chlorobenzyl)-3,4-dihydroquinoxalin-1(2H)-carboxylate was prepared in the same manner as in Preparation Example e-2, except that 2-chlorobenzyl bromide was used instead of benzyl bromide.

Yellow oil (yield 94%); ¹H NMR (400 MHz, CDCl₃) δ 7.53-7.43 (m, 1H), 7.40 (m, 1H), 7.24-7.14 (m, 3H), 6.92-6.87 (m, 1H), 6.68-6.64 (m, 1H), 6.45 (dd, 1H, *J =* 8.1, 0.8 Hz), 4.57 (s, 2H), 3.92-3.84 (m, 2H), 3.52-3.44 (m, 2H), 1.54 (s, 9H).

### <Preparation Example e-4> Preparation of tert-butyl-4-(3-chlorobenzyl)-3,4-dihydroquinoxalin-1(2H)-carboxylate

Tert-butyl-4-(3-chlorobenzyl)-3,4-dihydroquinoxalin-1(2H)-carboxylate was prepared in the same manner as in Preparation Example e-2, except that 3-chlorobenzyl bromide was used instead of benzyl bromide.

Yellow oil (yield 91.6%); ¹H NMR (400 MHz, CD₃CN) δ 7.41 (dd, 1H, *J=* 8.2, 1.2 Hz), 7.38-7.18 (m, 4H), 6.91-6.85 (m, 1H), 6.64-6.55 (m, 2H), 4.55 (s, 2H), 3.84-3.78 (m, 2H), 3.50-3.45 (m, 2H), 1.51 (s, 9H).

### <Preparation Example e-5> Preparation of tert-butyl-4-(4-chlorobenzyl)-3,4-dihydroquinoxalin-1(2H)-carboxylate

Tert-butyl-4-(3-chlorobenzyl)-3,4-dihydroquinoxalin-1(2H)-carboxylate was prepared in the same manner as in Preparation Example e-3, except that 4-chlorobenzyl bromide was used instead of benzyl bromide.

Pale yellow oil (yield 100%); ¹H NMR (400 MHz, CDCl₃) δ 7.46 (d, *J* = 8.0 Hz, 1H), 7.37-7.22 (m, 5H), 6.91 (td, *J* = 7.8, 1.6 Hz, 1H), 6.64 (t, *J* = 8.6 Hz, 2H), 4.52 (s, 2H), 3.87-3.80 (m, 2H), 3.47-3.40 (m, 2H), 1.53 (s, 9H).

### <Preparation Example e-6> Preparation of 1-benzyl-1,2,3,4-tetrahydroquinoxaline

A mixture in which tert-butyl-4-benzyl-3,4-dihydroquinoxalin-1(2H)-carboxylate (1.0 equiv) obtained in Preparation Example e-2 and TFA (10.0 equiv) were dissolved in dichloromethane (0.5 M) was stirred at room temperature for 12 hours. After termination of the reaction, toluene was added to the reaction solution to perform concentration under reduced pressured, water was added, and then extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-benzyl-1,2,3,4-tetrahydroquinoxaline.
Brown oil (yield 80%); ¹H NMR (400 MHz, DMSO-*d*₆) δ 7.40-7.16 (m, 5H), 6.44-6.30 (m, 4H), 5.47 (s, 1H), 4.39 (s, 2H), 3.30 (d, 4H, *J =* 1.5 Hz); LC/MS ESI (+): 202.7 (M+1)

### <Preparation Example e-7> Preparation of 1-(2-chlorobenzyl)-1,2,3,4-tetrahydroquinoxaline

1-(2-Chlorobenzyl)-1,2,3,4-tetrahydroquinoxaline was prepared in the same manner as in Preparation Example e-6, except that tert-butyl-4-(2-chlorobenzyl)-3,4-dihydroquinoxalin-1(2H)-carboxylate was used instead of tert-butyl-4-benzyl-3,4-dihydroquinoxalin-1(2H)-carboxylate.

Yellow solid (yield 90%); ¹H NMR (400 MHz, CDCl₃) δ 7.43-7.35 (m, 1H), 7.31 (dd, 1H, *J=* 5.5, 3.9 Hz), 7.23-7.14 (m, 2H), 6.66-6.48 (m, 3H), 6.35 (d, 1H, *J=* 8.6 Hz), 4.50 (s, 2H), 3.78 (brs, 1H), 3.49 (s, 4H).

### <Preparation Example e-8> Preparation of 1-(3-chlorobenzyl)-1,2,3,4-tetrahydroquinoxaline

1-(3-Chlorobenzyl)-1,2,3,4-tetrahydroquinoxaline was prepared in the same manner as in Preparation Example e-6, except that tert-butyl-4-(3-chlorobenzyl)-3,4-dihydroquinoxalin-1(2H)-carboxylate was used instead of tert-butyl-4-benzyl-3,4-dihydroquinoxalin-1(2H)-carboxylate.

Yellow solid (yield 87.4%); ¹H NMR (400 MHz, CDCl₃) δ 7.34-7.16 (m, 4H), 6.65-6.45 (m, 4H), 4.40 (s, 2H), 3.75 (br s, 1H), 3.49-3.44 (m, 2H), 3.43-3.38 (m, 2H).

### <Preparation Example e-9> Preparation of 1-(4-chlorobenzyl)-1,2,3,4-tetrahydroquinoxaline

1-(4-Chlorobenzyl)-1,2,3,4-tetrahydroquinoxaline was prepared in the same manner as in Preparation Example e-6, except that tert-butyl-4-(4-chlorobenzyl)-3,4-dihydroquinoxalin-1(2H)-carboxylate was used instead of tert-butyl-4-benzyl-3,4-dihydroquinoxalin-1(2H)-carboxylate.

Light brown oil (yield 94%); ¹H NMR (400 MHz, DMSO-*d*₆) δ 7.42-7.32 (m, 2H), 7.32-7.26 (m, 2H), 6.47-6.32 (m, 4H), 5.48 (s, 1H), 4.38 (s, 2H), 3.30 (d, *J=* 1.5 Hz, 4H)

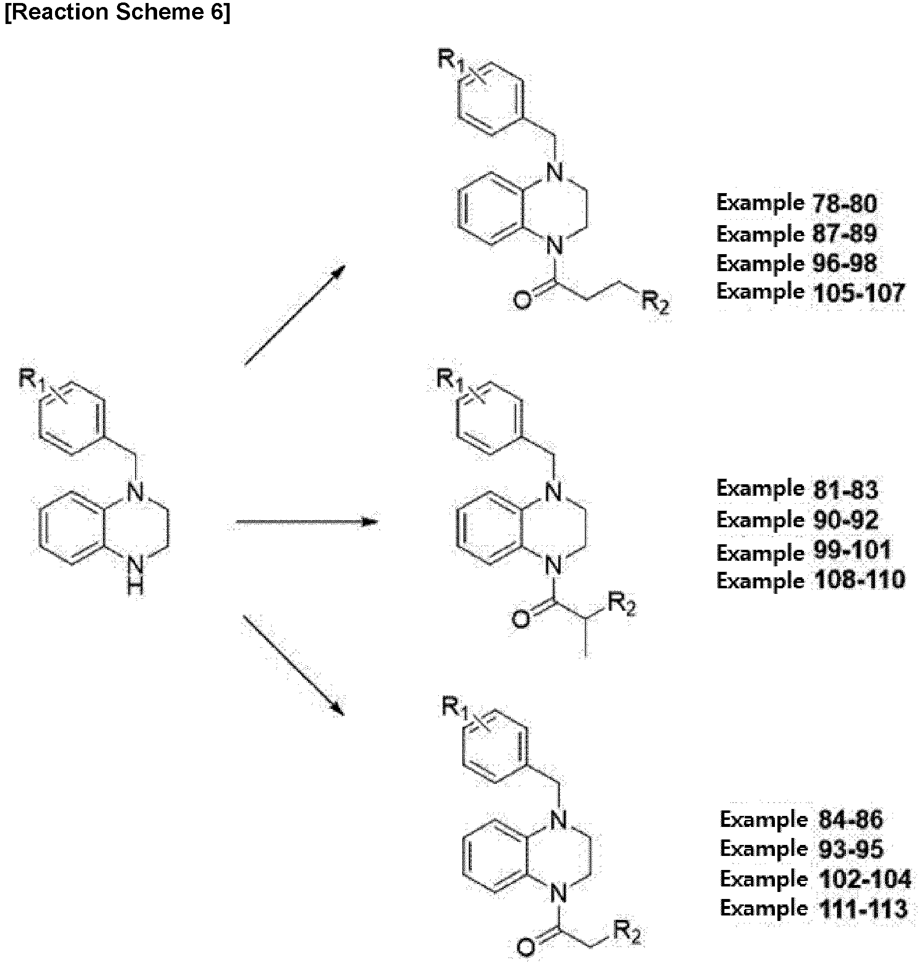

### <Example 1-78> Synthesis of 1-(4-benzyl-3,4-dihydroquinoxalin-1(2H)-yl)-3-(pyrrolidin-1-yl)propan-1-one

1-Benzyl-1,2,3,4-tetrahydroquinoxaline (1.0 equiv) obtained in Preparation Example e-6 was dissolved in dichloromethane (0.2 M), 3-bromopropionyl chloride (1.1 equiv) was slowly added thereto at -10 °C, and then the resulting solution was stirred at room temperature for 30 minutes. After terminating the reaction by adding water to the reaction solution, extraction was performed with dichloromethane. An organic layer was washed with brine, dried with MgSO₄, filtered, and then concentrated under reduced pressure, thereby synthesizing a yellow solid compound, 3-bromo-1-(4-benzyl-3,4-dihydroquinoxalin-1(2H)-yl)propan-1-one. TEA (3.0 equiv) and pyrrolidine (1.5 equiv) were added to a solution in which the obtained yellow solid compound (1.0 equiv) was dissolved in THF (0.2 M) and stirred at room temperature for 3 hours. After cooling to room temperature and terminating the reaction by adding water to the reaction solution, extraction was performed with ethyl acetate. An organic layer was washed with brine, dried with MgSO₄, filtered, and concentrated under reduced pressure. The obtained residue was purified by silica column chromatography using a dichloromethane/methanol mixture, thereby synthesizing 1-(4-benzyl-3,4-dihydroquinoxalin-1(2H)-yl)-3-(pyrrolidin-1-yl)propan-1-one.
Pale yellow oil (yield 41.5%); ¹H NMR (400 MHz, CD₃OD) δ 7.36-7.19 (m, 5H), 7.19-6.96 (m, 2H), 6.74 (d, 1H, *J =* 8.3 Hz), 6.68-6.30 (m, 1H), 4.58 (s, 2H), 3.92 (t, 2H, *J=* 5.5 Hz), 3.52-3.43 (m, 2H), 2.89-2.72 (m, 4H), 2.50-2.40 (m, 4H), 1.81-1.69 (m, 4H); LC/MS ESI (+): 350.2 (M+1)

### <Example 1-79> Synthesis of 1-(4-benzyl-3,4-dihydroquinoxalin-1(2H)-yl)-3-(piperidin-1-yl)propan-1-one

1-(4-Benzyl-3,4-dihydroquinoxalin-1(2H)-yl)-3-(piperidin-1-yl)propan-1-one was synthesized in the same manner as in Example 1-78, except that piperidine was used instead of pyrrolidine.
Pale yellow oil (yield 71.6%); ¹H NMR (400 MHz, CD₃OD) δ 7.39-7.18 (m, 5H), 7.18-6.97 (m, 2H), 6.73 (d, 1H, *J =* 8.2 Hz), 6.68-6.60 (m, 1H), 4.58 (s, 2H), 3.92 (t, 2H, *J* = 5.5 Hz,), 3.48 (d, 2H, *J* = 6.4 Hz), 2.80 (t, 2H, *J* = 7.3 Hz), 2.68-2.60 (m, 2H), 2.40-2.25 (m, 4H), 1.60-1.49 (m, 4H), 1.47-1.37 (m, 2H); LC/MS ESI (+): 364.1 (M+1)

### <Example 1-80> Synthesis of 1-(4-benzyl-3,4-dihydroquinoxalin-1(2H)-yl)-3-(4-methylpiperazin-1-yl)propan-1-one

1-(4-Benzyl-3,4-dihydroquinoxalin-1(2H)-yl)-3-(4-methylpiperazin-1-yl)propan-1-one was synthesized in the same manner as in Example 1-78, except that 1-methylpiperazine was used instead of pyrrolidine.
Pale yellow oil (yield 60.7%); ¹H NMR (400 MHz, CD₃OD) δ 7.38-7.19 (m, 5H), 7.19-6.93 (m, 2H), 6.74 (d, 1H, *J =* 8.3 Hz), 6.66-6.59 (m, 1H), 4.59 (s, 2H), 3.92 (t, 2H, *J* = 5.5 Hz), 3.49 (d, 2H, *J* = 5.5 Hz), 2.80 (t, 2H, *J* = 7.1 Hz), 2.69-2.60 (m, 2H), 2.54-2.28 (m, 8H), 2.25 (s, 3H); LC/MS ESI (+): 379.0 (M+1)

### <Example 1-81> Synthesis of 1-(4-benzyl-3,4-dihydroquinoxalin-1(2H)-yl)-2-(pyrrolidin-1-yl)propan-1-one

1-(4-Benzyl-3,4-dihydroquinoxalin-1(2H)-yl)-2-(pyrrolidin-1-yl)propan-1-one was synthesized in the same manner as in Example 1-78, except that 2-bromopropionyl chloride was used instead of 3-bromopropionyl chloride.
Yellow oil (yield 55%); ¹H NMR (400 MHz, CD₃OD) δ 7.31-7.20 (m, 5H), 7.11-6.97 (m, 2H), 6.82 (d, 1H, *J =* 7.8 Hz), 6.68-6.62 (m, 1H), 4.71-4.47 (m, 2H), 4.23-4.11 (m, 1H), 4.03-3.92 (m, 1H), 3.72-3.59 (m, 1H), 3.54-3.42 (m, 2H), 2.82-2.53 (m, 4H), 1.84-1.69 (m, 4H), 1.30 (d, 3H, *J* = 5.9 Hz); LC/MS ESI (+): 350.1 (M+1)

### <Example 1-82> Synthesis of 1-(4-benzyl-3,4-dihydroquinoxalin-1(2H)-yl)-2-(piperidin-1-yl)propan-1-one

1-(4-Benzyl-3,4-dihydroquinoxalin-1(2H)-yl)-2-(piperidin-1-yl)propan-1-one was synthesized in the same manner as in Example 1-81, except that piperidine was used instead of pyrrolidine.
Pale yellow oil (yield 54.0%); ¹H NMR (400 MHz, CD₃OD) δ 7.31-7.21 (m, 5H), 7.14-6.99 (m, 2H), 6.85-6.75 (m, 1H), 6.69-6.60 (m, 1H), 4.65-4.51 (m, 2H), 4.24-4.08 (m, 2H), 3.73-3.59 (m, 1H), 3.54-3.44 (m, 2H), 2.69-2.52 (m, 4H), 1.63-1.50 (m, 4H), 1.49-1.40 (m, 2H), 1.27 (d, 3H, *J* = 7.0 Hz); LC/MS ESI (+): 364.3 (M+1)

### <Example 1-83> Synthesis of 1-(4-benzyl-3,4-dihydroquinoxalin-1(2H)-yl)-2-(4-methylpiperazin-1-yl)propan-1-one

1-(4-Benzyl-3,4-dihydroquinoxalin-1(2H)-yl)-2-(4-methylpiperazin-1-yl)propan-1-one was synthesized in the same manner as in Example 1-81, except that 1-methylpiperazine was used instead of pyrrolidine.
Orange oil (yield 35.0%); ¹H NMR (400 MHz, CD₃OD) δ 7.32-7.21 (m, 5H), 7.15-7.07 (m, 1H), 7.07-6.98 (m, 1H), 6.81-6.73 (m, 1H), 6.67-6.58 (m, 1H), 4.70-4.50 (m, 2H), 4.34-4.20 (m, 1H), 4.12-3.96 (m, 1H), 3.61-3.40 (m, 3H), 2.61-2.31 (m, 8H), 2.22 (s, 3H), 1.22 (d, 3H, *J* = 6.7 Hz); LC/MS ESI (+): 379.2 (M+1)

### <Example 1-84> Synthesis of 1-(4-benzyl-3,4-dihydroquinoxalin-1(2H)-yl)-2-(pyrrolidin-1-yl)ethan-1-one

1-Benzyl-1,2,3,4-tetrahydroquinoxaline (1.0 equiv) obtained in Preparation Example e-6 was dissolved in dichloromethane (0.4 M), bromoacetyl chloride (1.1 equiv) was slowly added at -70 °C, and then stirred at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained concentrate was dissolved in THF (0.4 M), TEA (3.0 equiv) and pyrrolidine (1.0 equiv) were added, and then the resulting solution was stirred at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-benzyl-3,4-dihydroquinoxalin-1(2H)-yl)-2-(pyrrolidin-1-yl)ethan-1-one.
Brown solid (yield 39%); ¹H NMR (400 MHz, CD₃OD) δ 7.32-7.21 (m, 5H), 7.15-7.07 (m, 1H), 7.07-6.98 (m, 1H), 6.81-6.73 (m, 1H), 6.67-6.58 (m, 1H), 4.70-4.50 (m, 2H), 4.34-4.20 (m, 1H), 4.12-3.96 (m, 1H), 3.61-3.40 (m, 3H), 2.61-2.31 (m, 8H), 2.22 (s, 3H), 1.22 (d, 3H, *J* = 6.7 Hz); LC/MS ESI (+): 336.2 (M+1)

### <Example 1-85> Synthesis of 1-(4-benzyl-3,4-dihydroquinoxalin-1(2H)-yl)-2-(piperidin-1-yl)ethan-1-one

1-(4-Benzyl-3,4-dihydroquinoxalin-1(2H)-yl)-2-(piperidin-1-yl)ethan-1-one was synthesized in the same manner as in Example 1-84, except that piperidine was used instead of pyrrolidine.
Yellow oil (yield 49.3%); ¹H NMR (400 MHz, CD₃CN) δ 7.59-7.23 (m, 5H), 7.00-6.89 (m, 1H), 6.66 (d, 1H, *J* = 8.2 Hz), 6.63-6.55 (m, 1H), 4.58 (s, 2H), 3.93 (t, 2H, *J* = 5.5 Hz), 3.62-3.44 (m, 2H), 3.28-3.21 (m, 2H), 2.49-2.35 (m, 4H), 1.59-1.47 (m, 4H), 1.45-1.37 (m, 2H); LC/MS ESI (+): 350.1 (M+1)

### <Example 1-86> Synthesis of 1-(4-benzyl-3,4-dihydroquinoxalin-1(2H)-yl)-2-(4-methylpiperazin-1-yl)ethan-1-one

1-(4-Benzyl-3,4-dihydroquinoxalin-1(2H)-yl)-2-(4-methylpiperazin-1-yl)ethan-1-one was synthesized in the same manner as in Example 1-81, except that 1-methylpiperazine was used instead of pyrrolidine.
Yellow oil (yield 43.1%); ¹H NMR (400 MHz, CD₃OD) δ 7.37-7.09 (m, 5H), 7.00 (s, 1H), 6.79-6.70 (m, 1H), 6.67-6.56 (m, 1H), 4.62-4.51 (m, 2H), 3.98-3.85 (m, 2H), 3.48 (s, 2H), 3.42-3.35 (m, 2H), 2.82-2.31 (m, 8H), 2.29-2.20 (m, 3H); LC/MS ESI (+): 365.1 (M+1)

### <Example 1-87> Synthesis of 1-(4-(2-chlorobenzyl)-3,4-dihydroquinoxalin-1(2H)-yl)-3-(pyrrolidin-1-yl)propan-1-one

1-(4-(2-Chlorobenzyl)-3,4-dihydroquinoxalin-1(2H)-yl)-3-(pyrrolidin-1-yl)propan-1-one was synthesized in the same manner as in Example 78, except that 1-(2-chlorobenzyl)-1,2,3,4-tetrahydroquinoxaline obtained in Preparation Example e-7 was used instead of 1-benzyl-1,2,3,4-tetrahydroquinoxaline.
White solid (yield 46.4%); ¹H NMR (400 MHz, CDCl₃) δ 7.42-7.40 (m, 1H), 7.25-7.19 (m, 2H), 7.17-7.07 (m, 2H), 7.02 (d, 1H, *J* = 8.2 Hz), 6.77-6.69 (m, 1H), 6.51 (d, 1H, *J* = 8.2 Hz), 4.59 (s, 2H), 4.03-3.93 (m, 2H), 3.65-3.35 (m, 6H), 3.26 (d, 2H, *J* = 6.3 Hz), 2.23-2.04 (m, 6H); LC/MS ESI (+): 384.0 (M+1)

### <Example 1-88> Synthesis of 1-(4-(2-chlorobenzyl)-3,4-dihydroquinoxalin-1(2H)-yl)-3-(piperidin-1-yl)propan-1-one

1-(4-(2-Chlorobenzyl)-3,4-dihydroquinoxalin-1(2H)-yl)-3-(piperidin-1-yl)propan-1-one was synthesized in the same manner as in Example 1-87, except that piperidine was used instead of pyrrolidine.
Yellow oil (yield 29.1%); ¹H NMR (400 MHz, CD₃OD) δ ) δ 7.47-7.40 (m, 1H), 7.31-7.20 (m, 2H), 7.20-7.12 (m, 2H), 7.05-6.63 (m, 2H), 6.54 (d, 1H, *J =* 8.2 Hz), 4.64 (s, 2H), 3.97 (t, 2H, *J* = 5.3 Hz), 3.53-3.46 (m, 2H), 2.98-2.90 (m, 4H), 2.74-2.63 (m, 4H), 1.73-1.62 (m, 4H), 1.58-1.48 (m, 2H); LC/MS ESI (+): 398.0 (M+1)

### <Example 1-89> Synthesis of 1-(4-(2-chlorobenzyl)-3,4-dihydroquinoxalin-1(2H)-yl)-3-(4-methylpiperazin-1-yl)propan-1-one

1-(4-(2-Chlorobenzyl)-3,4-dihydroquinoxalin-1(2H)-yl)-3-(4-methylpiperazin-1-yl)propan-1-one was synthesized in the same manner as in Example 1-87, except that 1-methylpiperazine was used instead of pyrrolidine.
Colorless oil (yield 35.5%); ¹H NMR (400 MHz, CD₃OD) δ 7.47-7.40 (m, 1H), 7.30-7.20 (m, 2H), 7.16 (d, 2H, *J* = 6.3 Hz), 7.00 (d, 1H, *J* = 6.7 Hz), 6.70-6.62 (m, 1H, *J =* 7.4 Hz), 6.53 (d, 1H, *J* = 8.2 Hz), 4.64 (s, 2H), 3.96 (t, 2H, *J* = 5.3 Hz), 3.57-3.43 (m, 2H), 2.88-2.79 (m, 2H), 2.76-2.64 (m, 2H), 2.63-2.57 (m, 4H), 2.57-2.42 (m, 4H), 2.39 (s, 3H); LC/MS ESI (+): 413.0 (M+1)

### <Example 1-90> Synthesis of 1-(4-(2-chlorobenzyl)-3,4-dihydroquinoxalin-1(2H)-yl)-2-(pyrrolidin-1-yl)propan-1-one

1-(4-(2-Chlorobenzyl)-3,4-dihydroquinoxalin-1(2H)-yl)-2-(pyrrolidin-1-yl)propan-1-one was synthesized in the same manner as in Example 1-87, except that 2-bromopropionyl chloride was used instead of 3-bromopropionyl chloride.
Yellow oil (yield 55.2%); ¹H NMR (400 MHz, CD₃OD) 7.43 (d, 1H, *J* = 7.4 Hz), 7.30-7.12 (m, 3H), 7.05 (d, 2H, *J* = 9.0 Hz), 6.71-6.63 (m, 1H), 4.77-4.53 (m, 2H), 4.32-4.20 (m, 1H), 3.96-3.86 (m, 1H), 3.70-3.58 (m, 1H), 3.55-3.45 (m, 2H), 2.66-2.46 (m, 4H), 1.77-1.71 (m, 4H), 1.33 (d, 3H, *J* = 6.7 Hz); LC/MS ESI (+): 384.1 (M+1)

### <Example 1-91> Synthesis of 1-(4-(2-chlorobenzyl)-3,4-dihydroquinoxalin-1(2H)-yl)-2-(piperidin-1-yl)propan-1-one

1-(4-(2-Chlorobenzyl)-3,4-dihydroquinoxalin-1(2H)-yl)-2-(piperidin-1-yl)propan-1-one was synthesized in the same manner as in Example 1-90, except that piperidine was used instead of pyrrolidine.
White solid (yield 35.6%); ¹H NMR (400 MHz, CD₃OD) δ 7.43 (d, 1H, *J=* 7.4 Hz), 7.31-7.18 (m, 2H), 7.16-7.13 (m, 2H), 7.08-6.98 (m, 1H), 6.69-6.63 (m, 1H), 6.57 (d, 1H, *J =* 7.8 Hz), 4.77-4.48 (m, 2H), 4.37-4.31 (m, 1H), 4.03 (d, 1H, *J =* 5.5 Hz), 3.58-3.44 (m, 3H), 2.50-2.37 (m, 4H), 1.55-1.47 (m, 4H), 1.43-1.39 (m, 4H), 1.25-1.23 (m, 2H); LC/MS ESI (+): 398.0 (M+1)

### <Example 1-92> Synthesis of 1-(4-(2-chlorobenzyl)-3,4-dihydroquinoxalin-1(2H)-yl)-2-(4-methylpiperazin-1-yl)propan-1-one

1-(4-(2-Chlorobenzyl)-3,4-dihydroquinoxalin-1(2H)-yl)-2-(4-methylpiperazin-1-yl)propan-1-one was synthesized in the same manner as in Example 1-90, except that 1-methylpiperazine was used instead of pyrrolidine.
White solid (yield 63.2%); ¹H NMR (400 MHz, CD₃OD) δ 7.44 (d, 1H, *J* = 7.4 Hz), 7.31-7.19 (m, 2H), 7.16 (d, 2H, *J* = 6.7 Hz), 7.03 (d, 1H, *J =* 8.2 Hz), 6.71-6.63 (m, 1H), 6.59 (d, 1H, *J =* 8.2 Hz), 4.66 (s, 2H), 4.26-4.18 (m, 1H), 4.14 (d, 1H, *J =* 6.3 Hz), 3.78-3.61 (m, 1H), 3.54-3.46 (m, 2H), 2.96-2.58 (m, 8H), 2.55 (s, 3H), 1.25 (d, 3H, *J* = 6.7 Hz); LC/MS ESI (+): 413.0 (M+1)

### <Example 1-93> Synthesis of 1-(4-(2-chlorobenzyl)-3,4-dihydroquinoxalin-1(2H)-yl)-2-(pyrrolidin-1-yl)ethan-1-one

1-(4-(2-Chlorobenzyl)-3,4-dihydroquinoxalin-1(2H)-yl)-2-(pyrrolidin-1-yl)ethan-1-one was synthesized in the same manner as in Example 84, except that 1-(2-chlorobenzyl)-1,2,3,4-tetrahydroquinoxaline obtained in Preparation Example e-7 was used instead of 1-benzyl-1,2,3,4-tetrahydroquinoxaline.
Yellow oil (yield 43.4%); ¹H NMR (400 MHz, CD₃OD) δ 7.43-7.41 (m, 1H), 7.30-7.18 (m, 2H), 7.14 (d, 2H, *J* = 6.7 Hz), 7.05-6.93 (m, 1H), 6.66-6.62 (m, 1H), 6.53 (d, 1H, *J =* 8.2 Hz), 4.63 (s, 2H), 3.96 (t, 2H, *J* = 5.3 Hz), 3.57 (s, 2H), 3.53-3.47 (m, 2H), 2.67-2.59 (m, 4H), 1.84-1.76 (m, 4H); LC/MS ESI (+): 370.1 (M+1)

### <Example 1-94> Synthesis of 1-(4-(2-chlorobenzyl)-3,4-dihydroquinoxalin-1(2H)-yl)-2-(piperidin-1-yl)ethan-1-one

1-(4-(2-Chlorobenzyl)-3,4-dihydroquinoxalin-1(2H)-yl)-2-(piperidin-1-yl)ethan-1-one was synthesized in the same manner as in Example 1-93, except that piperidine was used instead of pyrrolidine.
Yellow solid (yield 41.5%); ¹H NMR (400 MHz, CD₃OD) δ 7.42 (d, 1H, *J* = 7.8 Hz), 7.27-7.19 (m, 2H), 7.14 (d, 2H, *J =* 7.0 Hz), 7.04-6.94 (m, 1H), 6.65-6.61 (m, 1H), 6.53 (d, 1H, *J* = 8.2 Hz), 4.63 (s, 2H), 4.03-3.91 (m, 2H), 3.57-3.43(m, 2H), 3.37 (s, 2H), 1.66-1.52 (m, 4H), 1.44 (m, 2H); LC/MS ESI (+): 384.0 (M+1)

### <Example 1-95> Synthesis of 1-(4-(2-chlorobenzyl)-3,4-dihydroquinoxalin-1(2H)-yl)-2-(4-methylpiperazin-1-yl)ethan-1-one

1-(4-(2-Chlorobenzyl)-3,4-dihydroquinoxalin-1(2H)-yl)-2-(4-methylpiperazin-1-yl)ethan-1-one was synthesized in the same manner as in Example 1-93, except that 1-methylpiperazine was used instead of pyrrolidine.
White solid (yield 39.1%); ¹H NMR (400 MHz, CD₃OD) δ 7.46-7.39 (m, 1H), 7.30-7.19 (m, 2H), 7.14 (d, 2H, *J* = 6.7 Hz), 7.03-6.95 (m, 1H), 6.65-6.61 (m, 1H), 6.54 (d, 1H, *J =* 8.2 Hz), 4.63 (s, 2H), 3.97 (t, 2H, *J* = 5.3 Hz), 3.55-3.45 (m, 2H), 3.42 (s, 2H), 2.66-2.39 (m, 8H), 2.26 (s, 3H); LC/MS ESI (+): 399.1 (M+1)

### <Example 1-96> Synthesis of 1-(4-(3-chlorobenzyl)-3,4-dihydroquinoxalin-1 (2H)-yl)-3-(pyrrolidin-1-yl)propan-1-one

1-(3-Chlorobenzyl)-1,2,3,4-tetrahydroquinoxaline (1.0 equiv) obtained in Preparation Example e-8 was dissolved in dichloromethane (0.3 M), 3-bromopropionyl chloride (1.5 equiv) was slowly added at -70 °C, and then stirred at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained concentrate was dissolved in THF (0.3 M), TEA (3.0 equiv) and pyrrolidine (1.0 equiv) were added, and then the resulting solution was stirred at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-(3-chlorobenzyl)-3,4-dihydroquinoxalin-1(2H)-yl)-3-(pyrrolidin-1-yl)propan-1-one.
Pale yellow oil (yield 27.1%); ¹H NMR (400 MHz, CD₃OD) δ 7.33-7.21 (m, 3H), 7.20-7.10 (m, 2H), 7.07-6.99 (m, 1H), 6.73-6.62 (m, 2H), 4.58 (s, 2H), 3.97-3.89 (m, 2H), 3.48 (t, 2H, *J* = 5.9 Hz), 2.89-2.71 (m, 4H), 2.59-2.35 (m, 4H), 1.86-1.66 (m, 4H); LC/MS ESI (+): 384.0 (M+1)

### <Example 1-97> Synthesis of 1-(4-(3-chlorobenzyl)-3,4-dihydroquinoxalin-1(2H)-yl)-3-(piperidin-1-yl)propan-1-one

1-(4-(3-Chlorobenzyl)-3,4-dihydroquinoxalin-1(2H)-yl)-3-(piperidin-1-yl)propan-1-one was synthesized in the same manner as in Example 1-96, except that piperidine was used instead of pyrrolidine.
Pale yellow oil (yield 33.1%); ¹H NMR (400 MHz, CD₃OD) δ 7.33-7.27 (m, 1H), 7.24 (dd, 2H, *J* = 4.9, 1.8 Hz), 7.20-7.11 (m, 2H), 7.08-6.98 (m, 1H), 6.73-6.62 (m, 2H), 4.58 (s, 2H), 3.92 (t, 2H, *J* = 5.5 Hz), 3.48 (t, 2H, *J* = 5.3 Hz), 2.86-2.76 (m, 2H), 2.74-2.58 (m, 2H), 2.53-2.23 (m, 4H), 1.64-1.48 (m, 4H), 1.48-1.35 (m, 2H); LC/MS ESI (+): 398.0 (M+1)

### <Example 1-98> Synthesis of 1-(4-(3-chlorobenzyl)-3,4-dihydroquinoxalin-1(2H)-yl)-3-(4-methylpiperazin-1-yl)propan-1-one

1-(4-(3-Chlorobenzyl)-3,4-dihydroquinoxalin-1(2H)-yl)-3-(4-methylpiperazin-1-yl)propan-1-one was synthesized in the same manner as in Example 1-96, except that 1-methylpiperazine was used instead of pyrrolidine.
Light brown solid (yield 41.2%); ¹H NMR (400 MHz, CDCl₃) δ 7.25-7.23 (m, 1H), 7.19 (s, 1H), 7.09 (dd, 1H, *J =* 8.4, 2.2 Hz), 7.04-6.98 (m, 1H), 6.69-6.63 (m, 1H), 6.61 (dd, 1H, *J* = 8.0, 1.0 Hz), 4.52 (s, 2H), 3.96 (t, 2H, *J* = 5.7 Hz), 3.45 (t, 2H, *J* = 5.5 Hz), 2.82-2.69 (m, 4H), 2.68-2.31 (m, 8H), 2.28 (s, 3H); LC/MS ESI (+): 413.0 (M+1)

### <Example 1-99> Synthesis of 1-(4-(3-chlorobenzyl)-3,4-dihydroquinoxalin-1(2H)-yl)-2-(pyrrolidin-1-yl)propan-1-one

1-(4-(3-Chlorobenzyl)-3,4-dihydroquinoxalin-1(2H)-yl)-2-(pyrrolidin-1-yl)propan-1-one was synthesized in the same manner as in Example 1-96, except that 2-bromopropionyl chloride was used instead of 3-bromopropionyl chloride.
Pale yellow oil (yield 26.2%); ¹H NMR (400 MHz, CD₃OD) δ 7.34-7.15 (m, 4H), 7.11-6.99 (m, 2H), 6.81-6.73 (m, 1H), 6.71-6.62 (m, 1H), 4.72-4.62 (m, 1H), 4.56-4.44 (m, 1H), 4.33-4.18 (m, 1H), 3.96-3.83 (m, 1H), 3.70-3.54 (m, 1H), 3.53-3.42 (m, 2H), 2.75-2.41 (m, 4H), 1.85-1.65 (m, 4H), 1.39-1.26 (m, 3H); LC/MS ESI (+): 384.1 (M+1)

### <Example 1-100> Synthesis of 1-(4-(3-chlorobenzyl)-3,4-dihydroquinoxalin-1(2H)-yl)-2-(piperidin-1-yl)propan-1-one

1-(4-(3-Chlorobenzyl)-3,4-dihydroquinoxalin-1(2H)-yl)-2-(piperidin-1-yl)propan-1-one was synthesized in the same manner as in Example 1-99, except that piperidine was used instead of pyrrolidine.
Pale yellow oil (yield 10.0%); ¹H NMR (400 MHz, CD₃OD) δ 7.32-7.21 (m, 3H), 7.20-7.15 (m, 1H), 7.15-7.01 (m, 2H), 6.78-6.71 (m, 1H), 6.70-6.62 (m, 1H), 4.71-4.44 (m, 3H), 4.40-4.23 (m, 1H), 4.06-3.95 (m, 1H), 3.92-3.75 (m, 1H), 3.57-3.42 (m, 2H), 2.59-2.34 (m, 4H), 1.60-1.43 (m, 4H), 1.42-1.33 (m, 2H), 1.26-1.15 (m, 3H); LC/MS ESI (+): 398.0 (M+1)

### <Example 1-101> Synthesis of 1-(4-(3-chlorobenzyl)-3,4-dihydroquinoxalin-1(2H)-yl)-2-(4-methylpiperazin-1-yl)propan-1-one

1-(4-(3-Chlorobenzyl)-3,4-dihydroquinoxalin-1(2H)-yl)-2-(4-methylpiperazin-1-yl)propan-1-one was synthesized in the same manner as in Example 1-99, except that 1-methylpiperazine was used instead of pyrrolidine.
Pale yellow solid (yield 32.0%); ¹H NMR (400 MHz, CD₃OD) δ 7.33-7.22 (m, 3H), 7.21-7.16 (m, 1H), 7.16-7.09 (m, 1H), 7.09-7.01 (m, 1H), 6.77-6.70 (m, 1H), 6.70-6.61 (m, 1H), 4.68-4.51 (m, 2H), 4.35-4.20 (m, 1H), 4.14-4.00 (m, 1H), 3.97-3.74 (m, 1H), 3.63-3.43 (m, 3H), 2.70-2.28 (m, 8H), 2.27-2.19 (m, 3H), 1.25-1.19 (m, 3H); LC/MS ESI (+): 413.0 (M+1)

### <Example 1-102> Synthesis of 1-(4-(3-chlorobenzyl)-3,4-dihydroquinoxalin-1(2H)-yl)-2-(pyrrolidin-1-yl)ethan-1-one

1-(4-(3-Chlorobenzyl)-3,4-dihydroquinoxalin-1(2H)-yl)-2-(pyrrolidin-1-yl)ethan-1-one was synthesized in the same manner as in Example 1-96, except that bromoacetyl chloride was used instead of 3-bromopropionyl chloride.
Pale yellow solid (yield 16.9%); ¹H NMR (400 MHz, CD₃OD) δ 7.31-7.21 (m, 2H), 7.17 (s, 1H), 7.13-7.03 (m, 2H), 6.94 (d, 1H, *J* = 8.6 Hz), 6.72-6.62 (m, 2H), 4.53 (s, 2H), 4.05 (s, 2H), 4.01-3.91 (m, 2H), 3.49 (t, 2H, *J* = 5.5 Hz), 3.45-3.21 (m, 4H), 2.18-2.04 (m, 4H); LC/MS ESI (+): 370.1 (M+1)

### <Example 1-103> Synthesis of 1-(4-(3-chlorobenzyl)-3,4-dihydroquinoxalin-1(2H)-yl)-2-(piperidin-1-yl)ethan-1-one

1-(4-(3-Chlorobenzyl)-3,4-dihydroquinoxalin-1(2H)-yl)-2-(piperidin-1-yl)ethan-1-one was synthesized in the same manner as in Example 1-102, except that piperidine was used instead of pyrrolidine.
Pale yellow oil (yield 62.4%); ¹H NMR (400 MHz, CD₃OD) δ 7.36-6.86 (m, 6H), 6.74-6.68 (m, 1H), 6.67-6.60 (m, 1H), 4.63-4.53 (m, 2H), 3.99-3.88 (m, 2H), 3.60-3.42 (m, 2H), 3.40-3.33 (m, 2H), 2.55-2.34 (m, 4H), 1.63-1.51 (m, 4H), 1.48-1.37 (m, 2H); LC/MS ESI (+): 384.1 (M+1)

### <Example 1-104> Synthesis of 1-(4-(3-chlorobenzyl)-3,4-dihydroquinoxalin-1(2H)-yl)-2-(4-methylpiperazin-1-yl)ethan-1-one

1-(4-(3-Chlorobenzyl)-3,4-dihydroquinoxalin-1(2H)-yl)-2-(4-methylpiperazin-1-yl)ethan-1-one was synthesized in the same manner as in Example 1-102, except that 1-methylpiperazine was used instead of pyrrolidine.

White solid (yield 37.1%); ¹H NMR (400 MHz, CD₃OD) δ 7.34-7.20 (m, 3H), 7.20-6.91 (m, 3H), 6.74-6.68 (m, 1H), 6.67-6.59 (m, 1H), 4.61-4.55 (m, 2H), 3.97-3.89 (m, 2H), 3.56-3.44 (m, 2H), 3.44-3.38 (m, 2H), 2.81-2.32 (m, 8H), 2.31-2.23 (m, 3H); LC/MS ESI (+): 399.1 (M+1),

### <Example 1-105> Synthesis of 1-(4-(4-chlorobenzyl)-3,4-dihydroquinoxalin-1(2H)-yl)-3-(pyrrolidin-1-yl)propan-1-one

1-(4-(4-Chlorobenzyl)-3,4-dihydroquinoxalin-1(2H)-yl)-3-(pyrrolidin-1-yl)propan-1-one was synthesized in the same manner as in Example 1-96, except that 1-(4-chlorobenzyl)-1,2,3,4-tetrahydroquinoxaline obtained in Preparation Example e-9 was used instead of 1-(3-chlorobenzyl)-1,2,3,4-tetrahydroquinoxaline.
Colorless oil (yield 63.9%); ¹H NMR (400 MHz, CDCl₃) δ 7.33-7.27 (m, 2H), 7.14 (d, 2H, *J* = 8.1 Hz), 7.10-6.96 (m, 2H), 6.70-6.57 (m, 2H), 4.50 (s, 2H), 3.95 (t, 2H, *J* = 5.5 Hz), 3.43 (t, 2H, *J* = 5.4 Hz), 2.87-2.75 (m, 4H), 2.49-2.39 (m, 4H), 1.81-1.60 (m, 4H); LC/MS ESI (+): 384.2 (M+1)

### <Example 1-106> Synthesis of 1-(4-(4-chlorobenzyl)-3,4-dihydroquinoxalin-1(2H)-yl)-3-(piperidin-1-yl)propan-1-one

1-(4-(4-Chlorobenzyl)-3,4-dihydroquinoxalin-1(2H)-yl)-3-(piperidin-1-yl)propan-1-one was synthesized in the same manner as in Example 1-105, except that piperidine was used instead of pyrrolidine.
Colorless oil (yield 78.6%); ¹H NMR (400 MHz, CDCl₃) δ 7.29 (d, 2H, *J* = 8.3 Hz), 7.14 (d, 2H, *J* = 8.2 Hz), 7.11-7.03 (m, 1H), 7.03-6.95 (m, 1H), 6.69-6.57 (m, 2H), 4.50 (s, 2H), 3.94 (d, 2H, *J* = 5.7 Hz), 3.43 (t, 2H, *J* = 5.5 Hz), 2.75 (d, 2H, *J* = 7.0 Hz), 2.69 (d, 2H, *J* = 7.2 Hz), 2.37-2.27 (m, 3H), 1.66-1.35 (m, 7H); LC/MS ESI (+): 398.1 (M+1)

### <Example 1-107> Synthesis of 1-(4-(4-chlorobenzyl)-3,4-dihydroquinoxalin-1(2H)-yl)-3-(4-methylpiperazin-1-yl)propan-1-one

1-(4-(4-Chlorobenzyl)-3,4-dihydroquinoxalin-1(2H)-yl)-3-(4-methylpiperazin-1-yl)propan-1-one was synthesized in the same manner as in Example 1-105, except that 1-methylpiperazine was used instead of pyrrolidine.
Colorless oil (yield 89%); ¹H NMR (400 MHz, CDCl₃) δ 7.29 (d, 2H, *J* = 8.2 Hz), 7.14 (d, 2H, *J* = 8.2 Hz), 7.10-6.96 (m, 2H), 6.69-6.57 (m, 2H), 4.51 (s, 2H), 4.00-3.95 (m, 2H), 3.43 (t, 2H, *J* = 5.4 Hz), 2.74 (d, 3H, *J* = 4.6 Hz), 2.53-2.21 (m, 10H), 1.69-1.57 (m, 2H); LC/MS ESI (+): 413.1 (M+1)

### <Example 1-108> Synthesis of 1-(4-(4-chlorobenzyl)-3,4-dihydroquinoxalin-1(2H)-yl)-2-(pyrrolidin-1-yl)propan-1-one

1-(4-(4-Chlorobenzyl)-3,4-dihydroquinoxalin-1(2H)-yl)-2-(pyrrolidin-1-yl)propan-1-one was synthesized in the same manner as in Example 1-105, except that 2-bromopropionyl chloride was used instead of 3-bromopropionyl chloride.
Brown oil (yield 30.2%); ¹H NMR (400 MHz, CD₃OD) δ 7.33-7.19 (m, 4H), 7.06 (s, 2H), 6.80 (d, 1H, *J* = 8.3 Hz), 6.70-6.61 (m, 1H), 4.72-4.57 (m, 1H), 4.57-4.43 (m, 1H), 4.30-4.18 (m, 1H), 3.88 (d, 1H, *J* = 7.0 Hz), 3.63-3.40 (m, 3H), 2.56-2.39 (m, 4H), 1.81-1.61 (m, 4H), 1.30 (d, 3H, *J* = 6.7 Hz); LC/MS ESI (+): 383.9 (M+1)

### <Example 1-109> Synthesis of 1-(4-(4-chlorobenzyl)-3,4-dihydroquinoxalin-1(2H)-yl)-2-(piperidin-1-yl)propan-1-one

1-(4-(4-Chlorobenzyl)-3,4-dihydroquinoxalin-1(2H)-yl)-2-(piperidin-1-yl)propan-1-one was synthesized in the same manner as in Example 1-108, except that piperidine was used instead of pyrrolidine.
Pale yellow oil (yield 68%); ¹H NMR (400 MHz, CD₃OD) δ 7.34-7.27 (m, 2H), 7.24 (d, 2H, *J* = 8.3 Hz), 7.14-7.01 (m, 2H), 6.76 (d, 1H, *J* = 8.2 Hz), 6.68-6.57 (m, 1H), 4.72-4.57 (m, 1H), 4.57-4.43 (m, 1H), 4.37-4.25 (m, 1H), 4.01 (d, 1H, *J* = 7.2 Hz), 3.53-3.42 (m, 3H), 2.57-2.21 (m, 5H), 1.58-1.16 (m, 8H); LC/MS ESI (+): 397.8 (M+1)

### <Example 1-110> Synthesis of 1-(4-(4-chlorobenzyl)-3,4-dihydroquinoxalin-1(2H)-yl)-2-(4-methylpiperazin-1-yl)propan-1-one

1-(4-(4-Chlorobenzyl)-3,4-dihydroquinoxalin-1(2H)-yl)-2-(4-methylpiperazin-1-yl)propan-1-one was synthesized in the same manner as in Example 1-108, except that 1-methylpiperazine was used instead of pyrrolidine.
Pale yellow oil (yield 31%); ¹H NMR (400 MHz, CD₃OD) δ 7.35-7.21 (m, 4H), 7.14-7.01 (m, 2H), 6.78 (d, 1H, *J* = 8.3 Hz), 6.68-6.60 (m, 1H), 4.73-4.58 (m, 1H), 4.57-4.45 (m, 1H), 4.39-4.23 (m, 1H), 4.03 (d, 1H, *J* = 6.9 Hz), 3.49 (d, 2H, *J* = 5.0 Hz), 2.68-2.28 (m, 9H), 2.23 (s, 3H), 1.23 (d, 3H, *J* = 6.7 Hz); LC/MS ESI (+): 413.1 (M+1)

### <Example 1-111> Synthesis of 1-(4-(4-chlorobenzyl)-3,4-dihydroquinoxalin-1(2H)-yl)-2-(pyrrolidin-1-yl)ethan-1-one

1-(4-(4-Chlorobenzyl)-3,4-dihydroquinoxalin-1(2H)-yl)-2-(pyrrolidin-1-yl)ethan-1-one was synthesized in the same manner as in Example 1-106, except that bromoacetyl chloride was used instead of 3-bromopropionyl chloride.
Pale yellow oil (yield 52%); ¹H NMR (400 MHz, CD₃OD) δ 7.36-7.27 (m, 2H), 7.23-7.17 (m, 3H), 7.10-6.95 (m, 1H), 6.74-6.59 (m, 2H), 4.57 (s, 2H), 3.92 (t, 2H, *J =* 5.4 Hz), 3.56 (s, 2H), 3.53-3.44 (m, 2H), 2.69-2.55 (m, 4H), 1.83-1.75 (m, 4H); LC/MS ESI (+): 369.9 (M+1)

### <Example 1-112> Synthesis of 1-(4-(4-chlorobenzyl)-3,4-dihydroquinoxalin-1(2H)-yl)-2-(piperidin-1-yl)ethan-1-one

1-(4-(4-Chlorobenzyl)-3,4-dihydroquinoxalin-1(2H)-yl)-2-(piperidin-1-yl)ethan-1-one was synthesized in the same manner as in Example 1-111, except that piperidine was used instead of pyrrolidine.
Pale yellow oil (yield 64.9%); ¹H NMR (400 MHz, CD₃OD) δ 7.30 (d, 2H, *J* = 8.4 Hz), 7.26-7.08 (m, 3H), 7.08-6.90 (m, 1H), 6.71 ( d, 1H, *J* = 8.3 Hz), 6.67-6.59 (m, 1H), 4.57 (s, 2H), 3.94 (t, 2H, *J* = 5.5 Hz), 3.56-3.42 (m, 2H), 3.35 (s, 2H), 2.51-2.35 (m, 4H), 1.63-1.52 (m, 4H), 1.49-1.35 (m, 2H); LC/MS ESI (+): 383.8 (M+1)

### <Example 1-113> Synthesis of 1-(4-(4-chlorobenzyl)-3,4-dihydroquinoxalin-1(2H)-yl)-2-(4-methylpiperazin-1-yl)ethan-1-one

1-(4-(4-Chlorobenzyl)-3,4-dihydroquinoxalin-1(2H)-yl)-2-(4-methylpiperazin-1-yl)ethan-1-one was synthesized in the same manner as in Example 1-111, except that 1-methylpiperazine was used instead of pyrrolidine.
Pale yellow oil (yield 43.9%); ¹H NMR (400 MHz, CD₃OD) δ 7.39-6.90 (m, 5H), 6.72 (d, 1H, *J* = 8.3 Hz), 6.67-6.58 (m, 1H), 4.57 (s, 2H), 3.93 (t, 2H, *J* = 5.4 Hz), 3.52-3.43 (m, 2H), 3.40 (s, 2H), 2.61-2.23 (m, 11H); LC/MS ESI (+): 398.8 (M+1)

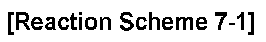

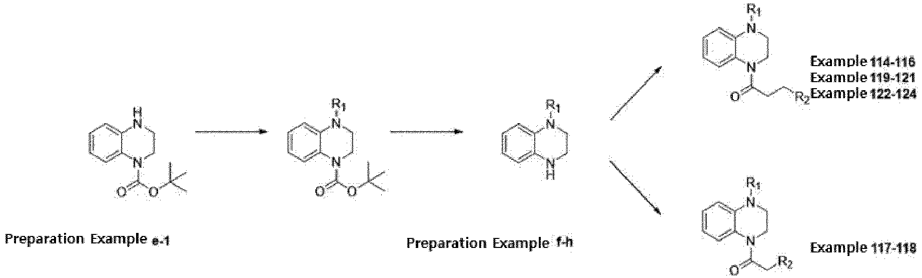

### <Preparation Method f> Preparation of 1-(pyridin-2-ylmethyl)-1,2,3,4-tetrahydroquinoxaline

A 2-methylpyridine group was introduced to tert-butyl 3,4-dihydroquinoxalin-1(2H)-carboxylate obtained in Preparation Example e-1 while stirring 2-(bromomethyl)pyridine hydrobromide, DIPEA, NaOH, and THF at room temperature for 1 hour, and then a BOC group was deprotected with TFA, thereby preparing 1-(pyridin-2-ylmethyl)-1,2,3,4-tetrahydroquinoxaline.
Light brown solid (yield 86.7%); ¹H NMR (400 MHz, CD₃CN) δ 8.55 (dd,1H, *J* = 5.1, 2.0 Hz), 7.69 (td, 1H, *J* = 7.6, 2.0 Hz), 7.31 (d, 1H, *J* = 7.8 Hz), 7.21 (dd, 1H, *J* = 7.6, 4.5 Hz), 6.51-6.39 (m, 3H), 6.38-6.33 (m, 1H), 4.51 (s, 2H), 3.57-3.35 (m, 5H); LC/MS ESI (+): 226.1 (M+1)

### <Preparation Method g> Preparation of 1-(pyridin-3-ylmethyl)-1,2,3,4-tetrahydroquinoxaline

A 3-methylpyridine group was introduced to tert-butyl 3,4-dihydroquinoxalin-1(2H)-carboxylate obtained in Preparation Example e-1 while stirring sodium triacetoxyborohydride, 3-pyridinecarboxalde, and dichloromethane at 60 °C for 4 hours, and then a BOC group was deprotected with TFA, thereby preparing 1-(pyridin-3-ylmethyl)-1,2,3,4-tetrahydroquinoxaline.
Light brown solid (yield 64.7%); ¹H NMR (400 MHz, CD₃CN) δ 8.56-8.52 (m, 1H), 8.47 (dd, 1H, *J* = 4.7, 1.6 Hz), 7.68 (td, 1H, *J =* 7.9, 2.3 Hz), 7.30 (dd, 1H, *J =* 7.8, 4.7 Hz), 6.52-6.43 (m, 4H)t, 4.45 (s, 2H), 4.34 (brs, 1H), 3.39 (s, 4H); LC/MS ESI (+): 226.1 (M+1)

### <Preparation Method h> Preparation of 1-(pyridin-4-ylmethyl)-1,2,3,4-tetrahydroquinoxaline

1-(Pyridin-3-ylmethyl)-1,2,3,4-tetrahydroquinoxaline was synthesized in the same manner as in Preparation Method C, except that 4-pyridinecarboxalde was used instead of 3-pyridinecarboxalde.
Light brown solid (yield 87.0%); ¹H NMR (400 MHz, CD₃CN) δ 8.52-8.48 (m, 2H), 7.31-7.25 (m, 2H), 6.52-6.42 (m, 3H), 6.36-6.29 (m, 1H), 4.45 (s, 2H), 3.42 (s, 5H); LC/MS ESI (+): 226.1 (M+1)

### <Example 1-114> Synthesis of 1-(4-(pyridin-2-ylmethyl)-3,4-dihydroquinoxalin-1(2H)-yl)-3-(pyrrolidin-1-yl)propan-1-one di-oxalate

1-(Pyridin-2-ylmethyl)-1,2,3,4-tetrahydroquinoxaline (1.0 equiv) obtained in Preparation Method f was dissolved in dichloromethane (0.3 M), 3-bromopropionyl chloride (1.2 equiv) was slowly added thereto at -70 °C, and then the resulting solution was stirred at room temperature for 1.5 hours. TEA (3.0 equiv) and pyrrolidine (1.1 equiv) were added to the reaction solution and then stirred at 70 °C for 16 hours. After cooling to room temperature and terminating the reaction by adding water, the reaction solution was extracted with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-(pyridin-2-ylmethyl)-3,4-dihydroquinoxalin-1(2H)-yl)-3-(pyrrolidin-1-yl)propan-1-one. The obtained 1-(4-(pyridin-2-ylmethyl)-3,4-dihydroquinoxalin-1(2H)-yl)-3-(pyrrolidin-1-yl)propan-1-one (1.0 equiv) was dissolved in isopropyl alcohol (0.5M) and stirred at room temperature for 10 minutes. Oxalic acid (2.0 equiv) was added to the prepared reaction solution and then stirred at room temperature for 1 hour. The resulting solid was filtered, washed, and dried, thereby synthesizing 1-(4-(pyridin-2-ylmethyl)-3,4-dihydroquinoxalin-1(2H)-yl)-3-(pyrrolidin-1-yl)propan-1-one di-oxalate.
Yellow solid (yield 40.1%); ¹H NMR (400 MHz, CD₃OD) δ 8.61-8.55 (m, 1H), 7.97-7.88 (m, 1H), 7.49-7.39 (m, 2H), 7.21 (d, 1H, *J* = 8.0 Hz), 7.11-6.96 (m, 1H), 6.77-6.58 (m, 2H), 4.74 (s, 2H), 4.07-3.90 (m, 2H), 3.80-3.45 (m, 6H), 3.25-2.93 (m, 4H), 2.28-1.92 (m, 4H); LC/MS ESI (+): 351.1 (M+1)

### <Example 1-115> Synthesis of 3-(piperidin-1-yl)-1-(4-(pyridin-2-ylmethyl)-3,4-dihydroquinoxalin-1(2H)-yl)propan-1-one

1-(Pyridin-2-ylmethyl)-1,2,3,4-tetrahydroquinoxaline (1.0 equiv) obtained in Preparation Method f was dissolved in dichloromethane (0.3 M), and 3-bromopropionyl chloride (1.2 equiv) was slowly added thereto at -70 °C, and then the resulting solution was stirred at room temperature for 1.5 hours. TEA (3.0 equiv) and piperidine (1.1 equiv) were added to the reaction solution and stirred at 70 °C for 16 hours. After cooling to room temperature and terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 3-(piperidin-1-yl)-1-(4-(pyridin-2-ylmethyl)-3,4-dihydroquinoxalin-1(2H)-yl)propan-1-one.
Colorless oil (yield 31.1%); ¹H NMR (400 MHz, CD₃CN) δ 8.55 (dd, 1H, *J* = 5.3, 1.8 Hz), 7.79-7.75 (m, 1H), 7.26-7.09 (m, 3H), 6.58-6.52 (m, 5H), 4.63 (s, 2H), 3.90 (t, 2H, *J* = 5.3 Hz), 3.63-3.53 (m, 2H), 2.73-2.66 (m, 2H), 2.65-2.52 (m, 2H), 2.48-2.12 (m, 10H); LC/MS ESI (+): 365.1 (M+1)

### <Example 1-116> Synthesis of 3-(4-methylpiperazin-1-yl)-1-(4-(pyridin-2-ylmethyl)-3,4-dihydroquinoxalin-1(2H)-yl)propan-1-one

3-(4-Methylpiperazin-1-yl)-1-(4-(pyridin-2-ylmethyl)-3,4-dihydroquinoxalin-1(2H)-yl)propan-1-one was synthesized in the same manner as in Example 1-115, except that 1-methylpiperazine was used instead of piperidine.
Colorless oil (yield 37.3%); ¹H NMR (400 MHz, CD₃CN) δ 8.56 (dd, 1H, *J =* 5.3, 2.2 Hz), 7.74-7.65 (m, 1H), 7.27-7.20 (m, 2H), 7.15 (s, 1H), 7.01-6.91 (m, 1H), 6.66-6.57 (m, 2H), 4.63 (s, 2H), 3.90 (t, 2H, *J* = 5.3 Hz), 3.58 (t, 2H, *J* = 5.5 Hz), 2.71-2.64 (m, 2H), 2.63-2.53 (m, 2H), 2.48-2.23 (m, 8H), 2.19 (s, 3H); LC/MS ESI (+): 380.1 (M+1)

### <Example 1-117> Synthesis of 1-(4-(pyridin-2-ylmethyl)-3,4-dihydroquinoxalin-1(2H)-yl)-2-(pyrrolidin-1-yl)ethan-1-one

1-(4-(Pyridin-2-ylmethyl)-3,4-dihydroquinoxalin-1(2H)-yl)-2-(pyrrolidin-1-yl)ethan-1-one was synthesized in the same manner as in Example 1-115, except that bromoacetyl chloride was used instead of 3-bromopropionyl chloride.
Brown oil (yield 31.6%); ¹H NMR (400 MHz, CDCl₃) δ 8.59 (d, 1H, *J* = 4.3 Hz), 7.60 (td, 1H, *J* = 7.7, 1.8 Hz), 7.20-7.09 (m, 2H), 6.99 (t, 1H, *J* = 8.0 Hz), 6.70-6.57 (m, 2H), 4.64 (s, 2H), 4.00 (t, 2H, *J* = 5.5 Hz), 3.56 (t, 2H, *J* = 5.3 Hz), 3.50 (s, 2H), 2.69-2.57 (m, 4H), 1.85-1.74 (m, 4H); LC/MS ESI (+): 337.3 (M+1)

### <Example 1-118> Synthesis of 2-(4-methylpiperazin-1-yl)-1-(4-(pyridin-2-ylmethyl)-3,4-dihydroquinoxalin-1(2H)-yl)ethan-1-one

2-(4-Methylpiperazin-1-yl)-1-(4-(pyridin-2-ylmethyl)-3,4-dihydroquinoxalin-1(2H)-yl)ethan-1-one was synthesized in the same manner as in Example 1-117, except that 1-methylpiperazine was used instead of pyrrolidine.
Off-white oil (yield 29.8%); ¹H NMR (400 MHz, CDCl₃) δ 8.59 (d, 1H, *J* = 4.3 Hz), 7.65-7.57 (m, 1H), 7.21-7.09 (m, 2H), 7.03-6.95 (m, 1H), 6.70-6.58 (m, 2H), 4.64 (s, 2H), 3.99 (t, 2H, *J* = 5.3 Hz), 3.56 (s, 2H), 3.35 (s, 2H), 2.75-2.41 (m, 8H), 2.31 (s, 3H); LC/MS ESI (+): 366.3 (M+1)

### <Example 1-119> Synthesis of 1-(4-(pyridin-3-ylmethyl)-3,4-dihydroquinoxalin-1(2H)-yl)-3-(pyrrolidin-1-yl)propan-1-one

1-(4-(Pyridin-3-ylmethyl)-3,4-dihydroquinoxalin-1(2H)-yl)-3-(pyrrolidin-1-yl)propan-1-one was synthesized in the same manner as in Example 1-115, except that 1-(pyridin-3-ylmethyl)-1,2,3,4-tetrahydroquinoxaline was used instead of 1-(pyridin-2-ylmethyl)-1,2,3,4-tetrahydroquinoxaline.
White solid (yield 47.3%); ¹H NMR (400 MHz, CD₃OD) δ 8.49-8.40 (m, 2H), 7.73 (d, 1H, *J* = 7.8 Hz), 7.39 (dd, 1H, *J* = 7.8, 5.1 Hz), 7.15 (s, 1H), 7.04 (t, 1H, *J* = 7.0 Hz), 6.74 (d, 1H, *J* = 8.2 Hz), 6.68 (t, 1H, *J* = 7.2 Hz), 4.66 (s, 2H), 3.94 (t, 2H, *J* = 5.3 Hz), 3.51 (d, 2H, *J* = 4.3 Hz), 2.87-2.71 (m, 4H), 2.55-2.35 (m, 4H), 1.82-1.67 (m, 4H); LC/MS ESI (+): 351.3 (M+1)

### <Example 1-120> Synthesis of 3-(piperidin-1-yl)-1-(4-(pyridin-3-ylmethyl)-3,4-dihydroquinoxalin-1(2H)-yl)propan-1-one

3-(Piperidin-1-yl)-1-(4-(pyridin-3-ylmethyl)-3,4-dihydroquinoxalin-1(2H)-yl)propan-1-one was synthesized in the same manner as in Example 1-119, except that piperidine was used instead of pyrrolidine.
Colorless oil (yield 46.8%); ¹H NMR (400 MHz, CD₃CN) δ 8.53-8.45 (m, 2H), 7.62 (d, 1H, *J* = 7.8 Hz), 7.30 (dd, 1H, *J* = 8.0, 4.9 Hz), 7.27-7.09 (m, 1H), 7.04-6.94 (m, 1H), 6.72-6.61 (m, 2H), 4.60 (s, 2H), 3.89 (t, 2H, *J* = 5.3 Hz), 3.49 (t, 2H, *J* = 5.3 Hz), 2.70-2.63 (m, 2H), 2.60-2.49 (m, 2H), 2.35-2.20 (m, 4H), 1.52-1.43 (m, 4H), 1.39 (d, 2H, *J* = 4.7 Hz); LC/MS ESI (+): 365.3 (M+1)

### <Example 1-121> Synthesis of 3-(4-methylpiperazin-1-yl)-1-(4-(pyridin-3-ylmethyl)-3,4-dihydroquinoxalin-1(2H)-yl)propan-1-one

3-(4-Methylpiperazin-1-yl)-1-(4-(pyridin-3-ylmethyl)-3,4-dihydroquinoxalin-1(2H)-yl)propan-1-one was synthesized in the same manner as in Example 1-119, except that 1-methylpiperazine was used instead of pyrrolidine.
Off-white oil (yield 20.4%); ¹H NMR (400 MHz, CDCl₃) δ 8.55-8.48 (m, 2H), 7.53 (d, 1H, *J* = 7.8 Hz), 7.13-6.97 (m, 2H), 6.71-6.61 (m, 2H), 4.57 (s, 2H), 3.95 (t, 2H, *J* = 4.9 Hz), 3.45 (t, 2H, *J* = 5.3 Hz), 2.80-2.73 (m, 4H), 2.68-2.36 (m, 8H), 2.33 (s, 3H); LC/MS ESI (+): 380.3 (M+1)

### <Example 1-122> Synthesis of 1-(4-(pyridin-4-ylmethyl)-3,4-dihydroquinoxalin-1(2H)-yl)-3-(pyrrolidin-1-yl)propan-1-one

1-(4-(Pyridin-4-ylmethyl)-3,4-dihydroquinoxalin-1(2H)-yl)-3-(pyrrolidin-1-yl)propan-1-one was synthesized in the same manner as in Example 1-115, except that 1-(pyridin-4-ylmethyl)-1,2,3,4-tetrahydroquinoxaline was used instead of 1-(pyridin-2-ylmethyl)-1,2,3,4-tetrahydroquinoxaline.
White solid (yield 49.8%); ¹H NMR (400 MHz, CD₃CN) δ 8.53-8.48 (m, 2H), 7.25-7.10 (m, 3H), 7.02-6.93 (m, 1H), 6.69-6.61 (m, 1H), 6.56 (d, 1H, *J* = 8.2 Hz), 4.59 (s, 2H), 3.91 (t, 2H, *J* = 5.3 Hz), 3.49 (t, 2H, *J* = 4.5 Hz), 2.73-2.67 (m, 4H), 2.45-2.28 (m, 4H),, 1.77-1.62 (m, 4H); LC/MS ESI (+): 351.3 (M+1)

### <Example 1-123> Synthesis of 3-(piperidin-1-yl)-1-(4-(pyridin-4-ylmethyl)-3,4-dihydroquinoxalin-1(2H)-yl)propan-1-one

3-(Piperidin-1-yl)-1-(4-(pyridin-4-ylmethyl)-3,4-dihydroquinoxalin-1(2H)-yl)propan-1-one was synthesized in the same manner as in Example 1-122, except that piperidine was used instead of pyrrolidine.
Colorless oil (yield 42.3%); ¹H NMR (400 MHz, CD₃CN) δ 8.53-8.48 (m, 2H), 7.22 (d, 2H, *J* = 5.9 Hz), 7.01-6.93 (m, 1H), 6.68-6.61 (m, 1H), 6.56 (d, 1H, *J* = 8.6 Hz), 4.59 (s, 2H), 3.91 (t, 2H, *J* = 5.3 Hz), 3.50 (t, 2H, *J* = 5.1 Hz), 2.71-2.64 (m, 2H), 2.61-2.50 (m, 2H), 2.42-2.19 (m, 4H), 1.54-1.44 (m, 4H), 1.39 (d, 2H, *J* = 5.1 Hz); LC/MS ESI (+): 365.3 (M+1)

### <Example 1-124> Synthesis of 3-(4-methylpiperazin-1-yl)-1-(4-(pyridin-4-ylmethyl)-3,4-dihydroquinoxalin-1(2H)-yl)propan-1-one

3-(4-Methylpiperazin-1-yl)-1-(4-(pyridin-4-ylmethyl)-3,4-dihydroquinoxalin-1(2H)-yl)propan-1-one was synthesized in the same manner as in Example 1-122, except that 1-methylpiperazine was used instead of pyrrolidine.
Colorless oil (yield 8.4%); ¹H NMR (400 MHz, CDCl₃) δ 8.58-8.52 (m, 2H), 7.14 (d, 2H, *J =* 5.9 Hz), 7.12-7.04 (m, 1H), 7.03-6.96 (m, 1H), 6.71-6.64 (m, 1H), 6.53-6.48 (m, 1H), 4.54 (s, 2H), 3.98 (t, 2H, *J* = 5.1 Hz), 3.48 (t, 2H, *J* = 5.3 Hz), 2.82-2.69 (m, 4H), 2.60-2.30 (m, 8H), 2.27 (s, 3H); LC/MS ESI (+): 380.3 (M+1)

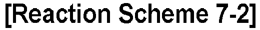

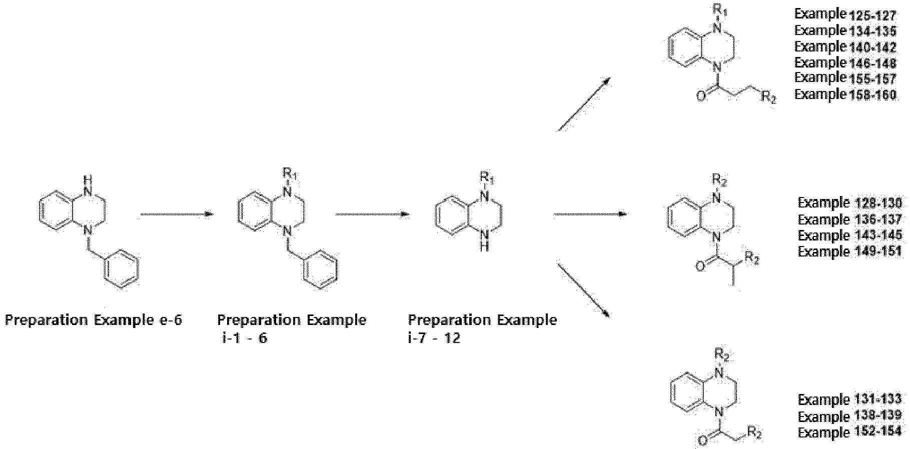

### <Preparation Method i> Preparation of chloro-substituted phenyl or pyrindin-1,2,3,4-tetrahydroquinoxaline

Chloro-substituted phenyl or pyrindin-1,2,3,4-tetrahydroquinoxaline according to the present invention was prepared through the processes of Preparation Examples i-1 to i-12 below.

### <Preparation Example i-1> Preparation of 1-benzyl-4-(2-chlorophenyl)-1,2,3,4-tetrahydroquinoxaline

1-Benzyl-1,2,3,4-tetrahydroquinoxaline (1.0 equiv) obtained in Preparation Example e-6, sodium butoxide (2.0 equiv), and 1-chloro-2-iodobenzene (3.0 equiv) were sufficiently dissolved in toluene (0.3M). The reaction solution was heated at 110 °C for 5 minutes, a mixture in which Pd₂dba (0.05 equiv) and Xphos (0.15 equiv) were dissolved in toluene (0.6M) was slowly added to the reaction solution and stirred at 110 °C for 15 hours. The reaction solution was cooled to room temperature and filtered through a Celite pad, water was added, and then extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-benzyl-4-(2-chlorophenyl)-1,2,3,4-tetrahydroquinoxaline.
Colorless oil (yield 99%); ¹H NMR (400 MHz, DMSO-*d*₆) δ 7.57 (dd, 1H, *J* = 8.0, 1.4 Hz), 7.47-7.31 (m, 6H), 7.30-7.21 (m, 2H), 6.61-6.49 (m, 2H), 6.37 (td, 1H, *J* = 7.4, 2.0 Hz), 5.99 (dd, 1H, *J* = 8.0, 1.0 Hz), 3.72-3.59 (m, 2H), 3.57-3.42 (m, 2H); LC/MS ESI (+): 335.1 (M+1)

### <Preparation Example i-2> Preparation of 1-benzyl-4-(3-chlorophenyl)-1,2,3,4-tetrahydroquinoxaline

1-Benzyl-4-(3-chlorophenyl)-1,2,3,4-tetrahydroquinoxaline was prepared in the same manner as in Preparation Example i-1, except that 1-chloro-3-iodobenzene was used instead of 1-chloro-2-iodobenzene.
Brown oil (yield 94%); ¹H-NMR (400 MHz, DMSO-*d*₆) δ 7.33-7.20 (m, 6H), 7.08-7.04 (m, 2H), 6.93 (dd, 1H, *J* = 7.8, 1.2 Hz), 6.81 (dd, 1H, *J* = 7.8, 1.2 Hz), 6.75-6.60 (m, 1H), 6.66-6.62 (m, 1H), 6.50-6.45 (m, 1H), 4.53 (s, 2H), 3.70 (t, 2H, *J* = 4.7 Hz), 3.38 (t, 2H, *J* = 4.7 Hz); LC/MS ESI (+): 335.2 (M+1)

### <Preparation Example i-3> Preparation of 1-benzyl-4-(4-chlorophenyl)-1,2,3,4-tetrahydroquinoxaline

1-Benzyl-4-(4-chlorophenyl)-1,2,3,4-tetrahydroquinoxaline was prepared in the same manner as in Preparation Example i-1, except that 1-chloro-4-iodobenzene was used instead of 1-chloro-2-iodobenzene.
Red oil (yield 60.4 %); ¹H-NMR (400 MHz, DMSO-*d*₆) δ 7.37-7.19 (m, 7H), 7.17-7.08 (m, 2H), 6.77-6.66 (m, 2H), 6.63 (dd, 1H, *J* = 8.3, 1.5 Hz), 6.45 (ddd, 1H, *J* = 7.9, 7.1, 1.5 Hz), 4.53 (s, 2H), 3.72-3.65 (m, 2H), 3.41 (dd, 2H, *J* = 5.9, 3.8 Hz); LC/MS ESI (+): 335.1 (M+1)

### <Preparation Example i-4> Preparation of 1-benzyl-4-(pyridin-2-yl)-1,2,3,4-tetrahydroquinoxaline

1-Benzyl-4-(pyridin-2-yl)-1,2,3,4-tetrahydroquinoxaline was prepared in the same manner as in Preparation Example i-1, except that 2-bromopyridine was used instead of 1-chloro-2-iodobenzene.
Yellow solid (yield 73%); ¹H NMR (400 MHz, DMSO-*d*₆) δ 8.22 (dd, 1H, *J=* 5.1, 1.2 Hz), 7.53 (ddd, 1H, *J=* 8.7, 7.0, 2.0 Hz), 7.35-7.28 (m, 2H), 7.27-7.19 (m, 3H), 7.15 (dd, 1H, *J* = 7.8, 1.6 Hz), 7.03 (d, 1H, *J* = 8.6 Hz), 6.86 (td, 1H, *J* = 7.8, 1.6 Hz), 6.77 (dd, 1H, *J* = 7.0, 5.5 Hz), 6.70 (dd, 1H, *J* = 8.2, 1.2 Hz), 6.60-6.53 (m, 1H), 4.65-4.50 (m, 2H), 4.10-4.02 (m, 2H), 3.40 (t, 2H, *J* = 5.1 Hz); LC/MS ESI (+): 302.2 (M+1)

### <Preparation Example i-5> Preparation of 1-benzyl-4-(pyridin-3-yl)-1,2,3,4-tetrahydroquinoxaline

1-Benzyl-4-(pyridin-3-yl)-1,2,3,4-tetrahydroquinoxaline was prepared in the same manner as in Preparation Example i-1, except that 3-iodopyridine was used instead of 1-chloro-2-iodobenzene.
Yellow oil (yield 100%); ¹H NMR (400 MHz, CD₃CN) δ 8.46 (d, 1H, *J* = 2.7 Hz), 8.19 (dd, 1H, *J* = 4.7, 1.2 Hz), 7.50 (ddd, 1H, *J* = 8.4, 2.9, 1.6 Hz), 7.40-7.31 (m, 4H), 7.31-7.24 (m, 2H), 6.82 (dd, 1H, *J =* 8.2, 1.6 Hz), 6.78-6.72 (m, 1H), 6.70-6.65 (m, 1H), 6.55-6.49 (m, 1H), 4.57 (s, 2H), 3.80-3.74 (m, 2H), 3.52-3.46 (m, 2H); LC/MS ESI (+): 302.2 (M+1)

### <Preparation Example i-6> Preparation of 1-benzyl-4-(pyridin-4-yl)-1,2,3,4-tetrahydroquinoxaline

1-Benzyl-4-(pyridin-4-yl)-1,2,3,4-tetrahydroquinoxaline was prepared in the same manner as in Preparation Example i-1, except that 4-iodopyridine was used instead of 1-chloro-2-iodobenzene.
Red oil (yield 100%); ¹H NMR (400 MHz, DMSO-*d*₆) δ 8.26 - 8.19 (m, 2H), 7.31 (dd, 2H, *J* = 8.1, 6.8 Hz), 7.26 - 7.18 (m, 3H), 7.09 (dd, 1H, *J* = 7.9, 1.5 Hz), 7.05 - 6.99 (m, 2H), 6.89 (ddd, 1H, *J* = 8.6, 7.2, 1.5 Hz), 6.73 (dd, 1H, *J* = 8.3, 1.3 Hz), 6.62 - 6.53 (m, 1H), 4.56 (s, 2H), 3.84 (q, 2H, *J* = 5.9 Hz), 3.40 (t, 2H, *J* = 5.1 Hz); LC/MS ESI (+): 302.2 (M+1)

### <Preparation Example i-7> Preparation of 1-(2-chlorophenyl)-1,2,3,4-tetrahydroquinoxaline

A mixture in which 1-benzyl-4-(2-chlorophenyl)-1,2,3,4-tetrahydroquinoxaline (1.0 equiv) obtained in Preparation Example i-4 and Pd(OH)₂ (0.6 equiv) were dissolved in MeOH/THF (v/v = 2: 1, 0.3M) was stirred under hydrogen gas conditions at room temperature for 3 hours. The reaction solution was filtered through a Celite pad, water was added, and extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-(2-chlorophenyl)-1,2,3,4-tetrahydroquinoxaline.
Gray solid (yield 62%); ¹H NMR (400 MHz, DMSO-*d*₆) δ 7.55 (dd, 1H, *J* = 8.0, 1.4 Hz), 7.43-7.35 (m, 1H), 7.35-7.30 (m, 1H), 7.28-7.21 (m, 1H), 6.54-6.49 (m, 2H), 6.31 (ddd, 1 H, *J* = 8.2, 5.1, 3.5 Hz), 5.96 (d, 1H, *J* = 7.8 Hz), 3.54-3.46 (m, 2H), 3.35 (dd, 2H, *J* = 4.9, 2.2 Hz); LC/MS ESI (+): 245.1 (M+1)

### <Preparation Example i-8> Preparation of 1-(3-chlorophenyl)-1,2,3,4-tetrahydroquinoxaline

1-(3-Chlorophenyl)-1,2,3,4-tetrahydroquinoxaline was synthesized in the same manner as in Preparation Example i-6, except that 1-benzyl-4-(3-chlorophenyl)-1,2,3,4-tetrahydroquinoxaline obtained in Preparation Example h-2 was used instead of 1-benzyl-4-(2-chlorophenyl)-1,2,3,4-tetrahydroquinoxaline.
Orange oil (yield 90%); ¹H-NMR (400 MHz, DMSO-*d*₆) δ 7.27 (t, 1H, *J* = 7.8 Hz), 7.09-7.05 (m, 2H), 6.94-6.90 (m, 1H), 6.82-6.78 (m, 1H), 6.74-6.68 (m, 1H), 6.62-6.58 (m, 1H), 6.45-6.39 (m, 1H), 5.92 (brs, 1H), 3.59-3.54 (m, 2H), 3.28-3.22 (m, 2H); LC/MS ESI (+): 245.1 (M+1)

### <Preparation Example i-9> Preparation of 1-(4-chlorophenyl)-1,2,3,4-tetrahydroquinoxaline

1-(4-Chlorophenyl)-1,2,3,4-tetrahydroquinoxaline was synthesized in the same manner as in Preparation Example i-6, except that 1-benzyl-4-(4-chlorophenyl)-1,2,3,4-tetrahydroquinoxaline obtained in Preparation Example h-3 was used instead of 1-benzyl-4-(2-chlorophenyl)-1,2,3,4-tetrahydroquinoxaline.
Light blue oil (yield 55%); ¹H-NMR (400 MHz, DMSO-*d*₆) δ 7.34-7.24 (m, 2H), 7.15-7.07 (m, 2H), 6.75-6.62 (m, 2H), 6.57 (dd, 1H, *J* = 7.9, 1.6 Hz), 6.39 (ddd, 1H, *J* = 7.9, 7.1, 1.6 Hz), 5.87 (s, 1H), 3.54 (dd, 2H, *J* = 5.8, 3.7 Hz), 3.28-3.21 (m, 2H); LC/MS ESI (+): 245.1 (M+1)

### <Preparation Example i-10> Preparation of 1-(pyridin-2-yl)-1,2,3,4-tetrahydroquinoxaline

A mixture in which 1-benzyl-4-(pyridin-2-yl)-1,2,3,4-tetrahydroquinoxaline (1.0 equiv) obtained in Preparation Example i-4, Pd(OH)₂ (0.5 equiv), and ammonium formate (7.0 equiv) were dissolved in MeOH/THF (v/v = 2: 1, 0.3M) was stirred at 75 °C for 3 hours. The reaction solution was filtered through a Celite pad, water was added, and extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-(pyridin-2-yl)-1,2,3,4-tetrahydroquinoxaline.
Yellow solid (yield 78.2%); ¹H-NMR (400 MHz, CDCl₃) δ 8.26 (d, 1H, *J* = 4.1 Hz), 7.42 (t, 1H, *J* = 7.8 Hz), 7.22 (d, 1H, *J* = 8.1 Hz), 7.16 (d, 1H, *J* = 8.5 Hz), 6.93-6.83 (m, 1H), 6.70 (t, 1H, *J* = 6.0 Hz), 6.67-6.55 (m, 2H), 4.08 (t, 2H, *J* = 4.8 Hz), 3.99 (brs, 1H), 3.41 (t, 2H, *J* = 4.8 Hz); LC/MS ESI (+): 212.2 (M+1)

### <Preparation Example i-11> Preparation of 1-(pyridin-3-yl)-1,2,3,4-tetrahydroquinoxaline

1-(Pyridin-3-yl)-1,2,3,4-tetrahydroquinoxaline was prepared in the same manner as in Preparation Example i-10, except that 1-benzyl-4-(pyridin-3-yl)-1,2,3,4-tetrahydroquinoxaline obtained in Preparation Example i-10 was used instead of 1-benzyl-4-(pyridin-2-yl)-1,2,3,4-tetrahydroquinoxaline.
Brown oil (yield 83.1%); ¹H NMR (400 MHz, CD₃CN) δ 8.45 (d, 1H, *J* = 2.7 Hz), 8.17 (dd, 1H, *J* = 4.7, 1.6 Hz), 7.50 (ddd, 1H, *J* = 8.3, 2.8, 1.8 Hz), 7.26 (dd, 1H, *J* = 8.2, 4.7 Hz), 6.81-6.71 (m, 2H), 6.66-6.61 (m, 1H), 6.54-6.47 (m, 1H), 4.63 (brs, 1H), 3.68-3.63 (m, 2H), 3.39-3.34 (m, 2H); LC/MS ESI (+): 212.2 (M+1)

### <Preparation Example i-12> Preparation of 1-(pyridin-4-yl)-1,2,3,4-tetrahydroquinoxaline

1-(Pyridin-4-yl)-1,2,3,4-tetrahydroquinoxaline was prepared in the same manner as in Preparation Example i-10, except that 1-benzyl-4-(pyridin-4-yl)-1,2,3,4-tetrahydroquinoxaline obtained in Preparation Example i-10 was used instead of 1-benzyl-4-(pyridin-2-yl)-1,2,3,4-tetrahydroquinoxaline.
Yellow solid (yield 76.7%); ¹H-NMR (400 MHz, DMSO-*d*₆) δ 8.23 - 8.17 (m, 2H), 7.06 - 6.97 (m, 3H), 6.83 (ddd, 1H, *J* = 8.4, 7.2, 1.4 Hz), 6.65 (dd, 1H, *J* = 8.1, 1.5 Hz), 6.49 (ddd, 1H, *J* = 8.4, 7.2, 1.5 Hz), 6.06 (t, 1H, *J=* 3.1 Hz), 3.65 (t, 2H, *J* = 5.0 Hz), 3.24 (td, 2H, *J* = 5.1, 3.1 Hz); LC/MS ESI (+): 212.1 (M+1)

### <Example 1-125> Synthesis of 1-(4-(2-chlorophenyl)-3,4-dihydroquinoxalin-1(2H)-yl)-3-(pyrrolidin-1-yl)propan-1-one

1-(2-Chlorophenyl)-1,2,3,4-tetrahydroquinoxaline (1.0 equiv) obtained in Preparation Example i-8 was dissolved in dichloromethane (0.3 M), 3-bromopropionyl chloride (1.5 equiv) was slowly added thereto at -70 °C, and then the resulting solution was stirred at room temperature for 1.5 hours. After terminating the reaction by adding water to the reaction solution, extraction was performed with dichloromethane, and an organic layer was washed with brine, dried with MgSO₄, filtered, and concentrated under reduced pressure. The obtained concentrate was dissolved in THF (0.3 M), and TEA (3.0 equiv) and pyrrolidine (1.0 equiv) were added, followed by stirring at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-(2-chlorophenyl)-3,4-dihydroquinoxalin-1(2H)-yl)-3-(pyrrolidin-1-yl)propan-1-one.
Light brown oil (yield 66%); ¹H-NMR (400 MHz, CD₃OD) δ 7.57 (d, 1H, *J* = 7.8 Hz,), 7.48-7.33 (m, 3H), 7.28-7.13 (m, 1H), 6.97-6.86 (m, 1H), 6.75-6.66 (m, 1H), 6.14 (d, 1H, *J* = 8.2 Hz), 4.04 (t, 2H, *J* = 4.9 Hz), 3.72-3.59 (m, 2H), 2.86-2.78 (m, 2H), 2.95-2.86 (m, 2H), 2.61-2.38 (m, 4H), 1.84-1.66 (m, 4H); LC/MS ESI (+): 370.3 (M+1)

### <Example 1-126> Synthesis of 1-(4-(2-chlorophenyl)-3,4-dihydroquinoxalin-1(2H)-yl)-3-(piperidin-1-yl)propan-1-one

1-(4-(2-Chlorophenyl)-3,4-dihydroquinoxalin-1(2H)-yl)-3-(piperidin-1-yl)propan-1-one was synthesized in the same manner as in Example 1-125, except that piperidine was used instead of pyrrolidine.
Light brown oil (yield 66%); ¹H-NMR (400 MHz, CD₃OD) δ 7.57 (d, 1H, *J* = 7.8 Hz), 7.48-7.32 (m, 3H), 7.29-7.14 (m, 1H), 6.97-6.87 (m, 1H), 6.75-6.66 (m, 1H), 6.14 (d, 1H, *J* = 7.8 Hz), 4.03 (t, 2H, *J* = 5.1 Hz), 3.72-3.59 (m, 2H), 2.93-2.81 (m, 2H), 2.77-2.66 (m, 2H), 2.51-2.25 (m, 4H), 1.63-1.51 (m, 4H), 1.50-1.38 (m, 2H); LC/MS ESI (+): 384.3 (M+1)

### <Example 1-127> Synthesis of 1-(4-(2-chlorophenyl)-3,4-dihydroquinoxalin-1(2H)-yl)-3-(4-methylpiperazin-1-yl)propan-1-one

1-(4-(2-Chlorophenyl)-3,4-dihydroquinoxalin-1(2H)-yl)-3-(4-methylpiperazin-1-yl)propan-1-one was synthesized in the same manner as in Example 1-125, except that 1-methylpiperazine was used instead of pyrrolidine.
Brown oil (yield 74%); ¹H-NMR (400 MHz, CD₃OD) δ 7.58 (d, 1H, *J* = 7.8 Hz), 7.49-7.32 (m, 3H), 7.28-7.15 (m, 1H), 6.97-6.86 (m, 1H), 6.75-6.66 (m, 1H), 6.15 (d, 1H, *J* = 8.2 Hz), 4.03 (t, 2H, *J* = 5.1 Hz), 3.73-3.60 (m, 2H), 2.91-2.82 (m, 2H), 2.74 (d, 2H, *J* = 6.7 Hz), 2.65-2.30 (m, 8H), 2.26 (s, 3H); LC/MS ESI (+): 399.4 (M+1)

### <Example 1-128> Synthesis of 1-(4-(2-chlorophenyl)-3,4-dihydroquinoxalin-1(2H)-yl)-2-(pyrrolidin-1-yl)propan-1-one

1-(4-(2-Chlorophenyl)-3,4-dihydroquinoxalin-1(2H)-yl)-2-(pyrrolidin-1-yl)propan-1-one was synthesized in the same manner as in Example 1-125, except that 2-bromopropionyl chloride was used instead of 3-bromopropionyl chloride.
Pale yellow oil (yield 50%); ¹H-NMR (400 MHz, CD₃OD) δ 7.58 (d, 1H, *J* = 7.8 Hz), 7.49-7.32 (m, 3H), 7.17-7.08 (m, 1H), 6.99-6.89 (m, 1H), 6.75-6.66 (m, 1H), 6.14 (d, 1H, *J* = 8.6 Hz), 4.39-4.22 (m, 1H), 4.05-3.94 (m, 1H), 3.87-3.74 (m, 2H), 3.73-3.63 (m, 1H), 2.73-2.50 (m, 4H), 1.84-1.67 (m, 4H), 1.37 (d, 3H, *J* = 6.7 Hz); LC/MS ESI (+): 370.3 (M+1)

### <Example 1-129> Synthesis of 1-(4-(2-chlorophenyl)-3,4-dihydroquinoxalin-1 (2H)-yl)-2-(piperidin-1-yl)propan-1-one

1-(4-(2-Chlorophenyl)-3,4-dihydroquinoxalin-1(2H)-yl)-2-(piperidin-1-yl)propan-1-one synthesized in the same manner as in Example 1-128, except that piperidine was used instead of pyrrolidine.
Pale yellow solid (yield 57%); ¹H-NMR (400 MHz, CD₃OD) δ 7.58 (d, 1H, *J* = 7.8 Hz), 7.43 (d, 1H, *J* = 7.0 Hz), 7.41-7.34 (m, 2H), 7.18 (d, 1H, *J* = 5.9 Hz), 7.00-6.88 (m, 1H), 6.75-6.63 (m, 1H), 6.13 (d, 1H, *J* = 8.6 Hz), 4.47-4.32 (m, 1H), 4.17-4.03 (m, 1H), 4.00-3.80 (m, 1H), 3.74-3.64 (m, 2H), 2.63-2.41 (m, 4H), 1.63-1.48 (m, 4H), 1.47-1.38 (m, 2H), 1.34-1.21 (m, 3H); LC/MS ESI (+): 384.3 (M+1)

### <Example 1-130> Synthesis of 1-(4-(2-chlorophenyl)-3,4-dihydroquinoxalin-1(2H)-yl)-2-(4-methylpiperazin-1-yl)propan-1-one

1-(4-(2-Chlorophenyl)-3,4-dihydroquinoxalin-1(2H)-yl)-2-(4-methylpiperazin-1-yl)propan-1-one was synthesized in the same manner as in Example 1-128, except that 1-methylpiperazine was used instead of pyrrolidine.
Pale yellow solid (yield 58%); ¹H-NMR (400 MHz, CD₃OD) δ 7.58 (d, 1H, *J* = 7.8 Hz), 7.43 (d, 1H, *J* = 7.0 Hz), 7.41-7.34 (m, 2H), 7.18 (d, 1H, *J* = 5.9 Hz), 7.00-6.88 (m, 1H), 6.75-6.63 (m, 1H), 6.13 (d, 1H, *J* = 8.6 Hz), 4.47-4.32 (m, 1H), 4.17-4.03 (m, 1H), 4.00-3.80(m, 1H), 3.74-3.64 (m, 2H), 2.63-2.41 (m, 4H), 1.63-1.48 (m, 4H), 1.47-1.38 (m, 2H), 1.34-1.21 (m, 3H); LC/MS ESI (+): 399.4 (M+1)

### <Example 1-131> Synthesis of 1-(4-(2-chlorophenyl)-3,4-dihydroquinoxalin-1(2H)-yl)-2-(pyrrolidin-1-yl)ethan-1-one

1-(4-(2-Chlorophenyl)-3,4-dihydroquinoxalin-1(2H)-yl)-2-(pyrrolidin-1-yl)ethan-1-one was synthesized in the same manner as in Example 1-125, except that bromoacetyl chloride was used instead of 3-bromopropionyl chloride.
Pale yellow oil (yield 56%); ¹H-NMR (400 MHz, CD₃OD) δ 7.57 (d, 1H, *J* = 7.8 Hz), 7.48-7.33 (m, 3H), 7.31-7.04 (m, 1H), 6.97-6.83 (m, 1H), 6.73-6.64 (m, 1H), 6.14 (d, 1H, *J* = 8.2 Hz), 4.04 (t, 2H, *J* = 5.1 Hz), 3.77-3.51 (m, 4H), 2.74-2.56 (m, 4H), 1.88-1.73 (m, 4H); LC/MS ESI (+): 356.3 (M+1)

### <Example 1-132> Synthesis of 1-(4-(2-chlorophenyl)-3,4-dihydroquinoxalin-1(2H)-yl)-2-(piperidin-1-yl)ethan-1-one

1-(4-(2-Chlorophenyl)-3,4-dihydroquinoxalin-1(2H)-yl)-2-(piperidin-1-yl)ethan-1-one was synthesized in the same manner as in Example 1-131, except that piperidine was used instead of pyrrolidine.
Pale yellow solid (yield 59%); ¹H-NMR (400 MHz, CD₃OD) δ 7.57 (d, 1H, *J* = 7.8 Hz), 7.49-7.32 (m, 3H), 7.32-7.09 (m, 1H), 6.96-6.83 (m, 1H), 6.73-6.63 (m, 1H), 6.14 (d, 1H, *J* = 8.2 Hz), 4.14-3.98 (m, 2H), 3.77-3.57 (m, 2H), 3.49-3.34 (m, 2H), 2.60-2.34 (m, 4H), 1.68-1.54 (m, 4H), 1.45 (d, 2H, *J* = 5.1 Hz); LC/MS ESI (+): 370.3 (M+1)

### <Example 1-133> Synthesis of 1-(4-(2-chlorophenyl)-3,4-dihydroquinoxalin-1(2H)-yl)-2-(4-methylpiperazin-1-yl)ethan-1-one

1-(4-(2-Chlorophenyl)-3,4-dihydroquinoxalin-1(2H)-yl)-2-(4-methylpiperazin-1-yl)ethan-1-one was synthesized in the same manner as in Example 1-131, except that 1-methylpiperazine was used instead of pyrrolidine.
Pale yellow oil (yield 53%); ¹H-NMR (400 MHz, CD₃OD) δ 7.57 (d, 1H, *J* = 7.8 Hz), 7.48-7.32 (m, 3H), 7.31-7.14 (m, 1H), 6.97-6.82 (m, 1H), 6.73-6.63 (m, 1H), 6.14 (d, 1H, *J =* 7.8 Hz), 4.05 (t, 2H, *J* = 4.9 Hz), 3.75-3.58 (m, 2H), 3.47 (s, 2H), 2.79-2.35 (m, 8H), 2.27 (s, 3H); LC/MS ESI (+): 385.4 (M+1)

### <Example 1-134> Synthesis of 1-(4-(3-chlorophenyl)-3,4-dihydroquinoxalin-1(2H)-yl)-3-(pyrrolidin-1-yl)propan-1-one

1-(4-(3-Chlorophenyl)-3,4-dihydroquinoxalin-1(2H)-yl)-3-(pyrrolidin-1-yl)propan-1-one was synthesized in the same manner as in Example 1-125, except that 1-(3-chlorophenyl)-1,2,3,4-tetrahydroquinoxaline was used instead of 1-(2-chlorophenyl)-1,2,3,4-tetrahydroquinoxaline.
Pale yellow oil (yield 50%); ¹H-NMR (400 MHz, CD₃OD) δ 7.35-7.24 (m, 3H), 7.17 (dd, 1H, *J* = 7.8, 1.6 Hz), 7.12-7.02 (m, 2H), 6.99-6.92 (m, 1H), 6.92-6.84 (m, 1H), 4.02 (t, 2H, *J* = 5.9 Hz), 3.77 (t, 2H, *J* = 5.9 Hz), 2.90-2.76 (m, 4H), 2.57-2.36 (m, 4H), 1.79-1.65 (m, 4H); LC/MS ESI (+): 370.3 (M+1)

### <Example 1-135> Synthesis of 1-(4-(3-chlorophenyl)-3,4-dihydroquinoxalin-1(2H)-yl)-3-(4-methylpiperazin-1-yl)propan-1-one

1-(4-(3-Chlorophenyl)-3,4-dihydroquinoxalin-1(2H)-yl)-3-(4-methylpiperazin-1-yl)propan-1-one was synthesized in the same manner as in Example 1-134, except that 1-methylpiperazine was used instead of pyrrolidine.
Pale yellow oil (yield 40%); ¹H-NMR (400 MHz, CD₃OD) δ 7.37-7.23 (m, 3H), 7.19 (dd, 1H, *J* = 7.8, 1.6 Hz), 7.10-7.02 (m, 2H), 7.01-6.94 (m, 1H), 6.92-6.84 (m, 1H), 4.02 (t, 2H, *J* = 5.7 Hz), 3.77 (t, 2H, *J* = 5.9 Hz), 2.87-2.76 (m, 2H), 2.75-2.65 (m, 2H), 2.59-2.24 (m, 8H), 2.19 (s, 3H); LC/MS ESI (+): 399.4 (M+1)

### <Example 1-136> Synthesis of 1-(4-(3-chlorophenyl)-3,4-dihydroquinoxalin-1(2H)-yl)-2-(pyrrolidin-1-yl)propan-1-one

1-(4-(3-Chlorophenyl)-3,4-dihydroquinoxalin-1(2H)-yl)-2-(pyrrolidin-1-yl)propan-1-one was synthesized in the same manner as in Example 1-134, except that 2-bromopropionyl chloride was used instead of 3-bromopropionyl chloride.
Pale yellow oil (yield 59%); ¹H-NMR (400 MHz, CD₃OD) δ 7.32-7.10 (m, 5H), 7.09-6.99 (m, 2H), 6.97-6.87 (m, 1H), 4.41-4.27 (m, 1H), 3.96-3.82 (m, 2H), 3.79-3.63 (m, 2H), 2.63-2.43 (m, 4H), 1.77-1.62 (m, 4H), 1.32 (d, 3H, *J* = 6.7 Hz); LC/MS ESI (+): 370.3 (M+1)

### <Example 1-137> Synthesis of 1-(4-(3-chlorophenyl)-3,4-dihydroquinoxalin-1(2H)-yl)-2-(4-methylpiperazin-1-yl)propan-1-one

1-(4-(3-Chlorophenyl)-3,4-dihydroquinoxalin-1(2H)-yl)-2-(4-methylpiperazin-1-yl)propan-1-one was synthesized in the same manner as in Example 1-136, except that 1-methylpiperazine was used instead of pyrrolidine.
Pale yellow oil (yield 59%); ¹H-NMR (400 MHz, CD₃OD) δ 7.38-7.23 (m, 3H), 7.21-7.16 (m, 1H), 7.15-7.03 (m, 3H), 6.96-6.85 (m, 1H), 4.59-4.46 (m, 1H), 4.19-4.06 (m, 1H), 3.97-3.88 (m, 1H), 3.74-3.51 (m, 2H), 2.63-2.26 (m, 8H), 2.18 (s, 3H), 1.26 (d, 3H, *J* = 6.7 Hz); LC/MS ESI (+): 399.4 (M+1)

### <Example 1-138> Synthesis of 1-(4-(3-chlorophenyl)-3,4-dihydroquinoxalin-1(2H)-yl)-2-(pyrrolidin-1-yl)ethan-1-one

1-(4-(3-Chlorophenyl)-3,4-dihydroquinoxalin-1(2H)-yl)-2-(pyrrolidin-1-yl)ethan-1-one was synthesized in the same manner as in Example 1-134, except that bromoacetyl chloride was used instead of 3-bromopropionyl chloride.
Pale yellow oil (yield 46%); ¹H-NMR (400 MHz, CD₃OD) δ 7.49-7.28 (m, 2H), 7.27-7.24 (m, 1H), 7.21-7.16 (m, 1H), 7.12-7.07 (m, 1H), 7.07-7.00 (m, 1H), 6.94-6.82 (m, 2H), 4.01 (t, 2H, *J* = 5.9 Hz), 3.76 (t, 2H, *J* = 5.9 Hz), 3.60 (s, 2H), 2.68-2.59 (m, 4H), 1.83-1.73 (m, 4H); LC/MS ESI (+): 356.3 (M+1)

### <Example 1-139> Synthesis of 1-(4-(3-chlorophenyl)-3,4-dihydroquinoxalin-1(2H)-yl)-2-(4-methylpiperazin-1-yl)ethan-1-one

1-(4-(3-Chlorophenyl)-3,4-dihydroquinoxalin-1(2H)-yl)-2-(4-methylpiperazin-1-yl)ethan-1-one was synthesized in the same manner as in Example 1-138, except that 1-methylpiperazine was used instead of pyrrolidine.
Pale yellow oil (yield 39%); ¹H-NMR (400 MHz, CD₃OD) 7.43-7.30 (m, 3H), 7.29-7.25 (m, 1H), 7.18 (dd, 1H, *J* = 8.0, 1.4 Hz), 7.17-7.00 (m, 2H), 6.97-6.90 (m, 1H), 6.89-6.82(m, 1H), 4.03 (t, 2H, *J* = 5.9 Hz), 3.82-3.73 (m, 2H), 3.45 (s, 2H), 2.67-2.34 (m, 8H), 2.25 (s, 3H); LC/MS ESI (+): 385.4 (M+1)

### <Example 1-140> Synthesis of 1-(4-(4-chlorophenyl)-3,4-dihydroquinoxalin-1(2H)-yl)-3-(pyrrolidin-1-yl)propan-1-one

1-(4-(4-Chlorophenyl)-3,4-dihydroquinoxalin-1(2H)-yl)-3-(pyrrolidin-1-yl)propan-1-one was synthesized in the same manner as in Example 1-125, except that 1-(4-chlorophenyl)-1,2,3,4-tetrahydroquinoxaline was used instead of 1-(2-chlorophenyl)-1,2,3,4-tetrahydroquinoxaline.
White solid (yield 66%); ¹H-NMR (400 MHz, CD₃OD) δ 7.41-7.32 (d, 2H, *J* = 8.7 Hz), 7.31-7.21 (d, 2H, *J* = 8.7 Hz), 7.09-6.93 (m, 1H), 6.93-6.77 (m, 2H), 4.02 (t, 2H, *J* = 5.7 Hz), 3.75 (t, 2H, *J* = 5.8 Hz), 2.91-2.77 (m, 4H), 2.62-2.37 (m, 4H), 1.84-1.63 (m, 4H); LC/MS ESI (+): 370.2 (M+1)

### <Example 1-141> Synthesis of 1-(4-(4-chlorophenyl)-3,4-dihydroquinoxalin-1(2H)-yl)-3-(piperidin-1-yl)propan-1-one

1-(4-(4-Chlorophenyl)-3,4-dihydroquinoxalin-1(2H)-yl)-3-(piperidin-1-yl)propan-1-one was synthesized in the same manner as in Example 1-140, except that piperidine was used instead of pyrrolidine.
White solid (yield 69%); ¹H-NMR (400 MHz, CD₃OD) δ 7.37 (d, 2H, *J* = 8.7 Hz), 7.30-7.22 (m, 3H), 7.07-6.97 (m, 1H), 6.92-6.76 (m, 2H), 4.02 (t, 2H, *J* = 5.7 Hz), 3.75 (t, 2H, *J* = 5.8 Hz), 2.84 (t, 2H, *J* = 7.2 Hz), 2.70 (t, 2H, *J* = 7.2 Hz), 2.47-2.23 (m, 4H), 1.57-1.47 (m, 4H), 1.46-1.34 (m, 2H); LC/MS ESI (+): 384.2 (M+1)

### <Example 1-142> Synthesis of 1-(4-(4-chlorophenyl)-3,4-dihydroquinoxalin-1(2H)-yl)-3-(4-methylpiperazin-1-yl)propan-1-one

1-(4-(4-Chlorophenyl)-3,4-dihydroquinoxalin-1(2H)-yl)-3-(4-methylpiperazin-1-yl)propan-1-one was synthesized in the same manner as in Example 1-140, except that 1-methylpiperazine was used instead of pyrrolidine.
Pale yellow solid (yield 78%); ¹H-NMR (400 MHz, CD₃OD) δ 7.41-7.32 (m, 2H), 7.26 (d, 2H, *J* = 8.8 Hz), 7.07-6.97 (m, 1H), 6.91-6.79 (m, 2H), 4.02 (t, 2H, *J* = 5.7 Hz), 3.76 (t, 2H, *J* = 5.8 Hz), 2.83 (t, 2H, *J* = 7.0 Hz), 2.71 (d, 2H, *J* = 7.2 Hz), 2.54-2.25 (m, 8H), 2.22 (s, 3H); LC/MS ESI (+): 399.2 (M+1)

### <Example 1-143> Synthesis of 1-(4-(4-chlorophenyl)-3,4-dihydroquinoxalin-1(2H)-yl)-2-(pyrrolidin-1-yl)propan-1-one

1-(4-(4-Chlorophenyl)-3,4-dihydroquinoxalin-1(2H)-yl)-2-(pyrrolidin-1-yl)propan-1-one was synthesized in the same manner as in Example 1-140, except that 2-bromopropionyl chloride was used instead of 3-bromopropionyl chloride.
Colorless oil (yield 65%); ¹H-NMR (400 MHz, CD₃OD) δ 7.35 (d, 2H, *J* = 8.8 Hz), 7.23 (d, 2H, *J* = 8.7 Hz), 7.12-7.03 (m, 1H), 6.97-6.80 (m, 2H), 4.39-4.25 (m, 1H), 3.98-3.60 (m, 4H), 2.64-2.44 (m, 4H), 1.77-1.647 (m, 5H), 1.33 (d, 3H, *J* = 6.6 Hz); LC/MS ESI (+): 370.3 (M+1)

### <Example 1-144> Synthesis of 1-(4-(4-chlorophenyl)-3,4-dihydroquinoxalin-1(2H)-yl)-2-(piperidin-1-yl)propan-1-one

1-(4-(4-Chlorophenyl)-3,4-dihydroquinoxalin-1(2H)-yl)-2-(piperidin-1-yl)propan-1-one was synthesized in the same manner as in Example 1-143, except that piperidine was used instead of pyrrolidine.
White solid (yield 32%); ¹H-NMR (400 MHz, CD₃OD) δ 7.35 (d, 2H, *J* = 8.5 Hz), 7.26-7.20 (m, 3H), 7.13-6.81 (m, 3H), 4.55-4.37 (m, 1H), 4.11-3.99 (m, 1H), 3.90 (dd, 1H, *J* = 10.6, 5.0 Hz), 3.73-3.52 (m, 2H), 2.62-2.35 (m, 3H), 1.56-1.17 (m, 10H); LC/MS ESI (+): 384.4 (M+1)

### <Example 1-145> Synthesis of 1-(4-(4-chlorophenyl)-3,4-dihydroquinoxalin-1(2H)-yl)-2-(4-methylpiperazin-1-yl)propan-1-one

1-(4-(4-Chlorophenyl)-3,4-dihydroquinoxalin-1(2H)-yl)-2-(4-methylpiperazin-1-yl)propan-1-one was synthesized in the same manner as in Example 1-143, except that 1-methylpiperazine was used instead of pyrrolidine.
Pale yellow solid (yield 67%); ¹H-NMR (400 MHz, CD₃OD) δ 7.35 (d, 2H, *J* = 8.7 Hz), 7.28-7.22 (m, 3H), 7.15-6.61 (m, 3H), 4.52-4.39 (m, 1H), 4.17-4.04 (m, 1H), 3.94-3.85 (m, 1H), 3.75-3.54 (m, 2H), 2.64-2.24 (m, 8H), 2.20 (s, 3H), 1.26 (d, 3H, *J* = 6.7 Hz); LC/MS ESI (+): 399.4 (M+1)

### <Example 1-146> Synthesis of 1-(4-pyridin-2-yl)-3,4-dihydroquinoxalin-1(2H)-yl)-3-(pyrrolidin-1-yl)propan-1-one

1-(Pyridin-2-yl)-1,2,3,4-tetrahydroquinoxaline (1.0 equiv) obtained in Preparation Example i-10 was dissolved in dichloromethane (0.3 M), 3-bromopropionyl chloride (1.5 equiv) was slowly added thereto at -70 °C, and then the resulting solution was stirred at room temperature for 1.5 hours. After terminating the reaction by adding water to the reaction solution, extraction was performed with dichloromethane, and an organic layer was washed with brine, dried with MgSO₄, filtered, and concentrated under reduced pressure. After dissolving the obtained concentrate in THF (0.3 M), TEA (3.0 equiv) and pyrrolidine (1.0 equiv) were added and stirred at room temperature for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-pyridin-2-yl)-3,4-dihydroquinoxalin-1(2H)-yl)-3-(pyrrolidin-1-yl)propan-1-one.
Colorless oil (yield 34.5%); ¹H-NMR (400 MHz, CDCl₃) δ 8.34-8.28 (m, 1H), 7.54-7.41 (m, 2H), 7.25-7.08 (m, 3H), 7.03-6.95 (m, 1H), 6.86-6.78 (m, 1H), 4.11-3.97 (m, 4H), 2.88-2.74 (m, 4H), 2.51-2.36 (m, 4H), 1.73-1.63 (m, 4H); LC/MS ESI (+): 337.3 (M+1)

### <Example 1-147> Synthesis of 3-(piperidin-1-yl)-1-(4-(pyridin-2-yl)-3,4-dihydroquinoxalin-1(2H)-yl)propan-1-one

3-(Piperidin-1-yl)-1-(4-(pyridin-2-yl)-3,4-dihydroquinoxalin-1(2H)-yl)propan-1-one was synthesized in the same manner as in Example 1-146, except that piperidine was used instead of pyrrolidine.
Colorless oil (yield 33.9%); ¹H-NMR (400 MHz, CDCl₃) δ 8.31 (dd, 1H, *J* = 5.1, 1.2 Hz), 7.53-7.43 (m, 2H), 7.25-7.08 (m, 3H), 7.03-6.95 (m, 1H), 6.82 (dd, 1H, *J* = 6.9, 5.3 Hz), 4.06-4.01 (m, 4H), 2.79-2.73 (m, 2H), 2.73-2.66 (m, 2H), 2.39-2.25 (m, 4H), 1.53-1.42 (m, 4H), 1.36 (d, 2H, *J* = 5.1 Hz); LC/MS ESI (+): 351.3 (M+1)

### <Example 1-148> Synthesis of 3-(4-methylpiperazin-1-yl)-1-(4-(pyridin-2-yl)-3,4-dihydroquinoxalin-1(2H)-yl)propan-1-one

3-(4-Methylpiperazin-1-yl)-1-(4-(pyridin-2-yl)-3,4-dihydroquinoxalin-1 (2H)-yl)propan-1-one was synthesized in the same manner as in Example 1-146, except that 1-methylpiperazine was used instead of pyrrolidine.
Colorless oil (yield 30.5%); ¹H-NMR (400 MHz, CDCl₃) δ 8.31 (dd, 1H, *J* = 4.7, 1.6 Hz), 7.55-7.43 (m, 2H), 7.25-7.09 (m, 3H), 7.03-6.95 (m, 1H), 6.82 (dd, 1H, *J* = 7.0, 5.1 Hz), 4.04 (m, 4H), 2.80-2.67 (m, 4H), 2.49-2.24 (m, 6H), 2.20 (s, 3H), 1.75-1.64 (m, 2H); LC/MS ESI (+): 366.3 (M+1)

### <Example 1-149> Synthesis of 1-(4-pyridin-2-yl)-3,4-dihydroquinoxalin-1(2H)-yl)-2-(pyrrolidin-1-yl)propan-1-one

1-(4-Pyridin-2-yl)-3,4-dihydroquinoxalin-1(2H)-yl)-2-(pyrrolidin-1-yl)propan-1-one was synthesized in the same manner as in Example 1-146, except that 2-bromopropionyl chloride was used instead of 3-bromopropionyl chloride.
Yellow oil (yield 11.2%); ¹H-NMR (400 MHz, CD₃OD) δ 8.23 (dd, 1H, *J* = 4.9, 1.0 Hz), 7.65-7.58 (m, 1H), 7.45 (d, 1H, *J* = 7.4 Hz), 7.35-7.16 (m, 3H), 7.14-7.04 (m, 1H), 6.90 (dd, 1H, *J* = 6.9, 5.3 Hz), 4.37-4.23 (m, 1H), 4.04-3.93 (m, 2H), 3.93-3.85 (m, 1H), 3.85-3.69 (m, 1H), 2.63-2.41 (m, 4H), 1.75-1.57 (m, 4H), 1.30 (d, 3H, *J* = 6.7 Hz); LC/MS ESI (+): 337.3 (M+1)

### <Example 1-150> Synthesis of 2-(piperidin-1-yl)-1-(4-pyridin-2-yl)-3,4-dihydroquinoxalin-1(2H)-yl)propan-1-one

2-(Piperidin-1-yl)-1-(4-pyridin-2-yl)-3,4-dihydroquinoxalin-1(2H)-yl)propan-1-one was synthesized in the same manner as in Example 1-149, except that piperidine was used instead of pyrrolidine.
Pale yellow oil (yield 15.8%); ¹H-NMR (400 MHz, CD₃OD) 8.24 (dd, 1H, *J* = 4.9, 1.0 Hz), 7.64-7.57 (m, 1H), 7.51-7.39 (m, 1H), 7.38-7.29 (m, 1H), 7.20 (d, 2H, *J* = 8.6 Hz), 7.14-7.00 (m, 1H), 6.89 (dd, 1H, *J* = 6.9, 5.7 Hz), 4.54-4.40 (m, 1H), 4.12-3.85 (m, 4H), 3.68-3.54 (m, 1H), 2.43-2.29 (m, 3H), 1.47-1.26 (m, 6H), 1.23 (d, 3H, *J* = 6.7 Hz); LC/MS ESI (+): 351.3 (M+1)

### <Example 1-151> Synthesis of 1-(4-pyridin-2-yl)-3,4-dihydroquinoxalin-1(2H)-yl)-2-(4-methylpiperazin-1-yl)propan-1-one

1-(4-Pyridin-2-yl)-3,4-dihydroquinoxalin-1(2H)-yl)-2-(4-methylpiperazin-1-yl)propan-1-one was synthesized in the same manner as in Example 1-149, except that 1-methylpiperazine was used instead of pyrrolidine.
White solid (yield 9.8%); ¹H-NMR (400 MHz, CD₃OD) δ 8.24 (d, 1H, *J* = 4.3 Hz), 7.66-7.57 (m, 1H), 7.52-7.40 (m, 1H), 7.39-7.28 (m, 1H), 7.22 (d, 2H, *J* = 8.6 Hz), 7.12-7.03 (m, 1H), 6.90 (dd, 1H, *J* = 6.7, 5.5 Hz), 4.56-4.41 (m, 1H), 4.13-3.85 (m, 4H), 3.69-3.52 (m, 1H), 2.55-2.35 (m, 4H), 2.34-2.19 (m, 3H), 2.18-2.08 (m, 3H), 1.23 (d, 3H, *J* = 6.7 Hz); LC/MS ESI (+): 366.3 (M+1)

### <Example 1-152> Synthesis of 1-(4-pyridin-2-yl)-3,4-dihydroquinoxalin-1(2H)-yl)-2-(pyrrolidin-1-yl)ethan-1-one

1-(4-Pyridin-2-yl)-3,4-dihydroquinoxalin-1(2H)-yl)-2-(pyrrolidin-1-yl)ethan-1-one was synthesized in the same manner as in Example 1-146, except that bromoacetyl chloride was used instead of 3-bromopropionyl chloride.
White solid (yield 14.1%); ¹H-NMR (400 MHz, CD₃OD) δ 8.23 (dd, 1H, *J* = 4.7, 1.2 Hz), 7.67-7.59 (m, 1H), 7.57-7.43 (m, 1H), 7.38 (d, 1H, *J =* 8.2 Hz), 7.24-7.12 (m, 2H), 7.08-7.00 (m, 1H), 6.95-6.87 (m, 1H), 4.08-3.93 (m, 4H), 3.61-3.50 (m, 2H), 2.68-2.50 (m, 4H), 1.80-1.68 (m, 4H); LC/MS ESI (+): 323.3 (M+1)

### <Example 1-153> Synthesis of 2-(piperidin-1-yl)-1-(4-(pyridin-2-yl)-3,4-dihydroquinoxalin-1(2H)-yl)ethan-1-one

2-(Piperidin-1-yl)-1-(4-(pyridin-2-yl)-3,4-dihydroquinoxalin-1(2H)-yl)ethan-1-one was synthesized in the same manner as in Example 1-152, except that piperidine was used instead of pyrrolidine.
Yellow oil (yield 20%); ¹H-NMR (400 MHz, CD₃OD) δ 8.24 (dd, 1H, *J* = 4.9, 1.0 Hz), 7.66-7.58 (m, 1H), 7.58-7.43 (m, 1H), 7.39 (d, 1H, *J* = 8.2 Hz), 7.25-7.10 (m, 2H), 7.08-7.00 (m, 1H), 6.91 (dd, 1H, *J* = 6.7, 5.1 Hz), 4.09-3.91 (m, 4H), 3.42-3.33 (m, 2H), 2.50-2.33 (m, 4H), 1.58-1.44 (m, 4H), 1.39 (d, 2H, *J* = 4.7 Hz); LC/MS ESI (+): 337.3 (M+1)

### <Example 1-154> Synthesis of 2-(4-methylpiperazin-1-yl)-1-(4-(pyridin-2-yl)-3,4-dihydroquinoxalin-1(2H)-yl)ethan-1-one di-L-tartrate

2-(4-Methylpiperazin-1-yl)-1-(4-(pyridin-2-yl)-3,4-dihydroquinoxalin-1 (2H)-yl)ethan-1-one was synthesized in the same manner as in Example 1-152, except that 1-methylpiperazine was used instead of pyrrolidine. The obtained 2-(4-methylpiperazin-1-yl)-1-(4-(pyridin-2-yl)-3,4-dihydroquinoxalin-1(2H)-yl)ethan-1-one (1.0 equiv) was dissolved in ethyl acetate (0.5M) and stirred at room temperature for 10 minutes. L-Tartaric acid (2.0 equiv) was dissolved in ethyl acetate and slowly added to the prepared reaction solution, and then stirred at room temperature for 1 hour. The resulting solid was filtered, washed, and dried, thereby synthesizing 2-(4-methylpiperazin-1-yl)-1-(4-(pyridin-2-yl)-3,4-dihydroquinoxalin-1(2H)-yl)ethan-1-one di-L-tartrate.
White solid (yield 19.9%); ¹H NMR (400 MHz, CD₃OD) δ 8.28-8.21 (m, 1H), 7.71-7.62 (m, 1H), 7.40 (d, 2H, *J* = 8.1 Hz), 7.25-7.12 (m, 2H), 7.09-7.00 (m, 1H), 6.96-6.91 (m, 1H), 4.46 (s, 4H), 4.04 (d, 2H, *J* = 4.9 Hz), 4.01-3.93 (m, 2H), 3.60 (s, 2H), 3.23-3.08 (m, 4H), 2.87-2.72 (m, 7H); LC/MS ESI (+): 352.3 (M+1)

### <Example 1-155> Synthesis of 1-(4-(pyridine-3-yl)-3,4-dihydroquinoxalin-1(2H)-yl)-3-(pyrrolidin-1-yl)propan-1-one

1-(4-(Pyridine-3-yl)-3,4-dihydroquinoxalin-1(2H)-yl)-3-(pyrrolidin-1-yl)propan-1-one was synthesized in the same manner as in Example 1-146, except that 1-(4-pyridin-3-yl)-1,2,3,4-dihydroquinoxaline obtained in Preparation Example i-11 was used instead of 1-(4-pyridin-2-yl)-1,2,3,4-dihydroquinoxaline.
Colorless oil (yield 26.2%); ¹H-NMR (400 MHz, CD₃CN) δ 8.55 (d, 1H, *J* = 2.7 Hz), 8.34 (dd, 1H, *J* = 4.7, 1.2 Hz), 7.65 (ddd, 1H, *J* = 8.3, 2.6, 1.6 Hz), 7.36 (dd, 2H, *J* = 8.2, 4.7 Hz), 7.05-6.98 (m, 1H), 6.89-6.82 (m, 2H), 3.99 (t, 2H, *J* = 5.5 Hz), 3.79-3.73 (m, 2H), 2.77-2.68 (m, 4H), 2.38 (s, 4H), 1.70-1.60 (m, 4H); LC/MS ESI (+): 337.3 (M+1)

### <Example 1-156> Synthesis of 3-(piperidin-1-yl)-1-(4-(pyridin-3-yl)-3,4-dihydroquinoxalin-1(2H)-yl)propan-1-one

3-(Piperidin-1-yl)-1-(4-(pyridin-3-yl)-3,4-dihydroquinoxalin-1(2H)-yl)propan-1-one was synthesized in the same manner as in Example 1-155, except that piperidine was used instead of pyrrolidine.
Colorless oil (yield 23.1%); ¹H-NMR (400 MHz, CD₃CN) δ 8.56 (d, 1H, *J* = 2.7 Hz), 8.34 (dd, 1H, *J* =4.9, 1.4 Hz), 7.66 (ddd, 1H, *J* = 8.3, 2.6, 1.6 Hz), 7.36 (dd, 2H, *J* = 8.4, 4.5 Hz), 7.05-6.98 (m, 1H), 6.89-6.81 (m, 2H), 4.02-3.96 (m, 2H), 3.77 (t, 2H, *J* = 5.5 Hz), 2.75-2.68 (m, 2H), 2.63-2.55 (m, 2H), 2.29 (s, 4H), 1.51-1.42 (m, 4H), 1.40-1.33 (m, 2H); LC/MS ESI (+): 351.3 (M+1)

### <Example 1-157> Synthesis of 3-(4-methylpiperazin-1-yl)-1-(4-(pyridin-3-yl)-3,4-dihydroquinoxalin-1(2H)-yl)propan-1-one

3-(4-Methylpiperazin-1-yl)-1-(4-(pyridin-3-yl)-3,4-dihydroquinoxalin-1(2H)-yl)propan-1-one was synthesized in the same manner as in Example 1-155, except that 1-methylpiperazine was used instead of pyrrolidine.
Yellow oil (yield 20%); ¹H-NMR (400 MHz, CD₃OD) δ 8.51 (d, 1H, *J* = 2.7 Hz), 8.26 (dd, 1H, *J* = 4.9, 1.4 Hz), 7.79-7.73 (m, 1H), 7.43 (dd, 1H, *J* = 8.4, 4.9 Hz), 7.37-7.27 (m, 1H), 7.13-7.04 (m, 1H), 7.00-6.88 (m, 2H), 4.05 (t, 2H, *J* = 5.9 Hz), 3.83 (t, 2H, *J* = 5.7 Hz), 2.88-2.79 (m, 2H), 2.77-2.65 (m, 2H), 2.60-2.25 (m, 8H), 2.20 (s, 3H); LC/MS ESI (+): 366.3 (M+1)

### <Example 1-158> Synthesis of 1-(4-(pyridin-4-yl)-3,4-dihydroquinoxalin-1(2H)-yl)-3-(pyrrolidin-1-yl)propan-1-one

1-(4-(Pyridin-4-yl)-3,4-dihydroquinoxalin-1(2H)-yl)-3-(pyrrolidin-1-yl)propan-1-one was synthesized in the same manner as in Example 1-146, except that 1-(4-pyridin-4-yl)-1,2,3,4-dihydroquinoxaline obtained in Preparation Example i-12 was used instead of 1-(4-pyridin-2-yl)-1,2,3,4-dihydroquinoxaline.
Yellow solid (yield 46%); ¹H-NMR (400 MHz, CD₃OD) δ 8.28-8.21 (m, 2H), 7.49 (d, 1H, *J* = 8.1 Hz), 7.27 (d, 1H, *J* = 7.8 Hz), 7.21-7.12 (m, 3H), 4.06 (t, 2H, *J* = 6.4 Hz), 3.88 (t, 2H, *J* = 6.5 Hz), 2.86-2.73 (m, 4H), 2.48-2.29 (m, 4H), 1.72-1.56 (m, 4H); LC/MS ESI (+): 337.3 (M+1)

### <Example 1-159> Synthesis of 3-(piperidin-1-yl)-1-(4-(pyridin-4-yl)-3,4-dihydroquinoxalin-1(2H)-yl)propan-1-one

3-(Piperidin-1-yl)-1-(4-(pyridin-4-yl)-3,4-dihydroquinoxalin-1(2H)-yl)propan-1-one was synthesized in the same manner as in Example 1-158, except that piperidine was used instead of pyrrolidine.
Colorless oil (yield 25.9%); ¹H-NMR (400 MHz, CD₃OD) δ 8.28-8.21 (m, 2H), 7.50 (d, 1H, *J* = 8.3 Hz), 7.27 (d, 1H, *J* = 7.9 Hz), 7.22-7.13 (m, 3H), 4.06 (t, 2H, *J* = 6.4 Hz), 3.88 (t, 2H, *J* = 6.6 Hz), 2.79 (t, 2H, *J* = 7.1 Hz), 2.66 (t, 2H, *J* = 7.2 Hz), 2.35-2.21 (m, 3H), 1.49-1.37 (m, 4H), 1.36-1.28 (m, 3H); LC/MS ESI (+): 351.3 (M+1)

### <Example 1-160> Synthesis of 3-(4-methylpiperazin-1-yl)-1-(4-(pyridin-4-yl)-3,4-dihydroquinoxalin-1(2H)-yl)propan-1-one

3-(4-Methylpiperazin-1-yl)-1-(4-(pyridin-4-yl)-3,4-dihydroquinoxalin-1(2H)-yl)propan-1-one was synthesized in the same manner as in Example 1-158, except that 1-methylpiperazine was used instead of pyrrolidine.
Colorless oil (yield 25.9%); ¹H-NMR (400 MHz, CD₃OD) δ 8.28-8.22 (m, 2H), 7.61-7.36 (m, 2H), 7.34-7.23 (m, 1H), 7.20-7.12 (m, 3H), 4.06 (t, 2H, *J* = 6.4 Hz), 3.88 (t, 2H, *J* = 6.5 Hz), 2.79 (t, 2H, *J* = 6.9 Hz), 2.66 (d, 2H, *J* = 7.0 Hz), 2.42-2.16 (m, 9H), 2.14 (s, 3H); LC/MS ESI (+): 366.4 (M+1)

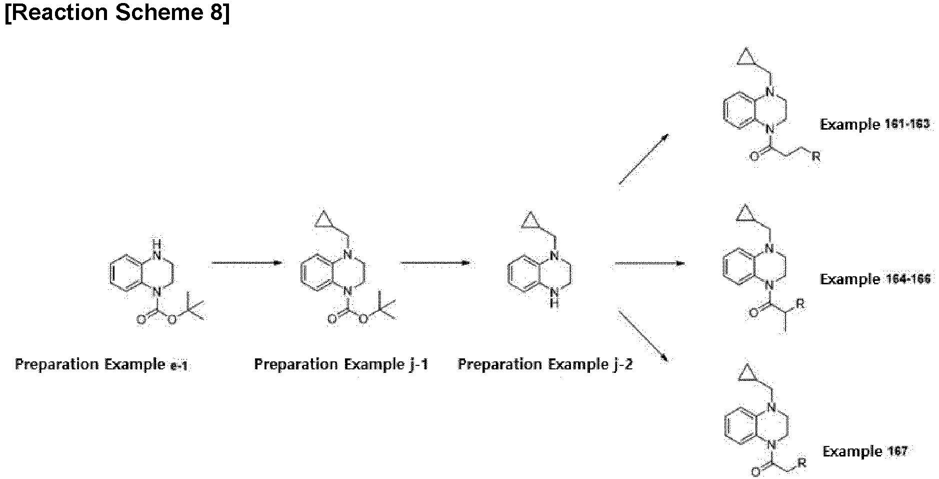

### <Preparation Method j> Preparation of 1-(cyclopropylmethyl)-1,2,3,4-tetrahydroquinoxaline

1-(Cyclopropylmethyl)-1,2,3,4-tetrahydroquinoxaline according to the present invention was prepared through the processes of Preparation Examples j-1 and j-2 below.

### <Preparation Example j-1> Preparation of tert-butyl 4-(cyclopropylmethyl)-3,4-dihydroquinoxaline-1(2H)-carboxylate

DIPEA (1.04 mL, 5.975 mmol) and (bromomethyl)cyclopropane (579 µL, 5.975 mmol) were added to a solution in which tert-butyl-3,4-dihydroquinoxaline-1(2H)-carboxylate (700 mg, 2.978 mmol) obtained in Preparation Example e-1 was dissolved in DMF (3 mL) and stirred at 100 °C for 24 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing tert-butyl 4-(cyclopropylmethyl)-3,4-dihydroquinoxaline-1(2H)-carboxylate.

Yellow oil (yield 89%); ¹H NMR (400 MHz, CDCl₃) δ 7.45 (d, 1H, *J=* 7.8 Hz), 6.96 (m, 1H), 6.76 (d, 1H, *J =* 8.2 Hz), 6.63 (m, 1H), 3.79 (m, 2H), 3.44 (m, 2H), 3.19 (d, 2H, *J =* 6.7 Hz), 1.52 (s, 9H), 1.06 (m, 1H), 0.53 (m, 2H), 0.22 (m, 2H).

### <Preparation Example j-2> Preparation of 1-(cyclopropylmethyl)-1,2,3,4-tetrahydroquinoxaline

Tert-butyl 4-(cyclopropylmethyl)-3,4-dihydroquinoxaline-1(2H)-carboxylate (870 mg, 3.01 mmol) obtained in Preparation Example j-1 was dissolved in dichloromethane (8 mL), TFA (1.85 mL, 24.1 mmol) was added, and then the resulting solution was stirred at room temperature for 24 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using an ethyl acetate/hexane mixture, thereby preparing 1-(cyclopropylmethyl)-1,2,3,4-tetrahydroquinoxaline.

Yellow oil (yield 89%); ¹H NMR (400 MHz, CDCl₃) δ 6.66 (m, 2H), 6.55 (m, 1H), 6.49 (m, 1H), 3.65 (brs, 1H), 3.44 (s, 4H), 3.13 (d, 2H, *J* = 6.3 Hz), 1.07 (m, 1H), 0.53 (m, 2H), 0.21 (q, 2H, *J* = 5.1 Hz).

### <Example 1-161> Synthesis of 1-(4-(cyclopropylmethyl)-3,4-dihydroquinoxaline-1(2H)-yl)-3-(pyrrolidin-1-yl)propan-1-one

1-(Cyclopropylmethyl)-1,2,3,4-tetrahydroquinoxaline (1.0 equiv) obtained in Preparation Example j-2 was dissolved in dichloromethane (0.2 M), 3-bromopropionyl chloride (1.1 equiv) was slowly added at -10 °C, and the resulting solution was stirred at room temperature for 30 minutes. After terminating the reaction by adding water to the reaction solution, extraction was performed with dichloromethane. An organic layer was washed with brine, dried with MgSO₄, filtered, and concentrated under reduced pressure, thereby synthesizing a brown oily compound, 3-bromo-1-(4-(cyclopropylmethyl)-3,4-dihydroquinoxalin-1(2H)-yl)propan-1-one. DIPEA (3.0 equiv) and pyrrolidine (1.5 equiv) were added to a solution in which the obtained brown oily compound (1.0 equiv) was dissolved in THF (0.2 M) and stirred at 70 °C for 3 hours. After cooling to room temperature and terminating the reaction by adding water to the reaction solution, extraction was performed with ethyl acetate. An organic layer was washed with brine, dried with MgSO₄, filtered, and concentrated under reduced pressure. The obtained residue was purified by silica column chromatography using a dichloromethane/methanol mixture, thereby synthesizing 1-(4-(cyclopropylmethyl)-3,4-dihydroquinoxaline-1(2H)-yl)-3-(pyrrolidin-1-yl)propan-1-one.
Ivory solid (yield 61.3%); ¹H NMR (400 MHz, CD₃OD) δ 7.15-6.98 (m, 2H), 6.85 (d, 1H, *J* = 8.6 Hz), 6.66-6.56 (m, 1H), 3.85 (t, 2H, *J* = 5.5 Hz), 3.54-3.43 (m, 2H), 3.24 (d, 2H, *J* = 6.3 Hz), 2.86-2.71 (m, 4H), 2.58-2.34 (m, 4H), 1.83-1.66 (m, 4H), 1.11-0.99 (m, 1H), 0.57-0.49 (m, 2H), 0.26 (q, 2H, *J* = 5.0 Hz); LC/MS ESI (+): 314.1 (M+1)

### <Example 1-162> Synthesis of 1-(4-(cyclopropylmethyl)-3,4-dihydroquinoxaline-1(2H)-yl)-3-(piperidin-1-yl)propan-1-one

1-(4-(Cyclopropylmethyl)-3,4-dihydroquinoxaline-1(2H)-yl)-3-(piperidin-1-yl)propan-1-one was synthesized in the same manner as in Example 1-161, except that piperidine was used instead of pyrrolidine.
Ivory solid (yield 66.5%); ¹H NMR (400 MHz, CD₃OD) δ 7.15-7.00 (m, 2H), 6.85 (d, 1H, *J* = 8.2 Hz), 6.66-6.56 (m, 1H), 3.85 (t, 2H, *J* = 5.5 Hz), 3.54-3.43 (m, 2H), 3.24 (d, 2H, *J* = 6.7 Hz), 2.84-2.76 (m, 2H), 2.74-2.63 (m, 2H), 2.49-2.23 (m, 4H), 1.62-1.49 (m, 4H), 1.47-1.36 (m, 2H), 1.12-0.99 (m, 1H), 0.58-0.49 (m, 2H), 0.26 (q, 2H, *J* = 5.0 Hz); LC/MS ESI (+): 328.1 (M+1)

### <Example 1-163> Synthesis of 1-(4-(cyclopropylmethyl)-3,4-dihydroquinoxaline-1(2H)-yl)-3-(4-methylpiperazin-1-yl)propan-1-one

1-(4-(Cyclopropylmethyl)-3,4-dihydroquinoxaline-1(2H)-yl)-3-(4-methylpiperazin-1-yl)propan-1-one was synthesized in the same manner as in Example 1-161, except that 1-methylpiperazine was used instead of pyrrolidine.
Yellow solid (yield 63.2%); ¹H NMR (400 MHz, CD₃OD) δ 7.14-7.00 (m, 2H), 6.85 (d, 1H, *J* = 8.2 Hz), 6.65-6.56 (m, 1H), 3.84 (t, 2H, *J* = 5.5 Hz), 3.49 (t, 2H, *J* = 5.1 Hz), 3.24 (d, 2H, *J* = 6.3 Hz), 2.81-2.74 (m, 2H), 2.69-2.59 (m,2H), 2.57-2.33 (m, 8H), 2.28 (s, 3H), 1.12-1.01 (m, 1H), 0.58-0.50 (m, 2H), 0.27 (q, 2H, *J* = 5.0 Hz); LC/MS ESI (+): 343.2 (M+1)

### <Example 1-164> Synthesis of 1-(4-(cyclopropylmethyl)-3,4-dihydroquinoxaline-1(2H)-yl)-2-(pyrrolidin-1-yl)propan-1-one

1-(4-(Cyclopropylmethyl)-3,4-dihydroquinoxaline-1(2H)-yl)-2-(pyrrolidin-1-yl)propan-1-one was synthesized in the same manner as in Example 1-161, except that a brown oily compound, 2-bromo-1-(4-(cyclopropylmethyl)-3,4-dihydroquinoxaline-1(2H)-yl)propan-1-one, was obtained using 2-bromopropionyl chloride instead of 3-bromopropionyl chloride, and then pyrrolidine was used.
Yellow oil (yield 23.5%); ¹H NMR (400 MHz, CD₃OD) δ 7.15-7.05 (m, 1H), 7.04-6.95 (m, 1H), 6.88 (d, 1H, *J* = 8.2 Hz), 6.66-6.56 (m, 1H), 4.23-4.09 (m, 1H), 3.93-3.80 (m, 1H), 3.62-3.42 (m, 3H), 3.40-3.30 (m, 1H), 3.21-3.09 (m, 1H), 2.71-2.44 (m 4H), 1.85-1.63 (m, 4H), 1.29 (d, 3H, *J* = 6.3 Hz), 1.10-0.98 (m, 1H), 0.55-0.43 (m, 2H), 0.31-0.20 (m, 2H); LC/MS ESI (+): 314.1 (M+1)

### <Example 1-165> Synthesis of 1-(4-(cyclopropylmethyl)-3,4-dihydroquinoxaline-1(2H)-yl)-2-(piperidin-1-yl)propan-1-one

1-(4-(Cyclopropylmethyl)-3,4-dihydroquinoxaline-1(2H)-yl)-2-(piperidin-1-yl)propan-1-one was synthesized in the same manner as in Example 1-164, except that piperidine was used instead of pyrrolidine.
Ivory solid (yield 48.2%); ¹H NMR (400 MHz, CD₃OD) δ 7.14-6.94 (m, 2H), 6.86 (d, 1H, *J* = 8.2 Hz), 6.66-6.51 (m, 1H), 4.37-4.20 (m, 1H), 4.16-3.95 (m, 1H), 3.85-3.61 (m, 1H), 3.57-3.34 (m, 4H), 3.19-3.05 (m, 1H), 2.59-2.31 (m, 4H), 1.65-1.31 (m, 6H), 1.22 (d, 3H, *J* = 6.7 Hz), 1.10-0.98 (m, 1H), 0.58-0.43 (m, 2H), 0.32-0.19 (m, 2H); LC/MS ESI (+): 328.1 (M+1)

### <Example 1-166> Synthesis of 1-(4-(cyclopropylmethyl)-3,4-dihydroquinoxaline-1(2H)-yl)-2-(4-methylpiperazin-1-yl)propan-1-one

1-(4-(Cyclopropylmethyl)-3,4-dihydroquinoxaline-1(2H)-yl)-2-(4-methylpiperazin-1-yl)propan-1-one was synthesized in the same manner as in Example 1-164, except that 1-methylpiperazine was used instead of pyrrolidine.
Ivory solid (yield 46.1%); ¹H NMR (400 MHz, CD₃OD) δ 7.15-6.94 (m, 2H), 6.86 (d, 1H, *J* = 8.2 Hz), 6.65-6.53 (m, 1H), 4.30-4.13 (m, 1H), 4.10-3.97 (m, 1H), 3.88-3.71 (m, 1H), 3.61-3.40 (m, 3H), 3.23-3.11 (m, 1H), 2.69-2.33 (m, 8H), 2.25 (s, 3H), 1.21 (d, 3H, *J* = 7.0 Hz), 1.12-1.00 (m, 1H), 0.58-0.43 (m, 2H), 0.26 (q, 2H, *J* = 5.0 Hz); LC/MS ESI (+): 343.1 (M+1)

### <Example 1-167> Synthesis of 1-(4-(cyclopropylmethyl)-3,4-dihydroquinoxaline-1(2H)-yl)-2-(pyrrolidin-1-yl)ethan-1-one

1-(4-(Cyclopropylmethyl)-3,4-dihydroquinoxaline-1(2H)-yl)-2-(pyrrolidin-1-yl)ethan-1-one was synthesized in the same manner as in Example 1-161, except that a brown oily compound, 2-bromo-1-(4-(cyclopropylmethyl)-3,4-dihydroquinoxaline-1(2H)-yl)ethanone, was obtained using bromoacetyl chloride instead of 3-bromopropionyl chloride, and then pyrrolidine was used.
Brown oil (yield 2.2%); ¹H NMR (400 MHz, CD₃OD) δ 7.15-7.03 (m, 2H), 6.85 (d, 1H, *J* = 8.5 Hz), 6.63-6.56 (m, 1H), 3.85 (t, 2H, *J* = 5.5 Hz), 3.56-3.48 (m, 4H), 3.24 (d, 2H, *J* = 6.3 Hz), 2.71-2.56 (m, 4H), 1.86-1.73 (m, 4H), 1.11-0.99 (m, 1H), 0.52 (q, 2H, *J* = 5.9 Hz), 0.26 (q, 2H, *J* = 5.0 Hz); LC/MS ESI (+): 300.1 (M+1)

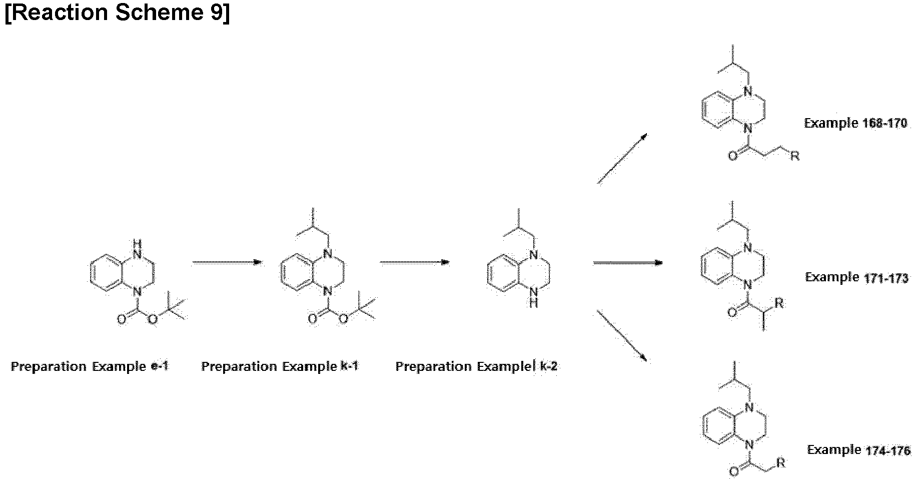

### <Preparation Method k> Preparation of 1-isobutyl-1,2,3,4-tetrahydroquinoxaline

1-Isobutyl-1,2,3,4-tetrahydroquinoxaline according to the present invention was prepared through the processes of Preparation Examples k-1 and k-2 below.

### <Preparation Example k-1> Preparation of tert-butyl 4-isobutyl-3,4-dihydroquinoxaline-1(2H)-carboxylate

Isobutyraldehyde (0.385 mL, 4.225 mmol) and sodium triacetoxyborohydride (976 mg, 4.609 mmol) were added to a solution in which tert-butyl-3,4-dihydroquinoxaline-1(2H)-carboxylate (900 mg, 3.841 mmol) obtained in Preparation Example e-1 was dissolved in dichloromethane (10 mL) and stirred at room temperature for 3 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing tert-butyl 4-isobutyl-3,4-dihydroquinoxaline-1(2H)-carboxylate.

Pale yellow solid (yield 91%); ¹H NMR (400 MHz, CDCl₃) δ 7.42 (d, 1H, *J* = 7.4 Hz) 6.95 (ddd, 1H, *J* = 8.4, 7.2, 1.6 Hz), 6.61 (m, 2H), 3.75 (m, 2H), 3.39 (m, 2H), 3.06 (d, 2H, *J* = 7.4 Hz), 2.09 (m, 1H), 1.51 (s, 9H), 0.94 (d, 6H, *J* = 6.7 Hz).

### <Preparation Example k-2> Preparation of 1-isobutyl-1,2,3,4-tetrahydroquinoxaline

Tert-butyl 4-isobutyl-3,4-dihydroquinoxaline-1(2H)-carboxylate (1000 mg, 3.443 mmol) obtained in Preparation Example k-1 was dissolved in dichloromethane (10 mL), TFA (2.635 mL, 34.43 mmol) was added, and then the resulting solution was stirred at room temperature for 1 hour. After terminating the reaction by adding water to the reaction solution, the resulting solution was neutralized with a saturated NaHCO₃ aqueous solution and extracted with dichloromethane. An organic layer was washed with brine, dried with MgSO₄, filtered, and concentrated under reduced pressure. The obtained residue was purified by silica column chromatography using a hexane/ethyl acetate mixture, thereby 1-isobutyl-1,2,3,4-tetrahydroquinoxaline.
Brown oil (yield 91%); ¹H NMR (400 MHz, CDCl₃) δ 6.66 (m, 1H), 6.51 (m, 3H), 3.64 (brs, 1H), 3.39 (s, 4H), 2.98 (d, 2H, *J* = 6.7 Hz), 2.07 (m, 1H), 0.95 (d, 6H, *J* = 7.4 Hz); LC/MS ESI (+): 191.2 (M+1)

### <Example 1-168> Synthesis of 1-(4-isobutyl-3,4-dihydroquinoxaline-1(2H)-yl)-2-(pyrrolidin-1-yl)propan-1-one

1-Isobutyl-1,2,3,4-tetrahydroquinoxaline (1.0 equiv) obtained in Preparation Example k-2 was dissolved in dichloromethane (0.2 M), 3-bromopropionyl chloride (1.1 equiv) was slowly added at -10 °C, and then the resulting solution was stirred at room temperature for 30 minutes. After terminating the reaction by adding water to the reaction solution, extraction was performed with dichloromethane. An organic layer was washed with brine, dried with MgSO₄, filtered, and concentrated under reduced pressure, thereby synthesizing a purple oily compound, 3-bromo-1-(4-isobutyl-3,4-dihydroquinoxaline-1(2H)-yl)propan-1-one. DIPEA (3.0 equiv) and pyrrolidine (1.5 equiv) were added to a solution in which the obtained purple oily compound (1.0 equiv) was dissolved in THF (0.2 M) and stirred at 70 °C for 3 hours. After cooling to room temperature and terminating the reaction by adding water to the reaction solution, extraction was performed with ethyl acetate. An organic layer was washed with brine, dried with MgSO₄, filtered, and concentrated under reduced pressure. The obtained residue was purified by silica column chromatography using a dichloromethane/methanol mixture, thereby synthesizing 1-(4-isobutyl-3,4-dihydroquinoxaline-1(2H)-yl)-2-(pyrrolidin-1-yl)propan-1-one.
Pale yellow oil (yield 53.4%); ¹H NMR (400 MHz, CDCl₃) δ 7.05-6.99 (m, 2H), 6.65 (d, 1H, *J* = 8.2 Hz), 6.60-6.56 (m, 1H), 3.89-3.85 (m, 2H), 3.39 (t, 2H, *J* = 5.3 Hz), 3.08 (d, 2H, *J* = 7.4 Hz), 2.83-2.74 (m, 4H), 2.48-2.37 (m, 4H), 2.13-2.06 (m, 1H), 1.71-1.68 (m, 4H), 0.92 (d, 6H, *J* = 6.7 Hz); LC/MS ESI (+): 316.3 (M+1)

### <Example 1-169> Synthesis of 1-(4-isobutyl-3,4-dihydroquinoxaline-1(2H)-yl)-3-(piperidin-1-yl)propan-1-one

1-(4-lsobutyl-3,4-dihydroquinoxaline-1(2H)-yl)-3-(piperidin-1-yl)propan-1-one was synthesized in the same manner as in Example 1-168, except that piperidine was used instead of pyrrolidine.
Pale yellow oil (yield 50%); ¹H NMR (400 MHz, CDCl₃) δ 7.06-6.96 (m, 2H), 6.65 (d, 1H, *J* = 8.2 Hz), 6.60-6.57 (m, 1H), 3.89-3.83 (m, 2H), 3.39 (t, 2H, *J* = 5.1 Hz), 3.08 (d, 2H, *J* = 7.4 Hz), 2.75-2.67 (m, 4H), 2.35-2.25 (m, 4H), 2.13-2.06 (m, 1H), 1.52-1.50 (m, 4H), 1.42-1.36 (m, 2H), 0.92 (d, 6H, *J* = 6.7 Hz); LC/MS ESI (+): 330.3 (M+1)

### <Example 1-170> Synthesis of 1-(4-isobutyl-3,4-dihydroquinoxaline-1(2H)-yl)-3-(4-methylpiperazin-1-yl)propan-1-one di-L-tartrate

1-(4-lsobutyl-3,4-dihydroquinoxaline-1(2H)-yl)-3-(4-methylpiperazin-1-yl)propan-1-one was synthesized in the same manner as in Example 1-168, except that 1-methylpiperazine was used instead of pyrrolidine. The obtained 1-(4-isobutyl-3,4-dihydroquinoxaline-1(2H)-yl)-3-(4-methylpiperazin-1-yl)propan-1-one (1.0 equiv) was dissolved in ethyl acetate (0.5 M) and stirred at room temperature for 10 minutes. L-Tartaric acid (2.0 equiv) was dissolved in ethyl acetate and slowly added to the prepared reaction solution, and then the resulting solution was stirred at room temperature for 1 hour. The resulting solid was filtered, washed, and dried, thereby synthesizing 1-(4-isobutyl-3,4-dihydroquinoxaline-1(2H)-yl)-3-(4-methylpiperazin-1-yl)propan-1-one di-L-tartrate.
White solid (yield 47.8%); ¹H NMR (400 MHz, CD₃OD) δ 7.15-7.03 (m, 2H), 6.78 (d, 1H, *J* = 8.3 Hz), 6.64-6.60 (m, 1H), 4.49-4.44 (m, 4H), 3.86 (t, 2H, *J* = 5.3 Hz), 3.50-3.39 (m, 2H), 3.18-3.16 (m, 6H), 2.87-2.84 (m, 4H), 2.78 (s, 3H), 2.74-2.62 (m, 4H), 2.18-2.07 (m, 1H), 0.97 (d, 6H, *J* = 6.6 Hz); LC/MS ESI (+): 345.3 (M+1)

### <Example 1-171> Synthesis of 1-(4-isobutyl-3,4-dihydroquinoxaline-1(2H)-yl)-2-(pyrrolidin-1-yl)propan-1-one

1-(4-lsobutyl-3,4-dihydroquinoxaline-1(2H)-yl)-2-(pyrrolidin-1-yl)propan-1-one was synthesized in the same manner as in Example 1-168, except that a green oily compound, 2-bromo-1-(4-isobutyl-3,4-dihydroquinoxalin-1(2H)-yl)propan-1-one, was obtained using 2-bromopropionyl chloride instead of 3-bromopropionyl chloride, and then pyrrolidine was used.
Brown oil (yield 71.6%); ¹H NMR (400 MHz, CDCl₃) δ 7.08-7.03 (m, 2H), 6.67 (d, 1H, *J =* 8.2 Hz), 6.60-6.58 (m, 1H), 4.16-4.08 (m, 1H), 3.94-3.90 (m, 1H), 3.64-3.60 (m, 1H), 3.48-3.35 (m, 2H), 3.18-3.13 (m, 1H), 3.06-3.00 (m, 1H), 2.56-2.53 (m, 4H), 2.12-2.05 (m, 1H), 1.77-1.65 (m, 4H), 1.31 (d, 2H, *J* = 6.7 Hz), 0.92 (d, 6H, *J* = 6.7 Hz); LC/MS ESI (+): 316.3 (M+1)

### <Example 1-172> Synthesis of 1-(4-isobutyl-3,4-dihydroquinoxaline-1(2H)-yl)-2-(piperidin-1-yl)propan-1-one

1-(4-lsobutyl-3,4-dihydroquinoxaline-1(2H)-yl)-2-(piperidin-1-yl)propan-1-one was synthesized in the same manner as in Example 1-171, except that piperidine was used instead of pyrrolidine.
Brown oil (yield 88.6%); ¹H NMR (400 MHz, CDCl₃) δ 7.15 (d, 1H, *J* = 7.0 Hz), 7.05-7.03 (m, 1H), 6.65 (d, 1H, *J* = 8.2 Hz), 6.60-6.57 (m, 1H), 4.14-4.04 (m, 1H), 4.01-3.94 (m, 1H), 3.69-3.61 (m, 1H), 3.47-3.35 (m, 2H), 3.18-3.13 (m, 1H), 3.05-3.00 (m, 1H), 2.53-2.38 (m, 4H), 2.14-2.04 (m, 1H), 1.56-1.43 (m, 4H), 1.41-1.34 (m, 2H), 1.22 (d, 3H, *J* = 7.0 Hz), 0.92 (d, 6H, *J* = 6.7 Hz); LC/MS ESI (+): 330.3 (M+1)

### <Example 1-173> Synthesis of 1-(4-isobutyl-3,4-dihydroquinoxaline-1(2H)-yl)-2-(4-methylpiperazin-1-yl)propan-1-one

1-(4-Isobutyl-3,4-dihydroquinoxaline-1 (2H)-yl)-2-(4-methylpiperazin-1-yl)propan-1-one was synthesized in the same manner as in Example 1-171, except that 1-methylpiperazine was used instead of pyrrolidine.
Brown oil (yield 89.5%); ¹H NMR (400 MHz, CDCl₃) δ 7.14 (d, 1H, *J* = 7.0 Hz), 7.04-6.99 (m, 1H), 6.66 (d, 1H, *J* = 8.2 Hz), 6.58-6.56 (m, 1H), 4.02-3.96 (m, 2H), 3.79-3.73 (m, 1H), 3.48-3.40 (m, 2H), 3.15-3.02 (m, 2H), 2.58-2.37 (m, 8H), 2.24 (s, 3H), 2.13-2.03 (m, 1H), 1.21 (d, 3H, *J* = 6.7 Hz), 0.92 (d, 6H, *J* = 6.7 Hz); LC/MS ESI (+): 345.3 (M+1)

### <Example 1-174> Synthesis of 1-(4-isobutyl-3,4-dihydroquinoxaline-1(2H)-yl)-2-(pyrrolidin-1-yl)ethan-1-one

1-(4-Isobutyl-3,4-dihydroquinoxaline-1(2H)-yl)-2-(pyrrolidin-1-yl)ethan-1-one was synthesized in the same manner as in Example 1-161, except that a yellow oily compound, 2-bromo-1-(4-isobutyl-3,4-dihydroquinoxalin-1(2H)-yl)ethanone, was obtained using bromoacetyl chloride instead of 3-bromopropionyl chloride, and then pyrrolidine was used.
Pale yellow oil (yield 66.1%); ¹H NMR (400 MHz, CDCl₃) δ 17.17-7.11 (m, 1H), 7.06-7.03 (m, 1H), 6.66 (d, 1H, *J =* 8.2 Hz), 6.61-6.57 (m, 1H), 3.88 (t, 2H, *J* = 5.5 Hz), 3.47 (s, 2H), 3.40 (t, 2H, *J* = 5.1 Hz), 3.08 (d, 2H, *J* = 7.4 Hz), 2.63-2.59 (m, 4H), 2.13-2.06 (m, 1H), 1.81-1.75 (m, 4H), 0.92 (d, 6H, *J* = 6.7 Hz); LC/MS ESI (+): 302.3 (M+1)

### <Example 1-175> Synthesis of 1-(4-isobutyl-3,4-dihydroquinoxaline-1(2H)-yl)-2-(piperidin-1-yl)ethan-1-one

1-(4-Isobutyl-3,4-dihydroquinoxaline-1(2H)-yl)-2-(piperidin-1-yl)ethan-1-one was synthesized in the same manner as in Example 1-174, except that piperidine was used instead of pyrrolidine.
Pale yellow oil (yield 72.5%); ¹H NMR (400 MHz, CDCl₃) δ 7.20-7.12 (m, 1H), 7.24-7.02 (m, 1H), 6.65 (d, 1H, *J* = 8.2 Hz), 6.61-6.57 (m, 1H), 3.88 (t, 2H, *J* = 5.3 Hz), 3.45-3.37 (m, 2H), 3.29 (s, 2H), 3.08 (d, 2H, *J* = 7.4 Hz), 2.50-2.40 (m, 4H), 2.13-2.06 (m, 1H), 1.61-1.54 (m, 4H), 1.43-1.37 (m, 2H), 0.92 (d, 6H, *J* = 6.7 Hz); LC/MS ESI (+): 316.3 (M+1)

### <Example 1-176> Synthesis of 1-(4-isobutyl-3,4-dihydroquinoxaline-1(2H)-yl)-2-(4-methylpiperazin-1-yl)ethan-1-one

1-(4-lsobutyl-3,4-dihydroquinoxaline-1(2H)-yl)-2-(4-methylpiperazin-1-yl)ethan-1-one was synthesized in the same manner as in Example 1-174, except that 1-methylpiperazine was used instead of pyrrolidine.
Pale yellow oil (yield 63.3%); ¹H NMR (400 MHz, CDCl₃) δ 7.20-7.14 (m, 1H), 7.06-7.02 (m, 1H), 6.66 (d, 1H, *J* = 8.2 Hz), 6.61-6.57 (m, 1H), 3.87 (t, 2H, *J* = 5.3 Hz), 3.43-3.37 (m, 2H), 3.31 (s, 2H), 3.08 (d, 2H, *J* = 7.4 Hz), 2.69-2.31 (m, 8H), 2.27 (s, 3H), 2.13-2.06 (m, 1H), 0.92 (d, 6H, *J=* 6.7 Hz); LC/MS ESI (+): 331.2 (M+1)

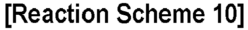

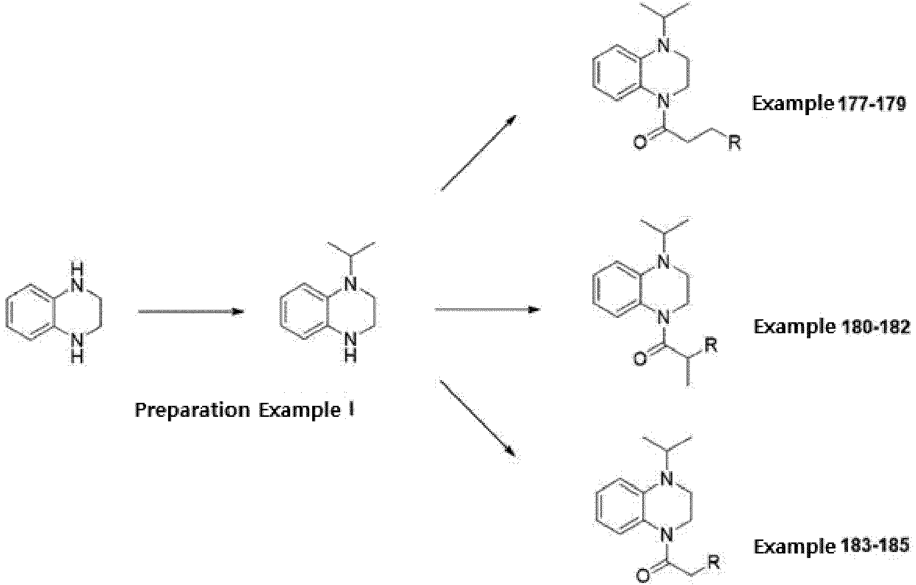

### <Preparation Method I> Preparation of 1-isopropyl-1,2,3,4-tetrahydroquinoxaline

Acetone (0.22 mL, 29.8 mmol) and sodium triacetoxyborohydride (2369 mg, 11.18 mmol) were added to a solution in which 1,2,3,4-tetrahydroquinoxaline (1000 mg, 7.457 mmol) was dissolved in THF (8 mL) and stirred at 70 °C for 24 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-isopropyl-1,2,3,4-tetrahydroquinoxaline.
Brown solid (yield 37%); ¹H NMR (400 MHz, CDCl₃) δ 6.66 (m, 2H), 6.51 (m, 2H), 4.07 (m, 1H), 3.65 (brs, 1H), 3.40 (m, 2H), 3.24 (m, 2H), 1.18 (d, 6H, *J* = 6.7 Hz); LC/MS ESI (+): 177.2 (M+1)

### <Example 1-177> Synthesis of 1-(4-isopropyl-3,4-dihydroquinoxaline-1(2H)-yl)-3-(pyrrolidin-1-yl)propan-1-one

1-lsopropyl-1,2,3,4-tetrahydroquinoxaline (1.0 equiv) obtained in Preparation Example I was dissolved in dichloromethane (0.2 M), 3-bromopropionyl chloride (1.1 equiv) was slowly added at -10 °C, and then the resulting solution was stirred at room temperature for 30 minutes. After terminating the reaction by adding water to the reaction solution, extraction was performed with dichloromethane. An organic layer was washed with brine, dried with MgSO₄, filtered, and concentrated under reduced pressure, thereby synthesizing a brown oily compound, 3-bromo-1-(4-isopropyl-3,4-dihydroquinoxalin-1(2H)-yl)propan-1-one. DIPEA (3.0 equiv) and pyrrolidine (1.5 equiv) were added to a solution in which the obtained brown oily compound (1.0 equiv) was dissolved in THF (0.2 M) and stirred at 70 °C for 3 hours. After cooling to room temperature and terminating the reaction by adding water to the reaction solution, extraction was performed with ethyl acetate. An organic layer was washed with brine, dried with MgSO₄, filtered, and concentrated under reduced pressure. The obtained residue was purified by silica column chromatography using a dichloromethane/methanol mixture, thereby synthesizing 1-(4-isopropyl-3,4-dihydroquinoxaline-1(2H)-yl)-3-(pyrrolidin-1-yl)propan-1-one. Pale yellow solid (yield 12.1%); ¹H NMR (400 MHz, CDCl₃) δ 7.15-7.04 (m, 2H) 6.81-6.75 (m, 1H,), 6.71-6.63 (m, 1H), 4.18-4.04 (m, 1H), 3.81 (t, 2H, *J* = 5.7 Hz), 3.54-3.19 (m, 10H), 2.21-2.01 (m, 4H), 1.20 (d, 6H, *J* = 6.7 Hz); LC/MS ESI (+): 302.2 (M+1)

### <Example 1-178> Synthesis of 1-(4-isopropyl-3,4-dihydroquinoxaline-1(2H)-yl)-3-(piperidin-1-yl)propan-1-one

1-(4-Isopropyl-3,4-dihydroquinoxaline-1(2H)-yl)-3-(piperidin-1-yl)propan-1-one was synthesized in the same manner as in Example 1-177, except that piperidine was used instead of pyrrolidine.
Yellow oil (yield 52.1%); ¹H NMR (400 MHz, CDCl₃) δ 7.12-6.97 (m, 2H), 6.80-6.73 (m, 1H,), 6.64-6.55 (m, 1H,), 4.19-4.05 (m, 1H), 3.82 (t, 2H, *J* = 5.5 Hz), 3.28 (t, 2H, *J* = 5.7 Hz), 2.78-2.66 (m, 4H), 2.39-2.22 (m, 4H), 1.57-1.44 (m, 4H), 1.43-1.32 (m, 2H), 1.19 (d, 6H, *J* = 6.7 Hz); LC/MS ESI (+): 316.3 (M+1)

### <Example 1-179> Synthesis of 1-(4-isopropyl-3,4-dihydroquinoxaline-1(2H)-yl)-3-(4-methylpiperazin-1-yl)propan-1-one

1-(4-Isopropyl-3,4-dihydroquinoxaline-1(2H)-yl)-3-(4-methylpiperazin-1-yl)propan-1-one was synthesized in the same manner as in Example 1-177, except that 1-methylpiperazine was used instead of pyrrolidine.
Yellow oil (yield 34.4%); ¹H NMR (400 MHz, CDCl₃) δ 7.11-6.98 (m, 2H), 6.80-6.73 (m, 1H,), 6.63-6.54 (m, 1H,), 4.17-4.06 (m, 1H), 3.82 (t, 2H, *J* = 5.5 Hz), 3.28 (t, 2H, *J* = 5.5 Hz), 2.78-2.67 (m, 4H), 2.54-2.29 (m, 8H), 2.24 (s, 3H), 1.20 (d, 6H, *J* = 6.7 Hz); LC/MS ESI (+): 331.3 (M+1)

### <Example 1-180> Synthesis of 1-(4-isopropyl-3,4-dihydroquinoxaline-1(2H)-yl)-2-(pyrrolidin-1-yl)propan-1-one

1-(4-Isopropyl-3,4-dihydroquinoxaline-1(2H)-yl)-2-(pyrrolidin-1-yl)propan-1-one was synthesized in the same manner as in Example 1-177, except that 2-bromopropionyl chloride was used instead of 3-bromopropionyl chloride.
Brown oil (yield 36.1%); ¹H NMR (400 MHz, CDCl₃) δ 7.15-6.94 (m, 2H), 6.79 (d, 1 H, *J* = 8.2 Hz), 6.66-6.55 (m, 1H,), 4.21-4.08 (m, 2H), 3.94-3.79 (m, 1H), 3.67-3.47 (m, 1H), 3.33-3.19 (m, 2H), 2.68-2.45 (m, 4H), 1.81-1.69 (m, 4H), 1.30 (d, 3H, *J* = 6.7 H), 1.23-1.15 (m, 6H); LC/MS ESI (+): 302.3 (M+1)

### <Example 1-181> Synthesis of 1-(4-isopropyl-3,4-dihydroquinoxaline-1(2H)-yl)-2-(piperidin-1-yl)propan-1-one

1-(4-Isopropyl-3,4-dihydroquinoxaline-1(2H)-yl)-2-(piperidin-1-yl)propan-1-one was synthesized in the same manner as in Example 1-180, except that piperidine was used instead of pyrrolidine.
Brown oil (yield 32.3%); ¹H NMR (400 MHz, CDCl₃) δ 7.22-7.03 (m, 2H), 6.77 (d, 1H, *J* = 8.2 Hz), 6.68-6.50 (m, 1H,), 4.21-4.05 (m, 2H), 4.02-3.83 (m, 1H), 3.82-3.49 (m, 2H), 3.45-3.22 (m, 2H), 2.66-2.25 (m, 4H), 1.56-1.44 (m, 4H), 1.40-1.30 (m, 2H), 1.21-1.12 (m, 8H); LC/MS ESI (+): 316.3 (M+1)

### <Example 1-182> Synthesis of 1-(4-isopropyl-3,4-dihydroquinoxaline-1(2H)-yl)-2-(4-methylpiperazin-1-yl)propan-1-one

1-(4-Isopropyl-3,4-dihydroquinoxaline-1(2H)-yl)-2-(4-methylpiperazin-1-yl)propan-1-one was synthesized in the same manner as in Example 1-180, except that 1-methylpiperazine was used instead of pyrrolidine.
Brown oil (yield 38.9%); ¹H NMR (400 MHz, CDCl₃) δ 7.22-6.97 (m, 2H,), 6.77 (d, 1H, *J* = 8.2 Hz), 6.62-6.49 (m, 1H,), 4.19-4.07 (m, 1H), 4.06-3.86 (m, 2H), 3.81-3.58 (m, 1H), 3.39-3.24 (m, 2H), 2.66-2.33 (m, 8H), 2.26 (s, 3H), 1.26-1.13 (m, 9H); LC/MS ESI (+): 331.3 (M+1)

### <Example 1-183> Synthesis of 1-(4-isopropyl-3,4-dihydroquinoxaline-1(2H)-yl)-3-(pyrrolidin-1-yl)ethan-1-one

1-(4-Isopropyl-3,4-dihydroquinoxaline-1(2H)-yl)-3-(pyrrolidin-1-yl)ethan-1-one was synthesized in the same manner as in Example 1-177, except that bromoacetyl chloride was used instead of 3-bromopropionyl chloride.
Pale yellow oil (yield 53%); ¹H NMR (400 MHz, CDCl₃) δ 7.23-7.01 (m, 2H), 6.77 (d, 1H, *J* = 8.2 Hz), 6.67-6.54 (m, 1H,), 4.17-4.03 (m, 1H), 3.84 (t, 2H, *J* = 5.5 Hz), 3.46 (s, 2H), 3.29 (t, 2H, *J* = 5.5 Hz), 2.70-2.53 (m, 4H), 1.85-1.69 (m, 4H), 1.22-1.16 (m, 6H); LC/MS ESI (+): 288.2 (M+1)

### <Example 1-184> Synthesis of 1-(4-isopropyl-3,4-dihydroquinoxaline-1(2H)-yl)-2-(piperidin-1-yl)ethan-1-one

1-(4-Isopropyl-3,4-dihydroquinoxaline-1(2H)-yl)-2-(piperidin-1-yl)ethan-1-one was synthesized in the same manner as in Example 1-183, except that piperidine was used instead of pyrrolidine.
Pale yellow oil (yield 58.6%); ¹H NMR (400 MHz, CDCl₃) δ 7.23-7.00 (m, 2H,), 6.81-6.74 (m, 1H,), 6.67-6.52 (m, 1H,), 4.19-4.05 (m, 1H), 3.85 (t, 2H, *J* = 5.3 Hz), 3.40-3.20 (m, 4H), 2.59-2.35 (m, 4H), 1.65-1.53 (m, 4H), 1.47-1.34 (m, 2H), 1.19 (d, 6H, *J* = 6.7 Hz); LC/MS ESI (+): 302.3 (M+1)

### <Example 1-185> Synthesis of 1-(4-isopropyl-3,4-dihydroquinoxaline-1(2H)-yl)-2-(4-methylpiperazin-1-yl)ethan-1-one

1-(4-Isopropyl-3,4-dihydroquinoxaline-1(2H)-yl)-2-(4-methylpiperazin-1-yl)ethan-1-one was synthesized in the same manner as in Example 1-183, except that 1-methylpiperazine was used instead of pyrrolidine.
Pale yellow oil (yield 37.2%); ¹H NMR (400 MHz, CDCl₃) δ 7.25-6.99 (m, 2H,), 6.77 (d, 1H, *J* = 8.2 Hz), 6.67-6.53 (m, 1H,), 4.18-4.07 (m, 1H), 3.84 (t, 2H, *J* = 5.5 Hz), 3.36-3.23 (m, 4H), 2.65-2.41 (m, 8H), 2.30-2.25 (m, 3H), 1.19 (d, 6H, *J* = 6.3 Hz); LC/MS ESI (+): 317.3 (M+1)

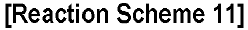

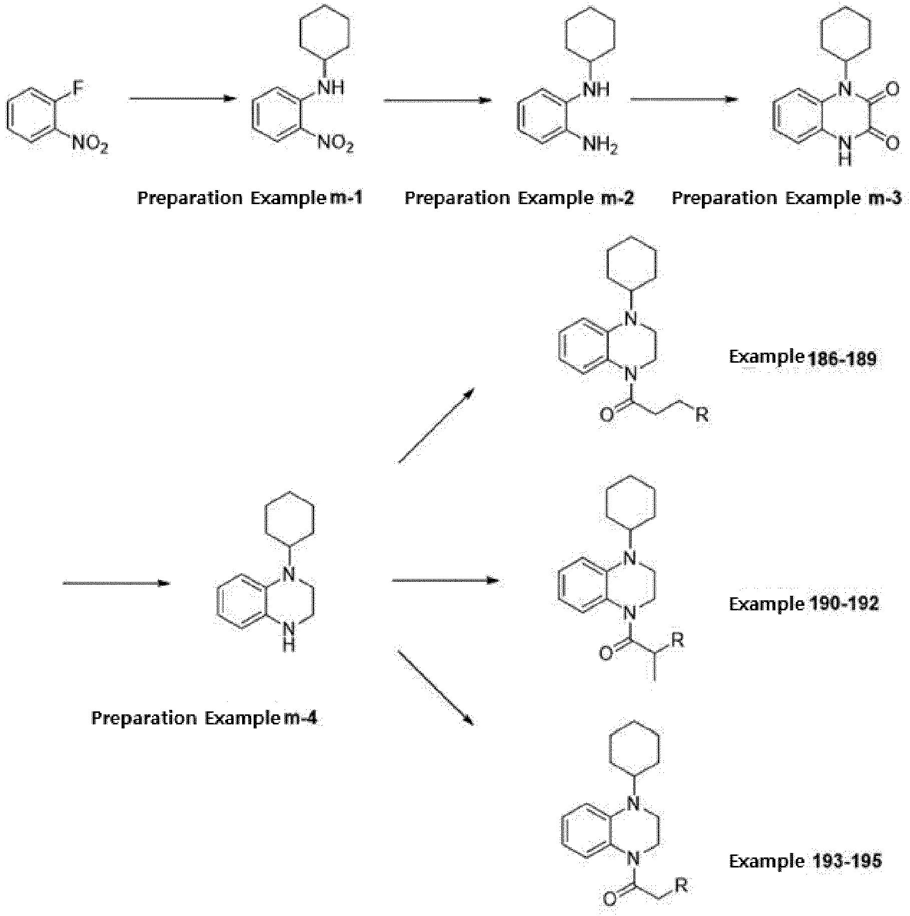

### <Preparation Method m> Preparation of 1-cyclohexyl-1,2,3,4-tetrahydroquinoxaline

1-Cyclohexyl-1,2,3,4-tetrahydroquinoxaline according to the present invention was synthesized through the processes of Preparation Examples m-1 to m-4 below.

### <Preparation Example m-1> Preparation of N-cyclohexyl-2-nitroaniline

A solution in which 1-fluoro-2-nitrobenzene (1 g, 7.087 mmol) and cyclohexylamine (0.973 mL, 8.504 mmol) were mixed was stirred at 110 °C for 6 hours and cooled to room temperature. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing N-cyclohexyl-2-nitroaniline.
Orange solid (yield 99%); ¹H NMR (400 MHz, CDCl₃) δ 8.20-8.08 (m, 2H), 7.42-7.36 (m, 1H), 6.86 (d, 1H, *J* = 8.6 Hz), 6.59 (ddd, 1H, *J* = 8.4, 6.8, 1.2 Hz), 3.55-3.48 (m, 1H), 2.10-2.01 (m, 2H), 1.85-1.76 (m, 2H), 1.70-1.62 (m, 1H), 1.48-1.25 (m, 5H); LC/MS ESI (+): 221.1 (M+1)

### <Preparation Example m-2> Preparation of N¹-cyclohexylbenzene-1,2-diamine

*N*-cyclohexyl-2-nitroaniline (1.55 g, 7.036 mmol) obtained in Preparation Example m-1 was dissolved in EtOAc/MeOH (1:3 = v/v, 20mL), 10% Pd/C (74 mg, 0.704 mmol) was added, and then the resulting solution was stirred under hydrogen gas conditions at room temperature for 6 hours. The reaction solution was filtered through a Celite pad and then concentrated under reduced pressure. The obtained residue was purified by silica column chromatography using an ethyl acetate/hexane mixture, thereby preparing *N*¹-cyclohexylbenzene-1,2-diamine.
Brown oil (yield 86%); ¹H NMR (400 MHz, CDCl₃) δ 6.83-6.77 (m, 1H), 6.74-6.70 (m, 1H), 6.69-6.62 (m, 2H), 3.27 (brs, 2H), 3.26-3.19 (m, 2H), 2.10-2.06 (m, 2H), 1.82-1.73 (m, 2H), 1.69-1.62 (m, 1H), 1.45-1.32 (m, 2H), 1.29-1.16 (m, 3H); LC/MS ESI (+): 191.2 (M+1)

### <Preparation Example m-3> Preparation of 1-cyclohexylquinoxalin-2,3(1H,4H)-dione

A reaction solution in which *N*¹-cyclohexylbenzene-1,2-diamine (1 g, 5.2554 mmol) obtained in Preparation Example m-2 was dissolved in diethyl oxalate (4.28 mL, 31.53 mmol) was stirred at 130 °C for 2 hours. The reaction solution was cooled to room temperature, filtered and dried, thereby preparing 1-cyclohexylquinoxalin-2,3(1H,4H)-dione.
Gray solid (yield 80%); ¹H NMR (400 MHz, DMSO-*d*₆) δ 11.96 (brs, 1H), 7.65-7.56 (m, 1H), 7.19-7.13 (m, 3H), 4.58-4.39 (m, 1H), 2.47-2.38 (m, 2H), 1.85-1.79 (m, 2H), 1.74-1.62 (m, 3H), 1.51-1.38 (m, 2H), 1.31-1.16 (m, 1H); LC/MS ESI (+): 245.1 (M+1)

### <Preparation Example m-4> Preparation of 1-cyclohexyl-1,2,3,4-tetrahydroquinoxaline

A borane-THF complex (1 M) (24 mL, 24.07 mmol) was slowly added to a solution in which 1-cyclohexylquinoxalin-2,3(1H,4H)-dione (980 mg, 4.011 mmol) obtained in Preparation Example m-3 was dissolved in THF (7 mL) at room temperature and then stirred at 65 °C for 16 hours. After cooling the reaction solution to room temperature and terminating the reaction by adding water, the resulting solution was neutralized with a saturated NaHCO₃ aqueous solution and extracted with ethyl acetate. An organic layer was washed with brine, dried with MgSO₄, filtered, and concentrated under reduced pressure. The obtained residue was purified by silica column chromatography using a hexane/ethyl acetate mixture, thereby 1-cyclohexyl-1,2,3,4-tetrahydroquinoxaline.
Orange oil (yield 91%); ¹H NMR (400 MHz, CDCl₃) δ 6.70-6.60 (m, 2H), 6.55-6.47 (m, 2H), 3.61-3.49 (m, 1H), 3.44-3.35 (m, 2H), 3.34-3.24 (m, 2H), 1.92-1.78 (m, 4H), 1.76-1.66 (m, 1H), 1.50-1.31 (m, 4H), 1.22-1.07 (m, 1H); LC/MS ESI (+): 217.2 (M+1)

### <Example 1-186> Synthesis of 1-(4-cyclohexyl-3,4-dihydroquinoxaline-1 (2H)-yl)-3-(pyrrolidin-1 -yl)propan-1 -one

1-Cyclohexyl-1,2,3,4-tetrahydroquinoxaline (1.0 equiv) obtained in Preparation Example m-4 was dissolved in dichloromethane (0.2 M), 3-bromopropionyl chloride (1.1 equiv) was slowly added at -10 °C, and then the resulting solution was stirred at room temperature for 30 minutes. After terminating the reaction by adding water to the reaction solution, extraction was performed with dichloromethane. An organic layer was washed with brine, dried with MgSO₄, filtered, and concentrated under reduced pressure, thereby synthesizing a white solid compound, 3-bromo-1-(4-cyclohexyl-3,4-dihydroquinoxalin-1(2H)-yl)propan-1-one. DIPEA (3.0 equiv) and pyrrolidine (1.5 equiv) were added to a solution in which the obtained white solid compound (1.0 equiv) was dissolved in THF (0.2 M) and stirred at 70 °C for 3 hours. After cooling to room temperature and terminating the reaction by adding water to the reaction solution, extraction was performed with ethyl acetate. An organic layer was washed with brine, dried with MgSO₄, filtered, and concentrated under reduced pressure. The obtained residue was purified by silica column chromatography using a dichloromethane/methanol mixture, thereby synthesizing 1-(4-cyclohexyl-3,4-dihydroquinoxaline-1(2H)-yl)-3-(pyrrolidin-1-yl)propan-1-one.
Ivory solid (yield 55.1%); ¹H NMR (400 MHz, CDCl₃) δ 7.11-6.93 (m, 2H), 6.77-6.69 (m, 1H), 6.63-6.54 (m, 1H), 3.82 (t, 2H, *J* = 5.3 Hz), 3.65-3.54 (m, 1H), 3.31 (t, 2H, *J* = 5.5 Hz), 2.87-2.72 (m, 4H), 2.54-2.32 (m, 4H), 1.91-1.77 (m, 4H), 1.75-1.68 (m, 5H), 1.52-1.30 (m, 4H), 1.22-1.08 (m, 1H); LC/MS ESI (+): 342.4 (M+1)

### <Example 1-187> Synthesis of 1-(4-cyclohexyl-3,4-dihydroquinoxaline-1(2H)-yl)-3-(piperidin-1-yl)propan-1-one

1-(4-Cyclohexyl-3,4-dihydroquinoxaline-1(2H)-yl)-3-(piperidin-1-yl)propan-1-one was synthesized in the same manner as in Example 1-186, except that piperidine was used instead of pyrrolidine.
Ivory solid (yield 59.9%); ¹H NMR (400 MHz, CDCl₃) δ 7.10-6.96 (m, 2H), 6.73 (d, 1H, *J* = 8.2 Hz), 6.62-6.54 (m, 1H), 3.81 (t, 2H, *J*= 5.3 Hz), 3.65-3.55 (m, 1H), 3.31 (t, 2H, *J* = 5.5 Hz), 2.77-2.63 (m, 4H), 2.39-2.20 (m, 4H), 1.91-1.79 (m, 4H), 1.75-1.67 (m, 1H), 1.56-1.31 (m, 10H), 1.21-1.08 (m, 1H); LC/MS ESI (+): 356.4 (M+1)

### <Example 1-188> Synthesis of 1-(4-cyclohexyl-3,4-dihydroquinoxaline-1(2H)-yl)-3-(4-methylpiperazin-1-yl)propan-1-one

1-(4-Cyclohexyl-3,4-dihydroquinoxaline-1(2H)-yl)-3-(4-methylpiperazin-1-yl)propan-1-one was synthesized in the same manner as in Example 1-186, except that 1-methylpiperazine was used instead of pyrrolidine.
Yellow solid (yield 57.5%); ¹H NMR (400 MHz, CDCl₃) δ 7.10-6.95 (m, 3H), 6.72 (d, 1H, *J* = 8.2 Hz), 6.61-6.53 (m, 1H), 3.81 (t, 2H, *J* = 5.3 Hz), 3.65-3.53 (m, 1H), 3.31 (t, 2H, *J* = 5.5 Hz), 2.77-2.65 (m, 4H), 2.61-2.27 (m, 8H), 2.24 (s, 3H), 1.91-1.78 (m, 4H) 1.76-1.69 (m, 1H), 1.52-1.31 (m, 4H), 1.23-1.09 (m, 1H); LC/MS ESI (+): 371.4 (M+1)

### <Example 1-189> Synthesis of 1-(4-cyclohexyl-3,4-dihydroquinoxaline-1(2H)-yl)-3-(piperazin-1-yl)propan-1-one

Tert-butyl 4-(3-(4-cyclohexyl-3,4-dihydroquinoxaline-1(2H)-yl)-3-oxopropyl)piperazin-1-carboxylate was synthesized in the same manner as in Example 1-186, except that 1-Boc-piperazine was used instead of pyrrolidine. The obtained tert-butyl 4-(3-(4-cyclohexyl-3,4-dihydroquinoxaline-1(2H)-yl)-3-oxopropyl)piperazin-1-carboxylate (100 mg, 0.22 mmol) was dissolved in dichloromethane (1 mL), TFA (0.167 mL, 2.19 mmol) was added, and then the resulting solution was stirred at room temperature for 3 hours. After terminating the reaction by adding water to the reaction solution, the resulting solution was neutralized with a saturated NaHCO₃ aqueous solution and extracted with dichloromethane. An organic layer was washed with brine, dried with MgSO₄, filtered, and concentrated under reduced pressure. The obtained residue was purified by silica column chromatography using a hexane/ethyl acetate mixture, thereby synthesizing 1-(4-cyclohexyl-3,4-dihydroquinoxaline-1(2H)-yl)-3-(piperazin-1-yl)propan-1-one.
Pale yellow solid (yield 60%); ¹H NMR (400 MHz, CDCl₃) δ 7.15-6.92 (m, 2H), 6.75 (d, 1H, *J* = 8.1 Hz), 6.66-6.56 (m, 1H), 3.88-3.74 (m, 2H), 3.68-3.56 (m, 1H), 3.40-3.26 (m, 3H), 3.05-2.91 (m, 3H), 2.79-2.65 (m, 4H), 2.55-2.35 (m, 5H), 1.90-1.82 (m, 4H), 1.77-1.68 (m, 1H), 1.52-1.35 (m, 4H), 1.24-1.08 (m, 1H); LC/MS ESI (+): 357.3 (M+1)

### <Example 1-190> Synthesis of 1-(4-cyclohexyl-3,4-dihydroquinoxaline-1(2H)-yl)-2-(pyrrolidin-1-yl)ethan-1-one

1-(4-Cyclohexyl-3,4-dihydroquinoxaline-1(2H)-yl)-2-(pyrrolidin-1-yl)ethan-1-one was synthesized in the same manner as in Example 1-186, except that a brown oily compound, 3-bromo-1-(4-(cyclopropylmethyl)-3,4-dihydroquinoxaline-1(2H)-yl)propan-1-one, was obtained using 2-bromopropionyl chloride instead of 3-bromopropionyl chloride, and then pyrrolidine was used.
Yellow oil (yield 51.8%); ¹H NMR (400 MHz, CDCl₃) δ 7.13-6.93 (m, 2H), 6.75 (d, 1H, *J* = 8.2 Hz), 6.63-6.55 (m, 1H), 4.21-4.00 (m, 1H), 3.94-3.80 (m, 1H), 3.68-3.49 (m, 2H), 3.38-3.26 (m, 2H), 2.66-2.40 (m, 4H), 1.88-1.68 (m, 9H), 1.53-1.25 (m, 7H), 1.21-1.08 (m, 1H); LC/MS ESI (+): 342.3 (M+1)

### <Example 1-191> Synthesis of 1-(4-cyclohexyl-3,4-dihydroquinoxaline-1(2H)-yl)-2-(piperidin-1-yl)propan-1-one

1-(4-Cyclohexyl-3,4-dihydroquinoxaline-1(2H)-yl)-2-(piperidin-1-yl)propan-1-one was synthesized in the same manner as in Example 1-190, except that piperidine was used instead of pyrrolidine.
Yellow oil (yield 40.7%); ¹H NMR (400 MHz, CDCl₃) δ 7.20-6.95 (m, 2H), 6.73 (d, 1H, *J* = 8.2 Hz), 6.63-6.53 (m, 1H), 4.16-4.04 (m, 1H), 4.02-3.88 (m, 1H), 3.69-3.52 (m, 2H), 3.42-3.26 (m, 2H), 2.60-2.35 (m, 4H), 1.90-1.66 (m, 5H), 1.51-1.32 (m, 10H), 1.26-1.18 (m, 3H), 1.18-1.07 (m, 1H); LC/MS ESI (+): 356.3 (M+1)

### <Example 1-192> Synthesis of 1-(4-cyclohexyl-3,4-dihydroquinoxaline-1(2H)-yl)-2-(4-methylpiperazin-1-yl)propan-1-one

1-(4-Cyclohexyl-3,4-dihydroquinoxaline-1(2H)-yl)-2-(4-methylpiperazin-1-yl)propan-1-one was synthesized in the same manner as in Example 1-190, except that 1-methylpiperazine was used instead of pyrrolidine.
Yellow oil (yield 40.7%); ¹H NMR (400 MHz, CDCl₃) δ 7.18-6.99 (m, 2H), 6.74 (d, 1H, *J* = 8.2 Hz), 6.62-6.52 (m, 1H), 4.10-3.88 (m, 2H), 3.73-3.54 (m, 2H), 3.32 (t, 2H, *J* = 5.5 Hz), 2.73-2.29 (m, 8H), 2.24 (s, 3H), 1.88-1.69 (m, 5H), 1.51-1.33 (m, 4H), 1.28-1.18 (m, 3H), 1.17-1.09 (m, 1H); LC/MS ESI (+): 371.4 (M+1)

### <Example 1-193> Synthesis of 1-(4-cyclohexyl-3,4-dihydroquinoxaline-1(2H)-yl)-2-(pyrrolidin-1-yl)ethan-1-one

1-(4-Cyclohexyl-3,4-dihydroquinoxaline-1(2H)-yl)-2-(pyrrolidin-1-yl)ethan-1-one was synthesized in the same manner as in Example 1-186, except that a green oily compound, 2-bromo-1-(4-(cyclopropylmethyl)-3,4-dihydroquinoxaline-1(2H)-yl)propan-1-one, was obtained using bromoacetyl chloride instead of 3-bromopropionyl chloride, and then pyrrolidine was used.
Yellow oil (yield 29.7%); ¹H NMR (400 MHz, CDCl₃) δ 7.21-7.01 (m, 2H), 6.74 (d, 1H, *J* = 8.2 Hz), 6.62-6.55 (m, 1H), 3.83 (t, 2H, *J* = 5.5 Hz), 3.64-3.55 (m, 1H), 3.46 (s, 2H), 3.32 (t, 2H, *J* = 5.5 Hz), 2.69-2.56 (m, 4H), 1.88-1.76 (m, 8H), 1.74-1.69 (m, 1H), 1.51-1.30 (m, 4H), 1.21-1.08 (m, 1H); LC/MS ESI (+): 328.3 (M+1)

### <Example 1-194> Synthesis of 1-(4-cyclohexyl-3,4-dihydroquinoxaline-1(2H)-yl)-2-(piperidin-1-yl)ethan-1-one

1-(4-Cyclohexyl-3,4-dihydroquinoxaline-1(2H)-yl)-2-(piperidin-1-yl)ethan-1-one was synthesized in the same manner as in Example 1-193, except that piperidine was used instead of pyrrolidine.
White solid (yield 52.2%); ¹H NMR (400 MHz, CDCl₃) δ 7.25-6.98 (m, 2H), 6.73 (d, 1H, *J* = 8.2 Hz), 6.63-6.53 (m, 1H), 3.83 (t, 2H, *J* = 5.1 Hz), 3.65-3.55 (m, 1H), 3.41-3.29 (m, 2H), 3.27 (s, 2H), 2.55-2.33 (m, 4H), 1.92-1.76 (m, 4H), 1.74-1.67 (m, 1H), 1.61-1.52 (m, 4H), 1.51-1.31 (m, 6H), 1.21-1.08 (m, 1H); LC/MS ESI (+): 342.3 (M+1)

### <Example 1-195> Synthesis of 1-(4-cyclohexyl-3,4-dihydroquinoxaline-1(2H)-yl)-2-(4-methylpiperazin-1-yl)ethan-1-one

1-(4-Cyclohexyl-3,4-dihydroquinoxaline-1(2H)-yl)-2-(4-methylpiperazin-1-yl)ethan-1-one was synthesized in the same manner as in Example 1-193, except that 1-methylpiperazine was used instead of pyrrolidine.
White solid (yield 52.2%); ¹H NMR (400 MHz, CDCl₃) δ 7.24-7.00 (m, 2H), 6.73 (d, 1H, *J* = 8.2 Hz), 6.62-6.54 (m, 1H), 3.82 (t, 2H, *J* = 5.3 Hz), 3.66-3.54 (m, 1H), 3.35-3.25 (m, 4H), 2.69-2.34 (m, 8H), 2.27 (s, 3H), 1.91-1.76 (m, 4H), 1.76-1.68 (m, 1H), 1.53-1.30 (m, 4H), 1.22-1.08 (m, 1H); LC/MS ESI (+): 357.3 (M+1)

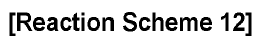

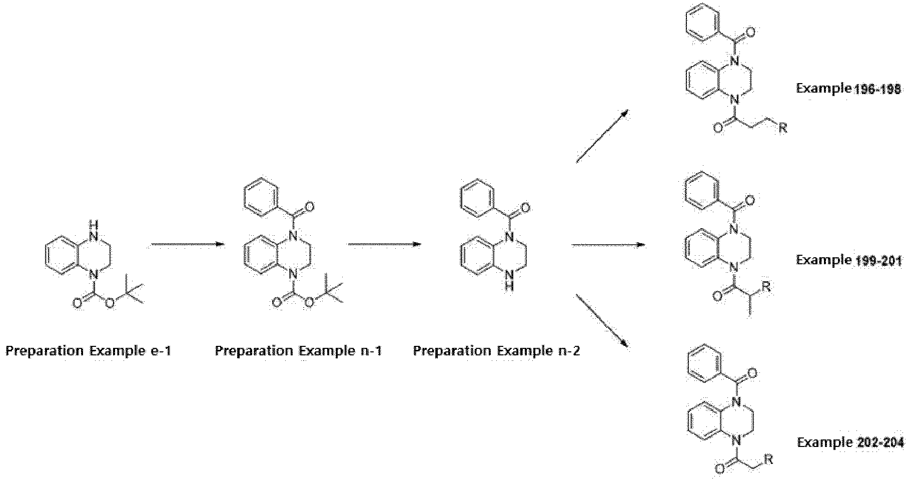

### <Preparation Method n> Preparation of (3,4-dihydroquinoxalin-1(2H)-yl)(phenyl)methanone

(3,4-Dihydroquinoxalin-1(2H)-yl)(phenyl)methanone according to the present invention was synthesized through the processes of Preparation Examples n-1 and n-2 below.

### <Preparation Example n-1> Preparation of tert-butyl 4-benzoyl-3,4-dihydroquinoxalin-1(2H)-carboxylate

Tert-butyl-3,4-dihydroquinoxaline-1(2H)-carboxylate (473 mg, 2.019 mmol) obtained in Preparation Example e-1 was dissolved in THF (20 mL), benzoyl chloride (117 µL, 1.010 mmol) was added, and then the resulting solution was stirred at room temperature for 3 hours. After terminating the reaction by adding water to the reaction solution, the resulting solution was neutralized with a saturated NaHCO₃ aqueous solution and extracted with dichloromethane. An organic layer was washed with brine, dried with MgSO₄, filtered, and concentrated under reduced pressure. The obtained residue was purified by silica column chromatography using a hexane/ethyl acetate mixture, thereby preparing tert-butyl 4-benzoyl-3,4-dihydroquinoxalin-1(2H)-carboxylate.
Yellow oil (yield 100%); ¹H NMR (400 MHz, CDCl₃) δ 7.92-7.83 (m, 1H),7.45- 7.34 (m, 3H), 7.31-7.26 (m, 2H), 7.05 (d, 1H, *J* = 1.6 Hz), 6.73 (s, 1H), 6.65-6.54 (m, 1H), 4.09-4.02 (m, 2H), 3.98-3.91 (m, 2H), 1.57 (s, 9H)

### <Preparation Example n-2> Preparation of (3,4-dihydroquinoxalin-1(2H)-yl)(phenyl)methanone

Tert-butyl 4-benzoyl-3,4-dihydroquinoxalin-1(2H)-carboxylate (683 mg, 2.01 8 mmol) obtained in Preparation Example n-1 was dissolved in dichloromethane (20 mL), TFA (1.54 mL, 20.183 mmol) was added and then the resulting solution was stirred at room temperature for 3 hours. After terminating the reaction by adding water to the reaction solution, the resulting solution was neutralized with a saturated NaHCO₃ aqueous solution and extracted with dichloromethane. An organic layer was washed with brine, dried with MgSO₄, filtered, and concentrated under reduced pressure. The obtained residue was purified by silica column chromatography using a hexane/ethyl acetate mixture, thereby preparing (3,4-dihydroquinoxalin-1(2H)-yl)(phenyl)methanone.
Pale yellow oil (yield 92%); ¹H NMR (400 MHz, CDCl₃) δ 7.49-7.42 (m, 2H), 7.42-7.35 (m, 1H), 7.35-7.28 (m, 2H), 6.91-6.83 (m, 1H), 6.59 (dd, 2H, *J* = 8.0, 1.0 Hz), 6.41-6.32 (m, 1H), 4.07 (brs, 1H, NH), 3.98 (t, 2H, *J* = 5.1 Hz), 3.56 (t, 2 H, *J* = 5.1 Hz); LC/MS ESI (+): 239.1 (M+1)

### <Example 1-196> Synthesis of 1-(4-benzoyl-3,4-dihydroquinoxaline-1(2H)-yl)-3-(pyrrolidin-1-yl)propan-1-one

(3,4-Dihydroquinoxalin-1(2H)-yl)(phenyl)methanone (1.0 equiv) obtained in Preparation Example m-2 was dissolved in dichloromethane (0.3 M), 3-bromopropionyl chloride (1.2 equiv) was slowly added at -70 °C, and then the resulting solution was stirred at room temperature for 1.5 hours. TEA (3.0 equiv) and pyrrolidine (1.1 equiv) were added to the reaction solution and stirred at 70 °C for 16 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a dichloromethane/methanol mixture, thereby synthesizing 1-(4-benzoyl-3,4-dihydroquinoxaline-1(2H)-yl)-3-(pyrrolidin-1-yl)propan-1-one.
Pale yellow oil (yield 81%); ¹H NMR (400 MHz, CDCl₃) δ 7.45-7.28 (m, 6H), 7.17-7.09 (m, 1H), 6,96-6.86 (m, 1H), 6.69 (d, 1H, *J* = 7.0 Hz), 4.13-4.02 (m, 4H), 3.33-3.17 (m, 2H), 3.17-3.06 (m, 2H), 3.05-2.75 (m, 4H), 2.03-1.92 (m, 4H); LC/MS ESI (+): 364.1 (M+1)

### <Example 1-197> Synthesis of 1-(4-benzoyl-3,4-dihydroquinoxaline-1(2H)-yl)-3-(piperidin-1-yl)propan-1-one

1-(4-Benzoyl-3,4-dihydroquinoxaline-1(2H)-yl)-3-(piperidin-1-yl)propan-1-one was synthesized in the same manner as in Example 1-196, except that piperidine was used instead of pyrrolidine.
Colorless oil (yield 87%); ¹H NMR (400 MHz, CDCl₃) δ 7.45-7.27 (m, 6H), 7.14-7.05 (m, 1H), 6.92-6.84 (m, 1H), 6.68 (d, 1H, *J* = 7.0 Hz), 4.14-4.01 (m, 4H), 2.87-2.75 (m, 4H), 2.47-2.35 (m., 4H), 1.67-1.53 (m, 4H), 1.48-1.38 (m, 2H); LC/MS ESI (+): 378.3 (M+1)

### <Example 1-198> Synthesis of 1-(4-benzoyl-3,4-dihydroquinoxaline-1(2H)-yl)-3-(4-methylpiperazin-1-yl)propan-1-one

1-(4-Benzoyl-3,4-dihydroquinoxaline-1(2H)-yl)-3-(4-methylpiperazin-1-yl)propan-1-one was synthesized in the same manner as in Example 1-196, except that 1-methylpiperazine was used instead of pyrrolidine.
Pale yellow oil (yield 63%); ¹H NMR (400 MHz, CDCl₃) δ 7.45-7.27 (m, 6H), 7.14-7.06 (m, 1H), 6.95-6.88 (m, 1H), 6.79-6.70 (m, 1H), 4.11-4.02 (m, 4H), 2.88 (d, 2H, *J* = 5.9 Hz), 2.86-2.79 (m, 2H), 2.70-2.45 (m., 8H), 2.34 (s, 3H); LC/MS ESI (+): 393.1 (M+1)

### <Example 1-199> Synthesis of 1-(4-benzoyl-3,4-dihydroquinoxaline-1(2H)-yl)-2-(pyrrolidin-1-yl)propan-1-one

1-(4-Benzoyl-3,4-dihydroquinoxaline-1(2H)-yl)-2-(pyrrolidin-1-yl)propan-1-one was synthesized in the same manner as in Example 1-196, except that 2-bromopropionyl chloride was used instead of 3-bromopropionyl chloride.
Pale yellow oil (yield 72%); ¹H NMR (400 MHz, CDCl₃) δ 7.47-7.27 (m, 6H), 7.14-7.06 (m, 1H), 6.96-6.85 (m, 1H), 6.77-6.66 (m, 1H), 4.29-3.87 (m, 5H), 2.97-2.50 (m, 4H), 1.88-1.73 (m, 4H), 1.43 (d, 3H, *J* = 3.9 Hz); LC/MS ESI (+): 364.1 (M+1)

### <Example 1-200> Synthesis of 1-(4-benzoyl-3,4-dihydroquinoxaline-1(2H)-yl)-2-(piperidin-1-yl)propan-1-one

1-(4-Benzoyl-3,4-dihydroquinoxaline-1(2H)-yl)-2-(piperidin-1-yl)propan-1-one was synthesized in the same manner as in Example 1-199, except that piperidine was used instead of pyrrolidine.
Colorless oil (yield 71%); ¹H NMR (400 MHz, CDCl₃) δ 7.63-7.43 (m, 1H), 7.43-7.33 (m, 3H), 7.30 (d, 2H, *J* = 7.4 Hz), 7.11-7.04 (m, 1H), 6.94-6.79 (m, 1H), 6.77-6.62 (m, 1H), 4.23-4.03 (m, 3H), 4.00-3.75 (m, 2H), 2.65-2.54 (m, 2H), 2.53-2.43 (m, 2H), 1.59-1.50 (m, 4H), 1.46-1.36 (m, 2H), 1.30 (d, 3H, *J* = 6.7 Hz); LC/MS ESI (+): 378.1 (M+1)

### <Example 1-201> Synthesis of 1-(4-benzoyl-3,4-dihydroquinoxaline-1(2H)-yl)-2-(4-methylpiperazin-1-yl)propan-1-one

1-(4-Benzoyl-3,4-dihydroquinoxaline-1(2H)-yl)-2-(4-methylpiperazin-1-yl)propan-1-one was synthesized in the same manner as in Example 1-199, except that 1-methylpiperazine was used instead of pyrrolidine.
Pale yellow oil (yield 60%); ¹H NMR (400 MHz, CDCl₃) δ 7.59-7.44 (m, 1H), 7.44-7.28 (m, 5H), 7.12-7.05 (m, 1H), 6.99-6.84 (m, 1H), 6.83-6.69 (m, 1H), 4.28-3.97 (m, 4H), 3.96-3.72 (m, 1H), 2.84-2.70 (m, 2H), 2.67-2.57 (m, 2H), 2.55-2.44 (m, 4H), 2.31 (s, 3H), 1.31 (d, 3H, *J* = 7.0 Hz); LC/MS ESI (+): 392.8 (M+1)

### <Example 1-202> Synthesis of 1-(4-benzoyl-3,4-dihydroquinoxaline-1(2H)-yl)-2-(pyrrolidin-1-yl)ethan-1-one

1-(4-Benzoyl-3,4-dihydroquinoxaline-1(2H)-yl)-2-(pyrrolidin-1-yl)ethan-1-one was synthesized in the same manner as in Example 1-196, except that bromoacetyl chloride was used instead of 3-bromopropionyl chloride.
Pale yellow oil (yield 72%); ¹H NMR (400 MHz, CDCl₃) δ 7.68-7.44 (m, 1H), 7.43-7.34 (m, 3H), 7.32-7.27 (m, 2H), 7.12-7.05 (m, 1H), 6.92-6.81 (m, 1H), 6.67 (d, 1H, *J* = 7.4 Hz), 4.11-4.07 (m, 4H), 3.56-3.53 (m, 2H), 2.69-2.61 (m. 4H), 1.84-1.76 (m, 4H); LC/MS ESI (+): 350.1 (M+1)

### <Example 1-203> Synthesis of 1-(4-benzoyl-3,4-dihydroquinoxaline-1(2H)-yl)-2-(piperidin-1-yl)ethan-1-one

1-(4-Benzoyl-3,4-dihydroquinoxaline-1(2H)-yl)-2-(piperidin-1-yl)ethan-1-one was synthesized in the same manner as in Example 1-202, except that piperidine was used instead of pyrrolidine.
Pale yellow oil (yield 83%); ¹H NMR (400 MHz, CDCl₃) δ 7.75-7.45 (m, 1H), 7.43-7.33 (m, 3H), 7.30 (d, 2H, *J* = 7.4 Hz), 7.12-7.03 (m, 1H), 6.92-6.78 (m, 1H), 6.68 (d, 1H, *J* = 7.0 Hz), 4.16-4.05 (m, 4H), 3.39-3.33 (m, 2H), 2.56-2.48 (m, 4H), 1.64-1.57 (m, 4H), 1.48-1.40 (m, 2H); LC/MS ESI (+): 364.1 (M+1)

### <Example 1-204> Synthesis of 1-(4-benzoyl-3,4-dihydroquinoxaline-1(2H)-yl)-2-(4-methylpiperazin-1-yl)ethan-1-one

1-(4-Benzoyl-3,4-dihydroquinoxaline-1(2H)-yl)-2-(4-methylpiperazin-1-yl)ethan-1-one was synthesized in the same manner as in Example 1-202, except that 1-methylpiperazine was used instead of pyrrolidine.
Pale yellow oil (yield 43%); ¹H NMR (400 MHz, CDCl₃) δ 7.63-7.45 (m, 1H), 7.44-7.34 (m, 3H), 7.34-7.27 (m, 2H), 7.13-7.05 (m, 1H), 6.93-6.84 (m, 1H), 6.76-6.64 (m, 1H), 4.13-4.06 (m, 4H), 3.43-3.39 (s, 2H), 2.81-2.64 (m, 4H), 2.63-2.47 (m, 4H), 2.35 (s, 3H); LC/MS ESI (+): 379.0 (M+1)

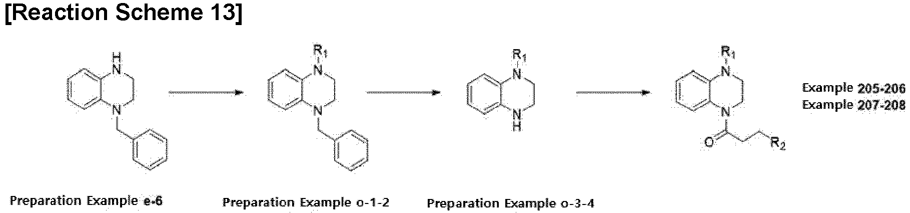

### <Preparation Method o> Preparation of 1-(3,4-dihydroquinoxaline-1(2H)-yl)-3-methylbutan-1-one or 1-(3,4-dihydroquinoxaline-1(2H)-yl)-3,3-dimethylbutan-1-one

1-(3,4-Dihydroquinoxaline-1(2H)-yl)-3-methylbutan-1-one or 1-(3,4-dihydroquinoxaline-1(2H)-yl)-3,3-dimethylbutan-1-one according to the present invention was synthesized through the processes of Preparation Examples o-1 to o-4 below.

### <Preparation Example o-1> Preparation of 1-(4-benzyl-3,4-dihydroquinoxaline-1(2H)-yl)-3-methylbutan-1-one

1-Benzyl-1,2,3,4-tetrahydroquinoxaline (1.0 equiv) obtained in Preparation Example e-6 was dissolved in THF (0.3 M), isovaleryl chloride (1.5 equiv) and triethylamine (2.0 equiv) were slowly added at 0 °C, and then stirred for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-(4-benzyl-3,4-dihydroquinoxaline-1(2H)-yl)-3-methylbutan-1-one.
Colorless oil (yield 100%); ¹H NMR (400 MHz, CD₃CN) δ 7.38-7.30 (m, 2H), 7.30-7.02 (m, 4H), 7.02-6.93 (m, 1H), 6.68 (d, 1H, *J* = 8.2 Hz), 6.65-6.58 (m, 1H), 4.59 (s, 2H), 3.89 (t, 2H, *J* = 5.3 Hz), 3.54-3.42 (m, 2H), 2.42 (d, 2H, *J* = 7.0 Hz), 2.12-2.00 (m, 1H), 0.89 (s, 6H); LC/MS ESI (+): 309.32 (M+1)

### <Preparation Example o-2> Preparation of 1-(4-benzyl-3,4-dihydroquinoxaline-1(2H)-yl)-3,3-dimethylbutan-1-one

1-Benzyl-1,2,3,4-tetrahydroquinoxaline (1.0 equiv) obtained in Preparation Example e-6 was dissolved in THF (0.4 M), 3,3-dimethylbutyryl chloride (1.5 equiv) and triethylamine (2.0 equiv) were slowly added at 0 °C, and stirred for 1.5 hours. After terminating the reaction by adding water to the reaction solution, extraction was performed with dichloromethane. An organic layer was washed with brine, dried with MgSO₄, filtered, and concentrated under reduced pressure. The obtained residue was purified by silica column chromatography using a hexane/ethyl acetate mixture, thereby preparing 1-(4-benzyl-3,4-dihydroquinoxaline-1(2H)-yl)-3,3-dimethylbutan-1-one.
Yellow oil (yield 100%); ¹H NMR (400 MHz, CD₃CN) δ 7.39-7.30 (m, 2H), 7.30-7.04 (m, 4H), 7.02-6.92 (m, 1H), 6.68 (d, 1H, *J* = 8.2 Hz), 6.65-6.58 (m, 1H), 4.58 (s, 2H), 3.89 (t, 2H, *J* = 5.5 Hz), 3.49 (t, 2H, *J* = 5.3 Hz), 2.50 (s, 2H), 0.96 (s, 9H); LC/MS ESI (+): 323.3 (M+1)

### <Preparation Example o-3> Preparation of 1-(3,4-dihydroquinoxaline-1(2H)-yl)-3-methylbutan-1-one

1-(4-Benzyl-3,4-dihydroquinoxaline-1(2H)-yl)-3-methylbutan-1-one (1.0 equiv) obtained in Preparation Example o-1 was dissolved in MeOH/THF (v/v = 2: 1, 0.4 M), 10% Pd/C (0.5 equiv) was added, and stirred under hydrogen gas conditions at room temperature for 1.5 hours. The reaction solution was filtered through a Celite pad and concentrated under reduced pressure. The obtained residue was purified by silica column chromatography using a hexane/ethyl acetate mixture, thereby preparing 1-(3,4-dihydroquinoxaline-1(2H)-yl)-3-methylbutan-1-one.
Yellow solid (yield 92.5%); ¹H NMR (400 MHz, CD₃CN) δ 7.21-6.88 (m, 2H), 6.68-6.53 (m, 2H), 4.78 (brs, 1H, NH), 3.77-3.66 (m, 2H), 3.42-3.31 (m, 2H), 2.42 (d, 2H, *J* = 7.0 Hz), 2.10-2.02 (m, 1H), 0.89 (s, 6H); LC/MS ESI (+): 219.1 (M+1)

### <Preparation Example o-4> Preparation of 1-(3,4-dihydroquinoxaline-1(2H)-yl)-3,3-dimethylbutan-1-one

1-(3,4-Dihydroquinoxaline-1(2H)-yl)-3,3-dimethylbutan-1-one was prepared in the same manner as in Preparation Example o-3, except that 1-(4-benzyl-3,4-dihydroquinoxaline-1(2H)-yl)-3,3-dimethylbutan-1-one obtained in Preparation Example o-2 was used instead of 1-(4-benzyl-3,4-dihydroquinoxaline-1(2H)-yl)-3-methylbutan-1-one.
Yellow solid (yield 80.1%); ¹H NMR (400 MHz, CD₃CN) δ 7.26-6.89 (m, 2H), 6.66-6.52 (m, 2H), 4.78 (brs, 1H, NH), 3.49-3.44 (m, 2H), 3.43-3.38 (m, 2H), 2.51 (s, 2H), 0.96 (s, 9H); LC/MS ESI (+): 233.1 (M+1)

### <Example 1-205> Synthesis of 3-methyl-1-(4-(3-(pyrrolidin-1-yl)propanoyl)-3,4-dihydroquinoxaline-1(2H)-yl)butan-1-one

1-(Pyridin-3-yl)-1,2,3,4-tetrahydroquinoxaline (1.0 equiv) obtained in Preparation Example o-3 was dissolved in dichloromethane (0.2 M), 3-bromopropionyl chloride (1.1 equiv) was slowly added at -0 °C, and then the resulting solution was stirred at 50 °C for 6 hours. The reaction solution was cooled to room temperature, pyrrolidine (1.1 equiv) and TEA (2.0 equiv) were added, and then the resulting solution was stirred at 55 °C for 5 hours. The reaction solution was poured into water and extracted with DCM. After terminating the reaction by adding water to the reaction solution, extraction was performed with dichloromethane. The obtained residue was purified by silica column chromatography using a hexane/ethyl acetate mixture, thereby synthesizing 3-methyl-1-(4-(3-(pyrrolidin-1-yl)propanoyl)-3,4-dihydroquinoxaline-1(2H)-yl)butan-1-one.
Colorless oil (yield 32.4%); ¹H NMR (400 MHz, CDCl₃) δ 7.52-7.15 (m, 4H), 3.97 (s, 4H), 2.89-2.79 (m, 2H), 2.79-2.70 (m, 2H), 2.57-2.45 (m, 4H), 2.42 (d, 2H, *J* = 7.0 Hz), 2.26-2.12 (m, 1H), 1.85-1.71 (m, 4H), 1.00-0.80 (m, 6H); LC/MS ESI (+): 344.3 (M+1)

### <Example 1-206> Preparation of 3-methyl-1-(4-(3-(4-methylpiperazin-1-yl)propanoyl)-3,4-dihydroquinoxaline-1(2H)-yl)butan-1-one

3-Methyl-1-(4-(3-(4-methylpiperazin-1-yl)propanoyl)-3,4-dihydroquinoxaline-1(2H)-yl)butan-1-one was prepared in the same manner as in Example 205, except that 1-methylpiperazine was used instead of pyrrolidine.
Colorless oil (yield 7.2%); ¹H NMR (400 MHz, CD₃OD) δ 7.76-7.18 (m, 4H), 4.04-3.89 (m, 4H), 2.84-2.66 (m, 4H), 2.59-2.36 (m, 8H), 2.33-2.26 (m, 3H), 2.23-1.96 (m, 2H), 1.39-1.23 (m, 1H), 1.01-0.76 (m, 6H); LC/MS ESI (+): 373.3 (M+1)

### <Example 1-207> Synthesis of 3,3-dimethyl-1-(4-(3-(pyrrolidin-1-yl)propanoyl)-3,4-dihydroquinoxaline-1(2H)-yl)butan-1-one

1-(3,4-Dihydroquinoxaline-1(2H)-yl)-3,3-dimethylbutan-1-one (1.0 equiv) obtained in Preparation Example o-4 was dissolved in dichloromethane (0.43 M), 3-bromopropionyl chloride (1.2 equiv) was slowly added at -0 °C, and then the resulting solution was stirred at 50 °C for 7 hours. The reaction solution was cooled to room temperature, pyrrolidine (1.1 equiv) and TEA (2.0 equiv) were added, and then the resulting solution was stirred at 55 °C for 7 hours. After terminating the reaction by adding water to the reaction solution, extraction was performed with dichloromethane. The obtained residue was purified by silica column chromatography using a hexane/ethyl acetate mixture, thereby synthesizing 3,3-dimethyl-1-(4-(3-(pyrrolidin-1-yl)propanoyl)-3,4-dihydroquinoxaline-1(2H)-yl)butan-1-one.
Colorless oil (yield 30.0%); ¹H NMR (400 MHz, CDCl₃) δ 7.53-7.11 (m, 4H), 4.03-3.87 (m, 4H), 2.94-2.81 (m, 2H), 2.80-2.70 (m, 2H), 2.61-2.51 (m, 4H), 2.49 (s, 2H), 1.82-1.78 (m, 4H), 0.96 (s, 9H); LC/MS ESI (+): 358.2 (M+1)

### <Example 1-208> Synthesis of 3,3-dimethyl-1-(4-(3-(4-methylpiperazin-1-yl)propanoyl)-3,4-dihydroquinoxaline-1(2H)-yl)butan-1-one

3,3-Dimethyl-1-(4-(3-(4-methylpiperazin-1-yl)propanoyl)-3,4-dihydroquinoxaline-1(2H)-yl)butan-1-one was synthesized in the same manner as in Example 207, except that 1-methylpiperazine was used instead of pyrrolidine.
Colorless oil (yield 36.5%); ¹H NMR (400 MHz, CDCl₃) δ 7.54-7.14 (m, 4H), 4.01-3.92 (m, 4H), 2.82-2.74 (m, 2H), 2.74-2.66 (m, 2H), 2.56-2.40 (m, 8H), 2.29 (s, 3H), 1.61 (s, 2H), 0.97 (s, 9H); LC/MS ESI (+): 387.4 (M+1)

Compounds 1 to 208 according to the present invention are shown in Table 1 below.

**[Table 1]**

| No. | Structure | IUPAC name |
|---|---|---|
| 1 | 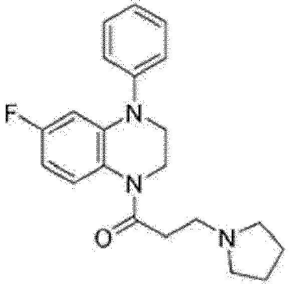 | 1-(6-fluoro-4-phenyl-3,4-dihydroquinoxaline-1(2H)-yl)-3-(pyrrolidin-1-yl)propan-1-one |
| 2 | 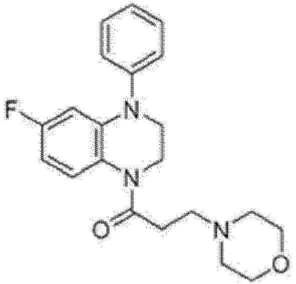 | 1-(6-fluoro-4-phenyl-3,4-dihydroquinoxaline-1(2H)-yl)-3-morpholinopropan-1-one |
| 3 | 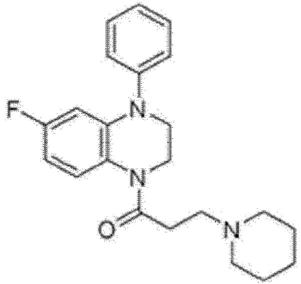 | 1-(6-fluoro-4-phenyl-3,4-dihydroquinoxaline-1(2H)-yl)-3-(piperidin-1-yl)propan-1-one |
| 4 | 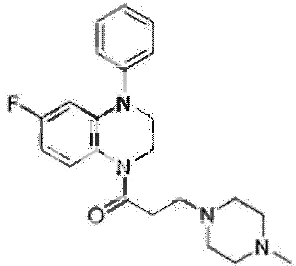 | 1-(6-fluoro-4-phenyl-3,4-dihydroquinoxaline-1(2H)-yl)-3-(4-methylpiperazin-1-yl)propan-1-one |
| 5 | 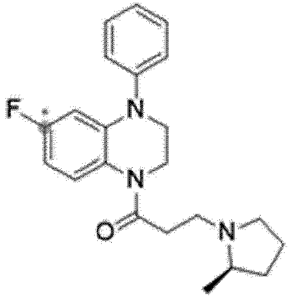 | (R)-1-(6-fluoro-4-phenyl-3,4-dihydroquinoxaline-1(2H)-yl)-3-(2-methylpyrrolidin-1-yl)propan-1-one |
| 6 | 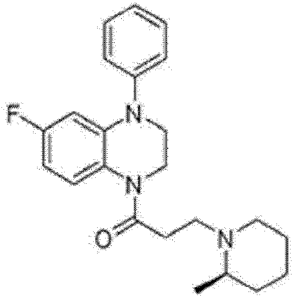 | (R)-1-(6-fluoro-4-phenyl-3,4-dihydroquinoxaline-1(2H)-yl)-3-(2-methylpiperidin-1-yl)propan-1-one |
| 7 | 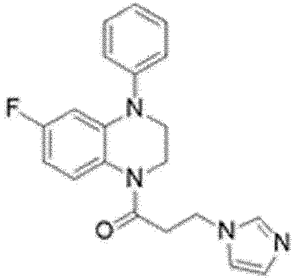 | 1-(6-fluoro-4-phenyl-3,4-dihydroquinoxaline-1(2H)-yl)-3-(1H-imidazol-1-yl)propan-1-one |
| 8 | 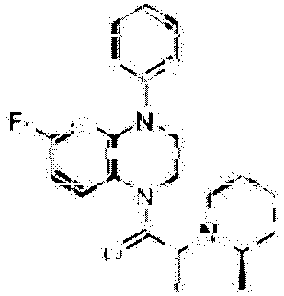 | 1-(6-fluoro-4-phenyl-3,4-dihydroquinoxaline-1(2H)-yl)-2-((R)-2-methylpiperidin-1-yl)propan-1-one |
| 9 | 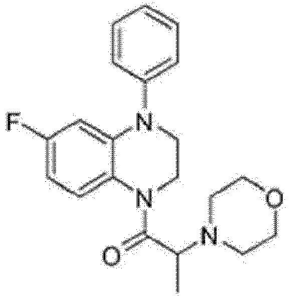 | 1-(6-fluoro-4-phenyl-3,4-dihydroquinoxaline-1(2H)-yl)-2-morpholinopropan-1-one |
| 10 | 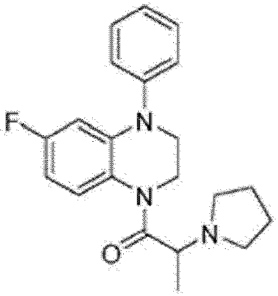 | 1-(6-fluoro-4-phenyl-3,4-dihydroquinoxaline-1(2H)-yl)-2-(pyrrolidin-1-yl)propan-1-one |
| 11 | 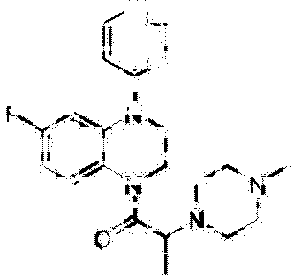 | 1-(6-fluoro-4-phenyl-3,4-dihydroquinoxaline-1(2H)-yl)-2-(4-methylpiperazin-1-yl)propan-1-one |
| 12 | 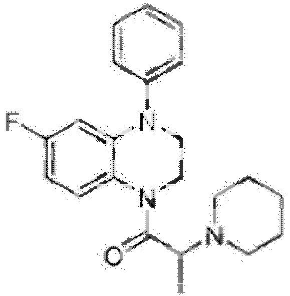 | 1-(6-fluoro-4-phenyl-3,4-dihydroquinoxaline-1(2H)-yl)-2-(piperidin-1-yl)propan-1-one |
| 13 | 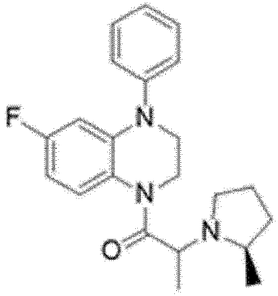 | 1-(6-fluoro-4-phenyl)-3,4-dihydroquinoxaline-1(2H)-yl)-2-((R)-2-methylpyrrolidin-1-yl)propan-1-one |
| 14 | 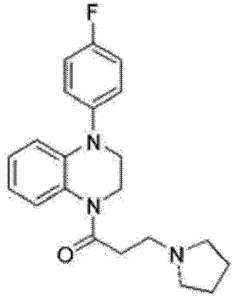 | 1-(4-(4-fluorophenyl)-3,4-dihydroquinoxaline-1(2H)-yl)-3-(pyrrolidin-1-yl)propan-1-one |
| 15 | 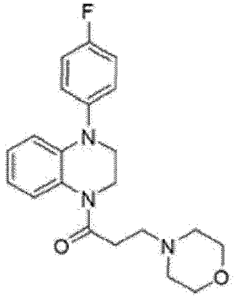 | 1-(4-(4-fluorophenyl)-3,4-dihydroquinoxaline-1(2H)-yl)-3-morpholinopropan-1-one |
| 16 | 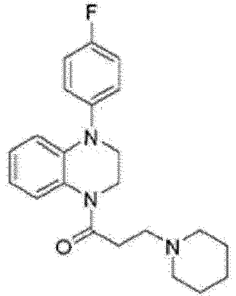 | 1-(4-(4-fluorophenyl)-3,4-dihydroquinoxaline-1(2H)-yl)-3-(piperidin-1-yl)propan-1-one |
| 17 | 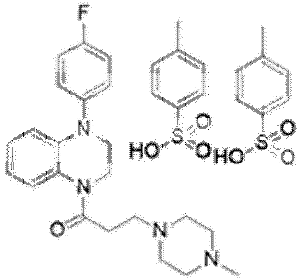 | 1-(4-(4-fluorophenyl)-3,4-dihydroquinoxaline-1(2H)-yl)-3-(4-methylpiperazin-1-yl)propan-1-one di-p-toluene sulfonate |
| 18 | 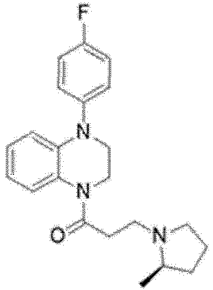 | (R)-1-(4-(4-fluorophenyl)-3,4-dihydroquinoxaline-1(2H)-yl)-3-(2-methylpyrrolidin-1-yl)propan-1-one |
| 19 | 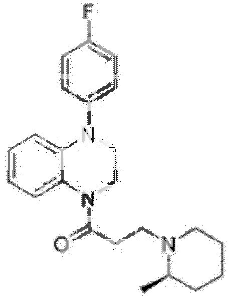 | (R)-1-(4-(4-fluorophenyl)-3,4-dihydroquinoxaline-1(2H)-yl)-3-(2-methylpiperidin-1-yl)propan-1-one |
| 20 | 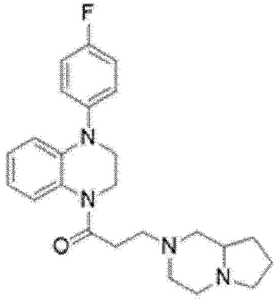 | 1-(4-(4-fluorophenyl)-3,4-dihydroquinoxaline-1(2H)-yl)-3-(hexahydropyrrolo[1,2-a]pyrazin-2(1H)-yl)propan-1-one |
| 21 | 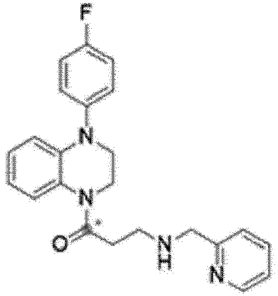 | 1-(4-(4-fluorophenyl)-3,4-dihydroquinoxaline-1(2H)-yl)-3-((pyridin-2-ylmethyl)amino)propan-1-one |
| 22 | 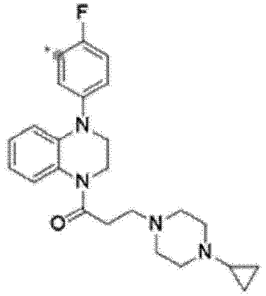 | 3-(4-cyclopropylpiperazin-1-yl)-1-(4-(4-fluorophenyl)-3,4-dihydroquinoxaline-1(2H)-yl)propan-1-one |
| 23 | 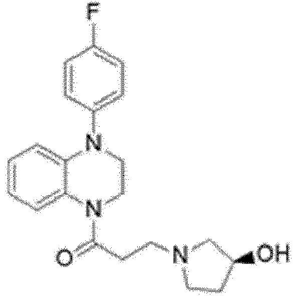 | (S)-1-(4-(4-fluorophenyl)-3,4-dihydroquinoxaline-1(2H)-yl)-3-(3-hydroxypyrrolidin-1-yl)propan-1-one |
| 24 | 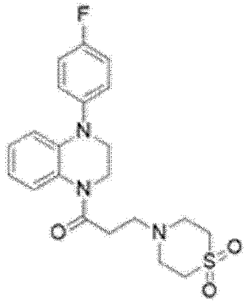 | 3-(1,1-dioxidothiomorpholino)-1-(4-(4-fluorophenyl)-3,4-dihydroquinoxaline-1(2H)-yl)propan-1-one |
| 25 | 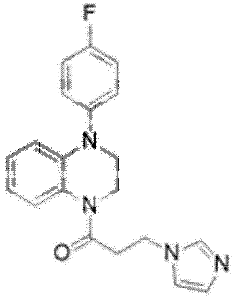 | 1-(4-(4-fluorophenyl)-3,4-dihydroquinoxaline-1(2H)-yl)-3-(1H-imidazol-1-yl)propan-1-one |
| 26 | 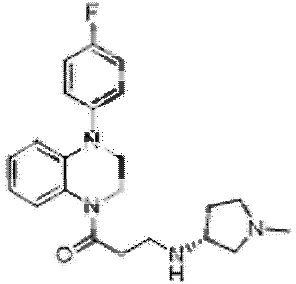 | (R)-1-(4-(4-fluorophenyl)-3,4-dihydroquinoxaline-1(2H)-yl)-3-((1-methylpyrrolidin-3-yl)amino)propan-1-one |
| 27 | 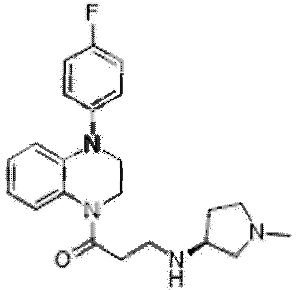 | (S)-1-(4-(4-fluorophenyl)-3,4-dihydroquinoxaline-1(2H)-yl)-3-((1-methylpyrrolidin-3-yl)amino)propan-1-one |
| 28 | 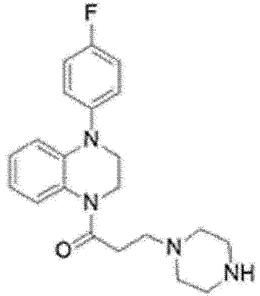 | 1-(4-(4-fluorophenyl)-3,4-dihydroquinoxaline-1(2H)-yl)-3-(piperazin-1-yl)propan-1-one |
| 29 | 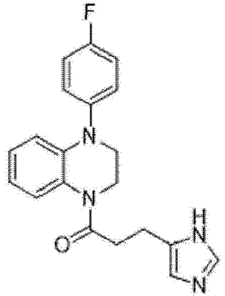 | 1-(4-(4-fluorophenyl)-3,4-dihydroquinoxaline-1(2H)-yl)-3-(1H-imidazol-5-yl)propan-1-one |
| 30 | 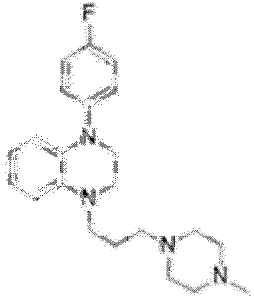 | 1-(4-fluorophenyl)-4-(3-(4-methylpiperazin-1-yl)propyl)-1,2,3,4-tetrahydroquinoxaline |
| 31 | 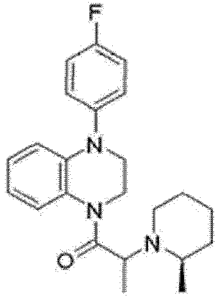 | 1-(4-(4-fluorophenyl)-3,4-dihydroquinoxaline-1(2H)-yl)-2-((R)-2-methylpiperidin-1-yl)propan-1-one |
| 32 | 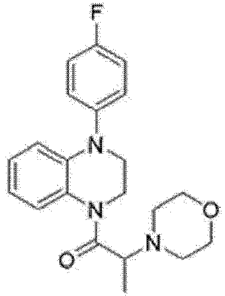 | 1-(4-(4-fluorophenyl)-3,4-dihydroquinoxaline-1(2H)-yl)-2-morpholinopropan-1-one |
| 33 | 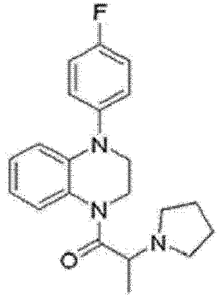 | 1-(4-(4-fluorophenyl)-3,4-dihydroquinoxaline-1(2H)-yl)-2-(pyrrolidin-1-yl)propan-1-one |
| 34 | 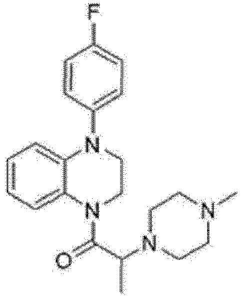 | 1-(4-(4-fluorophenyl)-3,4-dihydroquinoxaline-1(2H)-yl)-2-(4-methylpiperazin-1-yl)propan-1-one |
| 35 | 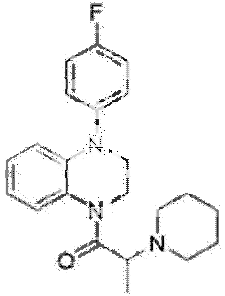 | 1-(4-(4-fluorophenyl)-3,4-dihydroquinoxaline-1(2H)-yl)-2-(piperidin-1-yl)propan-1-one |
| 36 | 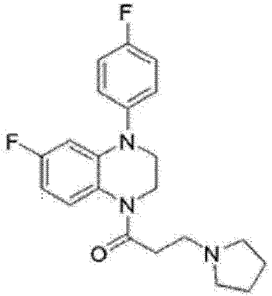 | 1-(6-fluoro-4-(4-fluorophenyl)-3,4-dihydroquinoxaline-1(2H)-yl)-3-(pyrrolidin-1-yl)propan-1-one |
| 37 | 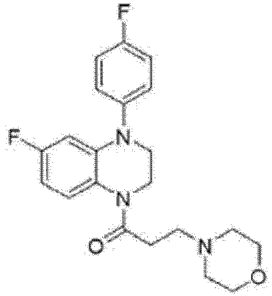 | 1-(6-fluoro-4-(4-fluorophenyl)-3,4-dihydroquinoxaline-1(2H)-yl)-3-morpholinopropan-1-one |
| 38 | 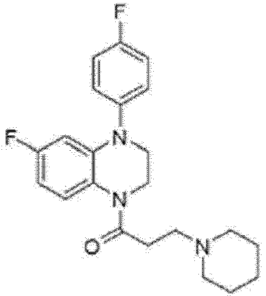 | 1-(6-fluoro-4-(4-fluorophenyl)-3,4-dihydroquinoxaline-1(2H)-yl)-3-(piperidin-1-yl)propan-1-one |
| 39 | 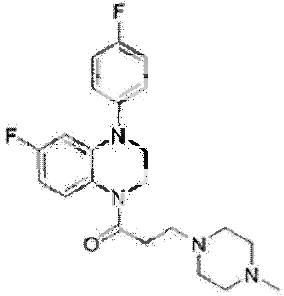 | 1-(6-fluoro-4-(4-fluorophenyl)-3,4-dihydroquinoxaline-1(2H)-yl)-3-(4-methylpiperazin-1-yl)propan-1-one |
| 40 | 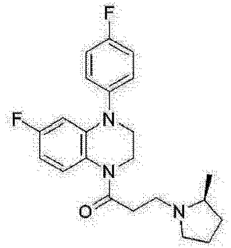 | (R)-1-(6-fluoro-4-(4-fluorophenyl)-3,4-dihydroquinoxaline-1(2H)-yl)-3-(2-methylpyrrolidin-1-yl)propan-1-one |
| 41 | 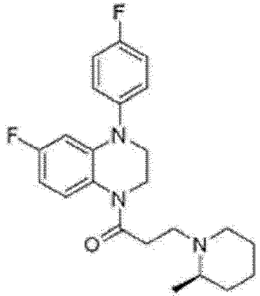 | (R)-1-(6-fluoro-4-(4-fluorophenyl)-3,4-dihydroquinoxaline-1(2H)-yl)-3-(2-methylpiperidin-1-yl)propan-1-one |
| 42 | 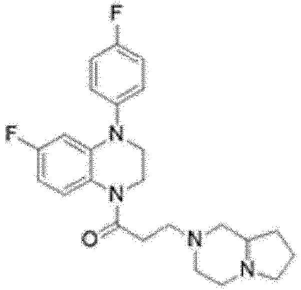 | 1-(6-fluoro-4-(4-fluorophenyl)-3,4-dihydroquinoxaline-1(2H)-yl)-3-(hexahydropyrrolo[1,2-a]pyrazin-2(1H)-yl)propan-1-one |
| 43 | 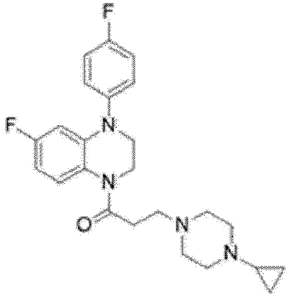 | 3-(4-cyclopropylpiperazin-1-yl)-1-(6-fluoro-4-(4-fluorophenyl)-3,4-dihydroquinoxaline-1(2H)-yl)propan-1-one |
| 44 | 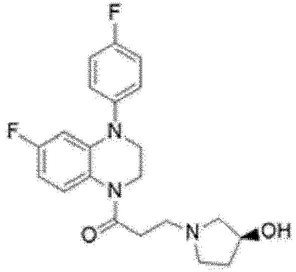 | (S)-1-(6-fluoro-4-(4-fluorophenyl)-3,4-dihydroquinoxaline-1(2H)-yl)-3-(3-hydroxypyrrolidin-1-yl)propan-1-one |
| 45 | 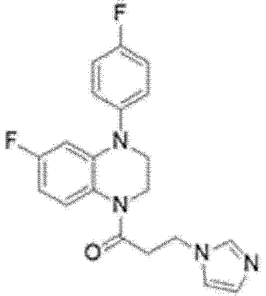 | 1-(6-fluoro-4-(4-fluorophenyl)-3,4-dihydroquinoxaline-1(2H)-yl)-3-(1H-imidazol-1-yl)propan-1-one |
| 46 | 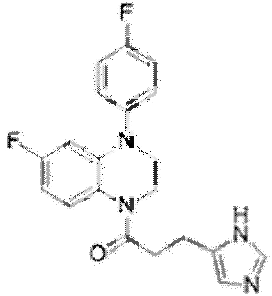 | 1-(6-fluoro-4-(4-fluorophenyl)-3,4-dihydroquinoxaline-1(2H)-yl)-3-(1H-imidazol-5-yl)propan-1-one |
| 47 | 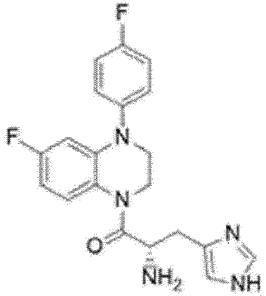 | (S)-2-amino-1-(6-fluoro-4-(4-fluorophenyl)-3,4-dihydroquinoxaline-1(2H)-yl)-3-(1H-imidazol-4-yl)propan-1-one |
| 48 | 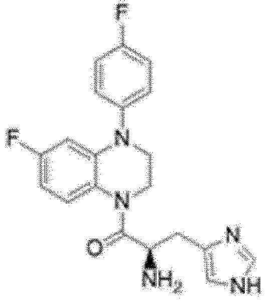 | (R)-2-amino-1-(6-fluoro-4-(4-fluorophenyl)-3,4-dihydroquinoxaline-1(2H)-yl)-3-(1H-imidazol-4-yl)propan-1-one |
| 49 | 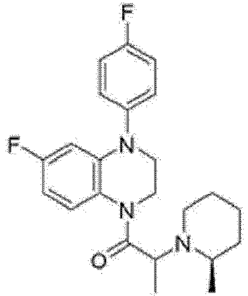 | 1-(6-fluoro-4-(4-fluorophenyl)-3,4-dihydroquinoxaline-1(2H)-yl)-2-((R)-2-methylpiperidin-1-yl)propan-1-one |
| 50 | 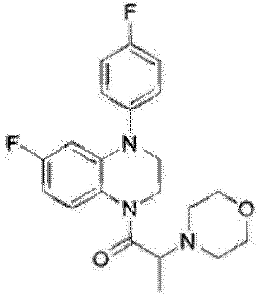 | 1-(6-fluoro-4-(4-fluorophenyl)-3,4-dihydroquinoxaline-1(2H)-yl)-2-morpholinopropan-1-one |
| 51 | 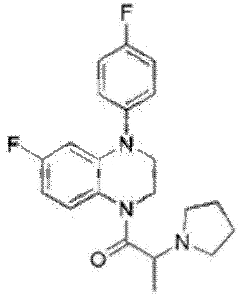 | 1-(6-fluoro-4-(4-fluorophenyl)-3,4-dihydroquinoxaline-1(2H)-yl)-2-(pyrrolidin-1-yl)propan-1-one |
| 52 | 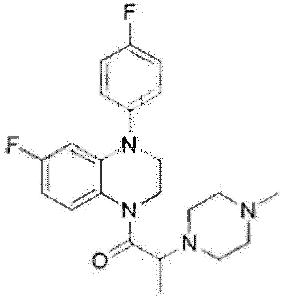 | 1-(6-fluoro-4-(4-fluorophenyl)-3,4-dihydroquinoxaline-1(2H)-yl)-2-(4-methylpiperazin-1-yl)propan-1-one |
| 53 | 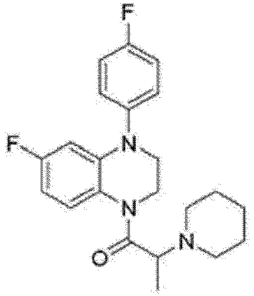 | 1-(6-fluoro-4-(4-fluorophenyl)-3,4-dihydroquinoxaline-1(2H)-yl)-2-(piperidin-1-yl)propan-1-one |
| 54 | 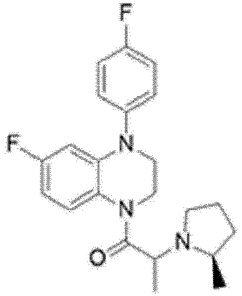 | 1-(6-fluoro-4-(4-fluorophenyl)-3,4-dihydroquinoxaline-1(2H)-yl)-2-((R)-2-methylpyrrolidin-1-yl)propan-1-one |
| 55 | 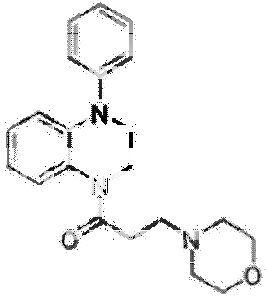 | 3-morpholino-1-(4-phenyl-3,4-dihydroquinoxaline-1(2H)-yl)propan-1-one |
| 56 | 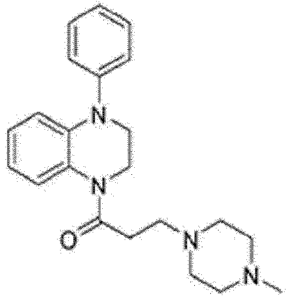 | 3-(4-methylpiperazin-1-yl)-1-(4-phenyl-3,4-dihydroquinoxaline-1(2H)-yl)propan-1-one |
| 57 | 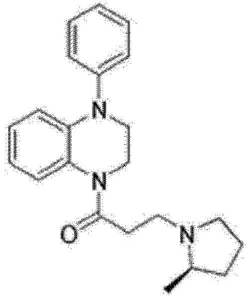 | (R)-3-(2-methylpyrrolidin-1-yl)-1-(4-phenyl-3,4-dihydroquinoxaline-1(2H)-yl)propan-1-one |
| 58 | 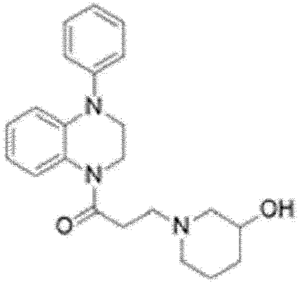 | 3-(3-hydroxypiperidin-1-yl)-1-(4-phenyl-3,4-dihydroquinoxaline-1(2H)-yl)propan-1-one |
| 59 | 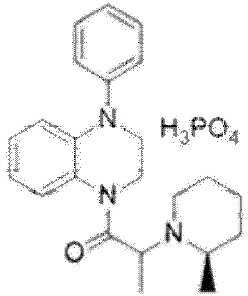 | 2-((R)-2-methylpiperidin-1-yl)-1-(4-phenyl-3,4-dihydroquinoxaline-1(2H)-yl)propan-1-one phosphate |
| 60 | 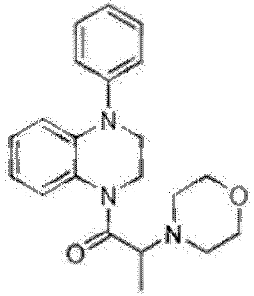 | 2-morpholino-1-(4-phenyl-3,4-dihydroquinoxaline-1(2H)-yl)propan-1-one |
| 61 | 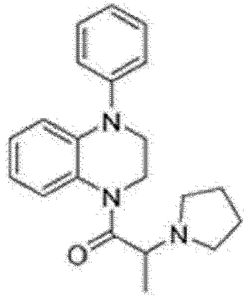 | 1-(4-phenyl-3,4-dihydroquinoxaline-1(2H)-yl)-2-(pyrrolidin-1-yl)propan-1-one |
| 62 | 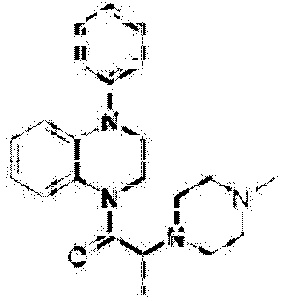 | 2-(4-methylpiperazin-1-yl)-1-(4-phenyl-3,4-dihydroquinoxaline-1(2H)-yl)propan-1-one |
| 63 | 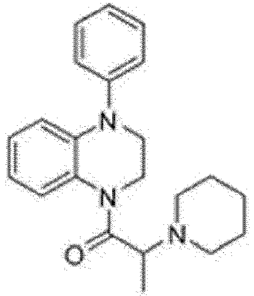 | 1-(4-phenyl-3,4-dihydroquinoxaline-1(2H)-yl)-2-(piperidin-1-yl)propan-1-one |
| 64 | 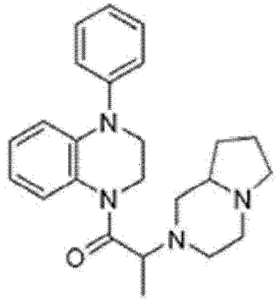 | 2-(hexahydropyrrolo[1,2-a]pyrazin-2(1H)-yl)-1-(4-phenyl-3,4-dihydroquinoxaline-1(2H)-yl)propan-1-one |
| 65 | 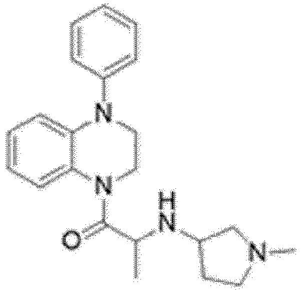 | 2-((1-methylpyrrolidin-3-yl)amino)-1-(4-phenyl-3,4-dihydroquinoxaline-1(2H)-yl)propan-1-one |
| 66 | 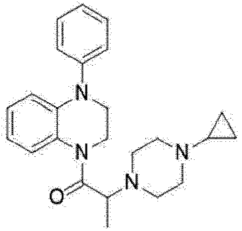 | 2-(4-cyclopropylpiperazin-1-yl)-1-(4-phenyl-3,4-dihydroquinoxaline-1(2H)-yl)propan-1-one |
| 67 | 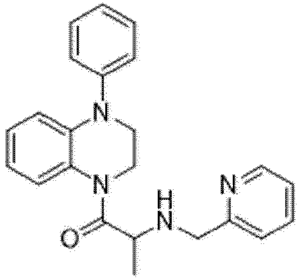 | 2-methyl-1-(4-phenyl-3,4-dihydroquinoxaline-1(2H)-yl)-3-((pyridin-2-ylmethyl)amino)propan-1-one |
| 68 | 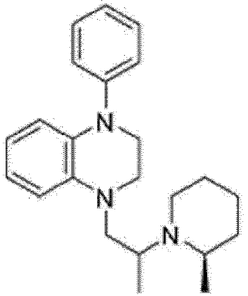 | 1-(2-((R)-2-methylpiperidin-1-yl)propyl)-4-phenyl-1,2,3,4-tetrahydroquinoxaline |
| 69 | 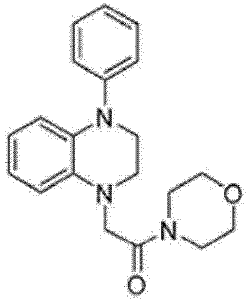 | 1-morpholino-2-(4-phenyl-3,4-dihydroquinoxaline-1 (2H)-yl)ethan-1-one |
| 70 | 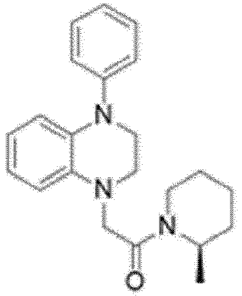 | (R)-1-(2-methylpiperidin-1-yl)-2-(4-phenyl-3,4-dihydroquinoxaline-1 (2H)-yl)ethan-1-one |
| 71 | 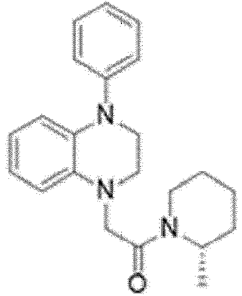 | (S)-1-(2-methylpiperidin-1-yl)-2-(4-phenyl-3,4-dihydroquinoxaline-1(2H)-yl)ethan-1-one |
| 72 | 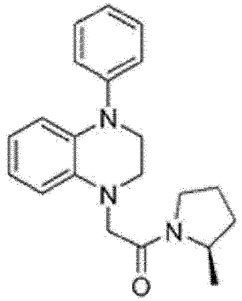 | (R)-1-(2-methylpyrrolidin-1-yl)-2-(4-phenyl-3,4-dihydroquinoxaline-1(2H)-yl)ethan-1-one |
| 73 | 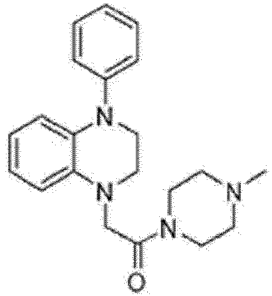 | 1-(4-methylpiperazin-1-yl)-2-(4-phenyl-3,4-dihydroquinoxaline-1(2H)-yl)ethan-1-one |
| 74 | 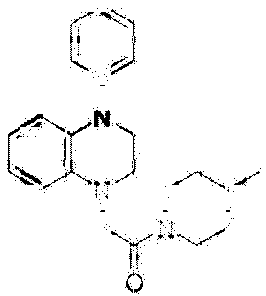 | 1-(4-methylpiperidin-1-yl)-2-(4-phenyl-3,4-dihydroquinoxaline-1(2H)-yl)ethan-1-one |
| 75 | 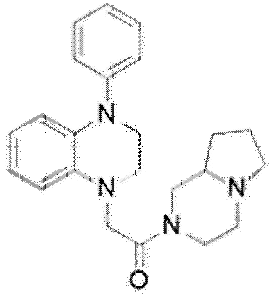 | 1-(hexahydropyrrolo[1,2-a]pyrazin-2(1H-yl)-2-(4-phenyl-3,4-dihydroquinoxaline-1(2H)-yl)ethan-1-one |
| 76 | 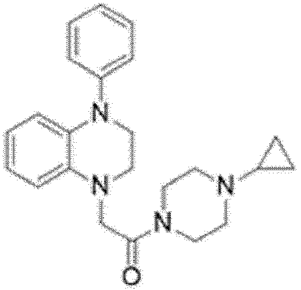 | 1-(4-cyclopropylpiperazin-1-yl)-2-(4-phenyl-3,4-dihydroquinoxaline-1(2H)-yl)ethan-1-one |
| 77 | 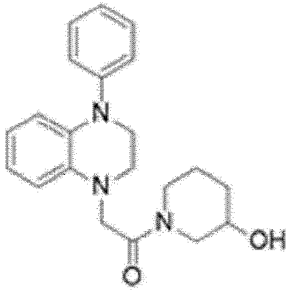 | 1-(3-hydroxypiperidin-1-yl)-2-(4-phenyl-3,4-dihydroquinoxaline-1(2H)-yl)ethan-1-one |
| 78 | 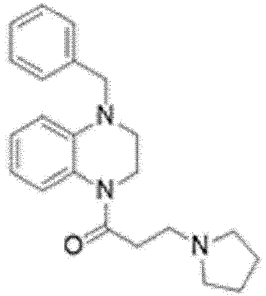 | 1-(4-benzyl-3,4-dihydroquinoxaline-1(2H)-yl)-3-(pyrrolidin-1-yl)propan-1-one |
| 79 | 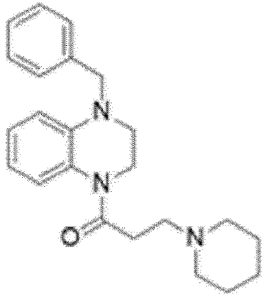 | 1-(4-benzyl-3,4-dihydroquinoxaline-1(2H)-yl)-3-(piperidin-1-yl)propan-1-one |
| 80 | 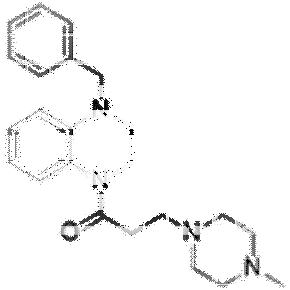 | 1-(4-benzyl-3,4-dihydroquinoxaline-1(2H)-yl)-3-(4-methylpiperazin-1-yl)propan-1-one |
| 81 | 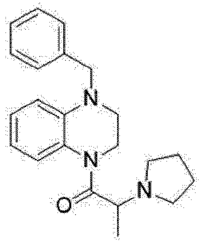 | 1-(4-benzyl-3,4-dihydroquinoxaline-1(2H)-yl)-2-(pyrrolidin-1-yl)propan-1-one |
| 82 | 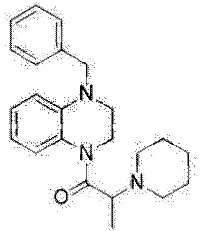 | 1-(4-benzyl-3,4-dihydroquinoxaline-1(2H)-yl)-2-(piperidin-1-yl)propan-1-one |
| 83 | 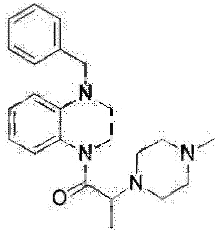 | 1-(4-benzyl-3,4-dihydroquinoxaline-1(2H)-yl)-2-(4-methylpiperazin-1-yl)propan-1-one |
| 84 | 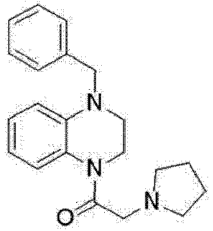 | 1-(4-benzyl-3,4-dihydroquinoxaline-1(2H)-yl)-2-(pyrrolidin-1-yl)ethan-1-one |
| 85 | 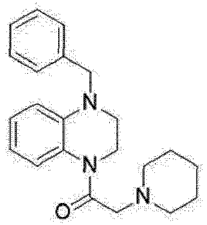 | 1-(4-benzyl-3,4-dihydroquinoxaline-1(2H)-yl)-2-(piperidin-1-yl)ethan-1-one |
| 86 | 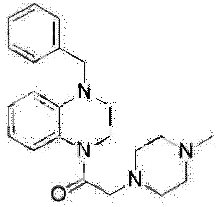 | 1-(4-benzyl-3,4-dihydroquinoxaline-1(2H)-yl)-2-(4-methylpiperazin-1-yl)ethan-1-one |
| 87 | 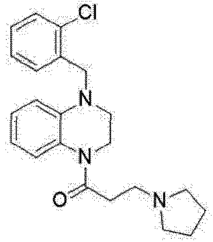 | 1-(4-(2-chlorobenzyl)-3,4-dihydroquinoxaline-1(2H)-yl)-3-(pyrrolidin-1-yl)propan-1-one |
| 88 | 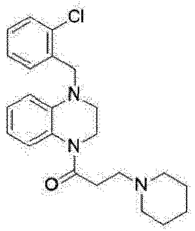 | 1-(4-(2-chlorobenzyl)-3,4-dihydroquinoxaline-1(2H)-yl)-3-(piperidin-1-yl)propan-1-one |
| 89 | 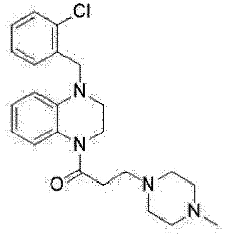 | 1-(4-(2-chlorobenzyl)-3,4-dihydroquinoxaline-1(2H)-yl)-3-(4-methylpiperazin-1-yl)propan-1-one |
| 90 | 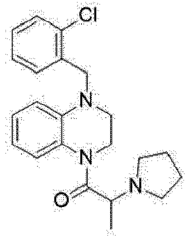 | 1-(4-(2-chlorobenzyl)-3,4-dihydroquinoxaline-1(2H)-yl)-2-(pyrrolidin-1-yl)propan-1-one |
| 91 | 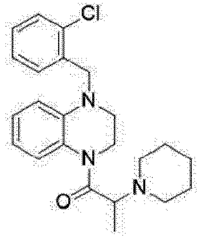 | 1-(4-(2-chlorobenzyl)-3,4-dihydroquinoxaline-1(2H)-yl)-2-(piperidin-1-yl)propan-1-one |
| 92 | 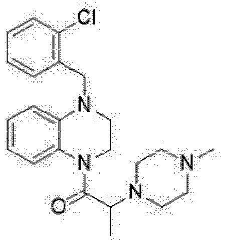 | 1-(4-(2-chlorobenzyl)-3,4-dihydroquinoxaline-1(2H)-yl)-2-(4-methylpiperazin-1-yl)propan-1-one |
| 93 | 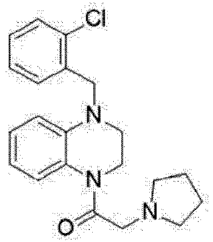 | 1-(4-(2-chlorobenzyl)-3,4-dihydroquinoxaline-1(2H)-yl)-2-(pyrrolidin-1-yl)ethan-1-one |
| 94 | 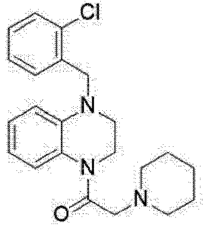 | 1-(4-(2-chlorobenzyl)-3,4-dihydroquinoxaline-1(2H)-yl)-2-(piperidin-1-yl)ethan-1-one |
| 95 | 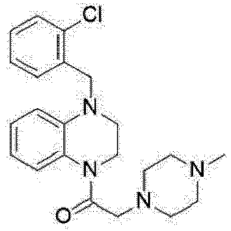 | 1-(4-(2-chlorobenzyl)-3,4-dihydroquinoxaline-1(2H)-yl)-2-(4-methylpiperazin-1-yl)ethan-1-one |
| 96 | 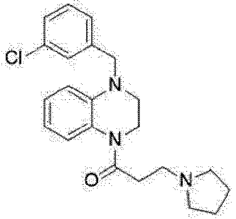 | 1-(4-(3-chlorobenzyl)-3,4-dihydroquinoxaline-1(2H)-yl)-3-(pyrrolidin-1-yl)propan-1-one |
| 97 | 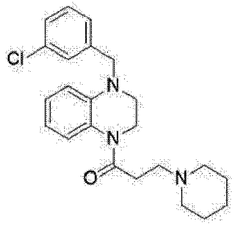 | 1-(4-(3-chlorobenzyl)-3,4-dihydroquinoxaline-1(2H)-yl)-3-(piperidin-1-yl)propan-1-one |
| 98 | 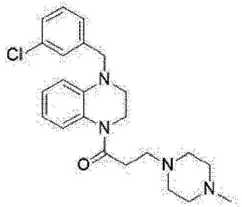 | 1-(4-(3-chlorobenzyl)-3,4-dihydroquinoxaline-1(2H)-yl)-3-(4-methylpiperazin-1-yl)propan-1-one |
| 99 | 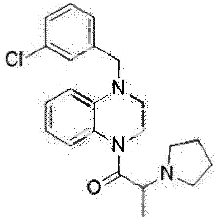 | 1-(4-(3-chlorobenzyl)-3,4-dihydroquinoxaline-1(2H)-yl)-2-(pyrrolidin-1-yl)propan-1-one |
| 100 | 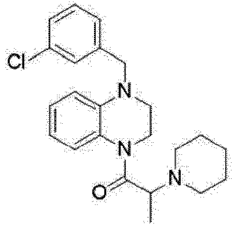 | 1-(4-(3-chlorobenzyl)-3,4-dihydroquinoxaline-1(2H)-yl)-2-(piperidin-1-yl)propan-1-one |
| 101 | 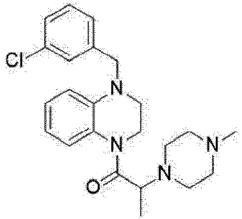 | 1-(4-(3-chlorobenzyl)-3,4-dihydroquinoxaline-1(2H)-yl)-2-(4-methylpiperazin-1-yl)propan-1-one |
| 102 | 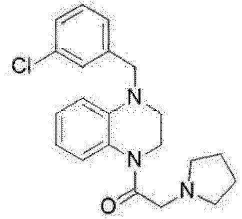 | 1-(4-(3-chlorobenzyl)-3,4-dihydroquinoxaline-1(2H)-yl)-2-(pyrrolidin-1-yl)ethan-1-one |
| 103 | 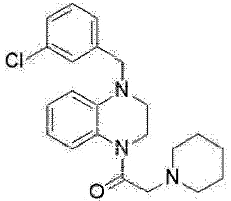 | 1-(4-(3-chlorobenzyl)-3,4-dihydroquinoxaline-1(2H)-yl)-2-(piperidin-1-yl)ethan-1-one |
| 104 | 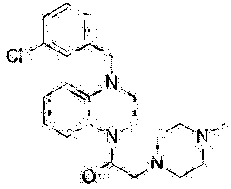 | 1-(4-(3-chlorobenzyl)-3,4-dihydroquinoxaline-1(2H)-yl)-2-(4-methylpiperazin-1-yl)ethan-1-one |
| 105 | 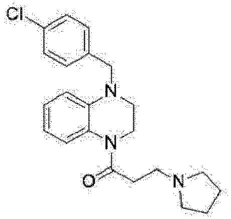 | 1-(4-(4-chlorobenzyl)-3,4-dihydroquinoxaline-1(2H)-yl)-3-(pyrrolidin-1-yl)propan-1-one |
| 106 | 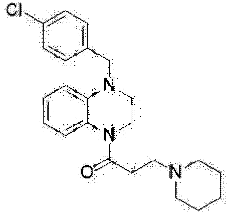 | 1-(4-(4-chlorobenzyl)-3,4-dihydroquinoxaline-1(2H)-yl)-3-(piperidin-1-yl)propan-1-one |
| 107 | 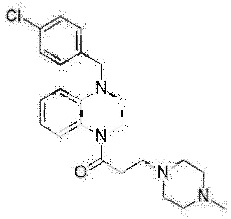 | 1-(4-(4-chlorobenzyl)-3,4-dihydroquinoxaline-1(2H)-yl)-3-(4-methylpiperazin-1-yl)propan-1-one |
| 108 | 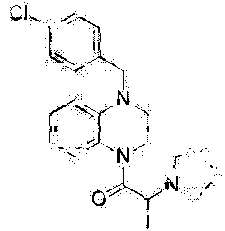 | 1-(4-(4-chlorobenzyl)-3,4-dihydroquinoxaline-1(2H)-yl)-2-(pyrrolidin-1-yl)propan-1-one |
| 109 | 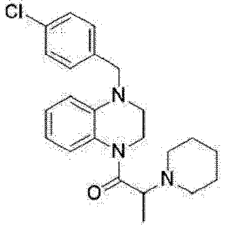 | 1-(4-(4-chlorobenzyl)-3,4-dihydroquinoxaline-1(2H)-yl)-2-(piperidin-1-yl)propan-1-one |
| 110 | 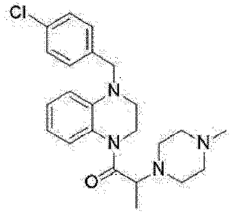 | 1-(4-(4-chlorobenzyl)-3,4-dihydroquinoxaline-1(2H)-yl)-2-(4-methylpiperazin-1-yl)propan-1-one |
| 111 | 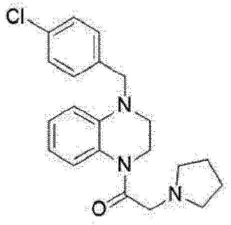 | 1-(4-(4-chlorobenzyl)-3,4-dihydroquinoxaline-1(2H)-yl)-2-(pyrrolidin-1-yl)ethan-1-one |
| 112 | 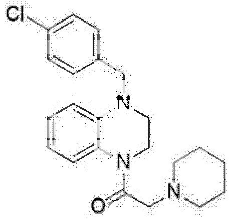 | 1-(4-(4-chlorobenzyl)-3,4-dihydroquinoxaline-1(2H)-yl)-2-(piperidin-1-yl)ethan-1-one |
| 113 | 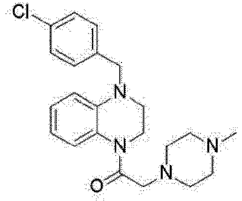 | 1-(4-(4-chlorobenzyl)-3,4-dihydroquinoxaline-1(2H)-yl)-2-(4-methylpiperazin-1-yl)ethan-1-one |
| 114 | 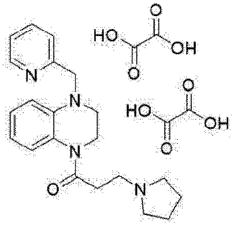 | 1-(4-(pyridin-2-ylmethyl)-3,4-dihydroquinoxaline-1(2H)-yl)-3-(pyrrolidin-1-yl)propan-1-one di-oxalate |
| 115 | 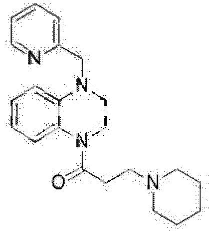 | 3-(piperidin-1-yl)-1-(4-(pyridin-2-ylmethyl)-3,4-dihydroquinoxaline-1(2H)-yl)propan-1-one |
| 116 | 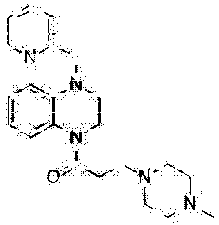 | 3-(4-methylpiperazin-1-yl)-1-(4-(pyridin-2-ylmethyl)-3,4-dihydroquinoxaline-1(2H)-yl)propan-1-one |
| 117 | 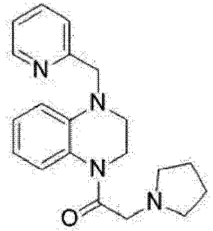 | 1-(4-(pyridin-2-ylmethyl)-3,4-dihydroquinoxaline-1(2H)-yl)-2-(pyrrolidin-1-yl)ethan-1-one |
| 118 | 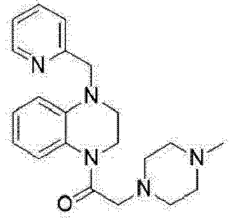 | 2-(4-methylpiperazin-1-yl)-1-(4-(pyridin-2-ylmethyl)-3,4-dihydroquinoxaline-1(2H)-yl)ethan-1-one |
| 119 | 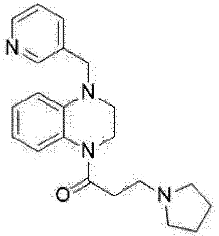 | 1-(4-(pyridin-3-ylmethyl)-3,4-dihydroquinoxaline-1(2H)-yl)-3-(pyrrolidin-1-yl)propan-1-one |
| 120 | 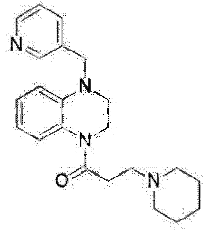 | 3-(piperidin-1-yl)-1-(4-(pyridin-3-ylmethyl)-3,4-dihydroquinoxaline-1(2H)-yl)propan-1-one |
| 121 | 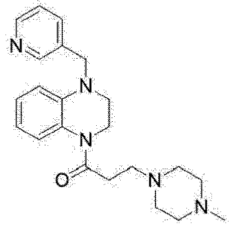 | 3-(4-methylpiperazin-1-yl)-1-(4-(pyridin-3-ylmethyl)-3,4-dihydroquinoxaline-1(2H)-yl)propan-1-one |
| 122 | 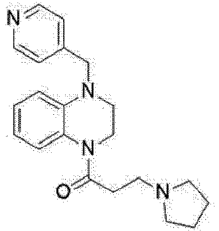 | 1-(4-(pyridin-4-ylmethyl)-3,4-dihydroquinoxaline-1(2H)-yl)-3-(pyrrolidin-1-yl)propan-1-one |
| 123 | 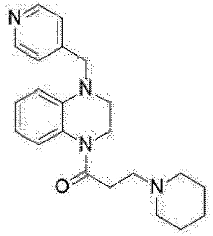 | 3-(piperidin-1-yl)-1-(4-(pyridin-4-ylmethyl)-3,4-dihydroquinoxaline-1(2H)-yl)propan-1-one |
| 124 | 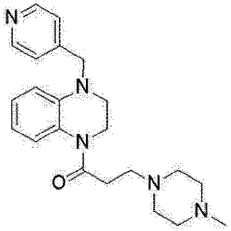 | 3-(4-methylpiperazin-1-yl)-1-(4-(pyridin-4-ylmethyl)-3,4-dihydroquinoxaline-1(2H)-yl)propan-1-one |
| 125 | 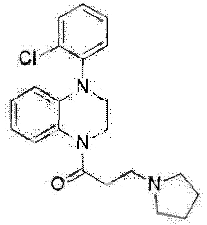 | 1-(4-(2-chlorophenyl)-3,4-dihydroquinoxaline-1(2H)-yl)-3-(pyrrolidin-1-yl)propan-1-one |
| 126 | 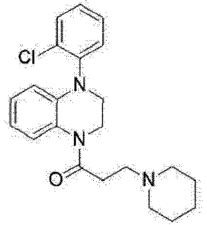 | 1-(4-(2-chlorophenyl)-3,4-dihydroquinoxaline-1(2H)-yl)-3-(piperidin-1-yl)propan-1-one |
| 127 | 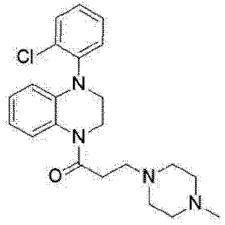 | 1-(4-(2-chlorophenyl)-3,4-dihydroquinoxaline-1(2H)-yl)-3-(4-methylpiperazin-1-yl)propan-1-one |
| 128 | 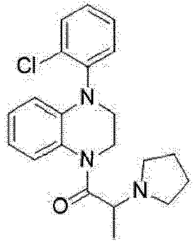 | 1-(4-(2-chlorophenyl)-3,4-dihydroquinoxaline-1(2H)-yl)-2-(pyrrolidin-1-yl)propan-1-one |
| 129 | 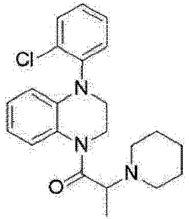 | 1-(4-(2-chlorophenyl)-3,4-dihydroquinoxaline-1(2H)-yl)-2-(piperidin-1-yl)propan-1-one |
| 130 | 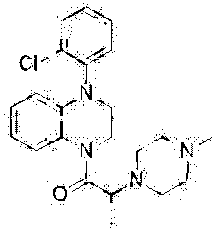 | 1-(4-(2-chlorophenyl)-3,4-dihydroquinoxaline-1(2H)-yl)-2-(4-methylpiperazin-1-yl)propan-1-one |
| 131 | 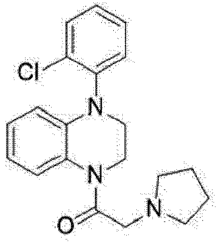 | 1-(4-(2-chlorophenyl)-3,4-dihydroquinoxaline-1(2H)-yl)-2-(pyrrolidin-1-yl)ethan-1-one |
| 132 | 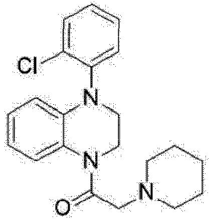 | 1-(4-(2-chlorophenyl)-3,4-dihydroquinoxaline-1(2H)-yl)-2-(piperidin-1-yl)ethan-1-one |
| 133 | 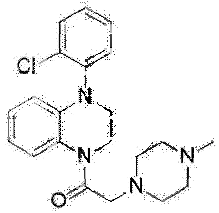 | 1-(4-(2-chlorophenyl)-3,4-dihydroquinoxaline-1(2H)-yl)-2-(4-methylpiperazin-1-yl)ethan-1-one |
| 134 | 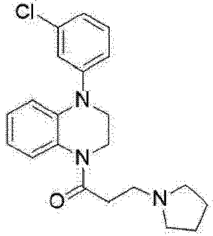 | 1-(4-(3-chlorophenyl)-3,4-dihydroquinoxaline-1(2H)-yl)-3-(pyrrolidin-1-yl)propan-1-one |
| 135 | 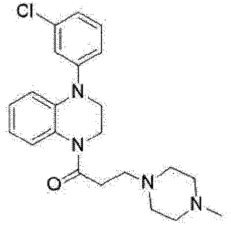 | 1-(4-(3-chlorophenyl)-3,4-dihydroquinoxaline-1(2H)-yl)-3-(4-methylpiperazin-1-yl)propan-1-one |
| 136 | 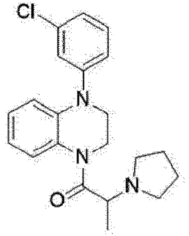 | 1-(4-(3-chlorophenyl)-3,4-dihydroquinoxaline-1(2H)-yl)-2-(pyrrolidin-1-yl)propan-1-one |
| 137 | 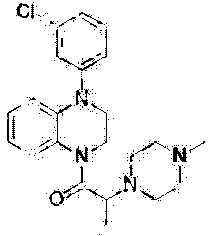 | 1-(4-(3-chlorophenyl)-3,4-dihydroquinoxaline-1(2H)-yl)-2-(4-methylpiperazin-1-yl)propan-1-one |
| 138 | 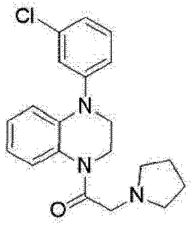 | 1-(4-(3-chlorophenyl)-3,4-dihydroquinoxaline-1(2H)-yl)-2-(pyrrolidin-1-yl)ethan-1-one |
| 139 | 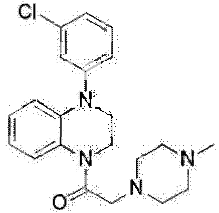 | 1-(4-(3-chlorophenyl)-3,4-dihydroquinoxaline-1(2H)-yl)-2-(4-methylpiperazin-1-yl)ethan-1-one |
| 140 | 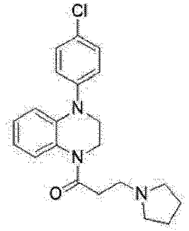 | 1-(4-(4-chlorophenyl)-3,4-dihydroquinoxaline-1(2H)-yl)-3-(pyrrolidin-1-yl)propan-1-one |
| 141 | 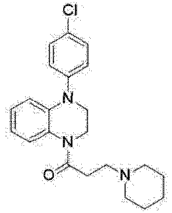 | 1-(4-(4-chlorophenyl)-3,4-dihydroquinoxaline-1(2H)-yl)-3-(piperidin-1-yl)propan-1-one |
| 142 | 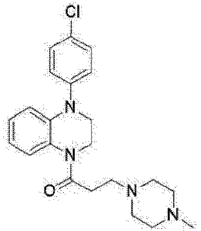 | 1-(4-(4-chlorophenyl)-3,4-dihydroquinoxaline-1(2H)-yl)-3-(4-methylpiperazin-1-yl)propan-1-one |
| 143 | 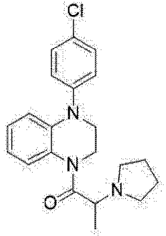 | 1-(4-(4-chlorophenyl)-3,4-dihydroquinoxaline-1(2H)-yl)-2-(pyrrolidin-1-yl)propan-1-one |
| 144 | 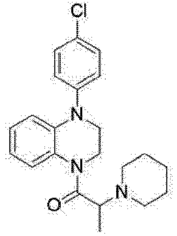 | 1-(4-(4-chlorophenyl)-3,4-dihydroquinoxaline-1(2H)-yl)-2-(piperidin-1-yl)propan-1-one |
| 145 | 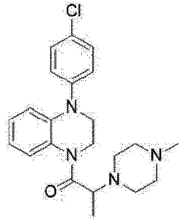 | 1-(4-(4-chlorophenyl)-3,4-dihydroquinoxaline-1(2H)-yl)-2-(4-methylpiperazin-1-yl)propan-1-one |
| 146 | 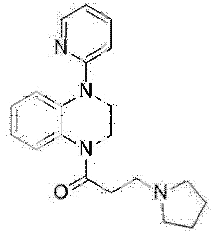 | 1-(4-pyridin-2-yl)-3,4-dihydroquinoxaline-1(2H)-yl)-3-(pyrrolidin-1-yl)propan-1-one |
| 147 | 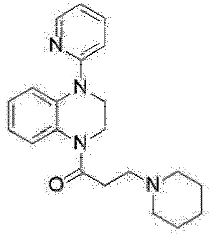 | 3-(piperidin-1-yl)-1-(4-(pyridin-2-yl)-3,4-dihydroquinoxaline-1(2H)-yl)propan-1-one |
| 148 | 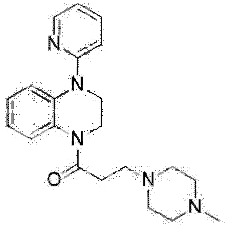 | 3-(4-methylpiperazin-1-yl)-1-(4-(pyridin-2-yl)-3,4-dihydroquinoxaline-1(2H)-yl)propan-1-one |
| 149 | 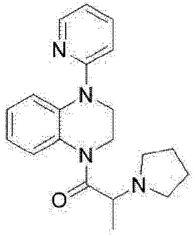 | 1-(4-pyridin-2-yl)-3,4-dihydroquinoxaline-1(2H)-yl)-2-(pyrrolidin-1-yl)propan-1-one |
| 150 | 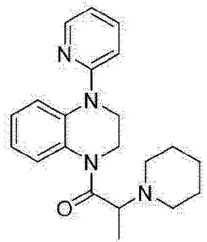 | 2-(piperidin-1-yl)-1-(4-pyridin-2-yl)-3,4-dihydroquinoxaline-1(2H)-yl)propan-1-one |
| 151 | 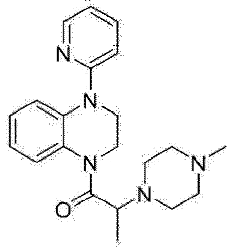 | 1-(4-pyridin-2-yl)-3,4-dihydroquinoxaline-1(2H)-yl)-2-(4-methylpiperazin-1-yl)propan-1-one |
| 152 | 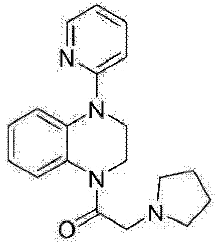 | 1-(4-pyridin-2-yl)-3,4-dihydroquinoxaline-1(2H)-yl)-2-(pyrrolidin-1-yl)ethan-1-one |
| 153 | 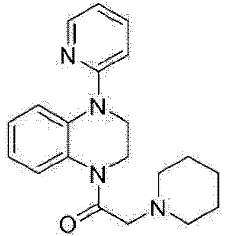 | 2-(piperidin-1-yl)-1-(4-(pyridin-2-yl)-3,4-dihydroquinoxaline-1(2H)-yl)ethan-1-one |
| 154 | 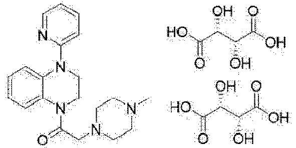 | 2-(4-methylpiperazin-1-yl)-1-(4-(pyridin-2-yl)-3,4-dihydroquinoxaline-1(2H)-yl)ethan-1-one di *L*-tartrate |
| 155 | 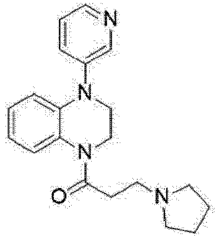 | 1-(4-(pyridin-3-yl)-3,4-dihydroquinoxaline-1(2H)-yl)-3-(pyrrolidin-1-yl)propan-1-one |
| 156 | 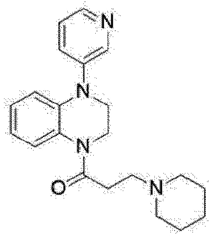 | 3-(piperidin-1-yl)-1-(4-(pyridin-3-yl)-3,4-dihydroquinoxaline-1(2H)-yl)propan-1-one |
| 157 | 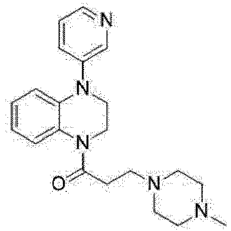 | 3-(4-methylpiperazin-1-yl)-1-(4-(pyridin-3-yl)-3,4-dihydroquinoxaline-1(2H)-yl)propan-1-one |
| 158 | 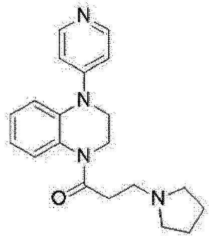 | 1-(4-(pyridin-4-yl)-3,4-dihydroquinoxaline-1(2H)-yl)-3-(pyrrolidin-1-yl)propan-1-one |
| 159 | 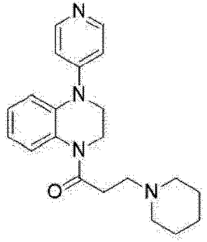 | 3-(piperidin-1-yl)-1-(4-(pyridin-4-yl)-3,4-dihydroquinoxaline-1(2H)-yl)propan-1-one |
| 160 | 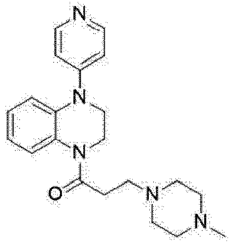 | 3-(4-methylpiperazin-1-yl)-1-(4-(pyridin-4-yl)-3,4-dihydroquinoxaline-1(2H)-yl)propan-1-one |
| 161 | 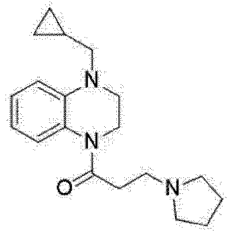 | 1-(4-(cyclopropylmethyl)-3,4-dihydroquinoxaline-1(2H)-yl)-3-(pyrrolidin-1-yl)propan-1-one |
| 162 | 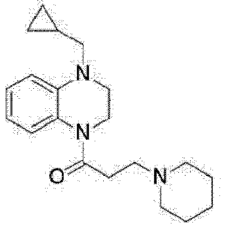 | 1-(4-(cyclopropylmethyl)-3,4-dihydroquinoxaline-1(2H)-yl)-3-(piperidin-1-yl)propan-1-one |
| 163 | 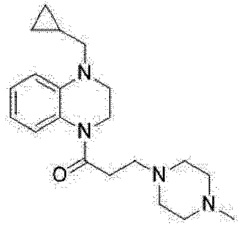 | 1-(4-(cyclopropylmethyl)-3,4-dihydroquinoxaline-1(2H)-yl)-3-(4-methylpiperazin-1-yl)propan-1-one |
| 164 | 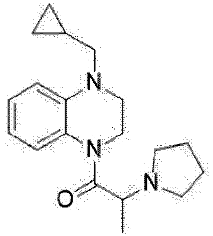 | 1-(4-(cyclopropylmethyl)-3,4-dihydroquinoxaline-1(2H)-yl)-2-(pyrrolidin-1-yl)propan-1-one |
| 165 | 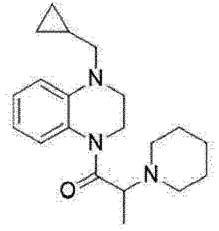 | 1-(4-(cyclopropylmethyl)-3,4-dihydroquinoxaline-1(2H)-yl)-2-(piperidin-1-yl)propan-1-one |
| 166 | 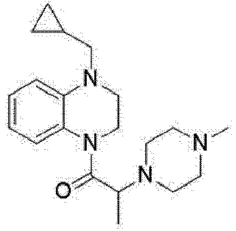 | 1-(4-(cyclopropylmethyl)-3,4-dihydroquinoxaline-1(2H)-yl)-2-(4-methylpiperazin-1-yl)propan-1-one |
| 167 | 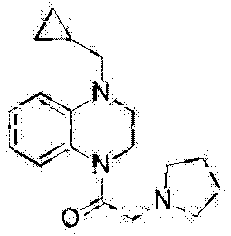 | 1-(4-(cyclopropylmethyl)-3,4-dihydroquinoxaline-1(2H)-yl)-2-(pyrrolidin-1-yl)ethan-1-one |
| 168 | 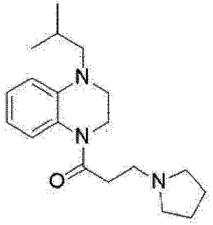 | 1-(4-isobutyl-3,4-dihydroquinoxaline-1(2H)-yl)-2-(pyrrolidin-1-yl)propan-1-one |
| 169 | 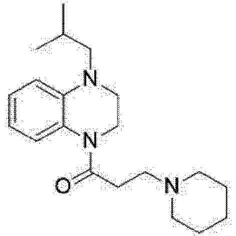 | 1-(4-isobutyl-3,4-dihydroquinoxaline-1(2H)-yl)-3-(piperidin-1-yl)propan-1-one |
| 170 | 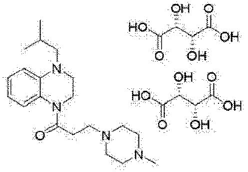 | 1-(4-isobutyl-3,4-dihydroquinoxaline-1(2H)-yl)-3-(4-methylpiperazin-1-yl)propan-1-one di *L-*tartrate |
| 171 | 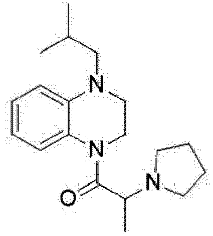 | 1-(4-isobutyl-3,4-dihydroquinoxaline-1(2H)-yl)-2-(pyrrolidin-1-yl)propan-1-one |
| 172 | 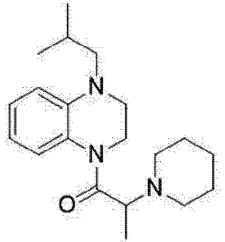 | 1-(4-isobutyl-3,4-dihydroquinoxaline-1(2H)-yl)-2-(piperidin-1-yl)propan-1-one |
| 173 | 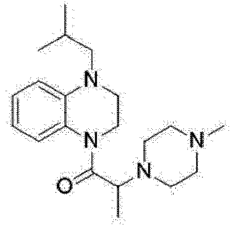 | 1-(4-isobutyl-3,4-dihydroquinoxaline-1(2H)-yl)-2-(4-methylpiperazin-1-yl)propan-1-one |
| 174 | 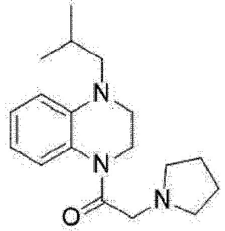 | 1-(4-isobutyl-3,4-dihydroquinoxaline-1(2H)-yl)-2-(pyrrolidin-1-yl)ethan-1-one |
| 175 | 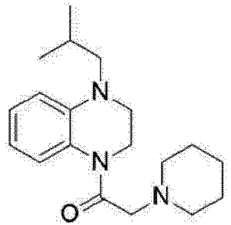 | 1-(4-isobutyl-3,4-dihydroquinoxaline-1(2H)-yl)-2-(piperidin-1-yl)ethan-1-one |
| 176 | 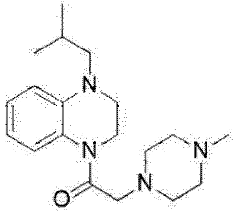 | 1-(4-isobutyl-3,4-dihydroquinoxaline-1(2H)-yl)-2-(4-methylpiperazin-1-yl)ethan-1-one |
| 177 | 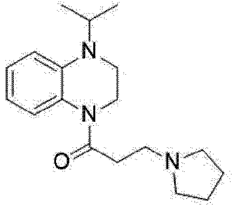 | 1-(4-isopropyl-3,4-dihydroquinoxaline-1(2H)-yl)-3-(pyrrolidin-1-yl)propan-1-one |
| 178 | 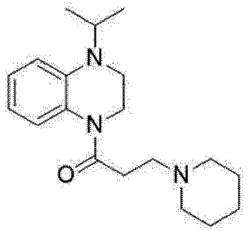 | 1-(4-isopropyl-3,4-dihydroquinoxaline-1(2H)-yl)-3-(piperidin-1-yl)propan-1-one |
| 179 | 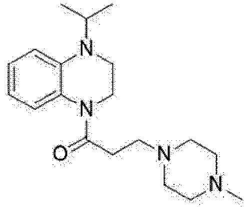 | 1-(4-isopropyl-3,4-dihydroquinoxaline-1(2H)-yl)-3-(4-methylpiperazin-1-yl)propan-1-one |
| 180 | 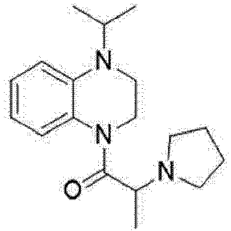 | 1-(4-isopropyl-3,4-dihydroquinoxaline-1(2H)-yl)-2-(pyrrolidin-1-yl)propan-1-one |
| 181 | 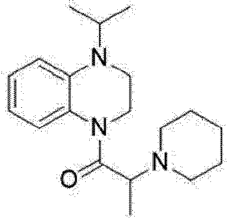 | 1-(4-isopropyl-3,4-dihydroquinoxaline-1(2H)-yl)-2-(piperidin-1-yl)propan-1-one |
| 182 | 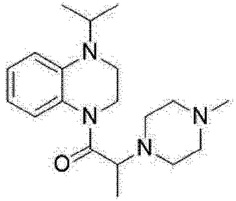 | 1-(4-isopropyl-3,4-dihydroquinoxaline-1(2H)-yl)-2-(4-methylpiperazin-1-yl)propan-1-one |
| 183 | 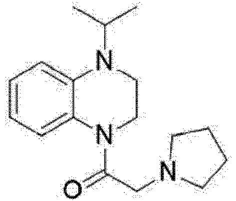 | 1-(4-isopropyl-3,4-dihydroquinoxaline-1(2H)-yl)-3-(pyrrolidin-1-yl)ethan-1-one |
| 184 | 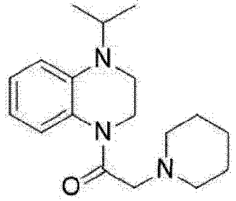 | 1-(4-isopropyl-3,4-dihydroquinoxaline-1(2H)-yl)-2-(piperidin-1-yl)ethan-1-one |
| 185 | 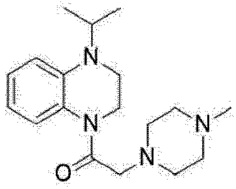 | 1-(4-isopropyl-3,4-dihydroquinoxaline-1(2H)-yl)-2-(4-methylpiperazin-1-yl)ethan-1-one |
| 186 | 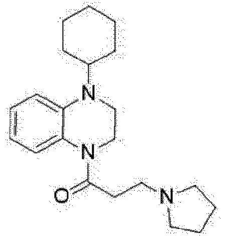 | 1-(4-cyclohexyl-3,4-dihydroquinoxaline-1(2H)-yl)-3-(pyrrolidin-1-yl)propan-1-one |
| 187 | 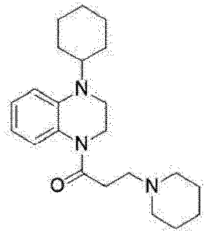 | 1-(4-cyclohexyl-3,4-dihydroquinoxaline-1(2H)-yl)-3-(piperidin-1-yl)propan-1-one |
| 188 | 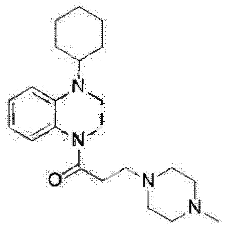 | 1-(4-cyclohexyl-3,4-dihydroquinoxaline-1(2H)-yl)-3-(4-methylpiperazin-1-yl)propan-1-one |
| 189 | 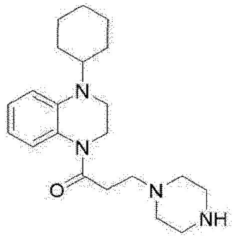 | 1-(4-cyclohexyl-3,4-dihydroquinoxaline-1(2H)-yl)-3-(piperazin-1-yl)propan-1-one |
| 190 | 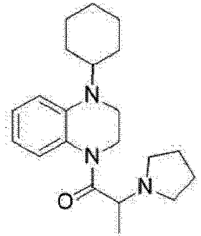 | 1-(4-cyclohexyl-3,4-dihydroquinoxaline-1(2H)-yl)-2-(pyrrolidin-1-yl)ethan-1-one |
| 191 | 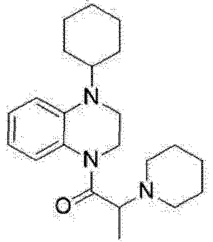 | 1-(4-cyclohexyl-3,4-dihydroquinoxaline-1(2H)-yl)-2-(piperidin-1-yl)propan-1-one |
| 192 | 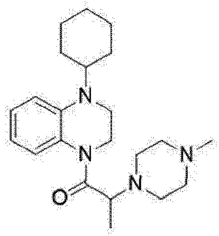 | 1-(4-cyclohexyl-3,4-dihydroquinoxaline-1(2H)-yl)-2-(4-methylpiperazin-1-yl)propan-1-one |
| 193 | 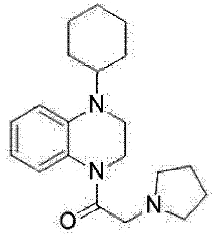 | 1-(4-cyclohexyl-3,4-dihydroquinoxaline-1(2H)-yl)-2-(pyrrolidin-1-yl)ethan-1-one |
| 194 | 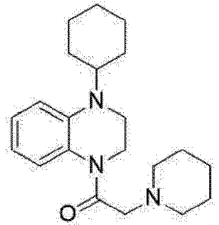 | 1-(4-cyclohexyl-3,4-dihydroquinoxaline-1(2H)-yl)-2-(piperidin-1-yl)ethan-1-one |
| 195 | 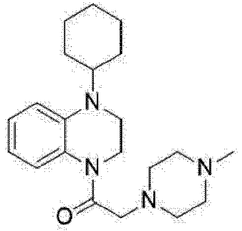 | 1-(4-cyclohexyl-3,4-dihydroquinoxaline-1(2H)-yl)-2-(4-methylpiperazin-1-yl)ethan-1-one |
| 196 | 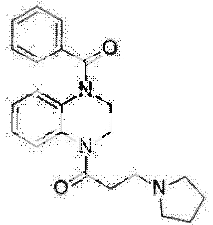 | 1-(4-benzoyl-3,4-dihydroquinoxaline-1(2H)-yl)-3-(pyrrolidin-1-yl)propan-1-one |
| 197 | 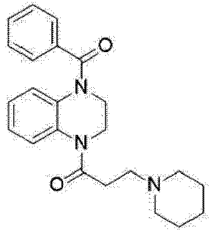 | 1-(4-benzoyl-3,4-dihydroquinoxaline-1(2H)-yl)-3-(piperidin-1-yl)propan-1-one |
| 198 | 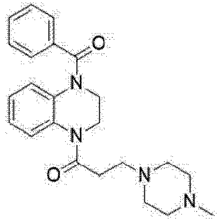 | 1-(4-benzoyl-3,4-dihydroquinoxaline-1(2H)-yl)-3-(4-methylpiperazin-1-yl)propan-1-one |
| 199 | 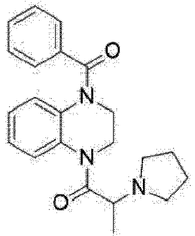 | 1-(4-benzoyl-3,4-dihydroquinoxaline-1(2H)-yl)-2-(pyrrolidin-1-yl)propan-1-one |
| 200 | 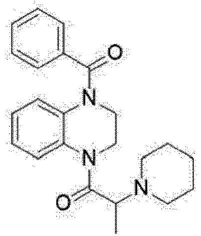 | 1-(4-benzoyl-3,4-dihydroquinoxaline-1(2H)-yl)-2-(piperidin-1-yl)propan-1-one |
| 201 | 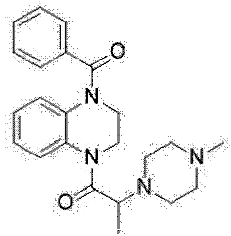 | 1-(4-benzoyl-3,4-dihydroquinoxaline-1(2H)-yl)-2-(4-methylpiperazin-1-yl)propan-1-one |
| 202 | 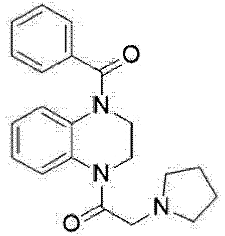 | 1-(4-benzoyl-3,4-dihydroquinoxaline-1(2H)-yl)-2-(pyrrolidin-1-yl)ethan-1-one |
| 203 | 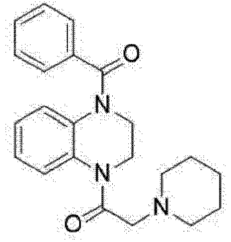 | 1-(4-benzoyl-3,4-dihydroquinoxaline-1(2H)-yl)-2-(piperidin-1-yl)ethan-1-one |
| 204 | 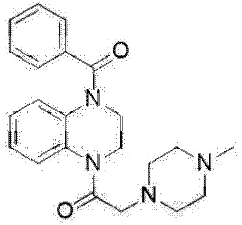 | 1-(4-benzoyl-3,4-dihydroquinoxaline-1(2H)-yl)-2-(4-methylpiperazin-1-yl)ethan-1-one |
| 205 | 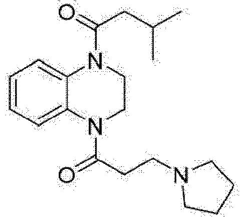 | 3-methyl-1-(4-(3-(pyrrolidin-1-yl)propanoyl)-3,4-dihydroquinoxaline-1(2H)-yl)butan-1-one |
| 206 | 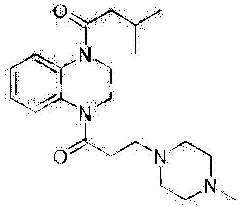 | 3-methyl-1-(4-(3-(4-methylpiperazin-1-yl)propanoyl)-3,4-dihydroquinoxaline-1(2H)-yl)butan-1-one |
| 207 | 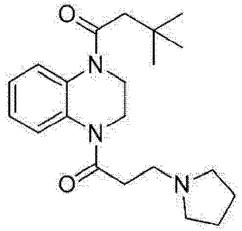 | 3,3-dimethyl-1-(4-(3-(pyrrolidin-1-yl)propanoyl)-3,4-dihydroquinoxaline-1(2H)-yl)butan-1-one |
| 208 | 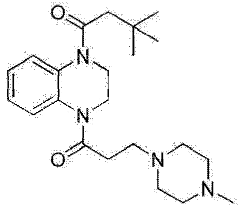 | 3,3-dimethyl-1-(4-(3-(4-methylpiperazin-1-yl)propanoyl)-3,4-dihydroquinoxaline-1(2H)-yl)butan-1-one |

### Example 2. Evaluation of actin polymerization inhibitory activity of compound according to the present invention

Although actin polymerization, which is a process of forming actin filaments from actin monomers, is involved in controlling cell movement, tissue fibrosis may be caused by the promotion of excessive α-SMA expression and induction of myofibroblast activation. Accordingly, an *in vitro* actin polymerization test was performed to confirm the actin polymerization controlling function of the compounds according to the present invention.

Specifically, a pyrene-labeled spherical actin stock was diluted with general actin buffer (5 mM Tris-HCl pH 8.0, 0.2 mM CaCl₂) and incubated at 4 °C for 1 hour, thereby producing spherical actin monomers. Afterward, 80% of a supernatant was recovered by centrifugation at 20,000X g and 4 °C for 3 hours, and 0.1 mg/mL spherical actin monomers were prepared. The Arp2/3 complex, the VCA domain protein, and Compound 12, 38, or 59 (10 µM each) according to the present invention were added to the spherical actin monomers and an actin polymerization buffer and reacted at room temperature for 1 hour. The increased fluorescence intensity during the reaction from the spherical actin monomer to a fibrous actin polymer was measured using a microplate reader at an excitation wavelength of 360 nm and an emission wavelength of 407 nm. The degree of actin polymerization was shown relative to the maximum polymerization rate (arbitrary units).

As a result, as shown in FIG. 1, it was confirmed that, in the experimental group treated with Compound 12, 38, or 59 according to the present invention, actin polymerization activity was decreased compared to the control. This result shows that the compound according to the present invention effectively inhibits actin polymerization.

### Example 3. Evaluation of intracellular F-actin polymerization inhibitory activity of compounds according to the present invention

F-actin polymerization activates transcription factors involved in the activation of myofibroblasts and the initiation of fibrosis. Accordingly, when F-actin polymerization decreases, the expression of fibrosis-causing genes by the transcription factors may be inhibited, and the activation and fibrosis of muscle fiber cells may be reduced. Therefore, in this example, it was confirmed whether the compounds according to the present invention are effective in inhibiting F-actin polymerization.

An MCR5 cell line was dispensed at 1×10³ cells/well in a 96-well black micro plate and cultured in Medium 1 (10% FBS-containing MEM) in 5% CO₂ at 37 °C for 24 hours. Subsequently, the above medium was exchanged with Medium 2 (0.5% FBS-containing MEM), a compound of the present invention was treated at 10 µM along with TGF-β1 (10 ng/mL), and the cells were incubated for 48 hours. After removing the entire batch, the cells were treated with each of CellMask Orange Actin Tracking Stain (Invitrogen) and NucBlue Live ReadyProbes (Invitrogen) to performing F-actin and cell nucleus staining in accordance with the manufacturer's recommended conditions. Cells treated with the staining reagents were incubated for 30 minutes at 5% CO₂ and 37 °C while blocking light. After completing the incubation, the staining reagents were removed, fresh Medium 1 was added to the cells at 100 µL/well, and then the cells were incubated for 5 minutes, followed by removal of the medium. The above process was repeated three times. To observe the stained F-actin and cell nucleus, PBS was added to the plate at 100 µL/well, and then the cells were photographed using a confocal microscope (Leica) and analyzed using Image J software.

The evaluation of intracellular F-actin polymerization of the compounds according to the present invention is shown in FIGS. 2A and 2B. In TGF-β1-treated cells, intracellular F-actin polymerization was greatly increased compared to the control (control material). On the other hand, in the cells also treated with Compound 12, 38, or 59 according to the present invention, intracellular F-actin polymerization was significantly reduced, confirming that TGF-β1-mediated F-actin polymerization is effectively inhibited. This result shows that the compound according to the present invention can effectively inhibit F-actin polymerization, which is a key factor in the activation and fibrosis of myofibroblasts.

### Example 4. Confirmation of inhibitory effect on α-SMA expression and fibrosis of compound according to the present invention

α-SMA protein is a marker of activated myofibroblasts, which are the main effector cells of fibrosis, myofibroblasts with increased α-SMA expression are known to produce a fibrotic protein such as collagen type 1 and cause fibrosis. Therefore, in this example, it was confirmed whether the compound according to the present invention is effective in inhibiting fibrosis by reducing the expression of α-SMA and other pro-fibrotic factors (COL1A1, COL4A1, fibronectin, and p-SMAD2).

Specifically, lung tissue-derived fibroblasts, MRC5 cells, were treated with Compound 59 according to the present invention (5, 10, or 20 µM), nintedanib (1 µM), or DMSO, and then 2 hours later, treated with TGF-β1 (10 ng/mL). After 70 hours, the cells were lysed in RIPA buffer (50 mM Tris-HCl, 150 mM NaCl, 1% NP-40, 0.5% sodium deoxycholate, 0.1% SDS, protease inhibitor & phosphatase inhibitor cocktail, pH 8.0), which had been cooled to 4 °C. The cell lysate was centrifuged at 4 °C at a speed of 15,000X g for 30 minutes, and then the supernatant was collected. After matching the total amount of proteins in the supernatant of each sample (15 to 30 µg), the proteins were separated according to molecular weight using SDS polyacrylamide gel electrophoresis (SDS-PAGE). The proteins on the gel were transferred to a PVDF membrane, the changes in the expression of biomarkers to be observed were compared by Western blotting using selective antibodies. As antibodies, anti-fibronectin (Santa Cruz Biotechnology), anti-α-SMA (Abcam), anti-phosphorylated SMAD2, anti-N-cadherin, anti-COL1A1, anti-COL4A1, and anti-GAPDH (Cell Signaling Technology) were used.

The results of evaluating the inhibition of fibrosis by the compound of the present invention are shown in FIG. 3. The α-SMA protein level in fibroblasts was increased by TGF-β1, but when also treated with Compound 59, it significantly decreased, and as the treatment concentration of Compound 59 increased, the α-SMA protein level also further decreased, thus a concentration-dependent decrease in α-SMA reduction by the compound according to the present invention was clearly shown. That is, this shows that the compound according to the present invention has a high inhibitory effect on α-SMA expression. Likewise, it was confirmed that the fibrosis biomarkers COL1A1, COL4A1, and fibronectin protein levels were also increased by TGF-β1 treatment, but when the compound of the present invention was treated, the protein levels greatly decreased. Particularly, the phosphorylation of SMAD2 protein was sharply increased by TGF-β1, and it can be confirmed that, when the compound of the present invention was treated, the phosphorylation was decreased in a concentration-dependent manner, and compared to when nintedanib was treated, expression was reduced to a greater extent.

Subsequently, the inhibitory effect on pulmonary fibrosis of the compounds according to the present invention was overall evaluated. An MRC5 cell line was treated with each compound at 10 µM, and 2 hours later, treated with TGF-β1 (10 ng/mL). After 72 hours, the cells were lysed using RIPA Buffer, and then an α-SMA protein level was measured through Western blotting. The α-SMA expression level when treating the cells with each compound and TGF-β1 compared to the α-SMA expression level when treating the cells only with TGF-β1 without the compound was calculated as the α-SMA expression inhibitory ability of the corresponding compound. The α-SMA expression inhibitory ability of each compound is summarized in Table 2 below (A: inhibitory ability ≥ 70%, B: 50% ≤ inhibitory ability < 70%, C: inhibitory ability < 50%).

**[Table 2]**

| Compound | Inhibitory ability | Compound | Inhibitory ability | Compound | Inhibitory ability |
|---|---|---|---|---|---|
| 1 | B | 65 | C | 140 | C |
| 2 | C | 67 | C | 141 | B |
| 3 | B | 70 | B | 145 | A |
| 4 | B | 70 | B | 146 | C |
| 5 | C | 71 | C | 149 | C |
| 6 | B | 73 | B | 150 | A |
| 8 | C | 75 | B | 152 | A |
| 10 | C | 76 | C | 153 | A |
| 11 | B | 77 | C | 154 | A |
| 12 | A | 78 | B | 158 | C |
| 13 | C | 79 | C | 159 | C |
| 14 | A | 80 | B | 161 | C |
| 16 | A | 82 | C | 162 | C |
| 17 | B | 83 | B | 163 | C |
| 19 | C | 85 | C | 165 | C |
| 20 | B | 88 | C | 166 | C |
| 21 | B | 89 | B | 168 | B |
| 23 | A | 94 | B | 169 | B |
| 26 | A | 95 | B | 170 | B |
| 27 | A | 98 | C | 172 | C |
| 28 | A | 100 | A | 173 | B |
| 29 | A | 101 | B | 174 | B |
| 30 | A | 102 | C | 175 | C |
| 33 | B | 103 | B | 176 | C |
| 34 | C | 104 | A | 177 | C |
| 36 | A | 110 | B | 178 | C |
| 37 | C | 114 | B | 179 | C |
| 38 | A | 115 | C | 181 | C |
| 39 | B | 117 | C | 182 | C |
| 40 | C | 118 | C | 184 | C |
| 41 | C | 121 | C | 186 | B |
| 42 | C | 122 | B | 187 | A |
| 43 | C | 123 | C | 188 | B |
| 44 | A | 125 | B | 190 | C |
| 46 | A | 126 | B | 191 | B |
| 47 | B | 127 | B | 192 | C |
| 48 | C | 128 | B | 193 | C |
| 49 | C | 129 | A | 194 | C |
| 51 | A | 130 | B | 196 | C |
| 52 | C | 131 | B | 198 | C |
| 53 | C | 132 | B | 199 | C |
| 54 | C | 133 | B | 200 | C |
| 57 | C | 134 | A | 201 | C |
| 58 | B | 135 | A | 202 | C |
| 59 | C | 136 | A | 203 | C |
| 61 | C | 137 | A | 204 | C |
| 62 | C | 138 | A | | |
| 64 | C | 139 | A | | |

The above results show that the compounds according to the present invention can inhibit the expression of α-SMA, which is a key factor in fibrosis, more effectively than the pulmonary fibrosis treatment, nintedanib, and thus can prevent and treat fibrosis by blocking the activation of myofibroblasts.

It should be understood by those of ordinary skill in the art that the above description of the present invention is for illustrative purposes, and the exemplary embodiments disclosed herein can be easily modified into other specific forms without changing the technical spirit or essential features of the present invention. Therefore, it should be interpreted that the exemplary embodiments described above are illustrative in all aspects, and are not limitative.

### [Industrial Applicability]

The present invention relates to a novel compound that is effective in inhibiting fibrosis. The inventors confirmed that the compound has an effect of inhibiting actin polymerization and not only reduces the levels of all types of fibrosis biomarkers but also effectively inhibits the expression of α-SMA, which is a major causative factor of fibrosis. Therefore, the novel compound according to the present invention is expected to be effectively used as a preparation having an excellent effect of alleviating and inhibiting fibrosis to prevent and treat a fibrosis disease.

## Claims

1. A compound represented by Chemical Formula 1 or 2 below, an enantiomer thereof, a diastereomer thereof, or a pharmaceutically acceptable salt thereof: 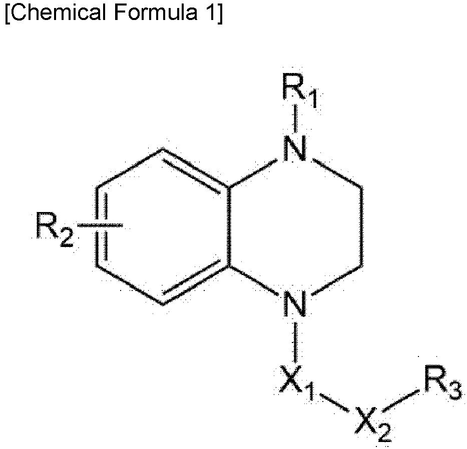 (In Chemical Formula 1,
X₁ is CO or CH₂;
X₂ is (CH₂)ₙ, n is an integer of 1 or 2, and X₂ is unsubstituted or has one H substituted with a methyl group or an amino group;
R₁ is a halogen-substituted or unsubstituted C₃-C₁₀ aryl group, a halogen-substituted or unsubstituted benzyl group, a 6-membered heteroaryl-substituted or unsubstituted C₁-C₁₀ alkyl group, a C₂-C₁₀ heteroaryl group, a C₃-C₁₀ cycloalkyl group, a C₂-C₁₀ heterocycloalkyl group, a C₁-C₅ alkyl-substituted or unsubstituted benzoyl group, or a C₁-C₅ alkyl-substituted or unsubstituted acyl group;
R₂ is H or a halogen; and
R₃ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a methyl-substituted or unsubstituted C₂-C₁₀ heterocycloalkylamine group, a 6-membered heteroaryl-substituted or unsubstituted C₁-C₃ alkylamine group, or 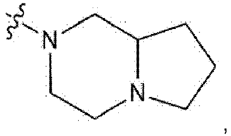 wherein the heterocycloalkyl group is substituted with one or more selected from the group consisting of O, OH, and a C₁-C₅ alkyl group or unsubstituted; and
the heterocycloalkyl group, the heteroaryl group, or the heterocycloalkylamine group each independently includes one or more selected from the group consisting of N, O, and SO₂.) 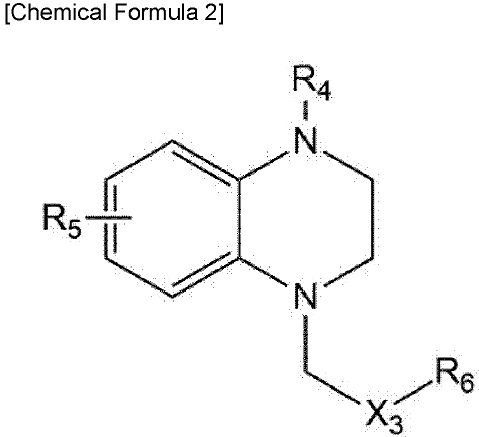 (In Chemical Formula 2,
X₃ is CO or CH₂;
R₄ is a halogen-substituted or unsubstituted C₃-C₁₀ aryl group, a halogen-substituted or unsubstituted benzyl group, a 6-membered heteroaryl-substituted or unsubstituted C₁-C₁₀ alkyl group, a C₂-C₁₀ heteroaryl group, a C₃-C₁₀ cycloalkyl group, a C₂-C₁₀ heterocycloalkyl group, a C₁-C₅ alkyl-substituted or unsubstituted benzoyl group, or a C₁-C₅ alkyl-substituted or unsubstituted acyl group;
R₅ is H or a halogen;
R₆ is a C₁-C₁₀ alkyl-substituted or unsubstituted amine group, a C₂-C₁₀ heterocycloalkyl group, a C₂-C₁₀ heteroaryl group, a C₃-C₁₀ cycloalkylamine group, a methyl-substituted or unsubstituted C₂-C₁₀ heterocycloalkylamine group, a 6-membered heteroaryl-substituted or unsubstituted C₁-C₃ alkylamine group, or 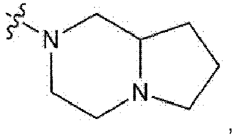 wherein the heterocycloalkyl group is substituted with one or more selected from the group consisting of O, OH, and a C₁-C₅ alkyl group or unsubstituted; and
the heterocycloalkyl group, the heteroaryl group, or the heterocycloalkylamine group each independently includes one or more selected from the group consisting of N, O, and SO₂.)

2. The compound of claim 1, an enantiomer thereof, a diastereomer thereof, or a pharmaceutically acceptable salt thereof, wherein,
in Chemical Formula 1,
R₁ is a halogen-substituted or unsubstituted phenyl group, a halogen-substituted or unsubstituted benzyl group, a pyridyl group, a C₃-C₆ cycloalkyl group, a benzoyl group, a C₄-C₅ alkyl-substituted or unsubstituted acyl group, or a pyridyl-substituted or unsubstituted C₁-C₄ alkyl group; and
R₃ is an N-containing C₂-C₆ heterocycloalkyl group, an N-containing C₂-C₆ heteroaryl group, a morphonyl group, 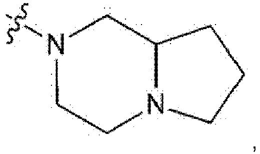 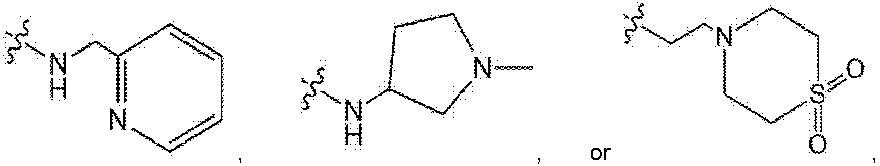 wherein the heterocycloalkyl group may be substituted with one or more selected from the group consisting of O, OH, and a C₁-C₃ alkyl group, or unsubstituted.

3. The compound of claim 1, an enantiomer thereof, a diastereomer thereof, or a pharmaceutically acceptable salt thereof, wherein,
in Chemical Formula 2,
R₄ is a halogen-substituted or unsubstituted phenyl group, and
R₆ is an N-containing C₂-C₆ heterocycloalkyl group, a morphonyl group, or 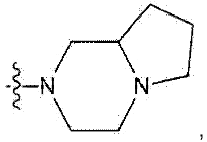 wherein the heterocycloalkyl group may be substituted with OH or a C₁-C₃ alkyl group or unsubstituted.

4. The compound of claim 1, an enantiomer thereof, a diastereomer thereof, or a pharmaceutically acceptable salt thereof, wherein
the compound represented by Chemical Formula 1 or 2 is selected from the group consisting of the following compounds:
(1) 1-(6-fluoro-4-phenyl-3,4-dihydroquinoxaline-1(2H)-yl)-3-(pyrrolidin-1-yl)propan-1-one;
(2) 1-(6-fluoro-4-phenyl-3,4-dihydroquinoxaline-1(2H)-yl)-3-morpholinopropan-1-one;
(3) 1-(6-fluoro-4-phenyl-3,4-dihydroquinoxaline-1 (2H)-yl)-3-(piperidin-1-yl)propan-1-one;
(4) 1-(6-fluoro-4-phenyl-3,4-dihydroquinoxaline-1(2H)-yl)-3-(4-methylpiperazin-1-yl)propan-1-one;
(5) (R)-1-(6-fluoro-4-phenyl-3,4-dihydroquinoxaline-1(2H)-yl)-3-(2-methylpyrrolidin-1-yl)propan-1-one;
(6) (R)-1-(6-fluoro-4-phenyl-3,4-dihydroquinoxaline-1(2H)-yl)-3-(2-methylpiperidin-1-yl)propan-1-one;
(7) 1-(6-fluoro-4-phenyl-3,4-dihydroquinoxaline-1(2H)-yl)-3-(1H-imidazol-1-yl)propan-1-one;
(8) 1-(6-fluoro-4-phenyl-3,4-dihydroquinoxaline-1(2H)-yl)-2-((R)-2-methylpiperidin-1-yl)propan-1-one;
(9) 1-(6-fluoro-4-phenyl-3,4-dihydroquinoxaline-1(2H)-yl)-2-morpholinopropan-1-one;
(10) 1-(6-fluoro-4-phenyl-3,4-dihydroquinoxaline-1(2H)-yl)-2-(pyrrolidin-1-yl)propan-1-one;
(11) 1-(6-fluoro-4-phenyl-3,4-dihydroquinoxaline-1(2H)-yl)-2-(4-methylpiperazin-1-yl)propan-1-one;
(12) 1-(6-fluoro-4-phenyl-3,4-dihydroquinoxaline-1 (2H)-yl)-2-(piperidin-1-yl)propan-1-one;
(13) 1-(6-fluoro-4-phenyl)-3,4-dihydroquinoxaline-1(2H)-yl)-2-((R)-2-methylpyrrolidin-1-yl)propan-1-one;
(14) 1-(4-(4-fluorophenyl)-3,4-dihydroquinoxaline-1(2H)-yl)-3-(pyrrolidin-1-yl)propan-1-one;
(15) 1-(4-(4-fluorophenyl)-3,4-dihydroquinoxaline-1(2H)-yl)-3-morpholinopropan-1-one;
(16) 1-(4-(4-fluorophenyl)-3,4-dihydroquinoxaline-1(2H)-yl)-3-(piperidin-1-yl)propan-1-one;
(17) 1-(4-(4-fluorophenyl)-3,4-dihydroquinoxaline-1(2H)-yl)-3-(4-methylpiperazin-1-yl)propan-1-one di-p-toluene sulfonate;
(18) (R)-1-(4-(4-fluorophenyl)-3,4-dihydroquinoxaline-1(2H)-yl)-3-(2-methylpyrrolidin-1-yl)propan-1-one;
(19) (R)-1-(4-(4-fluorophenyl)-3,4-dihydroquinoxaline-1(2H)-yl)-3-(2-methylpiperidin-1-yl)propan-1-one;
(20) 1-(4-(4-fluorophenyl)-3,4-dihydroquinoxaline-1(2H)-yl)-3-(hexahydropyrrolo[1,2-a]pyrazin-2(1H)-yl)propan-1-one;
(21) 1-(4-(4-fluorophenyl)-3,4-dihydroquinoxaline-1(2H)-yl)-3-((pyridin-2-ylmethyl)amino)propan-1-one;
(22) 3-(4-cyclopropylpiperazin-1-yl)-1-(4-(4-fluorophenyl)-3,4-dihydroquinoxaline-1(2H)-yl)propan-1-one;
(23) (S)-1-(4-(4-fluorophenyl)-3,4-dihydroquinoxaline-1(2H)-yl)-3-(3-hydroxypyrrolidin-1-yl)propan-1-one;
(24) 3-(1,1-dioxidothiomorpholino)-1-(4-(4-fluorophenyl)-3,4-dihydroquinoxaline-1(2H)-yl)propan-1-one;
(25) 1-(4-(4-fluorophenyl)-3,4-dihydroquinoxaline-1(2H)-yl)-3-(1H-imidazol-1-yl)propan-1-one;
(26) (R)-1-(4-(4-fluorophenyl)-3,4-dihydroquinoxaline-1(2H)-yl)-3-((1-methylpyrrolidin-3-yl)amino)propan-1-one;
(27) (S)-1-(4-(4-fluorophenyl)-3,4-dihydroquinoxaline-1(2H)-yl)-3-((1-methylpyrrolidin-3-yl)amino)propan-1-one;
(28) 1-(4-(4-fluorophenyl)-3,4-dihydroquinoxaline-1(2H)-yl)-3-(piperazin-1-yl)propan-1-one;
(29) 1-(4-(4-fluorophenyl)-3,4-dihydroquinoxaline-1(2H)-yl)-3-(1H-imidazol-5-yl)propan-1-one;
(30) 1-(4-fluorophenyl)-4-(3-(4-methylpiperazin-1-yl)propyl)-1,2,3,4-tetrahydroquinoxaline;
(31) 1-(4-(4-fluorophenyl)-3,4-dihydroquinoxaline-1(2H)-yl)-2-((R)-2-methylpiperidin-1-yl)propan-1-one;
(32) 1-(4-(4-fluorophenyl)-3,4-dihydroquinoxaline-1(2H)-yl)-2-morpholinopropan-1-one;
(33) 1-(4-(4-fluorophenyl)-3,4-dihydroquinoxaline-1(2H)-yl)-2-(pyrrolidin-1-yl)propan-1-one;
(34) 1-(4-(4-fluorophenyl)-3,4-dihydroquinoxaline-1(2H)-yl)-2-(4-methylpiperazin-1-yl)propan-1-one;
(35) 1-(4-(4-fluorophenyl)-3,4-dihydroquinoxaline-1(2H)-yl)-2-(piperidin-1-yl)propan-1-one;
(36) 1-(6-fluoro-4-(4-fluorophenyl)-3,4-dihydroquinoxaline-1(2H)-yl)-3-(pyrrolidin-1-yl)propan-1-one;
(37) 1-(6-fluoro-4-(4-fluorophenyl)-3,4-dihydroquinoxaline-1(2H)-yl)-3-morpholinopropan-1-one;
(38) 1-(6-fluoro-4-(4-fluorophenyl)-3,4-dihydroquinoxaline-1(2H)-yl)-3-(piperidin-1-yl)propan-1-one;
(39) 1-(6-fluoro-4-(4-fluorophenyl)-3,4-dihydroquinoxaline-1(2H)-yl)-3-(4-methylpiperazin-1-yl)propan-1-one;
(40) (R)-1-(6-fluoro-4-(4-fluorophenyl)-3,4-dihydroquinoxaline-1(2H)-yl)-3-(2-methylpyrrolidin-1-yl)propan-1-one;
(41) (R)-1-(6-fluoro-4-(4-fluorophenyl)-3,4-dihydroquinoxaline-1(2H)-yl)-3-(2-methylpiperidin-1-yl)propan-1-one;
(42) 1-(6-fluoro-4-(4-fluorophenyl)-3,4-dihydroquinoxaline-1(2H)-yl)-3-(hexahydropyrrolo[1,2-a]pyrazin-2(1H)-yl)propan-1-one;
(43) 3-(4-cyclopropylpiperazin-1-yl)-1-(6-fluoro-4-(4-fluorophenyl)-3,4-dihydroquinoxaline-1(2H)-yl)propan-1-one;
(44) (S)-1-(6-fluoro-4-(4-fluorophenyl)-3,4-dihydroquinoxaline-1(2H)-yl)-3-(3-hydroxypyrrolidin-1-yl)propan-1-one;
(45) 1-(6-fluoro-4-(4-fluorophenyl)-3,4-dihydroquinoxaline-1(2H)-yl)-3-(1H-imidazol-1-yl)propan-1-one;
(46) 1-(6-fluoro-4-(4-fluorophenyl)-3,4-dihydroquinoxaline-1(2H)-yl)-3-(1H-imidazol-5-yl)propan-1-one;
(47) (S)-2-amino-1-(6-fluoro-4-(4-fluorophenyl)-3,4-dihydroquinoxaline-1(2H)-yl)-3-(1H-imidazol-4-yl)propan-1-one;
(48) (R)-2-amino-1-(6-fluoro-4-(4-fluorophenyl)-3,4-dihydroquinoxaline-1(2H)-yl)-3-(1H-imidazol-4-yl)propan-1-one;
(49) 1-(6-fluoro-4-(4-fluorophenyl)-3,4-dihydroquinoxaline-1(2H)-yl)-2-((R)-2-methylpiperidin-1-yl)propan-1-one;
(50) 1-(6-fluoro-4-(4-fluorophenyl)-3,4-dihydroquinoxaline-1(2H)-yl)-2-morpholinopropan-1-one;
(51) 1-(6-fluoro-4-(4-fluorophenyl)-3,4-dihydroquinoxaline-1(2H)-yl)-2-(pyrrolidin-1-yl)propan-1-one;
(52) 1-(6-fluoro-4-(4-fluorophenyl)-3,4-dihydroquinoxaline-1(2H)-yl)-2-(4-methylpiperazin-1-yl)propan-1-one;
(53) 1-(6-fluoro-4-(4-fluorophenyl)-3,4-dihydroquinoxaline-1(2H)-yl)-2-(piperidin-1-yl)propan-1-one;
(54) 1-(6-fluoro-4-(4-fluorophenyl)-3,4-dihydroquinoxaline-1(2H)-yl)-2-((R)-2-methylpyrrolidin-1-yl)propan-1-one;
(55) 3-morpholino-1-(4-phenyl-3,4-dihydroquinoxaline-1(2H)-yl)propan-1-one;
(56) 3-(4-methylpiperazin-1-yl)-1-(4-phenyl-3,4-dihydroquinoxaline-1(2H)-yl)propan-1-one;
(57) (R)-3-(2-methylpyrrolidin-1-yl)-1-(4-phenyl-3,4-dihydroquinoxaline-1(2H)-yl)propan-1-one;
(58) 3-(3-hydroxypiperidin-1-yl)-1-(4-phenyl-3,4-dihydroquinoxaline-1(2H)-yl)propan-1-one;
(59) 2-((R)-2-methylpiperidin-1-yl)-1-(4-phenyl-3,4-dihydroquinoxaline-1(2H)-yl)propan-1-one phosphate;
(60) 2-morpholino-1-(4-phenyl-3,4-dihydroquinoxaline-1(2H)-yl)propan-1-one;
(61) 1-(4-phenyl-3,4-dihydroquinoxaline-1(2H)-yl)-2-(pyrrolidin-1-yl)propan-1-one;
(62) 2-(4-methylpiperazin-1-yl)-1-(4-phenyl-3,4-dihydroquinoxaline-1(2H)-yl)propan-1-one;
(63) 1-(4-phenyl-3,4-dihydroquinoxaline-1(2H)-yl)-2-(piperidin-1-yl)propan-1-one;
(64) 2-(hexahydropyrrolo[1,2-a]pyrazin-2(1H)-yl)-1-(4-phenyl-3,4-dihydroquinoxaline-1(2H)-yl)propan-1-one;
(65) 2-((1-methylpyrrolidin-3-yl)amino)-1-(4-phenyl-3,4-dihydroquinoxaline-1(2H)-yl)propan-1-one;
(66) 2-(4-cyclopropylpiperazin-1-yl)-1-(4-phenyl-3,4-dihydroquinoxaline-1(2H)-yl)propan-1-one;
(67) 2-methyl-1-(4-phenyl-3,4-dihydroquinoxaline-1 (2H)-yl)-3-((pyridin-2-ylmethyl)amino)propan-1-one;
(68) 1-(2-((R)-2-methylpiperidin-1-yl)propyl)-4-phenyl-1,2,3,4-tetrahydroquinoxaline;
(69) 1-morpholino-2-(4-phenyl-3,4-dihydroquinoxaline-1(2H)-yl)ethan-1-one;
(70) (R)-1-(2-methylpiperidin-1-yl)-2-(4-phenyl-3,4-dihydroquinoxaline-1(2H)-yl)ethan-1-one;
(71) (S)-1-(2-methylpiperidin-1-yl)-2-(4-phenyl-3,4-dihydroquinoxaline-1(2H)-yl)ethan-1-one;
(72) (R)-1-(2-methylpyrrolidin-1-yl)-2-(4-phenyl-3,4-dihydroquinoxaline-1(2H)-yl)ethan-1-one;
(73) 1-(4-methylpiperazin-1-yl)-2-(4-phenyl-3,4-dihydroquinoxaline-1(2H)-yl)ethan-1-one;
(74) 1-(4-methylpiperidin-1-yl)-2-(4-phenyl-3,4-dihydroquinoxaline-1(2H)-yl)ethan-1-one;
(75) 1-(hexahydropyrrolo[1,2-a]pyrazin-2(1H-yl)-2-(4-phenyl-3,4-dihydroquinoxaline-1(2H)-yl)ethan-1-one;
(76) 1-(4-cyclopropylpiperazin-1-yl)-2-(4-phenyl-3,4-dihydroquinoxaline-1(2H)-yl)ethan-1-one;
(77) 1-(3-hydroxypiperidin-1-yl)-2-(4-phenyl-3,4-dihydroquinoxaline-1(2H)-yl)ethan-1-one;
(78) 1-(4-benzyl-3,4-dihydroquinoxaline-1(2H)-yl)-3-(pyrrolidin-1-yl)propan-1-one;
(79) 1-(4-benzyl-3,4-dihydroquinoxaline-1(2H)-yl)-3-(piperidin-1-yl)propan-1-one;
(80) 1-(4-benzyl-3,4-dihydroquinoxaline-1(2H)-yl)-3-(4-methylpiperazin-1-yl)propan-1-one;
(81) 1-(4-benzyl-3,4-dihydroquinoxaline-1(2H)-yl)-2-(pyrrolidin-1-yl)propan-1-one;
(82) 1-(4-benzyl-3,4-dihydroquinoxaline-1(2H)-yl)-2-(piperidin-1-yl)propan-1-one;
(83) 1-(4-benzyl-3,4-dihydroquinoxaline-1(2H)-yl)-2-(4-methylpiperazin-1-yl)propan-1-one;
(84) 1-(4-benzyl-3,4-dihydroquinoxaline-1(2H)-yl)-2-(pyrrolidin-1-yl)ethan-1-one;
(85) 1-(4-benzyl-3,4-dihydroquinoxaline-1(2H)-yl)-2-(piperidin-1-yl)ethan-1-one;
(86) 1-(4-benzyl-3,4-dihydroquinoxaline-1(2H)-yl)-2-(4-methylpiperazin-1-yl)ethan-1-one;
(87) 1-(4-(2-chlorobenzyl)-3,4-dihydroquinoxaline-1(2H)-yl)-3-(pyrrolidin-1-yl)propan-1-one;
(88) 1-(4-(2-chlorobenzyl)-3,4-dihydroquinoxaline-1 (2H)-yl)-3-(piperidin-1-yl)propan-1-one;
(89) 1-(4-(2-chlorobenzyl)-3,4-dihydroquinoxaline-1(2H)-yl)-3-(4-methylpiperazin-1-yl)propan-1-one;
(90) 1-(4-(2-chlorobenzyl)-3,4-dihydroquinoxaline-1(2H)-yl)-2-(pyrrolidin-1-yl)propan-1-one;
(91) 1-(4-(2-chlorobenzyl)-3,4-dihydroquinoxaline-1(2H)-yl)-2-(piperidin-1-yl)propan-1-one;
(92) 1-(4-(2-chlorobenzyl)-3,4-dihydroquinoxaline-1(2H)-yl)-2-(4-methylpiperazin-1-yl)propan-1-one;
(93) 1-(4-(2-chlorobenzyl)-3,4-dihydroquinoxaline-1(2H)-yl)-2-(pyrrolidin-1-yl)ethan-1-one;
(94) 1-(4-(2-chlorobenzyl)-3,4-dihydroquinoxaline-1(2H)-yl)-2-(piperidin-1-yl)ethan-1-one;
(95) 1-(4-(2-chlorobenzyl)-3,4-dihydroquinoxaline-1(2H)-yl)-2-(4-methylpiperazin-1-yl)ethan-1-one;
(96) 1-(4-(3-chlorobenzyl)-3,4-dihydroquinoxaline-1(2H)-yl)-3-(pyrrolidin-1-yl)propan-1-one;
(97) 1-(4-(3-chlorobenzyl)-3,4-dihydroquinoxaline-1(2H)-yl)-3-(piperidin-1-yl)propan-1-one;
(98) 1-(4-(3-chlorobenzyl)-3,4-dihydroquinoxaline-1(2H)-yl)-3-(4-methylpiperazin-1-yl)propan-1-one;
(99) 1-(4-(3-chlorobenzyl)-3,4-dihydroquinoxaline-1(2H)-yl)-2-(pyrrolidin-1-yl)propan-1-one;
(100) 1-(4-(3-chlorobenzyl)-3,4-dihydroquinoxaline-1(2H)-yl)-2-(piperidin-1-yl)propan-1-one;
(101) 1-(4-(3-chlorobenzyl)-3,4-dihydroquinoxaline-1(2H)-yl)-2-(4-methylpiperazin-1-yl)propan-1-one;
(102) 1-(4-(3-chlorobenzyl)-3,4-dihydroquinoxaline-1(2H)-yl)-2-(pyrrolidin-1-yl)ethan-1-one;
(103) 1-(4-(3-chlorobenzyl)-3,4-dihydroquinoxaline-1(2H)-yl)-2-(piperidin-1-yl)ethan-1-one;
(104) 1-(4-(3-chlorobenzyl)-3,4-dihydroquinoxaline-1(2H)-yl)-2-(4-methylpiperazin-1-yl)ethan-1-one;
(105) 1-(4-(4-chlorobenzyl)-3,4-dihydroquinoxaline-1(2H)-yl)-3-(pyrrolidin-1-yl)propan-1-one;
(106) 1-(4-(4-chlorobenzyl)-3,4-dihydroquinoxaline-1(2H)-yl)-3-(piperidin-1-yl)propan-1-one;
(107) 1-(4-(4-chlorobenzyl)-3,4-dihydroquinoxaline-1(2H)-yl)-3-(4-methylpiperazin-1-yl)propan-1-one;
(108) 1-(4-(4-chlorobenzyl)-3,4-dihydroquinoxaline-1(2H)-yl)-2-(pyrrolidin-1-yl)propan-1-one;
(109) 1-(4-(4-chlorobenzyl)-3,4-dihydroquinoxaline-1(2H)-yl)-2-(piperidin-1-yl)propan-1-one;
(110) 1-(4-(4-chlorobenzyl)-3,4-dihydroquinoxaline-1(2H)-yl)-2-(4-methylpiperazin-1-yl)propan-1-one;
(111) 1-(4-(4-chlorobenzyl)-3,4-dihydroquinoxaline-1(2H)-yl)-2-(pyrrolidin-1-yl)ethan-1-one;
(112) 1-(4-(4-chlorobenzyl)-3,4-dihydroquinoxaline-1(2H)-yl)-2-(piperidin-1-yl)ethan-1-one;
(113) 1-(4-(4-chlorobenzyl)-3,4-dihydroquinoxaline-1(2H)-yl)-2-(4-methylpiperazin-1-yl)ethan-1-one;
(114) 1-(4-(pyridin-2-ylmethyl)-3,4-dihydroquinoxaline-1(2H)-yl)-3-(pyrrolidin-1-yl)propan-1-one di-oxalate;
(115) 3-(piperidin-1-yl)-1-(4-(pyridin-2-ylmethyl)-3,4-dihydroquinoxaline-1 (2H)-yl)propan-1-one;
(116) 3-(4-methylpiperazin-1-yl)-1-(4-(pyridin-2-ylmethyl)-3,4-dihydroquinoxaline-1(2H)-yl)propan-1-one;
(117) 1-(4-(pyridin-2-ylmethyl)-3,4-dihydroquinoxaline-1(2H)-yl)-2-(pyrrolidin-1-yl)ethan-1-one;
(118) 2-(4-methylpiperazin-1-yl)-1-(4-(pyridin-2-ylmethyl)-3,4-dihydroquinoxaline-1(2H)-yl)ethan-1-one;
(119) 1-(4-(pyridin-3-ylmethyl)-3,4-dihydroquinoxaline-1(2H)-yl)-3-(pyrrolidin-1-yl)propan-1-one;
(120) 3-(piperidin-1-yl)-1-(4-(pyridin-3-ylmethyl)-3,4-dihydroquinoxaline-1 (2H)-yl)propan-1-one;
(121) 3-(4-methylpiperazin-1-yl)-1-(4-(pyridin-3-ylmethyl)-3,4-dihydroquinoxaline-1(2H)-yl)propan-1-one;
(122) 1-(4-(pyridin-4-ylmethyl)-3,4-dihydroquinoxaline-1(2H)-yl)-3-(pyrrolidin-1-yl)propan-1-one;
(123) 3-(piperidin-1-yl)-1-(4-(pyridin-4-ylmethyl)-3,4-dihydroquinoxaline-1 (2H)-yl)propan-1-one;
(124) 3-(4-methylpiperazin-1-yl)-1-(4-(pyridin-4-ylmethyl)-3,4-dihydroquinoxaline-1(2H)-yl)propan-1-one;
(125) 1-(4-(2-chlorophenyl)-3,4-dihydroquinoxaline-1(2H)-yl)-3-(pyrrolidin-1-yl)propan-1-one;
(126) 1-(4-(2-chlorophenyl)-3,4-dihydroquinoxaline-1 (2H)-yl)-3-(piperidin-1-yl)propan-1-one;
(127) 1-(4-(2-chlorophenyl)-3,4-dihydroquinoxaline-1(2H)-yl)-3-(4-methylpiperazin-1-yl)propan-1-one;
(128) 1-(4-(2-chlorophenyl)-3,4-dihydroquinoxaline-1(2H)-yl)-2-(pyrrolidin-1-yl)propan-1-one;
(129) 1-(4-(2-chlorophenyl)-3,4-dihydroquinoxaline-1(2H)-yl)-2-(piperidin-1-yl)propan-1-one;
(130) 1-(4-(2-chlorophenyl)-3,4-dihydroquinoxaline-1(2H)-yl)-2-(4-methylpiperazin-1-yl)propan-1-one;
(131) 1-(4-(2-chlorophenyl)-3,4-dihydroquinoxaline-1(2H)-yl)-2-(pyrrolidin-1-yl)ethan-1-one;
(132) 1-(4-(2-chlorophenyl)-3,4-dihydroquinoxaline-1(2H)-yl)-2-(piperidin-1-yl)ethan-1-one;
(133) 1-(4-(2-chlorophenyl)-3,4-dihydroquinoxaline-1(2H)-yl)-2-(4-methylpiperazin-1-yl)ethan-1-one;
(134) 1-(4-(3-chlorophenyl)-3,4-dihydroquinoxaline-1(2H)-yl)-3-(pyrrolidin-1-yl)propan-1-one;
(135) 1-(4-(3-chlorophenyl)-3,4-dihydroquinoxaline-1(2H)-yl)-3-(4-methylpiperazin-1-yl)propan-1-one;
(136) 1-(4-(3-chlorophenyl)-3,4-dihydroquinoxaline-1(2H)-yl)-2-(pyrrolidin-1-yl)propan-1-one;
(137) 1-(4-(3-chlorophenyl)-3,4-dihydroquinoxaline-1(2H)-yl)-2-(4-methylpiperazin-1-yl)propan-1-one;
(138) 1-(4-(3-chlorophenyl)-3,4-dihydroquinoxaline-1(2H)-yl)-2-(pyrrolidin-1-yl)ethan-1-one;
(139) 1-(4-(3-chlorophenyl)-3,4-dihydroquinoxaline-1(2H)-yl)-2-(4-methylpiperazin-1-yl)ethan-1-one;
(140) 1-(4-(4-chlorophenyl)-3,4-dihydroquinoxaline-1(2H)-yl)-3-(pyrrolidin-1-yl)propan-1-one;
(141) 1-(4-(4-chlorophenyl)-3,4-dihydroquinoxaline-1(2H)-yl)-3-(piperidin-1-yl)propan-1-one;
(142) 1-(4-(4-chlorophenyl)-3,4-dihydroquinoxaline-1(2H)-yl)-3-(4-methylpiperazin-1-yl)propan-1-one;
(143) 1-(4-(4-chlorophenyl)-3,4-dihydroquinoxaline-1(2H)-yl)-2-(pyrrolidin-1-yl)propan-1-one;
(144) 1-(4-(4-chlorophenyl)-3,4-dihydroquinoxaline-1(2H)-yl)-2-(piperidin-1-yl)propan-1-one;
(145) 1-(4-(4-chlorophenyl)-3,4-dihydroquinoxaline-1(2H)-yl)-2-(4-methylpiperazin-1-yl)propan-1-one;
(146) 1-(4-pyridin-2-yl)-3,4-dihydroquinoxaline-1(2H)-yl)-3-(pyrrolidin-1-yl)propan-1-one;
(147) 3-(piperidin-1-yl)-1-(4-(pyridin-2-yl)-3,4-dihydroquinoxaline-1(2H)-yl)propan-1-one;
(148) 3-(4-methylpiperazin-1-yl)-1-(4-(pyridin-2-yl)-3,4-dihydroquinoxaline-1(2H)-yl)propan-1-one;
(149) 1-(4-pyridin-2-yl)-3,4-dihydroquinoxaline-1(2H)-yl)-2-(pyrrolidin-1-yl)propan-1-one;
(150) 2-(piperidin-1-yl)-1-(4-pyridin-2-yl)-3,4-dihydroquinoxaline-1(2H)-yl)propan-1-one;
(151) 1-(4-pyridin-2-yl)-3,4-dihydroquinoxaline-1(2H)-yl)-2-(4-methylpiperazin-1-yl)propan-1-one;
(152) 1-(4-pyridin-2-yl)-3,4-dihydroquinoxaline-1(2H)-yl)-2-(pyrrolidin-1-yl)ethan-1-one;
(153) 2-(piperidin-1-yl)-1-(4-(pyridin-2-yl)-3,4-dihydroquinoxaline-1 (2H)-yl)ethan-1-one;
(154) 2-(4-methylpiperazin-1-yl)-1-(4-(pyridin-2-yl)-3,4-dihydroquinoxaline-1(2H)-yl)ethan-1-one di L-tartrate;
(155) 1-(4-(pyridin-3-yl)-3,4-dihydroquinoxaline-1(2H)-yl)-3-(pyrrolidin-1-yl)propan-1-one;
(156) 3-(piperidin-1-yl)-1-(4-(pyridin-3-yl)-3,4-dihydroquinoxaline-1 (2H)-yl)propan-1-one;
(157) 3-(4-methylpiperazin-1-yl)-1-(4-(pyridin-3-yl)-3,4-dihydroquinoxaline-1(2H)-yl)propan-1-one;
(158) 1-(4-(pyridin-4-yl)-3,4-dihydroquinoxaline-1(2H)-yl)-3-(pyrrolidin-1-yl)propan-1-one;
(159) 3-(piperidin-1-yl)-1-(4-(pyridin-4-yl)-3,4-dihydroquinoxaline-1 (2H)-yl)propan-1-one;
(160) 3-(4-methylpiperazin-1-yl)-1-(4-(pyridin-4-yl)-3,4-dihydroquinoxaline-1(2H)-yl)propan-1-one;
(161) 1-(4-(cyclopropylmethyl)-3,4-dihydroquinoxaline-1(2H)-yl)-3-(pyrrolidin-1-yl)propan-1-one;
(162) 1-(4-(cyclopropylmethyl)-3,4-dihydroquinoxaline-1(2H)-yl)-3-(piperidin-1-yl)propan-1-one;
(163) 1-(4-(cyclopropylmethyl)-3,4-dihydroquinoxaline-1(2H)-yl)-3-(4-methylpiperazin-1-yl)propan-1-one;
(164) 1-(4-(cyclopropylmethyl)-3,4-dihydroquinoxaline-1(2H)-yl)-2-(pyrrolidin-1-yl)propan-1-one;
(165) 1-(4-(cyclopropylmethyl)-3,4-dihydroquinoxaline-1(2H)-yl)-2-(piperidin-1-yl)propan-1-one;
(166) 1-(4-(cyclopropylmethyl)-3,4-dihydroquinoxaline-1(2H)-yl)-2-(4-methylpiperazin-1-yl)propan-1-one;
(167) 1-(4-(cyclopropylmethyl)-3,4-dihydroquinoxaline-1(2H)-yl)-2-(pyrrolidin-1-yl)ethan-1-one;
(168) 1-(4-isobutyl-3,4-dihydroquinoxaline-1(2H)-yl)-2-(pyrrolidin-1-yl)propan-1-one;
(169) 1-(4-isobutyl-3,4-dihydroquinoxaline-1(2H)-yl)-3-(piperidin-1-yl)propan-1-one;
(170) 1-(4-isobutyl-3,4-dihydroquinoxaline-1(2H)-yl)-3-(4-methylpiperazin-1-yl)propan-1-one di L-tartrate;
(171) 1-(4-isobutyl-3,4-dihydroquinoxaline-1(2H)-yl)-2-(pyrrolidin-1-yl)propan-1-one;
(172) 1-(4-isobutyl-3,4-dihydroquinoxaline-1(2H)-yl)-2-(piperidin-1-yl)propan-1-one;
(173) 1-(4-isobutyl-3,4-dihydroquinoxaline-1(2H)-yl)-2-(4-methylpiperazin-1-yl)propan-1-one;
(174) 1-(4-isobutyl-3,4-dihydroquinoxaline-1(2H)-yl)-2-(pyrrolidin-1-yl)ethan-1-one;
(175) 1-(4-isobutyl-3,4-dihydroquinoxaline-1(2H)-yl)-2-(piperidin-1-yl)ethan-1-one;
(176) 1-(4-isobutyl-3,4-dihydroquinoxaline-1(2H)-yl)-2-(4-methylpiperazin-1-yl)ethan-1-one;
(177) 1-(4-isopropyl-3,4-dihydroquinoxaline-1(2H)-yl)-3-(pyrrolidin-1-yl)propan-1-one;
(178) 1-(4-isopropyl-3,4-dihydroquinoxaline-1(2H)-yl)-3-(piperidin-1-yl)propan-1-one;
(179) 1-(4-isopropyl-3,4-dihydroquinoxaline-1(2H)-yl)-3-(4-methylpiperazin-1-yl)propan-1-one;
(180) 1-(4-isopropyl-3,4-dihydroquinoxaline-1(2H)-yl)-2-(pyrrolidin-1-yl)propan-1-one;
(181) 1-(4-isopropyl-3,4-dihydroquinoxaline-1(2H)-yl)-2-(piperidin-1-yl)propan-1-one;
(182) 1-(4-isopropyl-3,4-dihydroquinoxaline-1(2H)-yl)-2-(4-methylpiperazin-1-yl)propan-1-one;
(183) 1-(4-isopropyl-3,4-dihydroquinoxaline-1(2H)-yl)-3-(pyrrolidin-1-yl)ethan-1-one;
(184) 1-(4-isopropyl-3,4-dihydroquinoxaline-1(2H)-yl)-2-(piperidin-1-yl)ethan-1-one;
(185) 1-(4-isopropyl-3,4-dihydroquinoxaline-1(2H)-yl)-2-(4-methylpiperazin-1-yl)ethan-1-one;
(186) 1-(4-cyclohexyl-3,4-dihydroquinoxaline-1(2H)-yl)-3-(pyrrolidin-1-yl)propan-1-one;
(187) 1-(4-cyclohexyl-3,4-dihydroquinoxaline-1(2H)-yl)-3-(piperidin-1-yl)propan-1-one;
(188) 1-(4-cyclohexyl-3,4-dihydroquinoxaline-1(2H)-yl)-3-(4-methylpiperazin-1-yl)propan-1-one;
(189) 1-(4-cyclohexyl-3,4-dihydroquinoxaline-1(2H)-yl)-3-(piperazin-1-yl)propan-1-one;
(190) 1-(4-cyclohexyl-3,4-dihydroquinoxaline-1(2H)-yl)-2-(pyrrolidin-1-yl)ethan-1-one;
(191) 1-(4-cyclohexyl-3,4-dihydroquinoxaline-1(2H)-yl)-2-(piperidin-1-yl)propan-1-one;
(192) 1-(4-cyclohexyl-3,4-dihydroquinoxaline-1(2H)-yl)-2-(4-methylpiperazin-1-yl)propan-1-one;
(193) 1-(4-cyclohexyl-3,4-dihydroquinoxaline-1(2H)-yl)-2-(pyrrolidin-1-yl)ethan-1-one;
(194) 1-(4-cyclohexyl-3,4-dihydroquinoxaline-1(2H)-yl)-2-(piperidin-1-yl)ethan-1-one;
(195) 1-(4-cyclohexyl-3,4-dihydroquinoxaline-1(2H)-yl)-2-(4-methylpiperazin-1-yl)ethan-1-one;
(196) 1-(4-benzoyl-3,4-dihydroquinoxaline-1(2H)-yl)-3-(pyrrolidin-1-yl)propan-1-one;
(197) 1-(4-benzoyl-3,4-dihydroquinoxaline-1(2H)-yl)-3-(piperidin-1-yl)propan-1-one;
(198) 1-(4-benzoyl-3,4-dihydroquinoxaline-1(2H)-yl)-3-(4-methylpiperazin-1-yl)propan-1-one;
(199) 1-(4-benzoyl-3,4-dihydroquinoxaline-1(2H)-yl)-2-(pyrrolidin-1-yl)propan-1-one;
(200) 1-(4-benzoyl-3,4-dihydroquinoxaline-1(2H)-yl)-2-(piperidin-1-yl)propan-1-one;
(201) 1-(4-benzoyl-3,4-dihydroquinoxaline-1(2H)-yl)-2-(4-methylpiperazin-1-yl)propan-1-one;
(202) 1-(4-benzoyl-3,4-dihydroquinoxaline-1(2H)-yl)-2-(pyrrolidin-1-yl)ethan-1-one;
(203) 1-(4-benzoyl-3,4-dihydroquinoxaline-1(2H)-yl)-2-(piperidin-1-yl)ethan-1-one;
(204) 1-(4-benzoyl-3,4-dihydroquinoxaline-1(2H)-yl)-2-(4-methylpiperazin-1-yl)ethan-1-one;
(205) 3-methyl-1-(4-(3-(pyrrolidin-1-yl)propanoyl)-3,4-dihydroquinoxaline-1(2H)-yl)butan-1-one;
(206) 3-methyl-1-(4-(3-(4-methylpiperazin-1-yl)propanoyl)-3,4-dihydroquinoxaline-1(2H)-yl)butan-1-one;
(207) 3,3-dimethyl-1-(4-(3-(pyrrolidin-1-yl)propanoyl)-3,4-dihydroquinoxaline-1(2H)-yl)butan-1-one; and
(208) 3,3-dimethyl-1-(4-(3-(4-methylpiperazin-1-yl)propanoyl)-3,4-dihydroquinoxaline-1(2H)-yl)butan-1-one.

5. A pharmaceutical composition for preventing or treating a pulmonary fibrosis-related disease, comprising the compound represented by Chemical Formula 1 or 2 according to claim 1, an enantiomer thereof, a diastereomer thereof, or a pharmaceutically acceptable salt thereof as an active ingredient.

6. The pharmaceutical composition of claim 5, wherein the composition inhibits actin polymerization.

7. The pharmaceutical composition of Claim 5, wherein the composition reduces the level or activity of one or more selected from the group consisting of α-SMA, COL1A1, COL4A1, fibronectin, and phosphorylated SMAD2 (p-SMAD2).

8. The pharmaceutical composition of claim 5, wherein the pulmonary fibrosis-related disease includes one or more selected from the group consisting of pulmonary fibrosis, idiopathic pulmonary fibrosis (IPF), desquamative interstitial pneumonia, nonspecific interstitial pneumonia, cryptogenic organizing pneumonia, respiratory bronchiolitis-associated interstitial lung disease, acute interstitial pneumonia, lymphatic interstitial pneumonia, idiopathic pleuroparenchymal fibroelastosis, and chronic obstructive pulmonary disease (COPD).

9. The pharmaceutical composition of claim 5, wherein the composition is used for treating a pulmonary fibrosis-related disease accompanied by hyperactivation of actin polymerization.

10. A kit for preventing or treating a pulmonary fibrosis-related disease, comprising the composition of any one of claims 5 to 9.

11. A method of preventing or treating a pulmonary fibrosis-related disease, comprising administering the compound of claim 1, an enantiomer thereof, a diastereomer thereof, or a pharmaceutically acceptable salt thereof to a subject in need thereof.

12. A use of the compound of claim 1, an enantiomer thereof, a diastereomer thereof, or a pharmaceutically acceptable salt thereof for preventing or treating a pulmonary fibrosis-related disease.

13. A use of the compound of claim 1, an enantiomer thereof, a diastereomer thereof, or a pharmaceutically acceptable salt thereof for preparing a drug for treating a pulmonary fibrosis-related disease.
